# EUROPEAN PATENT APPLICATION

(11) **EP 3 378 931 A1**
(43) Date of publication of application: **26.09.2018**
(21) Application number: 17162104.8
(22) Date of filing: 21.03.2017
(51) Int. Cl.: C12N 1/15, C12N 1/19, C07K 14/385, C12N 9/02, C12P 17/04

(54) **FDCA-DECARBOXYLATING MONOOXYGENASE-DEFICIENT HOST CELLS FOR PRODUCING FDCA**

(71) Applicant: Purac Biochem B.V., 4206 AC Gorinchem (NL)
(72) Inventor: de Bont, Johannes Adrianus Maria, 6707 AK Wageningen (NL); Ruijssenaars, Harald Johan, 3941 VL Doorn (NL); Werij, Jan, 7213 TD Gorssel (NL)
(74) Representative: Nederlandsch Octrooibureau

(57) **Abstract**

The invention relates to fungal cells for the production of FDCA. The fungal cell has genetic modification that reduces specific 2,5-furandicarboxylic acid (FDCA) decarboxylating monooxygenase activity in the cell, as compared to a corresponding parent cell lacking the genetic modification. The fungal cell can further be genetically modified to increase the cell's ability to oxidize furanic aldehydes to the corresponding furanic carboxylic acids. The invention also relates to a process for the production of 2,5-furan-dicarboxylic acid (FDCA) wherein the cells of the invention are used for oxidation of a furanic precursors of FDCA to FDCA.

SEQUENCE LISTING

<110>  Purac Biochem B.V.

<120>  FDCA-decarboxylating monooxygenase-deficient host cells for
       producing FDCA

<130>  P6065528EP

<160>  55

<170>  PatentIn version 3.3

<210>  1
<211>  351
<212>  PRT
<213>  Penicillium brasilianum

<400>  1

Met Gly Ser Leu Ser Leu Pro Glu Thr Ser Leu Ala Ala Ile Gln Asp
1               5                   10                  15

Lys Glu Thr Lys Ala Ile Ser Val Ala Lys Arg Pro Thr Pro Val Pro
            20                  25                  30

Val Gly Thr Gln Val Leu Val Lys Leu His Tyr Ser Gly Val Cys Ala
        35                  40                  45

Thr Asp Leu His Leu Ala Arg Gly Ser Val Pro Tyr Leu Gln Pro Lys
    50                  55                  60

Val Ser Val Gly Gly His Glu Gly Thr Gly Val Ile Ala Ser Leu Gly
65                  70                  75                  80

Pro Asp Val Asp Ala Ala Glu Trp His Val Gly Asp Arg Val Ala Val
                85                  90                  95

Arg Trp Val His Ile Val Cys Gly Lys Cys Glu Val Cys Thr Thr Gly
            100                 105                 110

Phe Glu Asn Leu Cys Gln Ser Arg Lys Leu Ala Gly Lys Asp Val Glu
        115                 120                 125

Gly Thr Phe Ala Glu Tyr Ala Ile Ala Asp Ser Ser Tyr Met Val Arg
    130                 135                 140

Leu Pro Ala Gly Val Ser Asp Ala Asp Ala Ala Pro Ile Leu Cys Ala
145                 150                 155                 160

Gly Val Thr Val Tyr Lys Ala Leu Lys Ile Ala Ser Leu Arg Ala Gly
                165                 170                 175

Ser Trp Val Ala Val Ala Gly Ala Gly Gly Gly Leu Gly His Leu Ala
        180                 185                 190

Ile Gln Tyr Ala Arg Ala Met Gly Leu Lys Val Val Ala Leu Asp Ala
    195                 200                 205

Arg Lys Arg Asp Leu Cys Leu Ser Leu Gly Ala Glu Ser Tyr Ile Asp
    210                 215                 220

Val Leu Glu Thr Asp Asp Cys Val Ala Gln Val Ile Lys Val Thr Asp
225                 230                 235                 240

Gly Gly Ala His Gly Ala Leu Ile Cys Ala Ser Ser Gly Gln Ala Tyr
                245                 250                 255

Asp Asp Ala Val Lys Phe Leu Arg Trp Thr Gly Thr Leu Val Cys Ile
            260                 265                 270

Gly Leu Pro Pro Lys Pro Thr Leu Leu Ser Leu Gly Pro Ala Asp Phe
        275                 280                 285

Val Ala Arg Gly Ile Lys Val Met Gly Thr Ser Thr Gly Asp Arg Gln
    290                 295                 300

Asp Thr Val Glu Ala Leu Ala Phe Val Ala Lys Gly Gln Val Lys Pro
305                 310                 315                 320

Gln Leu Thr Glu Arg Arg Leu Glu Asp Val Glu Glu Ile Leu Lys Glu
                325                 330                 335

Ile Glu Asn Gly Thr Met Gln Gly Lys Ala Val Ile Arg Ile Ala
            340                 345                 350

<210>  2
<211>  339
<212>  PRT
<213>  Penicillium brasilianum

<400>  2

Met Ser Leu Pro Ser His Tyr Lys Arg Ala Ala Phe Lys Glu Ala Gly
1               5                   10                  15

Gly Pro Leu Thr Ile Glu Glu Val Asp Leu Thr Met Pro Asp Ala Gly
            20                  25                  30

Glu Val Leu Val Lys Val Glu Ala Cys Gly Val Cys Phe Ser Asp Thr
        35                  40                  45

Val Pro Gln Ala His Gly Leu Gly Gly Lys Phe Pro Ile Val Pro Gly
    50                  55                  60

His Glu Ile Ile Gly His Val Val Ala Thr Gly Asp Gly Val Ser Asp
65                  70                  75                  80

Trp Glu Val Gly Asp Arg Ile Gly Glu Gly Trp His Gly Gly His Asp
                85                  90                  95

Gly Thr Cys Pro Ser Cys Arg Gln Gly His Phe Gln Met Cys Asp Asn
            100                 105                 110

Gln Ser Ile Asn Gly Val Thr Lys Asn Gly Gly Tyr Ala Gln Tyr Cys
        115                 120                 125

Ile Leu Arg Ser Glu Ala Ala Val Arg Ile Pro Thr His Val Ser Ala
    130                 135                 140

Ala Glu Tyr Ala Pro Ile Leu Cys Ala Gly Val Thr Val Phe Asn Ser
145                 150                 155                 160

Met Arg Gln Ile Gly Val Lys Pro Gly Ser Thr Val Ala Ile Gln Gly
                165                 170                 175

Leu Gly Gly Leu Gly His Leu Ala Ile Gln Tyr Ala Asn Arg Phe Gly
            180                 185                 190

Phe Arg Val Val Ala Ile Ser Arg Asp Asp Gln Lys Glu Arg Phe Val
        195                 200                 205

Arg Asp Leu Gly Ala His Glu Tyr Ile Asn Thr Ser Glu Glu Asp Val
    210                 215                 220

Gly Ser Ala Leu Gln Lys Leu Gly Gly Ala Ser Leu Ile Val Ala Thr
225                 230                 235                 240

Ala Pro Asn Ala Arg Ala Ile Ser Pro Leu Leu Lys Gly Leu Arg Pro
                245                 250                 255

Leu Gly Lys Leu Leu Ile Leu Ala Val Pro Gly Glu Ile Pro Leu Asp
            260                 265                 270

Thr Arg Leu Met Val Ala Arg Gly Leu Ser Val His Gly Trp Pro Ser
        275                 280                 285

Gly His Ala Leu Asp Ser Glu Glu Thr Ile Arg Phe Thr Glu Leu Glu
    290                 295                 300

Asp Ile Lys Cys Met Ile Gln Thr Tyr Ser Leu Asp Arg Ala Asn Glu
305                 310                 315                 320

Ala Phe Asp Ala Met Ile Ser Gly Ser Val Arg Phe Arg Ala Val Ile
                325                 330                 335

Thr Met Glu

<210>  3
<211>  505
<212>  PRT
<213>  Penicillium brasilianum

<400>  3

Met Thr Gln Thr Asn Val His Val Asn Lys Ser Asp Thr Ser Leu Ala
1               5                   10                  15

Ala Pro Gln Gln Leu Phe Ile Ser Gly Lys Tyr Gln Asn Ser Gln Arg
            20                  25                  30

Asn Gly Thr Phe Pro Val Lys Asn Pro Met Thr Gly Glu Thr Ile Tyr
        35                  40                  45

Glu Cys Val Ser Ala Ser Leu Asp Asp Tyr Ala Ala Ala Ile Glu Glu
    50                  55                  60

Ala Asp Ala Ala Gln Pro Ser Trp Ala Arg Leu Gly Pro Ser Ala Arg
65                  70                  75                  80

Arg Leu Ile Leu Leu Lys Ala Ala Asp Ile Met Glu Thr Tyr Ile Glu
                85                  90                  95

Thr Asp Ala Pro Ala Ile Leu Ser Ala Glu Val Ser Ala Thr Arg Gly
            100                 105                 110

Trp Val Arg Ala Asn Ile Leu Ser Thr Ala Gly Val Phe Arg Glu Thr
        115                 120                 125

Ala Ala Leu Ala Thr His Ile Lys Gly Glu Ile Val Pro Ala Asp Arg
    130                 135                 140

Pro Gly Thr Thr Ile Leu Val Ser Arg Glu Pro Val Gly Val Val Leu
145                 150                 155                 160

Ala Ile Ser Pro Trp Asn Met Pro Ala Thr Leu Thr Ala Arg Ala Ile
                165                 170                 175

Cys Cys Pro Leu Ile Cys Gly Asn Ser Val Val Leu Arg Pro Ser Glu
            180                 185                 190

Phe Ser Pro Lys Ser Gln His Leu Val Val Arg Ala Leu Thr Glu Ala
        195                 200                 205

Gly Leu Pro Ala Gly Cys Leu Gln Phe Leu Pro Thr Ser Thr Ala Asp
    210                 215                 220

Thr Pro Arg Ala Ile Glu Phe Ala Ile Arg His Pro Lys Val Ser Arg
225                 230                 235                 240

Ala Asn Phe Thr Gly Ser Asp Arg Val Gly Arg Ile Ile Ala Gly Leu
                245                 250                 255

Ser Ala Ser Cys Leu Lys Pro Cys Val Leu Glu Leu Gly Gly Lys Ala
            260                 265                 270

Pro Val Val Val Leu Glu Asp Ala Asp Val Glu Ala Ala Val Glu Ala
        275                 280                 285

Val Val Tyr Gly Ala Met Ser Asn Ser Gly Gln Ile Cys Met Ser Thr
    290                 295                 300

Glu Arg Ala Ile Val His Arg Ser Leu Ala Ala Asp Phe Lys Ala Leu
305                 310                 315                 320

Leu Val Lys Arg Ala Glu Ser Leu Arg Val Gly Asn His Leu Glu Asp
                325                 330                 335

Pro Asp Val Gln Leu Ser Gly Leu Phe Thr Ala Ala Ser Ala Glu Arg
            340                 345                 350

Val Leu Gly Leu Ile Lys Gly Ala Val Asn Ala Gly Ala Thr Leu Leu
        355                 360                 365

Ala Gly Asp Leu Ala Leu His Gly Pro Cys Gln Thr Ile Met Ala Pro
    370                 375                 380

His Ile Leu Thr Gly Val Thr Arg Asp Met Asp Leu Phe His Arg Glu
385                 390                 395                 400

Thr Phe Gly Pro Val Leu Phe Val Ser Glu Phe Asp Thr Asp Asp Glu
                405                 410                 415

Ala Ile Ala Gln Ala Asn Asp Thr Glu Phe Ser Leu Cys Ala Ser Val

420                 425                 430

Phe Ser Arg Asp Val Leu Arg Ala Met Asp Thr Ala Lys Arg Ile Arg
        435                 440                 445

Thr Gly Ser Cys His Val Asn Gly Pro Thr Val Tyr Ile Glu Ala Pro
    450                 455                 460

Leu Pro Asn Gly Gly Val Gly Gly Gly Ser Gly Tyr Gly Arg Phe Gly
465                 470                 475                 480

Gly Val Ala Gly Ile Glu Glu Phe Thr Glu Arg Gln Ile Val Ser Leu
                485                 490                 495

Ala Lys Pro Gly Ile Lys Tyr Ala Phe
            500                 505

<210>  4
<211>  427
<212>  PRT
<213>  Penicillium brasilianum

<400>  4

Met Pro His Ala Ser Arg Ser Leu Asn Val Leu Ile Val Gly Ala Gly
1               5                   10                  15

Leu Gly Gly Leu Ala Ala Gly Leu Ala Leu Gln Thr Asp Gly His Lys
            20                  25                  30

Val Thr Ile Ile Asp Ala Ala Pro Glu Phe Ala Glu Ala Gly Ala Gly
        35                  40                  45

Ile Arg Ile Pro Pro Asn Ser Ser Arg Leu Leu Met Arg Trp Gly Val
    50                  55                  60

Asp Leu Glu Arg Met Lys Lys Ser Thr Ser Gln Arg Tyr His Phe Ile
65                  70                  75                  80

Arg Trp Lys Asp Gly Ser Thr Ile Phe Asp Leu Pro Phe Asn Asn Ile
                85                  90                  95

Val Glu Thr His Gly Ala Pro Tyr Trp Leu Val His Arg Ala Asp Leu
            100                 105                 110

His Ala Ala Leu Leu Asp Ala Thr Leu Lys Ala Gly Val Lys Val Leu
        115                 120                 125

Asn Asn Lys Leu Val Thr Ser Tyr Asp Phe Glu Ala Pro Ser Ala Thr

    130                 135                 140

Thr Gln Asp Gly Glu Thr Phe Lys Ala Asp Leu Ile Val Gly Ala Asp
145                 150                 155                 160

Gly Ile Lys Ser Ile Cys Arg Pro Leu Leu Thr Gly Gln Pro Asp Val
                165                 170                 175

Pro Arg Asp Thr Gly Asp Val Ala Tyr Arg Ile Leu Ile Pro Gly Glu
            180                 185                 190

Lys Leu Leu Ala Asp Pro Asp Leu Ala His Leu Ile Arg Asp Pro Cys
        195                 200                 205

Thr Thr Ser Trp Cys Gly Pro Asp Ala His Leu Val Gly Tyr Pro Ile
    210                 215                 220

Arg Asn Gly Glu Met Tyr Asn Ile Val Met Cys Ala Thr Ser Tyr Asn
225                 230                 235                 240

Glu Thr Thr Asp Glu Val Trp Val Val Lys Gly Asp Asn Ser Glu Leu
                245                 250                 255

Cys Lys Arg Phe Ala Ser Trp Glu Pro Gln Val Arg Lys Leu Cys Ala
            260                 265                 270

Leu Thr Gly Asp Phe Met Lys Trp Arg Leu Cys Asp Leu Pro Asn Leu
        275                 280                 285

Ala Arg Trp Thr His Pro Ser Gly Lys Ala Val Leu Leu Gly Asp Ser
    290                 295                 300

Cys His Pro Met Leu Pro Tyr Leu Ala Gln Gly Ala Ala Gln Ala Val
305                 310                 315                 320

Glu Asp Ala Ala Val Leu Arg Gln Val Leu Ala Gln Asp Met Asp Met
                325                 330                 335

Ala Ala Ala Leu Lys Gln Tyr Glu Gln Ile Arg Met Pro Arg Ala Ser
            340                 345                 350

Leu Val Gln Ala Lys Thr Arg Glu His Gln Tyr Ile Leu His Val Asp
        355                 360                 365

Asp Gly His Glu Gln Gln Asp Arg Asp Lys Lys Leu Ala Leu Asp Ala
    370                 375                 380

Ala Glu Asn Pro Val Phe Trp Gly Tyr Asp Asp Arg Arg Lys Trp Leu
385                 390                 395                 400

Phe Ser His Asp Ala Glu Val Ile Gln Lys Glu Gly Ala Asn Trp Arg
                405                 410                 415

Asp Gly Pro Asn Met Asn Gly Val His Val Ala
            420                 425

<210>  5
<211>  245
<212>  PRT
<213>  Penicillium brasilianum

<400>  5

Met Ser Leu Ser Gly Lys Val Val Leu Ile Thr Gly Ser Ser Lys Gly
1               5                   10                  15

Ile Gly Lys Ala Ala Ala Leu Arg Val Ala Ser Glu Gly Ala Asn Val
            20                  25                  30

Val Ile Asn Tyr Leu Arg Asp Pro Val Ala Ala Asn Asn Leu Val Asp
        35                  40                  45

Gln Ile Gly Ala Asp Arg Ala Leu Ala Val Gln Ala Asp Ala Ser Lys
    50                  55                  60

Leu Ala Asp Leu Asp Arg Leu Val Asn Ala Ala Val Ala Gln Phe Gly
65                  70                  75                  80

Lys Ile Asp Val Leu Ile Pro Asn Ala Gly Ile Leu Pro Leu Arg Asp
                85                  90                  95

Leu Glu His Thr Ser Glu Glu Asp Phe Asp Arg Thr Tyr Asn Leu Met
            100                 105                 110

Val Lys Gly Pro Tyr Phe Leu Ala Gln Lys Ala Val Lys His Met Pro
        115                 120                 125

Pro Gly Gly Arg Ile Ile Phe Val Ser Thr Ser Thr Ala Arg Phe Ala
    130                 135                 140

Ser Val Ala Pro Ala Tyr Leu Leu Tyr Thr Ser Ser Lys Gly Ala Ile
145                 150                 155                 160

Glu Gln Met Thr Arg Ile Met Ala Lys Asp Leu Ala Arg Lys Gly Ile
                165                 170                 175

Leu Val Asn Ala Val Ala Pro Gly Pro Thr Ser Thr Glu Leu Phe Leu
        180                 185                 190

Glu Gly Lys Pro Glu Gln Met Ile Lys Ala Ile Ser Gly Phe Ser Pro
        195                 200                 205

Phe Asn Arg Ile Gly Glu Pro Glu Glu Ile Ala Ala Val Met Ala Phe
    210                 215                 220

Leu Ser Gly Lys Asp Ser Ser Trp Ile Ser Gly Gln Val Val Ala Val
225                 230                 235                 240

Asn Gly Ala Met Ala
                245

<210>  6
<211>  581
<212>  PRT
<213>  Penicillium brasilianum

<400>  6

Met Ala Ser Leu Ile Arg Glu Ala Pro Phe Gly Gln Ile Val Arg Tyr
1               5                   10                  15

Leu Thr Asn Asn Lys Tyr Phe Gln Tyr Pro Glu Glu Lys Pro Asp Phe
            20                  25                  30

Lys Leu Pro Asp Thr Trp Leu Gln Leu Leu Asn Glu Ser Asp Ala Ala
        35                  40                  45

Thr Ile Ala Asp Pro Glu Lys Thr Glu Pro Glu Pro Glu Gly Gln Gly
    50                  55                  60

Tyr Asp Ala Thr Ser Glu Ala Ile Ser Arg Ala Ser Thr Gln Asn Ser
65                  70                  75                  80

Leu Pro Phe Thr Glu Ala Arg Leu Glu Ala Asp Glu Gln His Glu Ile
                85                  90                  95

Glu Lys Ile Lys Ser Ile Pro Ile Gln Pro Lys Lys Thr Lys Asp Gly
            100                 105                 110

Ala Ile Leu Val Asp Trp Tyr Tyr Thr Asp Asp Ala Glu Asn Pro His
        115                 120                 125

Asn Trp Ser Asn Arg Lys Arg Ala Leu Leu Thr Thr Leu Ile Cys Leu
    130                 135                 140

Tyr Thr Phe Val Val Tyr Thr Thr Ser Ala Ile Tyr Thr Ser Ser Val
145                 150                 155                 160

Pro Gly Ile Met Lys Glu Phe Gly Val Ser Asp Leu Val Ala Thr Leu
                165                 170                 175

Gly Leu Ser Leu Tyr Val Leu Gly Tyr Gly Thr Gly Pro Leu Ile Phe
            180                 185                 190

Ser Pro Leu Ser Glu Ile Pro Val Ile Gly Arg Asn Pro Val Tyr Ile
        195                 200                 205

Val Thr Met Phe Leu Phe Val Ile Leu Ser Ile Pro Thr Ala Phe Val
    210                 215                 220

Gly Asn Phe Ala Gly Leu Met Val Leu Arg Phe Leu Gln Gly Phe Phe
225                 230                 235                 240

Gly Ser Pro Cys Leu Ala Ser Gly Gly Ala Ser Ile Gly Asp Met Tyr
                245                 250                 255

Ser Leu Met Ser Leu Pro Tyr Ala Met Met Ser Trp Val Ser Ala Ala
            260                 265                 270

Tyr Cys Gly Pro Ala Leu Gly Pro Leu Ile Ser Gly Phe Ala Val Pro
        275                 280                 285

Ala Glu Thr Trp Arg Trp Ser Leu Phe Glu Ser Ile Trp Met Ser Ala
    290                 295                 300

Pro Val Leu Ile Leu Met Phe Phe Phe Leu Pro Glu Thr Ser Ser Ala
305                 310                 315                 320

Thr Ile Leu Leu Arg Arg Ala Ala Arg Leu Arg Lys Ile His Asn Asn
                325                 330                 335

Ala Arg Phe Met Ala Gln Ser Glu Ile Asp Gln Arg Asn Met Lys Val
            340                 345                 350

Ser Ala Val Ala Val Asp Ala Leu Ile Lys Pro Leu Glu Ile Thr Ile
        355                 360                 365

Lys Asp Pro Ala Val Leu Phe Val Gln Val Tyr Thr Ala Ile Ile Tyr
    370                 375                 380

Gly Ile Tyr Tyr Ser Phe Phe Glu Val Phe Pro Leu Val Tyr Pro Val
385                 390                 395                 400

Asp Tyr Gly Met Asn Leu Gly Gln Val Gly Leu Val Phe Leu Cys Ile
                405                 410                 415

Leu Val Ser Cys Ile Ile Gly Ile Ala Ile Tyr Trp Ser Tyr Leu Tyr
            420                 425                 430

Phe Trp Met Asn Pro Arg Ile Glu Arg Phe Gly Phe Pro Ala Gln Glu
        435                 440                 445

Ser Arg Leu Ile Pro Ala Leu Pro Ala Ser Ile Gly Pro Thr Ile Gly
    450                 455                 460

Leu Phe Leu Phe Ala Trp Thr Ala Arg Ala Ser Ile His Trp Ile Ala
465                 470                 475                 480

Pro Thr Ile Gly Ile Thr Ile Tyr Gly Ala Thr Val Phe Ile Val Met
                485                 490                 495

Gln Cys Leu Phe Val Tyr Ile Pro Leu Ser Tyr Pro Met Tyr Ala Ala
            500                 505                 510

Ser Leu Phe Ala Ala Asn Asp Phe Phe Arg Ser Ala Leu Ala Cys Gly
        515                 520                 525

Ser Val Leu Phe Ala His Pro Leu Phe Gly Asn Leu Gly Val Ala Arg
    530                 535                 540

Gly Thr Ser Leu Leu Gly Gly Leu Ser Val Ile Gly Ile Ile Gly Ile
545                 550                 555                 560

Trp Leu Leu Tyr Tyr Tyr Gly Ala Arg Leu Arg Ser Leu Ser Lys Phe
                565                 570                 575

Ala Ile Ser Asp Asp
            580

<210> 7
<211> 513
<212> PRT
<213> Penicillium brasilianum

<400> 7

Met Ser Thr Thr Lys Glu Ala Phe Pro His Thr Asp Ser Asp Ile Met
1               5                   10                  15

Glu Asp Ser Glu Lys Asn Leu Pro Glu Cys Glu His Ile Val Ser Val
            20                  25                  30

Glu Pro Thr Leu Lys Met Arg Asp Gly Ile Val Leu Met Pro Gln Pro
        35                  40                  45

Ser Asp Asp Pro Asn Asp Pro Leu Asn Trp Ser Trp Phe Arg Lys His
    50                  55                  60

Ala Ala Met Phe Thr Leu Ser Tyr Leu Ala Leu Val Cys Tyr Val Ala
65                  70                  75                  80

Val Thr Thr Leu Val Thr Gly Thr Val Pro Leu Ala Lys Ser Met His
                85                  90                  95

Val Ser Lys Ser Thr Ala Val Tyr Leu Gly Asn Thr Pro Val Ala Leu
            100                 105                 110

Tyr Ala Val Ala Pro Trp Phe Trp Ser Pro Leu Ser His Phe Ile Gly
        115                 120                 125

Arg Arg Pro Val Leu Leu Met Cys Asn Ile Ile Ala Val Val Gly Ala
    130                 135                 140

Val Val Val Thr Thr Ser Lys Thr Tyr Ala Ser Cys Met Val Gly Arg
145                 150                 155                 160

Val Ile Leu Gly Ala Gly Gly Ser Ala Phe Trp Thr Leu Gly Pro Ala
                165                 170                 175

Ser Ile Gly Asp Ile Phe Phe Arg His Glu Lys Gly Lys Lys Ile Gly
            180                 185                 190

Val Ser Thr Leu Ala Ile Val Ile Ala Pro Phe Leu Gly Thr Ile Ile
        195                 200                 205

Gly Gly Pro Ile Ile Glu Asn Glu Lys Leu Gly Trp Pro Ala Ser Gln
    210                 215                 220

Trp Ile Pro Leu Ile Phe Met Ala Ala Gly Phe Ile Met Gln Ile Phe
225                 230                 235                 240

Phe Leu Pro Glu Thr Ile Tyr Ile Arg Glu Thr Arg Ala His Pro Ala
                245                 250                 255

Ile Met Ser Thr Ser Thr Pro Gly Lys Pro Thr Phe Trp Asp Arg Tyr
            260                 265                 270

Gly Ile His Ile Pro Lys Arg Ser Glu Glu Lys Gln His Ser Phe Leu
        275                 280                 285

Phe Ile Ala Thr Arg Pro Phe Val Leu Phe Lys Phe Pro Ala Val Ile
    290                 295                 300

Leu Ser Ala Phe Trp Phe Gly Ile Ala Tyr Met Met His Val Gly Ile
305                 310                 315                 320

Thr Ser Glu Ile Pro Leu Ile Phe Glu Glu His Tyr Asp Phe Ser Val
                325                 330                 335

Leu Glu Ile Gly Leu Ser Gly Phe Ser Gly Leu Ile Gly Ala Leu Leu
            340                 345                 350

Gly Glu Val Tyr Ala Gly Pro Ser Leu Asp Phe Ile Ala Lys Arg Thr
        355                 360                 365

Met Lys Gln Gly Arg Glu Trp Arg Pro Glu Tyr Arg Leu Gln Ala Ile
    370                 375                 380

Trp Pro Ala Leu Ile Thr Val Pro Ala Gly Leu Ile Met Phe Gly Thr
385                 390                 395                 400

Ser Ile Gln Phe Gly Asn Val Trp Ile Thr Pro Leu Ile Gly Gln Ala
                405                 410                 415

Val Tyr Ile Phe Gly Ile Glu Ile Ala Thr Thr Val Ile Gln Thr Tyr
            420                 425                 430

Ile Leu Glu Cys Tyr Pro Arg Gln Gly Ala Glu Ala Asn Leu Val Phe
        435                 440                 445

Asn Leu Ile Arg Asn Leu Phe Ser Tyr Ile Ser Pro Phe Phe Val Gln
    450                 455                 460

Pro Met Ile Ala Thr Leu Gly Thr Thr Ser Pro Phe Gly Leu Ser Ala
465                 470                 475                 480

Ala Leu Thr Ala Phe Phe Phe Pro Phe Thr Val Gly Val Leu Met Trp
                485                 490                 495

Arg Gly Lys Gln Ile Arg Asp Lys Gly Gly Asp Pro Gly Trp Ser Arg
            500                 505                 510

Asp

<210>  8

<211>  1435
<212>  PRT
<213>  Penicillium brasilianum

<400>  8

Met Glu Asp His Glu Lys Glu Tyr Asp Ser Thr Ser Pro Pro Gly Thr
1               5                   10                  15

Ala Thr Glu Glu Gly Asn Gly Gly Tyr Phe Asn Thr Leu Thr Val Pro
            20                  25                  30

Glu Ile Asn Leu Arg Glu Ala Ser Ser Ala Glu Thr Leu Thr Pro His
        35                  40                  45

Ala Ser Val Val Gln Pro Pro Lys Lys Ala Ala Glu Trp His Met Thr
    50                  55                  60

Pro Gln Val Ile Arg Asn Ala Glu Arg Asp Glu Ala Ala Gly Phe Lys
65                  70                  75                  80

Arg Arg Glu Leu Gly Val Thr Trp Gln Asp Leu Ser Val Glu Val Leu
                85                  90                  95

Ala Ala Glu Ala Ala Val Lys Glu Asn Met Ile Ser Gln Phe Asn Val
            100                 105                 110

Pro Gln Leu Ile Lys Asp Phe Arg Arg Lys Pro Pro Leu Lys Ser Ile
        115                 120                 125

Leu Ser Asn Ser His Gly Cys Val Lys Pro Gly Glu Met Leu Leu Val
    130                 135                 140

Leu Gly Arg Pro Gly Ser Gly Cys Thr Thr Leu Leu Lys Met Leu Ala
145                 150                 155                 160

Asn Arg Arg Glu Gly Tyr Gln Asn Ile Thr Gly Asp Val Arg Phe Gly
                165                 170                 175

Asn Met Thr Pro Glu Glu Ala Ser Arg Tyr Gln Gly Gln Ile Val Met
            180                 185                 190

Asn Thr Glu Glu Glu Leu Phe Tyr Pro Arg Leu Thr Val Gly Gln Thr
        195                 200                 205

Met Asp Phe Ala Thr Lys Leu Lys Val Pro Tyr His Leu Pro Gly Glu
    210                 215                 220

Gly Lys Ser Val Ala Glu Tyr Thr Ala Glu Thr Lys Gln Phe Leu Leu

225                 230                 235                 240

Glu Ser Met Gly Ile Ala His Thr Ala Asp Thr Lys Val Gly Asn Glu
                245                 250                 255

Phe Val Arg Gly Val Ser Gly Gly Glu Arg Lys Arg Val Ser Ile Ile
            260                 265                 270

Glu Cys Leu Ala Thr Arg Gly Ser Val Phe Thr Trp Asp Asn Ser Thr
        275                 280                 285

Arg Gly Leu Asp Ala Ser Thr Ala Leu Glu Trp Ala Lys Ala Leu Arg
    290                 295                 300

Ala Met Thr Asp Val Gln Gly Leu Ser Thr Ile Val Thr Leu Tyr Gln
305                 310                 315                 320

Ala Gly Asn Gly Ile Tyr Asn Leu Phe Asp Lys Val Leu Val Leu Asp
                325                 330                 335

Glu Gly Lys Gln Ile Tyr Tyr Gly Pro Ala Ala Glu Ala Lys Pro Phe
            340                 345                 350

Met Glu Asn Leu Gly Phe Val Tyr Thr Asp Gly Ala Asn Ile Gly Asp
        355                 360                 365

Phe Leu Thr Gly Leu Thr Val Pro Thr Glu Arg Lys Ile Arg Pro Gly
    370                 375                 380

Trp Glu Asn Arg Phe Pro Arg Thr Ala Asp Ala Ile Leu Thr Glu Tyr
385                 390                 395                 400

Gln Asn Ser Ala Thr Tyr Lys Asn Glu Val Ser Leu Tyr Gly Tyr Pro
                405                 410                 415

Asp Thr Asp Leu Ala Ala Glu Arg Thr Glu Ala Phe Lys Glu Ser Val
            420                 425                 430

Ala Trp Glu Lys Ser Lys His Leu Pro Lys Gly Ser Asp Leu Thr Thr
        435                 440                 445

Ser Phe Trp Ala Gln Leu Met Ser Cys Thr Ala Arg Gln Tyr Gln Ile
    450                 455                 460

Leu Trp Gly Glu Lys Ser Thr Phe Leu Ile Lys Gln Ile Leu Ser Cys
465                 470                 475                 480

Val Met Ala Leu Ile Ala Gly Ser Cys Phe Tyr Asn Ser Pro Asp Thr
                485                 490                 495

Ser Ala Gly Leu Phe Thr Lys Gly Gly Ala Val Phe Phe Ser Leu Leu
            500                 505                 510

Tyr Asn Cys Ile Val Ala Met Ser Glu Val Thr Glu Ser Phe Lys Gly
        515                 520                 525

Arg Pro Ile Leu Thr Lys His Lys Ser Phe Ala Met Tyr His Pro Ala
    530                 535                 540

Ala Phe Cys Leu Ala Gln Ile Thr Ala Asp Phe Pro Val Leu Leu Phe
545                 550                 555                 560

Gln Cys Thr Ile Phe Ser Val Val Ile Tyr Trp Met Val Gly Leu Lys
                565                 570                 575

His Thr Ala Ala Ala Phe Phe Thr Phe Trp Ala Ile Leu Phe Thr Thr
            580                 585                 590

Thr Leu Cys Ile Thr Ala Leu Phe Arg Phe Ile Gly Ala Ala Phe Ser
        595                 600                 605

Ser Phe Glu Ala Ala Ser Lys Ile Ser Gly Thr Ala Val Lys Ala Ile
    610                 615                 620

Val Met Tyr Ala Gly Tyr Met Ile Pro Lys Pro Glu Ile Lys Asn Trp
625                 630                 635                 640

Phe Leu Glu Phe Tyr Tyr Thr Asn Pro Phe Ala Tyr Ala Phe Gln Ala
                645                 650                 655

Ala Leu Thr Asn Glu Phe His Asp Gln His Ile Asp Cys Val Gly Gly
            660                 665                 670

Asn Leu Ile Pro Ser Gly Pro Gly Tyr Glu Asp Val Gly Ser Gly Tyr
        675                 680                 685

Lys Ala Cys Ala Gly Val Gly Gly Ala Leu Pro Gly Ala Asp Tyr Val
    690                 695                 700

Thr Gly Asp Gln Tyr Leu Ser Ser Leu His Tyr Lys His Ser Gln Leu
705                 710                 715                 720

Trp Arg Asn Phe Gly Val Val Trp Ala Trp Trp Gly Phe Phe Ala Val
                725                 730                 735

Leu Thr Val Val Phe Thr Cys Phe Trp Lys Ser Gly Ala Ala Ser Gly
        740                 745                 750

Ser Ser Leu Leu Ile Pro Arg Glu Asn Leu Lys Lys His Gln Val Gly
    755                 760                 765

Asn Asp Glu Glu Ala Gln Asn Asn Glu Lys His Ala Ala Arg Thr Thr
    770                 775                 780

Thr Asp Glu Pro Val Gln Val Glu Asp Asp Asn Leu Val Arg Asn Thr
785                 790                 795                 800

Ser Ile Phe Thr Trp Lys Asn Leu Thr Tyr Thr Val Lys Thr Pro Thr
                805                 810                 815

Gly Asp Arg Val Leu Leu Asp Asn Ile Asn Gly Trp Val Lys Pro Gly
        820                 825                 830

Met Leu Gly Ala Leu Met Gly Ser Ser Gly Ala Gly Lys Thr Thr Leu
    835                 840                 845

Leu Asp Val Leu Ala Gln Arg Lys Thr Glu Gly Thr Ile Lys Gly Ser
    850                 855                 860

Ile Leu Val Asp Gly Arg Glu Leu Pro Val Ser Phe Gln Arg Met Ala
865                 870                 875                 880

Gly Tyr Cys Glu Gln Leu Asp Val His Glu Ser Tyr Ala Thr Val Arg
                885                 890                 895

Glu Ala Leu Glu Phe Ser Ala Leu Leu Arg Gln Ser Arg Asp Thr Pro
        900                 905                 910

Lys Ala Glu Lys Leu Lys Tyr Val Asp Thr Ile Ile Asp Leu Leu Glu
    915                 920                 925

Leu His Asp Leu Ala Asp Thr Leu Ile Gly Ser Val Gly Asn Gly Leu
    930                 935                 940

Ser Val Glu Gln Arg Lys Arg Val Thr Ile Gly Val Glu Leu Val Ser
945                 950                 955                 960

Lys Pro Ser Ile Leu Ile Phe Leu Asp Glu Pro Thr Ser Gly Leu Asp
                965                 970                 975

Gly Gln Ser Ala Tyr Asn Thr Val Arg Phe Leu Arg Lys Leu Ala Asp
        980                 985                 990

Val Gly Gln Ala Val Leu Val Thr Ile His Gln Pro Ser Ala Gln Leu
        995                 1000                1005

Phe Ala Gln Phe Asp Thr Leu Leu Leu Leu Ala Arg Gly Gly Lys
    1010                1015                1020

Thr Val Tyr Phe Gly Asp Ile Gly Asp Asn Gly Ser Thr Ile Lys
    1025                1030                1035

Gln Tyr Phe Gly Asn Tyr Gly Ala Ile Cys Pro Gln Glu Ala Asn
    1040                1045                1050

Pro Ala Glu Phe Met Ile Asp Val Val Thr Gly Gly Ile Gln Glu
    1055                1060                1065

Val Lys Asp Lys Asp Trp His Gln Ile Trp Leu Asp Ser Pro Glu
    1070                1075                1080

Gln His Gln Met Ile Thr Glu Leu Asp Arg Met Ile Ala Asp Ala
    1085                1090                1095

Ala Ser Lys Pro Pro Gly Thr Val Asn Asp Gly Tyr Glu Phe Ser
    1100                1105                1110

Met Pro Leu Trp Glu Gln Ile Lys Ile Val Thr Gln Arg Met Asn
    1115                1120                1125

Val Ser Leu Phe Arg Asn Thr Ala Tyr Val Asn Asn Lys Phe Ser
    1130                1135                1140

Leu His Ile Ile Ser Ala Leu Leu Asn Gly Phe Ser Phe Trp Arg
    1145                1150                1155

Pro Gly Pro Ser Val Ser Ala Leu Gln Leu Lys Met Phe Thr Ile
    1160                1165                1170

Phe Asn Phe Val Phe Val Ala Pro Gly Val Ile Asn Gln Leu Gln
    1175                1180                1185

Pro Leu Phe Ile Gln Arg Arg Asp Ile Tyr Asp Ala Arg Glu Lys
    1190                1195                1200

Lys Ser Lys Met Tyr Ser Trp Val Ala Phe Val Thr Gly Leu Ile
    1205                1210                1215

Val Ser Glu Phe Pro Tyr Leu Cys Ile Cys Ala Val Leu Tyr Phe

1220                1225                1230

Val Cys  Trp Tyr Trp Pro Val  Trp Arg Leu Pro His  Asp Ser Asp
    1235                1240                1245

Arg Ser  Gly Ala Ile Phe Phe  Met Met Leu Ile Tyr  Glu Phe Ile
    1250                1255                1260

Tyr Thr  Gly Ile Gly Gln Phe  Ile Ala Ala Tyr Ala  Pro Asn Pro
    1265                1270                1275

Thr Phe  Ala Ala Leu Val Asn  Pro Leu Ile Ile Ser  Val Leu Val
    1280                1285                1290

Leu Phe  Cys Gly Val Phe Val  Pro Tyr Asp Gln Leu  Asn Val Phe
    1295                1300                1305

Trp Lys  Tyr Trp Met Tyr Tyr  Leu Asn Pro Phe Asn  Tyr Val Val
    1310                1315                1320

Asn Gly  Met Leu Thr Phe Gly  Leu Trp Gly Gln Lys  Val Thr Cys
    1325                1330                1335

Asn Glu  Ser Glu Tyr Ala Val  Phe Asp Pro Leu Asn  Gly Thr Cys
    1340                1345                1350

Gly Glu  Tyr Leu Ala Thr Tyr  Met Ser Gly Lys Gly  Ser Gly Val
    1355                1360                1365

Asn Leu  Leu Asn Pro Asp Ala  Thr Ser Ser Cys Lys  Val Cys Glu
    1370                1375                1380

Tyr Thr  Thr Gly Ser Asp Phe  Leu Gln Thr Leu Asn  Ile Asn His
    1385                1390                1395

Tyr Tyr  Tyr Gly Trp Arg Asp  Ala Gly Ile Thr Val  Ile Tyr Ala
    1400                1405                1410

Ile Ser  Gly Tyr Ala Leu Val  Phe Gly Leu Met Lys  Leu Arg Thr
    1415                1420                1425

Lys Ala  Ser Lys Lys Ala Glu
    1430                1435

<210>  9
<211>  527
<212>  PRT
<213>  Penicillium brasilianum

<400>  9

Met Thr Pro Ile Ser Arg Leu Leu Ser Arg Ala Val Asn Lys Pro Tyr
1               5                   10                  15

Arg Thr Lys Asp Ile Thr Asp Glu Ile Pro Pro Thr Leu Asp Glu Asp
            20                  25                  30

Gly Phe Val Ser Phe Gly Pro Gly Asp Ile Glu Asn Pro Arg Asn Trp
        35                  40                  45

Ser Met Arg Arg Arg Ala Gly Val Thr Met Ser Ala Val Leu Leu Val
    50                  55                  60

Val Asn Ala Thr Phe Ala Ser Ser Ser Pro Ser Gly Cys Phe Pro Ser
65                  70                  75                  80

Ile Ser Lys His Phe Gly Val Ser Thr Glu Val Ala Gly Leu Thr Ile
                85                  90                  95

Thr Leu Phe Leu Leu Gly Tyr Cys Ala Gly Pro Leu Ile Phe Ala Pro
            100                 105                 110

Leu Ser Glu Phe Tyr Gly Arg Arg Trp Ile Phe Tyr Ile Thr Phe Leu
        115                 120                 125

Leu Tyr Leu Ala Phe Asn Phe Leu Cys Ala Phe Pro Pro Asn Phe Gly
    130                 135                 140

Ser Leu Leu Val Gly Arg Phe Leu Thr Gly Thr Phe Val Ser Ala Pro
145                 150                 155                 160

Leu Ser Asn Cys Pro Gly Val Leu Ala Asp Val Trp Asn Pro Leu Glu
                165                 170                 175

Arg Ala Asn Ala Met Ala Gly Phe Ser Ala Met Val Trp Ile Gly Pro
            180                 185                 190

Ala Leu Gly Pro Val Val Ala Gly Phe Leu Gln Leu Lys Glu Asp Trp
        195                 200                 205

Arg Trp Ser Phe Tyr Val Leu Leu Trp Leu Gly Gly Ala Ser Ala Val
    210                 215                 220

Ile Met Leu Thr Ile Pro Glu Thr Tyr Ala Pro Ile Val Leu Tyr Asn
225                 230                 235                 240

Lys Ala Arg Arg Ile Arg Glu Ala Gln Ile Pro Gly Tyr Glu Asn Val
                245                 250                 255

Lys Ala Pro Val Glu Asp Gly Asp Arg Thr Leu Val Gly Ile Tyr Lys
            260                 265                 270

Val Ala Leu Thr Arg Pro Trp Ile Ile Leu Phe Asp Pro Ile Ser Leu
        275                 280                 285

Leu Cys Ala Ile Tyr Met Ala Phe Val Tyr Thr Leu Leu Tyr Met Leu
    290                 295                 300

Phe Thr Ile Tyr Pro Ile Val Phe Gln Glu Lys Arg Gly Trp Asn Ser
305                 310                 315                 320

Gly Val Gly Glu Leu Pro Leu Leu Gly Thr Val Val Gly Ala Leu Phe
                325                 330                 335

Gly Gly Val Ile Val Val Ala Asp Thr Arg Met Arg Gln Lys Arg Ile
            340                 345                 350

Asp Asn Gly Thr Thr Lys Met Glu Asp Ala Val Pro Glu Asp Arg Leu
        355                 360                 365

Pro Leu Ala Met Gly Gly Gly Ile Gly Phe Ala Val Thr Met Phe Trp
    370                 375                 380

Phe Ala Trp Ser Ala Glu Phe Asn Ser Val His Trp Ile Val Pro Thr
385                 390                 395                 400

Leu Ala Gly Val Phe Leu Ser Ser Ala Leu Leu Leu Ile Phe Val Gly
                405                 410                 415

Phe Leu Asn Tyr Leu Val Asp Val Tyr Gln Met Tyr Ala Ala Ser Ala
            420                 425                 430

Ile Ala Ala Asn Thr Ile Ala Arg Ser Ala Cys Gly Ala Ala Ala Pro
        435                 440                 445

Leu Phe Thr Ser Gln Met Phe Ala Ala Leu Gly Val Gly Gly Gly Gly
    450                 455                 460

Ser Leu Ile Ala Gly Val Ala Thr Leu Leu Ala Ala Ile Pro Phe Leu
465                 470                 475                 480

Phe Tyr Lys Tyr Gly Lys Gln Ile Arg Met Arg Ser Lys Phe Ala Pro
                485                 490                 495

Thr Thr Lys Glu Glu Arg Pro Ala Glu Glu Asn Lys Asp Glu Glu Arg
            500                 505                 510

Gly Leu Gly Asp Gly Ala Val Ser Ser Ser Ile Leu Gly Ala Gln
        515                 520                 525

<210>  10
<211>  514
<212>  PRT
<213>  Penicillium brasilianum

<400>  10

Met Ala Ala Ser Asn Glu His Ala Thr Pro Ser Ile Asp Ser Ala Thr
1               5                   10                  15

Thr Lys Thr Ala Pro Ser Val Asp Ser Thr Pro Ala Met Ser Asp His
            20                  25                  30

Val Asn Glu Asp Leu Glu Lys Gly Thr Arg Thr Thr Asp Pro Val Glu
        35                  40                  45

Ala Glu Lys His Asp Leu Asn Val Asn Pro Leu Ser Glu Lys Ile Glu
    50                  55                  60

Gly Thr Thr Pro Ala Pro Ala Gly Pro Pro Gly Pro Gly Pro Pro Pro
65                  70                  75                  80

Asp Gly Gly Ala Glu Ala Trp Leu Val Val Leu Gly Ala Phe Cys Gly
                85                  90                  95

Leu Phe Val Ser Phe Gly Trp Ile Asn Cys Ile Gly Val Phe Gln Thr
            100                 105                 110

Tyr Tyr Glu Thr His Gln Leu Ser Asn Leu Ser Thr Ser Thr Val Thr
        115                 120                 125

Trp Ile Thr Ser Leu Glu Thr Phe Val Met Phe Phe Ala Gly Pro Val
    130                 135                 140

Phe Gly Thr Leu Phe Asp Ser Tyr Gly Pro Arg Tyr Ile Leu Leu Gly
145                 150                 155                 160

Gly Thr Phe Leu His Val Phe Gly Leu Met Met Thr Ser Leu Ser Thr
                165                 170                 175

Glu Tyr Tyr Gln Phe Ile Leu Ala Gln Gly Ile Cys Ser Pro Leu Gly
            180                 185                 190

Ala Ser Ala Ile Phe Asn Ala Ser Ile Asn Ser Val Ser Thr Trp Phe
        195                 200                 205

Ala Lys Arg Arg Ala Phe Ala Leu Gly Val Thr Ala Ser Gly Ser Ser
    210                 215                 220

Leu Gly Gly Val Ile Phe Pro Ile Met Val Thr Asn Leu Ile Pro Glu
225                 230                 235                 240

Val Gly Phe Pro Trp Ala Met Arg Ile Cys Ala Phe Leu Ile Leu Ala
                245                 250                 255

Met Leu Gly Val Ser Asn Leu Thr Leu Lys Ser Arg Leu Lys His Thr
            260                 265                 270

Arg Lys Pro Phe Asn Phe Met Asn Phe Val Arg Pro Leu Lys Asp Ile
        275                 280                 285

Lys Phe Val Val Thr Val Ala Ala Ala Phe Cys Phe Phe Trp Gly Met
    290                 295                 300

Phe Leu Pro Phe Thr Phe Val Ile Thr Gln Ala Gln Arg Tyr Gly Met
305                 310                 315                 320

Ser Glu His Leu Ser Gln Tyr Leu Ile Pro Ile Leu Asn Ala Ala Ser
                325                 330                 335

Val Phe Gly Arg Thr Leu Pro Gly Tyr Leu Ala Asp Arg Val Gly Arg
            340                 345                 350

Tyr Asn Val Met Ile Phe Phe Ser Tyr Leu Ser Gly Ile Leu Val Leu
        355                 360                 365

Ala Leu Trp Leu Pro Ser Arg Ser Asn Ala Pro Ala Ile Val Phe Ser
    370                 375                 380

Ala Leu Tyr Gly Phe Gly Ser Gly Ala Phe Val Ser Leu Ala Pro Ala
385                 390                 395                 400

Leu Ile Ala Gln Ile Ser Asp Val Arg Glu Val Gly Val Arg Asn Gly
                405                 410                 415

Thr Cys Phe Ser Ile Ile Ala Phe Ala Ala Leu Thr Gly Thr Pro Ile
            420                 425                 430

Gly Gly Ala Leu Val Pro Asp Val Leu Thr Gly Ser Tyr Thr Arg Leu
        435                 440                 445

Gln Val Phe Ser Gly Val Val Met Leu Ala Gly Ala Thr Leu Phe Val
    450                 455                 460

Val Ala Arg Leu Val Val Gly Gly Val Lys Phe Gly Lys Ser Leu Gly
465                 470                 475                 480

Ser Gly Val Leu Met Gly Cys Ile Ser Asn Gly Leu Ile Glu His Asp
                485                 490                 495

Arg Arg Met His Trp His Tyr His Val Val Ile Cys Cys Ala Ala Thr
            500                 505                 510

Asp Ser

<210>  11
<211>  872
<212>  PRT
<213>  Penicillium brasilianum

<400>  11

Met Cys Gln Asp His Asp Leu Glu Cys Ser Tyr Thr Leu Pro Arg Lys
1               5                   10                  15

Thr Arg Phe Tyr Gly Ser Val Asp Asp Leu Ser Asp Arg Tyr Lys Cys
            20                  25                  30

Leu Glu Ala Ile Val Arg Ala Ala Phe Pro Asn Asp Gly Ile Ser Thr
        35                  40                  45

Val Pro Glu Leu Ile Arg Leu Gly Glu Arg Met Gly Tyr Ala Met Pro
    50                  55                  60

Asp Leu Ser Gln Lys Ser Gly Glu Ser Pro Arg Ile Glu Glu Leu Val
65                  70                  75                  80

Arg Asp Phe Pro Thr Glu Ala Gly Asp Gln Gly Leu Ala Gly Ser Thr
                85                  90                  95

Gln Cys Thr Ser Ser Pro Pro Arg Thr Gly Ala Val Asn Val Pro Thr
            100                 105                 110

Glu Ser Glu Arg Arg His Ser Ser Ser Gln Val Gln Glu Asn Asn Ser
        115                 120                 125

Cys Pro Asp Glu Pro Val Gly Leu Ile Arg Asp Thr Thr Gly Arg Glu
    130                 135                 140

His Phe Ile Gly Pro Ser Gly Ser Leu Gln Phe Leu Gly Gln Leu Arg
145                 150                 155                 160

Arg Leu Leu Leu Ile Ser Arg Ser Gly Asp Ala Val Glu Ser Arg Ala
                165                 170                 175

Pro Ala Arg Leu Thr Ala Thr Phe Thr Asp Glu Asp Ala Ala Gln Ala
            180                 185                 190

Leu Glu Ala Asp Gly Asp Gln Ser Glu Leu Ala Ala Leu Pro Ser Gly
        195                 200                 205

Gly Thr Gly Asn Gly Gly Asp Glu Gly Gln Glu Ile Asp Glu Arg Ser
    210                 215                 220

Pro Ala Ser Leu Gly Ser Ala Leu Val Arg Asp Phe Ser Ser Ile Pro
225                 230                 235                 240

Val Asn Asp Ile Asp Glu Met Arg Arg Gln Leu Pro Pro Arg His Val
                245                 250                 255

Leu Asp Ser Leu Met Arg Val Tyr Phe Lys Asn Val His Pro Asp Phe
            260                 265                 270

Ala Leu Phe His Arg Gly Thr Phe Glu Glu Glu Tyr Glu Thr Phe Met
        275                 280                 285

Ser Lys Gly Arg Tyr Tyr His Gln His Ala Arg Ala Gly Val His Leu
    290                 295                 300

Ser Ser Pro Thr Leu Pro Glu Pro Gly Trp Leu Gly Cys Leu His Met
305                 310                 315                 320

Met Ile Ala Phe Ala Ser Leu Asn Gly Ser Val Asp Val Ala Pro Asp
                325                 330                 335

Leu Asp Leu Thr Ser Leu Cys Arg His Cys Ala Ser Leu Thr Arg Gln
            340                 345                 350

Leu Leu Pro Gln Phe Ile Ser Lys Cys Thr Leu Ser Asn Val Arg Ala
        355                 360                 365

Leu Leu Leu Leu Ser Leu Phe Leu His Asn His Asn Glu Arg Asn Ala
    370                 375                 380

Ala Trp Asn Leu Val Gly Thr Ala Met Arg Leu Ser Phe Ala Met Gly

385                 390                 395                 400

Leu His Arg Ala Ser Asp Asn Gly Ser His Phe Arg Pro Ile Glu Arg
                405                 410                 415

Glu Val Arg Lys Arg Val Phe Cys Thr Leu Tyr Gly Phe Glu Gln Phe
            420                 425                 430

Leu Ala Ser Ser Leu Gly Arg Pro Ser Gly Phe Tyr Asp Phe Glu Asp
        435                 440                 445

Val Glu Ile Val Pro Pro Arg Glu Gly Val Leu Asp Ser Gly Gln Asp
    450                 455                 460

Glu Asp Asp Glu Val Met Lys Leu Ser Leu Arg Leu Gln Val Ile Leu
465                 470                 475                 480

Ala Lys Ala Arg Val Ser Leu Ala Val Lys Thr Leu Ala Val Ala Asn
                485                 490                 495

Glu Arg Gly Asn Ile Asp Gly Leu Ala Arg Gln Gln Gln Ser Ser Arg
            500                 505                 510

Glu Thr Leu Glu Ile Leu Lys Ala Trp Arg Glu Asp Leu Ala Ser His
        515                 520                 525

His Ile Leu Asn Ile Pro Leu Ile Ser Glu Thr Asp Asp Pro Leu Cys
    530                 535                 540

Gln Tyr Ala Glu Glu Ile Pro Arg Met Ser Leu Gln Asp Leu Lys Ala
545                 550                 555                 560

Met Met Gly Trp Gln Ser Arg Pro Arg Leu Arg Ala Ala Leu Val Leu
                565                 570                 575

His Leu Gln Tyr Arg Tyr Ile Ala Val Leu Val Thr Arg Ser Ser Leu
            580                 585                 590

Leu Arg Tyr Val Ala Ser Ala Gln Arg Gly Glu Pro Glu His Glu Ala
        595                 600                 605

Leu Leu Ser Arg Asn Glu Ala Arg Thr Asp Pro Tyr Asn Ser Glu Ala
    610                 615                 620

Gly Glu Arg Leu Ser Asp Ile Cys Val Thr His Ala Thr Gln Leu Cys
625                 630                 635                 640

Arg Leu Ile Leu Leu Ala Asp Ser Phe Gly Leu Val Asn Gly Ile Ser
                645                 650                 655

Ala Met Asp Val Phe Tyr Val Tyr Cys Gly Val Met Val Leu Ile Leu
            660                 665                 670

Arg Ser Leu Arg Ile Ser Ser Ser Ala Ser His Tyr His Asp Gln Arg
        675                 680                 685

Glu Ala His Leu Gln Leu Glu Leu Arg Lys Leu Ile Ala Gln Thr Arg
    690                 695                 700

Glu Val Leu Ile Arg Val Asn Lys Cys Ser Thr Met Lys Arg Phe Ala
705                 710                 715                 720

Arg Val Val Ala Thr Phe Glu Asp Gly Ser Arg Gln Asp Asn Ile Arg
                725                 730                 735

Pro Ala Asp Gly Ser Thr Asn Arg Ser Thr Ala Asn Cys Glu Met Arg
            740                 745                 750

Thr Ala Arg Gln Ala Ser Arg Asp Pro Arg Gly Arg Phe Asn His Ser
        755                 760                 765

Ile His Ala Ala Leu Asp Gly Gly Arg Ala Ser Asn Leu Ala Ile Phe
    770                 775                 780

Pro Gly Ala Gly Gly Ser Leu Asp Thr Ser Ser Ser Leu Pro Val Ser
785                 790                 795                 800

Gln Gln Glu Pro Leu Asn Phe Gln His Gly Tyr Gly Asn Gly Ile Gly
                805                 810                 815

Pro Arg Leu Gly Ile Ser Asp Pro Phe Trp Gln Pro Asn Leu Leu Thr
            820                 825                 830

Ser Phe Asp Gly Glu Pro Glu Ala Asn Gly Trp Met Met Asp Pro Phe
        835                 840                 845

Leu Ala Met Asp Gly Thr Gly Val Val Asp Trp Gly Asp Ile Glu Ser
    850                 855                 860

Leu Leu Ser Arg Asn Pro Gly Gln
865                 870

<210>  12
<211>  1056
<212>  DNA

<213>  Penicillium brasilianum

<400>  12
atgggctcgc tatctctacc ggagacgtct cttgcggcaa tccaagataa ggagaccaag      60

gccatctccg tcgccaaacg cccaactcca gtccccgtcg gcactcaagt cctcgtcaag     120

ctacactact ccggtgtctg cgccacagat ctccatctgg cccgcggcag cgtcccctat     180

ctgcagccca aggtctcggt aggcggccat gagggcacgg gggtaatcgc cagcctcggc     240

cccgacgtgg acgccgcgga gtggcacgtc ggcgaccggg tcgcggtgcg gtgggtgcac     300

atcgtctgcg ggaagtgcga ggtgtgcact accggctttg agaacctgtg ccaaagccgg     360

aagctcgctg gcaaggacgt tgagggcacc tttgccgagt acgctattgc ggacagctcg     420

tacatggtcc gcctgccggc tggggtgagc gacgccgatg ccgctccgat actgtgcgcc     480

ggtgtcaccg tctacaaggc gctcaagatc gccagcctcc gggcgggctc gtgggtcgcc     540

gtggccgggg ctggtggcgg cttgggtcat ctggctattc aatatgcacg tgccatgggg     600

ctcaaggtcg tggccctgga tgctaggaaa cgggatctct gcctgagcct gggcgcggaa     660

tcgtacatcg acgtgctcga aacggacgac tgcgtagccc aggtgatcaa ggtcacggat     720

gggggcgctc atggcgcact catctgcgcc tcgtccggcc aggcctatga cgatgccgtc     780

aaattcctaa gatggaccgg caccctggtg tgtatcggcc tcccccccaa gccgacactg     840

ctctccctag gccccgcgga cttcgtcgcc cgtggcatca aggtcatggg cacatccaca     900

ggtgaccgcc aggacacggt ggaggccctc gcctttgtcg ccaagggcca ggtaaaaccg     960

cagttaactg agagaaggct ggaggatgtt gaggagatct taaaggagat cgagaatggg    1020

actatgcagg gtaaagctgt gatcaggata gcctga                              1056


<210>  13
<211>  1020
<212>  DNA
<213>  Penicillium brasilianum

<400>  13
atgagtctgc cgtctcacta caaacgggcc gcctttaaag aggctggcgg cccgctcacc      60

attgaagaag tcgacttgac catgccggat gctggcgaag tgctggtgaa ggtggaagct     120

tgcggagttt gtttctccga cacggttcca caggctcatg gcctgggggg gaaatttcca     180

atcgtacccg gtcatgaaat catcggacat gtggttgcga caggtgacgg tgtctcggac     240

tgggaggttg gcgatcgtat cggcgaagga tggcacggcg gccatgacgg aacttgtccc     300

tcctgtcgcc agggccattt ccaaatgtgc gacaatcaga gcatcaatgg ggtgaccaaa     360

aacggcggat atgcccaata ctgcattctc cgtagtgaag ctgctgtcag aattcccact     420

catgtctcag ccgccgagta tgccccaatt ctctgcgcgg gcgtgaccgt cttcaactca     480

atgcgtcaaa taggggtcaa acctggctct accgttgcca ttcagggtct gggaggtctc     540

ggccatcttg ccatccaata tgcgaatcgc ttcggcttcc gagtggtggc catctctcgc 600

gatgatcaaa aggagcgatt tgtgcgagac ctcggtgccc acgagtatat caatacaagc 660

gaagaagatg tcggaagcgc gctccagaaa ctgggaggtg cttctctgat tgtagccacc 720

gcgcccaatg cccgggccat ttcccctcta ttgaagggcc ttagacctct gggtaagctt 780

cttattctcg ccgttccggg tgaaatcccc ctcgatacac ggcttatggt cgcccgaggt 840

ctttctgtcc acggatggcc gagtgggcat gcacttgatt ctgaggaaac cattcgcttt 900

acagaattag aggatatcaa gtgtatgatc cagacatatt ctcttgatcg tgccaatgaa 960

gcattcgatg ccatgatttc ggggtcggtt cgattccgtg cggtgattac catggagtaa 1020

<210> 14
<211> 1518
<212> DNA
<213> Penicillium brasilianum

<400> 14
atgactcaaa ccaacgtcca tgtcaacaag tctgatactt cactggcagc tccgcaacag 60

ttattcatct cgggaaaata ccagaacagc cagaggaatg ggaccttccc cgtgaagaac 120

cccatgacgg gcgagaccat atacgagtgc gtctcagcct ctctagacga ctacgccgcc 180

gccatcgagg aagcagatgc agcccaacct tcgtgggctc gcctcggccc ctcagcccgg 240

cggttgatcc ttctcaaggc cgccgacatc atggagacgt acatcgagac agacgcgccg 300

gccatccttt cagccgaggt ttcggccact aggggatggg tcagggccaa cattctgtcc 360

acagccggcg tcttccgcga gaccgccgcc ctggcaacgc atatcaaggg agagatcgtt 420

cccgcggacc ggccgggcac cacgatcctg gtgagccgcg agcccgtcgg cgtggtcctg 480

gccatcagcc cctggaatat gcccgccaca ctgacggcca gggcgatctg ctgcccgctg 540

atctgcggga atagcgtggt cctgaggccg tctgagttca gccccaagtc gcagcacctc 600

gtcgtgcgcg ctctgaccga ggctggactg ccggcggggt gcctgcagtt cctgcccacc 660

agcaccgccg acacgccgcg agcgatcgag tttgcaatcc gacacccgaa ggtgagccgc 720

gcaaacttca cgggcagcga ccgcgttggc cgcatcatcg ccgggctatc ggcatcctgt 780

ctgaagccgt gcgtgctgga gctaggcggc aaggcgcccg tcgtggtcct ggaggatgcc 840

gatgtggagg ccgccgtgga ggcagtggtg tatggcgcga tgtccaacag cgggcagata 900

tgtatgtcca cagagcgggc gatcgtgcat cgctccctgg cagcggactt caaggccctg 960

ttggtgaaac gggcggagag cctgcgggta gggaaccacc tcgaggaccc ggacgtgcag 1020

ctctcgggcc ttttcactgc cgcctccgcc gagcgtgtac tcggcctgat caagggcgcc 1080

gtcaacgcag gtgccacgct cctggcgggc gatctggctc ttcacggacc gtgccagaca 1140

atcatggctc cccacatcct cacgggcgtc acgcgggata tggacctctt ccatcgggag 1200

acgttcggcc ccgtgctctt cgtgtccgag ttcgacacgg acgatgaggc catcgcgcag 1260

gccaacgaca ccgagttctc tctgtgtgcc agcgtcttct cgcgtgacgt cctgcgcgcc 1320

atggacacgg ccaagcggat ccggacaggg agctgccacg tcaatgggcc gaccgtgtac 1380

atcgaggcgc cgctgcccaa cgggggtgtc ggcggcggga gcggctacgg ccggttcggt 1440

ggtgtggcgg gcatcgagga gtttacggag aggcagatcg tgagcttagc gaagccgggg 1500

attaagtatg cgttctag 1518

<210> 15
<211> 1284
<212> DNA
<213> Penicillium brasilianum

<400> 15
atgcctcacg catccagatc attgaacgtt ctcatcgtcg gtgctggctt gggcggcctc 60

gcggcgggat tagctttgca gacggacggc cacaaggtca caatcattga tgccgctcct 120

gagttcgcag aggccggagc ggggatcagg atcccaccca actcgagccg gctgctcatg 180

cgatggggcg ttgatctgga gaggatgaag aagtcgactt cacagagata tcacttcatc 240

cgctggaagg atggcagcac catcttcgac ttgcccttca acaatatcgt cgagacacac 300

ggggcgcctt actggcttgt ccatagagcc gacttgcacg ccgctctgct cgatgcgacc 360

ctgaaggccg gtgtcaaggt tctaaacaac aagcttgtca cgtcctacga cttcgaggca 420

ccgagcgcca ccacacagga tggcgagacc ttcaaggccg atcttatcgt cggtgcggac 480

ggcataaagt ccatctgccg accccttctt accggtcagc cggacgtccc gcgggacacg 540

ggcgacgtcg cctatcggat tctcatccct ggtgagaaac tgctggccga cccggacctg 600

gcccatctga tccgcgaccc ttgcacgaca tcgtggtgtg gcccggacgc gcacctggtc 660

ggctacccga tccgcaacgg cgagatgtac aacatcgtca tgtgtgccac ctcctacaac 720

gagaccacgg acgaggtctg ggtcgtcaag ggcgacaaca gcgagctgtg caagcgcttc 780

gccagctggg aaccccaggt gcggaagctc tgcgccctca cgggcgactt catgaagtgg 840

cgcctgtgcg acctgcccaa cctcgcccgc tggacgcacc cctcgggcaa ggccgtgctg 900

ctgggcgaca gctgccaccc catgctgcct tacctggccc agggcgctgc ccaggccgtc 960

gaggatgccg ccgtcctgcg ccaggtgctc gcccaggaca tggacatggc cgcggcccta 1020

aagcagtatg agcagatccg catgccgcgg gcgagcctgg tgcaggccaa gactcgcgag 1080

caccagtaca tcctccacgt cgacgacggt cacgagcagc aagaccggga caagaagttg 1140

gctctcgatg cggcggagaa cccggtcttc tggggctacg acgaccggag aaaatggctc 1200

tttagccatg atgcagaggt gatccaaaaa gaaggtgcga actggagaga cgggcccaac 1260

atgaatggcg tgcatgttgc ttag 1284

<210> 16
<211> 738
<212> DNA
<213> Penicillium brasilianum

<400> 16
atgtctctgt ctggcaaagt cgtcctcatc accggctcct ccaagggaat cggcaaagct 60

gctgcccttc gtgtcgccag cgaaggtgcc aacgtcgtga tcaactatct tcgtgacccg 120

gttgcagcca acaatctcgt cgaccaaatc ggtgccgacc gcgcccttgc tgttcaagct 180

gatgcttcaa agctggccga cctcgatcgc ctcgtcaatg ccgctgtcgc ccagttcggc 240

aagatagatg ttcttattcc aaatgccgga atcctcccgc ttagagactt ggagcatact 300

agcgaagagg actttgacag aacctacaat ctaatggtaa agggaccata cttcctggcc 360

cagaaagccg tgaagcatat gccccctgga ggacgaatca tctttgtctc tacctcaacc 420

gcccgattcg caagcgtggc tcccgcatac ctactctaca cctcctccaa aggcgcgatc 480

gagcagatga cccgaatcat ggccaaggat ttggcgcgaa agggaatctt ggtgaatgcg 540

gtcgctcccg gtccaacttc aaccgaactc ttcctcgagg gaaagccgga gcaaatgatc 600

aaggctatct ctgggtttag tccattcaat cggatcggag agcccgagga aattgccgca 660

gtcatggcgt tcttgtctgg gaaggacagc agttggatct caggacaggt tgtggctgtg 720

aacggagcta tggcatga 738

<210> 17
<211> 1746
<212> DNA
<213> Penicillium brasilianum

<400> 17
atggcatctc tcattcgaga ggcccccttt gggcaaatag ttcgatatct caccaacaac 60

aaatatttcc aatatcccga agaaaagccg gacttcaaac ttcccgatac atggcttcaa 120

ttattgaacg aatcggatgc cgcgacgata gccgatcccg agaaaacgga gccggagcca 180

gagggtcagg gctacgatgc aaccagcgag gcgattagtc gcgcatcaac ccaaaactcc 240

ttgcccttca cagaagctcg actggaagcg gatgagcagc atgagatcga aaagatcaag 300

tccattccta ttcagcctaa aaagaccaag gatggtgcga ttttggtcga ctggtactat 360

accgatgatg cagagaaccc acacaattgg tcgaatcgaa agcgagcact tctgacgacg 420

ctcatttgtc tttatacctt tgtggtatac acgacctctg caatttacac atcctccgtg 480

cccgggatca tgaaggagtt cggcgtcagt gacttggttg ctacactggg actgtccttg 540

tatgtccttg gctatggaac gggtcccctg atcttctcgc ctctgagtga gatccccgtg 600

attggtcgga acccggtcta tatcgtgacc atgttcctct ttgtgattct ctccattccc 660

actgcttttg tgggtaactt tgcaggactc atggtgcttc gtttcctgca aggattcttc 720

ggctcgccct gtctcgcttc tggaggtgct tcaattggcg acatgtacag tctcatgtct 780

ctcccttatg ccatgatgag ctgggtgtct gccgcttact gtggtcccgc cctgggtcct 840

ctcatcagtg gcttcgcagt tcccgccgag acctggcgct ggtccctgtt tgaatccatt 900

tggatgtcag ctccagtcct cattctgatg ttcttcttcc tccccgagac tagcagtgca 960

actatcctgc tccgtcgtgc cgctcgtctc cgcaagatcc ataacaacgc acgcttcatg 1020

gctcagtccg agattgacca gcgtaacatg aaggtctcgg ccgttgctgt cgacgccctg 1080

attaagcctt tggagattac catcaaggat cccgcggtgc tcttcgtcca ggtctacacc 1140

gccattatct acggcatcta ctactccttc ttcgaggtct tccccctggt ctaccccgtc 1200

gattatggca tgaatcttgg ccaagttggc ctggtcttcc tgtgtatcct ggtgtcctgc 1260

atcatcggta tcgccatcta ctggtcttac ctttacttct ggatgaaccc tcgcattgaa 1320

cgcttcggat tcccagctca agagtcccgt cttatccccg ccctgccagc ttctattgga 1380

cccaccattg gcttgttcct ctttgcctgg acagcccgtg cctcaatcca ctggattgcc 1440

ccgacaatcg gaatcaccat ctacggtgcg acggtcttca tcgtgatgca gtgcttgttt 1500

gtctacatcc ccttgagcta tccaatgtac gccgctagtc tgttcgctgc gaacgacttc 1560

ttccgcagtg ctctggcttg cggtagtgtc ctgtttgctc acccgttgtt tggcaacctc 1620

ggtgtcgctc ggggtaccag tctactcggt ggtctgagtg tgattggtat tatcggaata 1680

tggctgcttt actactatgg tgcccggctt cgctctttga gcaagtttgc catctccgat 1740

gattga 1746

<210> 18
<211> 1542
<212> DNA
<213> Penicillium brasilianum

<400> 18
atgagcacga ccaaggaagc tttcccacat acggatagcg acataatgga ggactcggag 60

aagaatctcc cagagtgcga gcatatcgtc tccgtggagc ccaccctcaa gatgcgcgac 120

ggcatcgtcc tgatgccgca accgtccgac gaccccaacg acccgctcaa ctggtcctgg 180

ttccgcaagc acgccgccat gttcaccctc tcgtatttgg ccctcgtctg ttacgtggct 240

gtgaccacgc tggttacggg aacagtgccc ctagccaagt ccatgcatgt ctccaagtcg 300

acggccgtct atctgggcaa cacgcccgtc gctctctacg ccgtggcgcc ctggttctgg 360

agcccgctga gccactttat aggccgtcgc ccggtgctgc tgatgtgtaa tatcatcgcc 420

gtcgtcgggg cggtcgttgt tacgacgtcc aagacatatg cgtcttgcat ggttggccgc 480

gtcatcctcg gtgccggtgg ttcggccttc tggacactgg ggccagccag tattggggac 540

attttcttcc gccacgagaa gggcaagaag attggtgtgt cgaccttggc cattgtgatc 600

gccccattct tggggacaat catcggcgga cccatcatag aaaacgagaa gctgggctgg 660

ccggcctccc agtggatccc cctgattttc atggccgccg gcttcatcat gcagatcttc 720

ttcctcccgg agaccatcta catccgagag acacgcgcgc atcctgcaat catgtccaca 780

tctacgccgg gcaagcccac gttctgggac cgctatggga tccacatccc caagcgctcg 840

gaggagaagc agcacagctt tctcttcatc gcgacgcgcc ctttcgtcct cttcaagttc 900

cccgcggtga tactgtcggc cttctggttc ggcatcgcct acatgatgca cgtgggcatc 960

acgtccgaga tcccgctcat cttcgaggag cactacgatt tctccgtgct cgagatcggg 1020

ctgtcgggct tctcgggact catcggcgcc ctgctcggcg aggtatacgc gggaccctcg 1080

ctggatttca tcgctaagcg gaccatgaag cagggtcgcg agtggcgccc cgagtaccgc 1140

ctgcaggcga tctggccggc gcttattacc gtgccggctg gtctgatcat gttcggcaca 1200

tcgatccagt tcggaaatgt ttggatcact cctctgatcg ggcaggccgt ttatatcttc 1260

ggcatcgaga ttgccaccac ggttattcaa acctacattt tagaatgtta ccctcgccag 1320

ggcgccgagg cgaacttggt cttcaacctc atccgcaacc tgttctccta tataagtccg 1380

ttctttgtgc agccaatgat cgccaccctc ggtaccacct ctccgtttgg tctctctgct 1440

gccttgactg ccttcttctt ccctttcact gtgggtgtct tgatgtggcg gggcaaacag 1500

atccgggata aaggaggcga cccgggctgg agcagggatt aa 1542

<210> 19
<211> 4308
<212> DNA
<213> Penicillium brasilianum

<400> 19
atggaggacc acgaaaaaga atacgatagt acctcacctc ccggaacggc cacggaagaa 60

gggaatggag gttacttcaa taccctcacc gttcctgaaa tcaacctccg agaagccagc 120

agtgccgaaa ccttaactcc tcacgcctcc gtcgtccaac ctcccaaaaa ggccgcagaa 180

tggcatatga caccacaagt cattcgcaat gccgaacgtg atgaagcagc tggttttaaa 240

aggcgtgagc tgggtgtcac ttggcaagac ctgtcggtcg aggttctcgc tgctgaagcg 300

gctgtcaaag agaacatgat ctcccaattc aacgttcctc aactcatcaa ggatttccgc 360

cgcaaaccac cactcaagtc gatcttgtcc aacagccatg gatgtgttaa gcctggagaa 420

atgcttctcg ttcttggaag acccggatcc ggatgcacta cccttctcaa aatgcttgcc 480

aaccgtcgtg agggatatca gaacatcacc ggagatgtaa gattcggaaa tatgactccg 540

gaagaagcat caagatatca aggccagatt gtgatgaata ccgaggagga gcttttctac 600

cctcgtttga cggtgggtca gacaatggat tttgctacca agcttaaggt cccatatcac 660

cttcctggag aagggaagag cgttgctgag tataccgccg aaacgaagca attcctcctc 720

gagtctatgg gaatcgccca tactgccgat acaaaagttg gcaatgaatt cgtccgaggt 780

gtcagtggtg gagaaagaaa gcgagtgtct attattgagt gccttgccac aagaggctct 840

gtttttactt gggataactc aacgagagga ctcgatgctt ccacggcctt agagtgggcc 900

aaagcccttc gcgccatgac cgacgtccaa ggtctttcga ctattgtaac gctctatcaa 960

gctggaaatg gaatttataa tcttttcgac aaagttctcg tcctcgacga aggaaagcag 1020

atctattacg gccctgccgc ggaagcgaaa cctttcatgg agaaccttgg ctttgtttac 1080

actgatggtg ccaacattgg tgatttcctc acggggttga ctgttccgac cgagcgaaag 1140

atcagacctg gttgggaaaa tcggttcccc aggacggccg acgccatttt gaccgagtac 1200

cagaactcgg cgacatataa gaatgaagtc tcactatacg gatatcccga cactgacctt 1260

gctgccgaac gcactgaggc cttcaaggaa tctgtggcct gggaaaagtc taaacactta 1320

cccaagggta gtgacctgac tactagtttc tgggcccagc tcatgtcatg cacggctaga 1380

cagtaccaga tcctctgggg cgagaagagc acgttcctga tcaaacagat tctgtcttgt 1440

gtcatggcct tgattgccgg gtcttgcttc tacaactctc cagatacctc tgcaggtctc 1500

ttcaccaagg gtggtgccgt tttcttctcg ttgctttaca actgcattgt ggccatgtcc 1560

gaggtcaccg aatctttcaa aggtcgtcct attttgacga aacacaaatc ttttgccatg 1620

tatcacccgg ctgctttctg tctggcccaa attactgcgg atttcccggt gttactgttc 1680

caatgcacga tcttctcggt cgttatctat tggatggttg gattgaagca taccgcggct 1740

gcatttttca ctttctgggc aatccttttc actacgacct tgtgcatcac agcgttattc 1800

agatttatcg gcgctgcttt cagtagcttc gaagctgcat ccaagatcag tggcaccgct 1860

gttaaggcaa tcgtcatgta tgcaggttac atgattccaa agccagagat caagaactgg 1920

ttcctcgagt tctactacac caatccgttt gcttatgcat tccaagctgc tttgaccaac 1980

gaattccacg accagcatat cgactgcgtt ggcggtaatc ttattcccag tggtcctgga 2040

tacgaggatg ttggatccgg ctataaagca tgtgctggag ttggtggtgc tcttcctggt 2100

gcagattatg tgactggaga tcagtatctt tcttctctac actacaagca ttctcaattg 2160

tggcgaaact tcggcgttgt ctgggcctgg tggggcttct tcgctgttct cacggtcgtc 2220

ttcacttgct tctggaaatc tggtgctgca tctggatctt cgcttctcat tccccgcgag 2280

aaccttaaga aacaccaagt tggcaatgat gaggaagccc aaaacaatga gaaacatgcc 2340

gctcggacga ccaccgatga gccagttcaa gtcgaggatg acaatcttgt gcgcaacaca 2400

tctatcttca catggaagaa tctcacctat acagtcaaaa caccaactgg cgaccgagtc 2460

ctcctggaca acatcaatgg atgggtgaaa cctggtatgc ttggtgcact catgggatct 2520

tccggagccg gcaaaacaac tctacttgat gttcttgcac agcgcaagac ggaaggtacc 2580

atcaaaggct ccattttggt tgacggtcgc gaattacctg tctctttcca aagaatggcc 2640

ggctactgtg agcaattgga tgttcatgag tcttacgcca ctgtgagaga agccctggag 2700

ttttctgctc tcctgcgaca gtctcgagat actcctaaag ctgagaaact taaatacgtc 2760

gacacaatta ttgacctctt ggagctgcat gatctcgccg acactctgat cggttccgtg 2820

ggtaatggct tgtctgttga acagcgcaaa cgtgtgacaa tcggtgtgga gctcgtgtcg 2880

aagcctagta ttctcatctt cttggatgag cccacttcag gtcttgatgg tcaatctgcg 2940

tataacactg tcagatttct tcggaaacta gccgatgtgg gccaagcagt tcttgtcacg 3000

attcaccagc cttcagctca gctattcgct caattcgata cccttctact ccttgcgaga 3060

ggtggcaaaa cggtctactt tggtgacatc ggggacaatg gatccacgat taagcagtac 3120

tttggcaatt acggggctat ctgccctcaa gaggcaaatc cagcagagtt catgattgac 3180

gtcgttaccg gtggtatcca agaagtgaag gacaaggatt ggcatcagat ctggcttgac 3240

tctcccgagc agcatcagat gatcaccgaa ttggacagaa tgattgcaga tgccgctagt 3300

aagccaccgg gaactgtcaa tgatggctat gagttttcaa tgcctctctg ggaacagatc 3360

aagattgtca ctcagcgcat gaacgtctcg cttttccgta atacggccta tgtcaacaac 3420

aagttctcgc ttcacatcat ctcagcattg ttgaacggat tctctttctg gcgacctggt 3480

cctagtgtga gtgcgttgca gctgaagatg ttcaccatct tcaattttgt tttcgtcgct 3540

ccaggtgtca tcaatcaact ccaacccctc ttcatccagc gccgcgatat ctacgatgct 3600

cgcgaaaaga agtccaagat gtattcctgg gtagctttcg tcactgggct tatcgtttcg 3660

gagttcccat atctctgcat ctgcgcagtt ctatactttg tttgctggta ctggcccgtc 3720

tggagattgc ctcatgactc tgaccgttct ggagccatct tcttcatgat gttgatctac 3780

gagttcatct acactggtat tggccagttc attgctgcgt atgcaccgaa cccaaccttt 3840

gcggcacttg tcaacccact tatcatcagt gttcttgttc tcttctgcgg tgtgtttgtg 3900

ccatatgacc agctgaacgt attctggaag tactggatgt attacctcaa cccattcaac 3960

tatgtcgtca acggcatgtt gactttcggt ctctggggcc agaaagtcac ctgcaatgag 4020

agcgagtatg cagtctttga tccgctcaat ggtacttgcg gcgagtatct ggcgacttac 4080

atgagtggca agggcagcgg agtcaatctg ctcaacccgg acgctacctc gagctgcaag 4140

gtctgtgagt acacaactgg aagtgacttc ctacagacgc tcaacatcaa ccattactat 4200

tatggatgga gagatgcggg catcactgtt atctatgcta tctcgggcta tgcacttgtg 4260

tttggtctga tgaagctccg gaccaaggcg tccaagaagg cagagtaa 4308

<210> 20
<211> 1584
<212> DNA

<213>  Penicillium brasilianum

<400>  20
atgacaccta tctcgcgcct attgtcccgt gcggtcaata aaccctaccg aacgaaagat      60

atcacggacg agattccgcc tacactcgat gaagatggat ttgtgagctt tggcccgggg     120

gacattgaga accctcggaa ttggtcgatg cgccgccgag ctggtgtgac aatgtctgcg     180

gtgcttttag ttgtgaatgc cacttttgcc tcgagctcgc cgagcggatg tttcccatca     240

atctcaaagc atttcggtgt ttccactgaa gttgccggat tgacgataac tctgttcctg     300

ctagggtact gtgctgggcc tttgattttc gctccgctga gcgagttcta cggtcgccga     360

tggatcttct acattacctt tctgctttat ctggccttca actttctttg tgcattcccg     420

ccgaattttg gaagcttgtt ggttggaaga tttctgacgg ggacctttgt gtctgctcca     480

ttgagtaact gccccggtgt tttggccgat gtatggaatc cgcttgagcg cgccaatgct     540

atggctgggt tctcggcaat ggtctggatt gggccagcac tcggacctgt ggttgcaggt     600

ttcctgcagt tgaaagaaga ttggagatgg agcttctatg tgcttctctg gctcggtgga     660

gcatcagccg tcatcatgtt gacaataccc gagacttacg cgccgattgt tctgtataac     720

aaagcacgac ggatccgcga ggcacagatt cccggatatg aaaacgtgaa agcccctgtc     780

gaggacggtg accggactct cgtgggcatc tataaggttg ctctgactcg gccgtggatc     840

attctgttcg acccgatctc cttgctgtgt gcaatttaca tggcgttcgt ctatacattg     900

ctctacatgt tgtttaccat ataccccatt gtgtttcagg aaaaacgggg ctggaactct     960

ggtgttgggg agctgccgtt gctgggaacc gtcgtaggtg ccctatttgg tggtgttatc    1020

gttgtagcag acacccggat gcgccagaaa aggattgaca acggtacgac aaagatggaa    1080

gatgctgtgc cggaagaccg tctcccgcta gcgatgggtg gaggcatcgg ctttgccgtg    1140

acgatgttct ggttcgcctg gtcggctgag ttcaactctg tccattggat tgtcccaact    1200

ctggcaggag tgtttctctc cagtgccctg ctactcatct tcgttgggtt cctcaactat    1260

ctcgtggatg tttatcaaat gtacgctgca tccgccattg ctgcgaacac gatcgctcgt    1320

tctgcctgtg gagcggcagc accactattc acatcccaaa tgttcgcggc cttgggcgtg    1380

ggaggaggag gcagcttgat cgcgggtgtg gccaccctcc ttgccgctat tcctttcttg    1440

ttctataagt atgggaagca gattagaatg cgcagcaaat ttgccccgac taccaaagaa    1500

gaacggcccg ccgaagagaa taaagatgaa gagcgtggac tgggagacgg tgctgtcagc    1560

agtagtatct tgggggcgca ataa                                           1584

<210>  21
<211>  1545
<212>  DNA
<213>  Penicillium brasilianum

<400> 21
atggctgcca gtaatgagca tgcgactccc tccatcgaca gtgctaccac aaaaaccgct 60

ccgtcagtgg attcaacgcc ggccatgagc gaccatgtga acgaggacct tgagaagggg 120

actcgtacga ccgatcccgt cgaggcagag aagcacgatc tcaatgtaaa cccgctatca 180

gagaaaattg agggcaccac tccagctccc gctggtcctc cggggcctgg ccctccaccg 240

gatggaggag ctgaagcatg gttggtcgtg ctaggagcct tttgcggact gttcgtcagt 300

tttggctgga tcaactgtat cggtgtcttc caaacgtact acgaaaccca ccagctcagt 360

aatttgtcca ccagtacggt gacatggatc acatcattgg agactttcgt catgttcttc 420

gctggtcccg tctttggtac tctctttgat agctatggcc cccgatacat cctcctgggt 480

ggcacattcc tccacgtctt cggcctcatg atgacctccc tctcaacgga gtactaccaa 540

ttcatcctcg cgcaaggaat ctgcagtcca ctcggtgcga gtgccatatt caacgccagc 600

attaactctg tcagtacctg gttcgccaaa cgccgcgcct tcgcactggg cgtcacagct 660

tccggatcga gtctgggagg agtgatcttc cccatcatgg tgacaaatct gattccagag 720

gttgggttcc cttgggcaat gcgcatttgc gcgttcttga tcctagctat gctgggcgtt 780

tccaatctca ccctcaagtc gaggctgaag catacaagga agccgtttaa ttttatgaat 840

ttcgttcggc cgctgaagga tatcaaattc gtcgtcacgg ttgctgcggc cttttgtttc 900

ttctggggta tgttcttgcc ttttacgttt gttatcactc aggcgcagcg atatggcatg 960

tcggagcatt tgtcgcagta tctcattccg atcttgaatg ctgctagtgt ctttggccgt 1020

acactcccag gttacctggc agaccgcgtt ggtcgatata acgtgatgat cttcttctct 1080

tatctctccg gcatcctcgt cctagccctc tggcttccat cacgcagcaa cgcccccgcc 1140

atcgtcttta gcgcattgta cggctttggc tccggtgcct tcgtgtccct cgccccagcc 1200

cttatcgccc aaatctctga tgttcgtgag gttggtgtgc gcaacggcac ctgcttctcg 1260

atcattgctt tcgccgctct taccggtacc cctattggcg gtgccttggt tcctgatgtt 1320

ctgactggct cgtacacgag gctgcaggtc ttcagtggcg tggtcatgct tgcaggtgcg 1380

acactgtttg tggtcgctcg gcttgttgtt ggtggcgtga agtttggcaa gtccctgggc 1440

tctggtgttt taatggggtg catttcaaac ggtttaattg agcatgatag acggatgcat 1500

tggcattatc acgtagtgat ttgttgtgct gccactgaca gctaa 1545

<210> 22
<211> 2619
<212> DNA
<213> Penicillium brasilianum

<400> 22
atgtgccagg accacgacct ggaatgttct tatacacttc cgcgaaagac caggttctac 60

ggcagcgtgg atgatctgag tgatcgatat aaatgcttag aggccatagt acgtgctgca 120

tttcccaatg acggcatctc caccgtcccg gagctcatcc ggctgggaga gcgcatggga 180

tatgccatgc cggacctatc tcagaaatca ggggagtcac ctaggataga ggagctggtg 240

agagacttcc ccaccgaagc tggagaccag ggccttgctg ggtccacaca gtgtacctct 300

tcgccaccaa gaacgggtgc cgtgaatgtc cctactgaga gtgaaagaag gcactcttcc 360

agccaagtcc aggaaaacaa ctcctgtcca gatgaacctg tcggactaat tagagacacc 420

actgggcgag aacatttcat cggcccgtct ggaagcctgc agtttctggg ccagctccgg 480

aggctacttc ttatatcccg cagcggggat gccgtggagt cccgggcacc cgctcgcctt 540

acggccacgt tcaccgatga agatgcagcg caagccctgg aagcagacgg tgaccagagt 600

gagctcgccg ctttgccttc tggaggcact ggcaacggtg gcgatgaagg ccaggagatt 660

gacgagcgct cccctgcctc cttgggttct gctcttgtca gggatttttc cagcattccc 720

gtcaatgaca tcgacgagat gagaaggcag cttccgcctc gccacgttct cgattctctg 780

atgcgagtat atttcaagaa tgtccatccg gactttgccc tattccaccg cggtaccttc 840

gaggaagaat acgagacctt catgtccaaa gggcggtact atcatcagca cgctcgagct 900

ggtgtacatt tatcctctcc cacgttacct gaaccaggct ggttaggctg tttgcacatg 960

atgattgcct ttgcctcgct aaatggctct gtcgatgtcg ctcccgacct ggatcttact 1020

tccctctgtc gccattgtgc cagtcttacc cgccagctcc tgcctcaatt tatctcaaag 1080

tgcaccctct ccaacgtccg ggccctccta cttctatccc ttttccttca taaccacaat 1140

gaacgtaacg cggcttggaa cctggttggg actgccatgc gcctttcctt cgccatgggg 1200

ctgcaccggg ccagcgacaa cgggtcgcac ttccggccta tagaaaggga ggtgcgcaag 1260

cgcgtcttct gcacgctcta cggctttgag caattcctcg cgtccagcct ggggagacca 1320

agcgggttct acgacttcga ggatgtggag atagttcctc cgcgtgaggg agtgctggac 1380

agtgggcagg acgaggacga cgaggtcatg aaactttcgc tgaggctgca agttatcctg 1440

gccaaggcca gggtctccct tgccgtcaag acactggccg tggccaatga gaggggcaac 1500

atcgacggtc tggctcggca acagcagtct tcaagggaga cgctggagat cttgaaggcg 1560

tggagggagg atcttgcctc ccaccacatc ttaaacatcc cgttgatcag tgagacagat 1620

gatccgcttt gccagtacgc cgaggagata ccgcgaatgt cactccaaga tctcaaggcc 1680

atgatgggtt ggcaaagccg acctcgactt cgggctgccc tggtcctgca cctccaatac 1740

aggtacattg ctgtcttggt gacgcggtct tctttgctgc gatacgtcgc gtccgcgcaa 1800

cgtggtgaac cagagcacga agccctgctc agccggaacg aggctagaac cgacccgtat 1860

aacagcgagg caggagaacg gctatcggac atctgcgtca cgcacgccac gcagctatgc 1920

cggctcatct tgcttgcaga ctcctttggc ttggtgaatg gcatatcggc catggacgtc 1980

ttctacgtct actgcggcgt catggtgctc atattgcggt cgctacggat ttctagctct 2040

gcaagccact accatgacca gcgcgaggcg catctgcagc tggagttgcg caagctgatc 2100

gcacaaacga gagaagtgct catacgtgtg aacaaatgca gcacaatgaa gcgattcgcg 2160

cgcgtggtgg ctaccttcga ggatggatca aggcaggata acatcaggcc tgccgatggt 2220

tctaccaatc ggtcaacggc caactgtgag atgcgaacgg ctcggcaggc atctcgtgac 2280

cctcggggcc ggttcaacca ttctatccac gccgccttag atggcgggcg agcgagcaac 2340

ctggcaatct ttccgggtgc aggaggatcg ctggacacgt cctcctccct gccagtctcg 2400

cagcaagagc cgcttaactt ccagcacgga tatggtaatg gaattgggcc gaggctcggt 2460

atatcagatc ctttctggca accaaactta ttgacgtcct ttgacgggga gccagaggca 2520

aacggttgga tgatggaccc attcctcgcg atggacggga ccggcgttgt ggactggggc 2580

gatattgagt ccctcttgtc acggaatcct ggtcaatag 2619

<210> 23
<211> 458
<212> PRT
<213> Penicillium brasilianum

<400> 23

Met Ala Asn Gln Arg Val His Val Gly Ile Val Gly Ala Gly Ile Gly
1               5                   10                  15

Gly Leu Ala Ala Ala Ile Ala Phe Arg Arg Ala Gly Val Asp Val Thr
            20                  25                  30

Val Leu Glu Ala Ala Asp Glu Leu Leu Glu Met Thr Pro Asn Val Ser
        35                  40                  45

Val Leu Leu Gln Lys Trp Gly Val Ala Asp Val Ile Gly Asp Asn Leu
    50                  55                  60

Val Gln Ile Glu Glu Leu Asn Met Arg Arg Lys Asp Gly Thr Leu Val
65                  70                  75                  80

Gly His Thr Glu Ile Pro Ile Val Glu Lys Ala Leu Gly Arg Pro Trp
                85                  90                  95

Trp Leu Val His Arg Ala His Leu His Glu Gly Leu Ala Thr Val Ala
            100                 105                 110

Lys Arg Leu Gly Ala Val Ile His Ile Asn Ser Arg Val Ile Ser Leu
        115                 120                 125

Asp Tyr Gln Glu Gly Gly Glu Val Thr Val Glu Thr Gln Lys Gly Asp

130                 135                 140

Lys Tyr His Phe Asp Leu Cys Val Gly Ala Asp Gly Val Asn Ser Ile
145                 150                 155                 160

Val Arg Ser Thr Leu Phe Pro Gly Ile Lys Pro Asp Ala Lys Thr Thr
                165                 170                 175

Asn Cys Ala Tyr Arg Ala Ile Ile Pro Tyr Glu Arg Ile Arg Gln Asp
            180                 185                 190

Pro Ile Ala Lys Glu Leu Ile Gln Lys Leu Thr Met Glu Val Trp Met
        195                 200                 205

Gly His Asn Ala Tyr Ile Ile Thr Tyr Pro Ile Ser Gly Gly Glu Thr
    210                 215                 220

Phe Asn Leu Val Leu Ser His His Arg Pro Glu Lys Leu Tyr Ser Thr
225                 230                 235                 240

Gln Pro Asn Val Pro Val Glu Glu Met Arg Asp Ala Tyr Lys Asp Phe
                245                 250                 255

Asp Pro Arg Ile Lys Arg Ile Val Asp Met Ile Thr Glu Thr Ser Arg
            260                 265                 270

Trp Pro Leu Leu Val Thr Gly Pro Leu Gln Ser Trp Ser Ser Pro Gln
        275                 280                 285

Lys Asn Val Val Leu Met Gly Asp Ala Ala His Ser Met Val Asn His
    290                 295                 300

Met Ala Gln Gly Ala Ala Thr Ser Met Glu Asp Gly Ala Phe Leu Ala
305                 310                 315                 320

Lys Cys Val Gly Ala Val Thr Arg Gly Lys Leu Ser Leu Arg Glu Ala
                325                 330                 335

Ile Asn Ile Tyr Glu Leu Glu Arg Met Pro Lys Ala Phe Met Lys Gln
            340                 345                 350

Gln Val Ser Phe Leu Asn Gly Ala Ile Trp His Leu Pro Asp Gly Pro
        355                 360                 365

Lys Gln Gln Ala Arg Asp Ala Ala Met Ala Pro Ser Leu Asp Gly Lys
    370                 375                 380

Tyr Leu Val Arg Ser Ser Asn Leu Tyr Asp Asp Pro Gln Thr Val Leu
385                 390                 395                 400

Asp Val Tyr Gly Tyr Asp Val Glu Ala His Ala Glu His Ala Leu Ala
                405                 410                 415

Lys Phe Ala His Gly Arg Glu Ala Leu Tyr Pro Gly Thr Gly Leu Ala
            420                 425                 430

Pro Phe Met Glu Asp Lys Tyr Met Gly Trp Phe Ser Lys Cys Gln Gln
        435                 440                 445

Asn Asp Arg Tyr Ala Ala Val Gly Lys Leu
    450                 455

<210>  24
<211>  1377
<212>  DNA
<213>  Penicillium brasilianum

<400>  24
atggccaatc agcgtgttca tgttggaatc gtcggcgcag gaataggagg gctggcagcc      60

gccattgcct ttcgacgtgc aggagtggat gtcactgtcc tggaagcagc ggatgaattg     120

ctcgaaatga cgccgaatgt atcggttctg ctgcagaaat ggggcgttgc agatgtgatt     180

ggagacaact tggttcaaat tgaagaactg aatatgcgcc gcaaagatgg cactcttgtc     240

ggacataccg aaataccgat tgttgaaaag gcattgggca gaccgtggtg gcttgttcat     300

cgtgctcatc tccacgaagg cctcgcgact gtggccaagc gtctgggagc cgtgattcat     360

atcaacagtc gcgtcatttc acttgactat caggaggggg gtgaagtcac ggtcgaaaca     420

caaaaagggg ataagtatca tttcgacctc tgtgtgggtg ctgatggcgt caacagcatt     480

gtccgaagca cactgtttcc gggcataaag ccggacgcga aaaccaccaa ctgtgcttac     540

cgggcgatta taccgtatga gcgtatccga caggacccaa ttgcaaagga gctcattcaa     600

aagttgacga tggaggtctg gatgggacat aatgcctata ttatcaccta tcccatcagt     660

ggaggcgaaa cattcaacct ggtgctctcg catcatcgtc cagagaagct ctatagtacc     720

caaccgaatg ttcctgtcga ggaaatgcgg gatgcgtaca aggactttga ccctcggatc     780

aaacgcattg ttgatatgat caccgagact tcacgctggc cgctattggt gaccgggccc     840

ttgcaatcat ggtcctcccc acagaagaat gtggttttga tgggggatgc agcgcactca     900

atggtaaacc atatggcgca aggggctgct acgtccatgg aagatggcgc ttttctcgca     960

aagtgcgtag gggcagttac tcgtggaaag ctgagtcttc gcgaagccat caatatctac    1020

gagctcgagc ggatgccaaa agccttcatg aaacaacagg tctcttttct gaatggtgcc    1080

atctggcact taccggatgg acctaagcaa caggctcgtg atgcggcaat ggcaccgtca    1140

cttgatggga aatatttggt tcgcagcagc aatctctacg atgaccctca gacggtgctg 1200

gatgtatatg gatatgacgt tgaagcgcat gcagaacatg cgctggcaaa gtttgcccac 1260

gggcgtgagg cgttgtatcc aggcactggt ttggcgcctt tcatggagga caaatacatg 1320

gggtggttct caaagtgtca gcaaaatgac cgctacgccg cagttgggaa gctttag 1377

<210> 25
<211> 360
<212> PRT
<213> Saccharomyces cerevisiae

<400> 25

Met Ser Tyr Pro Glu Lys Phe Glu Gly Ile Ala Ile Gln Ser His Glu
1               5                   10                  15

Asp Trp Lys Asn Pro Lys Lys Thr Lys Tyr Asp Pro Lys Pro Phe Tyr
            20                  25                  30

Asp His Asp Ile Asp Ile Lys Ile Glu Ala Cys Gly Val Cys Gly Ser
        35                  40                  45

Asp Ile His Cys Ala Ala Gly His Trp Gly Asn Met Lys Met Pro Leu
    50                  55                  60

Val Val Gly His Glu Ile Val Gly Lys Val Val Lys Leu Gly Pro Lys
65                  70                  75                  80

Ser Asn Ser Gly Leu Lys Val Gly Gln Arg Val Gly Val Gly Ala Gln
                85                  90                  95

Val Phe Ser Cys Leu Glu Cys Asp Arg Cys Lys Asn Asp Asn Glu Pro
            100                 105                 110

Tyr Cys Thr Lys Phe Val Thr Thr Tyr Ser Gln Pro Tyr Glu Asp Gly
        115                 120                 125

Tyr Val Ser Gln Gly Gly Tyr Ala Asn Tyr Val Arg Val His Glu His
    130                 135                 140

Phe Val Val Pro Ile Pro Glu Asn Ile Pro Ser His Leu Ala Ala Pro
145                 150                 155                 160

Leu Leu Cys Gly Gly Leu Thr Val Tyr Ser Pro Leu Val Arg Asn Gly
                165                 170                 175

Cys Gly Pro Gly Lys Lys Val Gly Ile Val Gly Leu Gly Gly Ile Gly
            180                 185                 190

Ser Met Gly Thr Leu Ile Ser Lys Ala Met Gly Ala Glu Thr Tyr Val
        195                 200                 205

Ile Ser Arg Ser Ser Arg Lys Arg Glu Asp Ala Met Lys Met Gly Ala
    210                 215                 220

Asp His Tyr Ile Ala Thr Leu Glu Glu Gly Asp Trp Gly Glu Lys Tyr
225                 230                 235                 240

Phe Asp Thr Phe Asp Leu Ile Val Val Cys Ala Ser Ser Leu Thr Asp
                245                 250                 255

Ile Asp Phe Asn Ile Met Pro Lys Ala Met Lys Val Gly Gly Arg Ile
            260                 265                 270

Val Ser Ile Ser Ile Pro Glu Gln His Glu Met Leu Ser Leu Lys Pro
        275                 280                 285

Tyr Gly Leu Lys Ala Val Ser Ile Ser Tyr Ser Ala Leu Gly Ser Ile
    290                 295                 300

Lys Glu Leu Asn Gln Leu Leu Lys Leu Val Ser Glu Lys Asp Ile Lys
305                 310                 315                 320

Ile Trp Val Glu Thr Leu Pro Val Gly Glu Ala Gly Val His Glu Ala
                325                 330                 335

Phe Glu Arg Met Glu Lys Gly Asp Val Arg Tyr Arg Phe Thr Leu Val
            340                 345                 350

Gly Tyr Asp Lys Glu Phe Ser Asp
        355                 360

<210>  26
<211>  422
<212>  PRT
<213>  Aspergillus niger

<400>  26

Met Cys Leu Lys Phe Glu Pro Leu Asn Ile Leu Ile Val Gly Ala Gly
1               5                   10                  15

Leu Gly Gly Leu Phe Ala Ser Leu Ala Leu Arg Gln Asp Gly His Arg
            20                  25                  30

Ile Thr Leu Leu Asp Ala Ser Ala Glu Phe Lys Glu Ala Gly Ala Gly
        35                  40                  45

Ile Arg Ile Pro Pro Asn Ser Thr Arg Leu Leu Arg Arg Trp Gly Val
    50                  55                  60

Asp Phe Asp Lys Met Arg Lys Cys Val Cys Gln Gly Tyr His Phe Arg
65                  70                  75                  80

Arg Trp Gln Asp Gly Ser Thr Ile Thr Glu Ile Asn Pro Glu Asp Met
                85                  90                  95

Ile Ala Cys His Gly Ala Pro Tyr Tyr Leu Val His Arg Ala Asp Leu
            100                 105                 110

His Ala Thr Leu Leu Ala Val Ala Ile Glu Ala Gly Val Val Ile His
        115                 120                 125

Lys Asn Arg His Val Thr Tyr Tyr Asp Phe Ser Val Pro Ile Ala Val
    130                 135                 140

Thr Ala Asp Ser Glu Lys Trp Thr Ala Asp Leu Ile Ile Cys Ala Asp
145                 150                 155                 160

Gly Ile Lys Ser Thr Ala Arg Pro Leu Leu Thr Ser Gln Pro Asp His
                165                 170                 175

Pro Arg Asp Thr Gly Asp Ile Ala Tyr Arg Ile Leu Ile Asn Gly Gln
            180                 185                 190

Asp Leu Leu Ala Asp Pro Asp Leu Ala Gly Leu Ile Thr His Pro Ala
        195                 200                 205

Thr Thr Ser Trp Cys Gly Pro Asp Ala His Leu Val Gly Tyr Pro Ile
    210                 215                 220

Arg Asp Gly Glu Leu Tyr Asn Ile Val Val Cys Ala Thr Ser Lys Gly
225                 230                 235                 240

Glu Thr Thr Asp Glu Ile Trp Val Val Glu Gly Asp Asn Glu Glu Leu
                245                 250                 255

Cys Ala Arg Phe Ser Gly Trp Glu Pro Arg Val Gln Lys Leu Cys Arg
            260                 265                 270

Leu Thr Lys Ser Phe Met Lys Trp Arg Leu Cys Asp Leu Pro Ala Leu
        275                 280                 285

Ser Thr Trp Ile His Pro Ser Gly Lys Ala Cys Leu Leu Gly Asp Ser

290                 295                 300

Cys His Pro Met Leu Pro Tyr Leu Ala Gln Gly Ala Ala Gln Ala Ala
305                 310                 315                 320

Glu Asp Ala Ala Thr Ile Cys Arg Ala Leu Ala Glu Asp Thr Asp Ile
                325                 330                 335

Thr Ser Ala Leu Lys Arg Tyr Glu Arg Ile Arg Ser Pro Arg Ala Ser
            340                 345                 350

Leu Ile Gln Ser Lys Thr Arg Glu His Gln Tyr Ile Leu His Val Asp
        355                 360                 365

Asp Gly Glu Glu Gln Cys Gln Arg Asp Glu Leu Met Arg His Asp Ser
    370                 375                 380

Pro Ser Ser Pro Ile Phe Trp Gly Tyr Glu Leu Arg Arg Glu Trp Leu
385                 390                 395                 400

Phe Ser His Asp Ala Glu Lys Met Glu Glu Ala Thr Ala Glu Pro Arg
                405                 410                 415

Leu Arg Thr Ala Ala Leu
            420

<210>  27
<211>  1056
<212>  DNA
<213>  Artificial

<220>
<223>  hmfL1 coding sequence optimised for expression in yeast

<400>  27
atgggttcct tatccttacc agaaacatca ttagccgcaa tccaagacaa agaaacaaaa      60

gctatctcag tcgccaaaag acctacacca gtacctgttg gtacccaagt cttagtaaaa     120

ttgcattatt ccggtgtttg tgccactgat ttgcacttag ctagaggttc tgttccatac     180

ttacaaccta aggtttcagt cggtggtcat gaaggtaccg gtgttattgc ttctttgggt     240

ccagatgtcg acgcagcaga atggcatgta ggtgacagag tagcagttag atgggtacac     300

atagtttgtg gtaaatgcga agtttgtact acaggtttcg aaaatttgtg ccaatctaga     360

aagttggctg gtaaagatgt tgaaggtact ttcgccgaat atgcaattgc cgactcttca     420

tacatggtta gattaccagc tggtgtctca gatgcagacg ccgctcctat cttgtgtgct     480

ggtgtcacag tatacaaagc cttgaagatc gcttctttga gagcaggttc atgggttgct     540

gtcgcaggtg ctggtggtgg tttaggtcat ttggcaatcc aatatgctag agcaatgggt     600

ttaaaagttg tcgcattgga tgccagaaag agagacttgt gcttatcctt gggtgctgaa 660

agttacatcg acgttttaga aactgatgac tgtgtcgcac aagtaattaa agttacagat 720

ggtggtgctc acggtgcatt aatatgcgct tccagtggtc aagcctacga tgacgctgtt 780

aaatttttga gatggaccgg tactttagtc tgtataggtt tgccacctaa gccaacattg 840

ttatccttag gtcctgctga ttttgtagcc agaggtatca aggttatggg tacaagtacc 900

ggtgacagac aagacacagt tgaagccttg gctttcgtcg ctaaaggtca agtaaagcct 960

caattaaccg aaagaagatt ggaagatgtt gaagaaatct taaaggaaat agaaaatggt 1020

acaatgcaag gtaaagccgt aatcagaatc gcatag 1056

<210> 28
<211> 1518
<212> DNA
<213> Artificial

<220>
<223> hmfN1 coding sequence optimised for expression in yeast

<400> 28
atgacccaaa ccaatgtcca cgtaaacaaa tccgacactt ccttagctgc tccacaacaa 60

ttattcatct ccggtaaata tcaaaactct caaagaaatg gtacatttcc agtcaaaaac 120

cctatgactg gtgaaacaat ctatgaatgt gtttctgcat cattagatga ctacgctgct 180

gctatagaag aagctgatgc cgcacaacca tcatgggcta gattaggtcc ttccgcaaga 240

agattgattt tgttaaaggc cgctgatata atggaaacat acatcgaaac cgacgctcca 300

gcaatcttga gtgctgaagt ttctgcaaca agaggttggg tcagagccaa tatattatct 360

accgctggtg ttttcagaga aactgctgct ttggcaacac atatcaaagg tgaaattgtt 420

ccagctgata gacctggtac tacaatctta gtttcaagag aaccagtcgg tgttgtcttg 480

gctatttccc cttggaatat gcctgcaacc ttaactgcca gagctatctg ttgcccttta 540

atttgtggta actctgtagt tttaagacca tccgaatttt ctcctaaatc tcaacatttg 600

gtcgtaagag ccttaacaga agctggtttg ccagcaggtt gcttgcaatt cttaccaaca 660

tcaaccgcag atacccctag agccatagaa tttgctatca gacaccctaa ggttagtaga 720

gctaatttca ctggttctga tagagtcggt agaattatag caggtttatc cgccagttgt 780

ttgaaaccat gcgttttgga attgggtggt aaagcccctg ttgtcgtatt agaagatgct 840

gatgtcgaag ctgctgttga agcagttgtc tacggtgcca tgtctaactc aggtcaaatt 900

tgtatgagta cagaaagagc tatagttcat agatcattgg ccgctgattt taaagcattg 960

ttagtaaaga gagccgaatc attaagagtt ggtaatcact tggaagatcc agacgttcaa 1020

ttgtcaggtt tgtttactgc tgcttccgca gaaagagtct tgggtttgat taaaggtgct 1080

gtaaacgcag gtgccacctt gttagctggt gacttggcat tacatggtcc atgccaaact 1140

ataatggctc ctcacatctt aaccggtgtt actagagata tggacttgtt tcatagagaa 1200

acattcggtc cagtattgtt cgttagtgaa tttgatactg atgacgaagc tatagcacaa 1260

gccaatgaca cagaattttc tttatgtgct tcagtattct ccagagatgt tttgagagct 1320

atggataccg ctaagagaat aagaactggt tcatgccacg tcaatggtcc tactgtatat 1380

atcgaagcac cattgcctaa cggtggtgtt ggtggtggtt ctggttacgg tagatttggt 1440

ggtgttgctg gtattgaaga gtttacagaa agacaaatag ttagtttagc caagccaggt 1500

atcaagtatg ccttttag 1518

<210> 29
<211> 2139
<212> DNA
<213> Artificial

<220>
<223> synthetic fragment comprising hmfN1 for expression in yeast

<400> 29
ctcccccggg ttagtcaaaa aattagcctt ttaattctgc tgtaacccgt acatgcccaa 60

aatagggggc gggttacaca gaatatataa catcgtaggt gtctgggtga acagtttatt 120

cctggcatcc actaaatata atggagcccg ctttttaagc tggcatccag aaaaaaaaag 180

aatcccagca ccaaaatatt gttttcttca ccaaccatca gttcataggt ccattctctt 240

agcgcaacta cagagaacag gggcacaaac aggcaaaaaa cgggcacaac ctcaatggag 300

tgatgcaacc tgcctggagt aaatgatgac acaaggcaat tgacccacgc atgtatctat 360

ctcattttct tacaccttct attaccttct gctctctctg atttggaaaa agctgaaaaa 420

aaaggttgaa accagttccc tgaaattatt cccctacttg actaataagt atataaagac 480

ggtaggtatt gattgtaatt ctgtaaatct atttcttaaa cttcttaaat tctactttta 540

tagttagtct tttttttagt tttaaaacac caagaactta gtttcgaata aacacacata 600

aacaaacaaa aatgacccaa accaatgtcc acgtaaacaa atccgacact tccttagctg 660

ctccacaaca attattcatc tccggtaaat atcaaaactc tcaaagaaat ggtacatttc 720

cagtcaaaaa ccctatgact ggtgaaacaa tctatgaatg tgtttctgca tcattagatg 780

actacgctgc tgctatagaa gaagctgatg ccgcacaacc atcatgggct agattaggtc 840

cttccgcaag aagattgatt ttgttaaagg ccgctgatat aatggaaaca tacatcgaaa 900

ccgacgctcc agcaatcttg agtgctgaag tttctgcaac aagaggttgg gtcagagcca 960

atatattatc taccgctggt gttttcagag aaactgctgc tttggcaaca catatcaaag 1020

gtgaaattgt tccagctgat agacctggta ctacaatctt agtttcaaga gaaccagtcg 1080

gtgttgtctt ggctatttcc ccttggaata tgcctgcaac cttaactgcc agagctatct 1140

gttgcccttt aatttgtggt aactctgtag ttttaagacc atccgaattt tctcctaaat 1200

ctcaacattt ggtcgtaaga gccttaacag aagctggttt gccagcaggt tgcttgcaat 1260

tcttaccaac atcaaccgca gataccccta gagccataga atttgctatc agacacccta 1320

aggttagtag agctaatttc actggttctg atagagtcgg tagaattata gcaggtttat 1380

ccgccagttg tttgaaacca tgcgttttgg aattgggtgg taaagcccct gttgtcgtat 1440

tagaagatgc tgatgtcgaa gctgctgttg aagcagttgt ctacggtgcc atgtctaact 1500

caggtcaaat ttgtatgagt acagaaagag ctatagttca tagatcattg gccgctgatt 1560

ttaaagcatt gttagtaaag agagccgaat cattaagagt tggtaatcac ttggaagatc 1620

cagacgttca attgtcaggt ttgtttactg ctgcttccgc agaaagagtc ttgggtttga 1680

ttaaaggtgc tgtaaacgca ggtgccacct tgttagctgg tgacttggca ttacatggtc 1740

catgccaaac tataatggct cctcacatct taaccggtgt tactagagat atggacttgt 1800

ttcatagaga aacattcggt ccagtattgt tcgttagtga atttgatact gatgacgaag 1860

ctatagcaca agccaatgac acagaatttt ctttatgtgc ttcagtattc tccagagatg 1920

ttttgagagc tatggatacc gctaagagaa taagaactgg ttcatgccac gtcaatggtc 1980

ctactgtata tatcgaagca ccattgccta acggtggtgt tggtggtggt tctggttacg 2040

gtagatttgg tggtgttgct ggtattgaag agtttacaga aagacaaata gttagtttag 2100

ccaagccagg tatcaagtat gccttttaga ctagtgtga 2139

<210> 30
<211> 3011
<212> DNA
<213> Artificial

<220>
<223> Synthetic split marker fragment 5-flank hmfK1 with Hygromicin B
 marker

<400> 30
ctcggtcgct cttttgggta gatggtagga taactgacag gaagtctatg ccagtactgc 60

ggaatcaaag aagcgtcgga gcaatgaacc gtatgcacgt cggaatgggg gaatgacacg 120

gagttatact cccggttcaa gccccgctta tcaaccctgg gcaggaattg cggggaagca 180

atgcggggaa gctacaacgc ttccccgcat ggtctacgtc tactcttggc acaacacatg 240

gaaatatcca gcgaagtggt tgactggtat cgagacttgg tccgtttcca gtcaaaatca 300

caccttcgta gaatccctgg cgttaccgcc tctcacgtct tttggctctg cttgtcagct 360

ggacgcagca cgcatcaatt ggcaagatgt cgtctacaag cgagtccttc actctcccaa 420

atgggcgaca gatggcttat accctctcgc ccggtggttc ctccgatcgc gtggtccttc 480

tctccaactc cttggccgaa gatctgacct cctgggagcg cgtcgtacct gtggtggaaa 540

accaaggctt ccgcgtcctc cgctacgacc agccaggcca tggacgctcg ggtgcaccta 600

ctgaagccga attgacctcc atgacattcg agaccttggt cgatgacgtc taccgccttc 660

tcggacacct caaaatcaac aatttgcatg cctgggtcgg cgtctcgatg ggcggcatca 720

aggccgtgta cttcaccgcg cgccacccag gcatcgtgaa caagatcgtc gtggccgacg 780

ccatcgccgc ttccccgtcc gtggtctgca tccccgataa cttcgccgcg cgggtaagcg 840

ctgtgaagca gtcgggttcc atctcggacg acctgtcaaa tactcggaag cggtggttcg 900

gtgaagactg gatggcgaag catccggagg agacggcgag gatggagaag tccatggcga 960

caactacaat tcagggtctc gaggcgtgct gtgctgccct tagcagcccg tcttttgacc 1020

tgaggccgct atatacgaag gtgggacatg gctgcgaaga agcactcatt gtcgccgggg 1080

agaaggatgc ggaccttcca gtcaagatgc aggagatgcg gcaggcaatc gaggagagcc 1140

tccggagctg tggaaagaag gtgcccgtga ggatggagat tatcaagggt gccgggcatg 1200

ttccctacat tgatggtttc gaggacttct gcgaaatcat caccaaattc cttgcctaga 1260

tagcactcga tctgcttcac gattgtgaaa tgtcttaaat tttcttgtca atctagactt 1320

tgcattaaac caatcttgag acaaatcatt cttcagtcgt attcccggag tgtgtgatgt 1380

cctggtccca tatttctcat ttctcatctt cgctatggct agtccgggta gcaagtgcgc 1440

gcaagtgcgc gctagtaggc aaatagaagc aatcgtcgga agttgggccc gactaggata 1500

acctggacta ggggcctgct gtacagtgac cggtgactct ttctggcatg cggagagacg 1560

gacggacgca gagagaaggg ctgagtaata agcgccactg cgccagacag ctctggcggc 1620

tctgaggtgc agtggatgat tattaatccg ggaccggccg cccctccgcc ccgaagtgga 1680

aaggctggtg tgcccctcgt tgaccaagaa tctattgcat catcggagaa tatggagctt 1740

catcgaatca ccggcagtaa gcgaaggaga atgtgaagcc aggggtgtat agccgtcggc 1800

gaaatagcat gccattaacc taggtacaga agtccaattg cttccgatct ggtaaaagat 1860

tcacgagata gtaccttctc cgaagtaggt agagcgagta cccggcgcgt aagctcccta 1920

attggcccat ccggcatctg tagggcgtcc aaatatcgtg cctctcctgc tttgcccggt 1980

gtatgaaacc ggaaaggccg ctcaggagct ggccagcggc gcagaccggg aacacaagct 2040

ggcagtcgac ccatccggtg ctctgcactc gacctgctga ggtccctcag tccctggtag 2100

gcagctttgc cccgtctgtc cgcccggtgt gtcggcgggg ttgacaaggt cgttgcgtca 2160

gtccaacatt tgttgccata ttttcctgct ctccccacca gctgctcttt tcttttctct 2220

ttcttttccc atcttcagta tattcatctt cccatccaag aacctttatt tcccctaagt 2280

aagtactttg ctacatccat actccatcct tcccatccct tattcctttg aacctttcag 2340

ttcgagcttt cccacttcat cgcagcttga ctaacagcta ccccgcttga gcagacatca 2400

ccatgcctga actcaccgcg acgtctgtcg agaagtttct gatcgaaaag ttcgacagcg 2460

tctccgacct gatgcagctc tcggagggcg aagaatctcg tgctttcagc ttcgatgtag 2520

gagggcgtgg atatgtcctg cgggtaaata gctgcgccga tggtttctac aaagatcgtt 2580

atgtttatcg gcactttgca tcggccgcgc tcccgattcc ggaagtgctt gacattgggg 2640

aattcagcga gagcctgacc tattgcatct cccgccgtgc acagggtgtc acgttgcaag 2700

acctgcctga aaccgaactg cccgctgttc tgcagccggt cgcggaggcc atggatgcga 2760

tcgctgcggc cgatcttagc cagacgagcg ggttcggccc attcggaccg caaggaatcg 2820

gtcaatacac tacatggcgt gatttcatat gcgcgattgc tgatccccat gtgtatcact 2880

ggcaaactgt gatggacgac accgtcagtg cgtccgtcgc gcaggctctc gatgagctga 2940

tgctttgggc cgaggactgc cccgaagtcc ggcacctcgt gcacgcggat ttcggctcca 3000

acaatgtcct g 3011

<210> 31
<211> 2886
<212> DNA
<213> Artificial

<220>
<223> Synthetic split marker fragment 3'-flank hmfK1 with Hygromicin B
      marker

<400> 31
tccggaagtg cttgacattg gggaattcag cgagagcctg acctattgca tctcccgccg 60

tgcacagggt gtcacgttgc aagacctgcc tgaaaccgaa ctgcccgctg ttctgcagcc 120

ggtcgcggag gccatggatg cgatcgctgc ggccgatctt agccagacga gcgggttcgg 180

cccattcgga ccgcaaggaa tcggtcaata cactacatgg cgtgatttca tatgcgcgat 240

tgctgatccc catgtgtatc actggcaaac tgtgatggac gacaccgtca gtgcgtccgt 300

cgcgcaggct ctcgatgagc tgatgctttg ggccgaggac tgccccgaag tccggcacct 360

cgtgcacgcg gatttcggct ccaacaatgt cctgacggac aatggccgca taacagcggt 420

cattgactgg agcgaggcga tgttcgggga ttcccaatac gaggtcgcca acatcttctt 480

ctggaggccg tggttggctt gtatggagca gcagacgcgc tacttcgagc ggaggcatcc 540

ggagcttgca ggatcgccgc ggctccgggc gtatatgctc cgcattggtc ttgaccaact 600

ctatcagagc ttggttgacg gcaatttcga tgatgcagct tgggcgcagg gtcgatgcga 660

cgcaatcgtc cgatccggag ccgggactgt cgggcgtaca caaatcgccc gcagaagcgc 720

ggccgtctgg accgatggct gtgtagaagt actcgccgat agtggaaacc gacgccccag 780

cactcgtccg agggcaaagg aatagtgatt taatagctcc atgtcaacaa gaataaaacg 840

cgtttcgggt ttacctcttc cagatacagc tcatctgcaa tgcattaatg cattggacct 900

cgcaacccta gtacgccctt caggctccgg cgaagcagaa gaatagctta gcagagtcta 960
ttttcatttt cgggagacga gatcaagcag atcaacggtc gtcaagagac ctacgagact 1020
gaggaatccg ctcttggctc cacgcgacta tatatttgtc tctaattgta ctttgacatg 1080
ctcctcttct ttactctgat agcttgacta tgaaaattcc gtcaccagcc cctgggttcg 1140
caaagataat tgcactgttt cttccttgaa ctctcaagcc tacaggacac acattcatcg 1200
taggtataaa cctcgaaaat cattcctact aagatgggta tacaatagta accatgcatg 1260
gttgcctagt gaatgctccg taacacccaa tacgccggcc gaaacttttt tacaactctc 1320
ctatgagtcg tttacccaga atgcacaggt acacttgttt agaggtaatc cttctttcta 1380
gaagtcctcg tgtactgtgt aagcgcccac tccacatctc cactcgagcc tatcactgtg 1440
gcaatgcctt agactctttt attcttcaat cataccatac atcctcgcgt tttgcattag 1500
tcctcggcga ttcacctcgc agttttcctc ctcctttggt tacatcttct cagcttaggc 1560
ggctggtacg tcttctgtat ttatttgagg cagctgtagg aaactttcac gttgaaagtt 1620
gacaagacga cttccacagc gtctgtgacg agggccacaa gcctgttttt tggtccatag 1680
aagttttcta tctttgtttc gaacagcgtc ttcctgtaac gggccagacc cagcagtgaa 1740
accatgtgcc atggcctttc ggacaccgcc tcaacaggca ggattctagg ggattctatc 1800
gcgaagattc catcagggac gagaccgatt ctctccatct ccttcgacac taaggattct 1860
aaactattcc aaaggattat atcagagacg agactgactc actttccttt cttcaatatt 1920
gttgtaaaaa gcatataggc ctaacataat tgaattaatg ctacaacacc ccttcgtact 1980
ttcttcttgt aggctccagg gatatagtcg ttcctctatc gtaagagacg acgcctttct 2040
ttagccttct catcacgctt ttagaaaaaa ggccttttga cccataaccg gatattctaa 2100
aagtaagctc aaagggtagg aggcagactc agaggagtag attttgaaac aagcaatgaa 2160
atggacgttg cttttaatga ggccgtagat gggataaagt ctcctttaat attttgtcac 2220
aggaagcatg actagtgcaa aggcctcttt gaactgcatt atcttgaaca ggacgggcca 2280
cctcacttgt gtcgttttgg ccagttttgt ttagatggag acctctagaa tgggatattc 2340
acgcgctagt tatcacagaa tagatgaccc tgtggggcat gtcctttttg ggtatagtct 2400
gacttggtct aagtgatcac tgagcgatca catgactgca ctcatgagcg cgtgctgaca 2460
tttactcact gagcctgagc ctgagtgccc tcattaaagc ggcggtagcc cagacaggaa 2520
agccctaccg gacaaaatcc ggtgtatata ggcgcatatt gcatcattgg gtgtgagtct 2580
gaggtagcac aacttattat cttggccatg tggagaggtc tataatgggc tttgtccgga 2640
tgcggcgatc agtcgacatt gtactttgta gacgtgtatg ggtggatgga tggagatcgg 2700
attgcgaagt atggagatcg cgattcttca gatgacgcac ggagtcgatc tcccgagctg 2760
ctctcttatt tacgtgtcaa gagtacaata acaattgagc tggacgatag atagtcttgg 2820

aaatcaacta tgacattgtt aactagacat ctattctaat gtacctcgaa agaatactcg 2880

ggatgg 2886

<210> 32
<211> 2658
<212> DNA
<213> Artificial

<220>
<223> Synthetic split marker fragment 5-flank hmfK3 with Phleomycin
      marker

<400> 32
ccgtgcgcta taataacctt cgaatcctgg tgcatcgccg ctgcttggaa agccttcttg 60

acccagttca gatagaaggt gacacttcgg tgacgtccga aatgcggttg cttcagcaaa 120

tgggactgag cagcgtcatg atctgtgttg agtctgctat gtctatcatc tcgattgtac 180

acaatatgtc tgccggtagt ggctggcgac gtgagttctt gggtgcatgg aattattcac 240

tctattacag taggttgggt tctgtaagat ttgacctcca gggagttgaa actaacgtaa 300

cacagctttc aatgcagcac tagttatttt cggctccctc attgtggcat cgaaggagcg 360

tgaccgcaac ccgtcagcct ggatcatgat cagccattcc cggtcgtata ttgacacggc 420

agctgaagcg ctgcgtcagc ttgacccggg aaaccctgtt gttgaacggt gtcaagagta 480

tctttcgcag ctatctgcaa cattggatcc tctcagttag ttatcccgca cagatgcaaa 540

cctccctttc gtcccatccg tgccatttca agactggccc tgcccgcgct tcaacaattg 600

ttccaaaaac ctgctgacag cccttgccta cacaatgcac agtttcagaa gagccatatt 660

gtgataatag gcttgctttc gactttccta ccgaatattc caccgggctg ttttctcttc 720

acagtgccga cttagtaccg cctatggact ttggcgaatt catgattggt tagggttgta 780

gcttttcggt acggccctgg gaatagcgag gcattgactg ctagtcaact gtagtggagc 840

aaacagagaa gggaccacgt aggtacaagc tgggcgatgc agctgtccga ccctctcgag 900

atgggttcgc cgcggaagga gcaaatagtc tcagtatcca gctttgccgt aggaatgaga 960

aaactgaact caagacagaa acctcagcgt accttttcaa agatgatgta aaagatgtct 1020

ctctcagaga aggatagtat agtaatgatc agatccgctg gaaaatagtg cggcaccgaa 1080

cccctgacgg cccggcggcc cggcgggtat ggtaatgacg gtaaattatc tagggcatcg 1140

ctatgatcat ttgacaagac ccgtgtgtcg gcattatcat tcggacatga ttcaacgcct 1200

caattttacc ccatatgtgc caaccattcc atctcgcgtg ggcttagaca tcccacgggc 1260

aaacgtcccg tccagcccca gtcagcgaat gcggggataa tccggggttt cctaccccca 1320

tgggctgggc cggaatgcct aactccccga attggctgcc cggggagtgc aaataaaata 1380

gttgcttgta tacatatata tatatccttc ggtatatcca agacttcctg ttgcatcaag 1440

cgcaatccta agcttttttc tccctaccac aagtagctct acaaacccga aacctagaac 1500

aggcctgctg tacagtgacc ggtgactctt tctggcatgc ggagagacgg acggacgcag 1560

agagaagggc tgagtaataa gcgccactgc gccagacagc tctggcggct ctgaggtgca 1620

gtggatgatt attaatccgg gaccggccgc ccctccgccc cgaagtggaa aggctggtgt 1680

gcccctcgtt gaccaagaat ctattgcatc atcggagaat atggagcttc atcgaatcac 1740

cggcagtaag cgaaggagaa tgtgaagcca ggggtgtata gccgtcggcg aaatagcatg 1800

ccattaacct aggtacagaa gtccaattgc ttccgatctg gtaaaagatt cacgagatag 1860

taccttctcc gaagtaggta gagcgagtac ccggcgcgta agctccctaa ttggcccatc 1920

cggcatctgt agggcgtcca aatatcgtgc ctctcctgct ttgcccggtg tatgaaaccg 1980

gaaaggccgc tcaggagctg gccagcggcg cagaccggga acacaagctg gcagtcgacc 2040

catccggtgc tctgcactcg acctgctgag gtccctcagt ccctggtagg cagctttgcc 2100

ccgtctgtcc gcccggtgtg tcggcggggt tgacaaggtc gttgcgtcag tccaacattt 2160

gttgccatat tttcctgctc tccccaccag ctgctctttt cttttctctt tcttttccca 2220

tcttcagtat attcatcttc ccatccaaga acctttattt cccctaagta agtactttgc 2280

tacatccata ctccatcctt cccatccctt attcctttga acctttcagt tcgagctttc 2340

ccacttcatc gcagcttgac taacagctac cccgcttgag cagacatcac catggccaag 2400

ttgaccagtg ccgttccggt gctcaccgcg cgcgacgtcg ccggagcggt cgagttctgg 2460

accgaccggc tcgggttctc ccgggacttc gtggaggacg acttcgccgg tgtggtccgg 2520

gacgacgtga ccctgttcat cagcgcggtc caggaccagg tggtgccgga caacaccctg 2580

gcctgggtgt gggtgcgcgg cctggacgag ctgtacgccg agtggtcgga ggtcgtgtcc 2640

acgaacttcc gggacgcc 2658

<210> 33
<211> 2472
<212> DNA
<213> Artificial

<220>
<223> Synthetic split marker fragment 3-flank hmfK3 with Phleomycin
 marker

<400> 33
gcgcgacgtc gccggagcgg tcgagttctg gaccgaccgg ctcgggttct cccgggactt 60

cgtggaggac gacttcgccg gtgtggtccg ggacgacgtg accctgttca tcagcgcggt 120

ccaggaccag gtggtgccgg acaacaccct ggcctgggtg tgggtgcgcg gcctggacga 180

gctgtacgcc gagtggtcgg aggtcgtgtc cacgaacttc cgggacgcct ccgggccggc 240

catgaccgag atcggcgagc agccgtgggg gcgggagttc gccctgcgcg acccggccgg 300

caactgcgtg cacttcgtgg ccgaggagca ggactgatga tttaatagct ccatgtcaac 360
aagaataaaa cgcgtttcgg gtttacctct tccagataca gctcatctgc aatgcattaa 420
tgcattggac ctcgcaaccc tagtacgccc ttcaggctcc ggcgaagcag aagaatagct 480
tagcagagtc tattttcatt ttcgggagac gagatcaagc agatcaacgg tcgtcaagag 540
acctacgaga ctgaggaatc cgctcttggc tccacgcgac tatatatttg tctctaattg 600
tactttgaca tgctcctctt ctttactctg atagcttgac tatgaaaatt ccgtcaccag 660
cccctgggtt cgcaaagata attgcactgt ttcttccttg aactctcaag cctacaggac 720
acacattcat cgtaggtata aacctcgaaa atcattccta ctaagatggg tatacaatag 780
taaccatgca tggttgccta gtgaatgctc cgtaacaccc aatacgccgg ccgaaacttt 840
tttacaactc tcctatgagt cgtttaccca gaatgcacag gtacacttgt ttagaggtaa 900
tccttctttc tagaagtcct cgtgtactgt gtaagcgccc actccacatc tccactcgag 960
cctatcattc agcaatcaga ctctcctatc tcttccttgc atgcaataac tgtgtaaata 1020
tacgttttgc tctatgagat tgtacctttg gacgaacttg gagccccgcc gtggcctgtg 1080
tttgtttcta ttttgatctg tggagaggct agggtattta ggtgtgcaga ttcatcattg 1140
taaactggtt tgtatggtcg gggaactgtt ggatcactct tgcgccaccg ttgccctgtt 1200
gccccgttgc cccgttgccc tgttgcctgg caggtgacat gattagatcc actgcatttc 1260
ctcgccgttt ttaaattcaa ctatctcttt ctctctgata atttgcgtgg gtgacaatca 1320
tggcagtcca tagacatcgt cagcttcagc tggagctgat tctgatggtc gcagactatt 1380
tgcgaccttt agatctttta aatttggttc agggcatacc tcaaattgct tcccttctct 1440
cctctcgaca tatgcgagcc caagatgaca atggattcac tatatttcat ctgattgtgg 1500
aacaggggct taaaagccta atagaacgcc tcgataaatg gatcccacgg ggctttattc 1560
ccgacaacga agggtggaca cctcttcatc aagaagttag gaaggctaat taccggatgg 1620
taaaagcact gattgatgct ggctcagatt tatcagcccg gggttataat ggaaaaactt 1680
cccttcacct tgcctgcggt ggaaatgcag ttgaaattgt caggttgctc ttagaccatg 1740
gtgcaaaccc gtccgcaaag gattacgacg aaaggacgcc tttgcacgac gcctattctc 1800
atgacatatc tgtccgtcag atgcttatta cagccggcgc ggaccttgac ccctgccaag 1860
tgccacgggg gttgaccccc ctttattatg aacctatgat agcccgagaa agcgttatta 1920
ggattcttct tgaggctggg gcagaccttt ccattcaaga tatgagtggg gatactatat 1980
tacaccgcgc tacgatccac aatttcgcta atgtcgtccg gctgttactt gaattcggtg 2040
ttgatatcac tgtgcgaaat gtccacggtt ataccccggt tttggttgct gcaattggcg 2100
ggtcagatga atgtctacga ctgctcctca aggccggcgc agacctttca gtcatggata 2160

atcatggacg tagcgctttg catatagcag ctcgggcggg aagggaatct acagtccgac 2220

tgcttgtgaa acaaggaatg gacatcgccg cacgggacag ccgccgtggc tggactccga 2280

tgtgctgggc ggtaaactac gaacagaaag gggtaatcca agttttagaa gatacgcaaa 2340

caaaccgatt tgcgaagttt gcacggcgag tacgcatcaa attttaaatg ccatttttgc 2400

tcagagcgct aggcatgtcg tggttccgga tttcctgtag ccagataagc ctcatatatc 2460

actctttcac ca 2472

<210> 34
<211> 20
<212> DNA
<213> Artificial

<220>
<223> Primer 180

<400> 34
   ggtagaaaag gggttgcgat 20

<210> 35
<211> 18
<212> DNA
<213> Artificial

<220>
<223> Primer 181

<400> 35
gaagcaatcg tcggaagt 18

<210> 36
<211> 21
<212> DNA
<213> Artificial

<220>
<223> Primer 182

<400> 36
cgcgaggatg tatggtatga t 21

<210> 37
<211> 21
<212> DNA
<213> Artificial

<220>
<223> Primer 183

<400> 37
gaaagtgaga ttgtggatgg a 21

<210> 38
<211> 17

<212>  DNA
<213>  Artificial

<220>
<223>  Primer 184

<400>  38
tgtcgttcat ggctccc                                                    17


<210>  39
<211>  18
<212>  DNA
<213>  Artificial

<220>
<223>  Primer 185

<400>  39
aaggtgcgaa ctggagag                                                   18


<210>  40
<211>  22
<212>  DNA
<213>  Artificial

<220>
<223>  Primer 186

<400>  40
ggaacaatcg gcaaagaaag tg                                              22


<210>  41
<211>  19
<212>  DNA
<213>  Artificial

<220>
<223>  Primer 187

<400>  41
caacttcttg tggcgttgg                                                  19


<210>  42
<211>  20
<212>  DNA
<213>  Artificial

<220>
<223>  Primer 212

<400>  42
ctcggtcgct cttttgggta                                                 20


<210>  43
<211>  20
<212>  DNA
<213>  Artificial

<220>
<223>  Primer 213

<400>  43
caggacattg ttggagccga                                                   20


<210>  44
<211>  20
<212>  DNA
<213>  Artificial

<220>
<223>  Primer 214

<400>  44
tccggaagtg cttgacattg                                                   20


<210>  45
<211>  22
<212>  DNA
<213>  Artificial

<220>
<223>  Primer 215

<400>  45
ccatcccgag tattctttcg ag                                                22


<210>  46
<211>  22
<212>  DNA
<213>  Artificial

<220>
<223>  Primer 216

<400>  46
ccgtgcgcta taataacctt cg                                                22


<210>  47
<211>  16
<212>  DNA
<213>  Artificial

<220>
<223>  Primer 217

<400>  47
gcgtcccgga agttcg                                                       16


<210>  48
<211>  18
<212>  DNA
<213>  Artificial

<220>
<223>  Primer 218

<400>  48
gagcggtcga gttctgga                                                    18


<210>  49
<211>  23
<212>  DNA
<213>  Artificial

<220>
<223>  Primer 219

<400>  49
ggtgaaagag tgatatatga ggc                                              23


<210>  50
<211>  21
<212>  DNA
<213>  Artificial

<220>
<223>  Primer 220

<400>  50
tctcaggact tgcagatgtt g                                                21


<210>  51
<211>  19
<212>  DNA
<213>  Artificial

<220>
<223>  Primer 221

<400>  51
gccaagtcat catcctcgc                                                   19


<210>  52
<211>  20
<212>  DNA
<213>  Artificial

<220>
<223>  Primer 222

<400>  52
cttactgccg gtgattcgat                                                  20


<210>  53
<211>  22
<212>  DNA
<213>  Artificial

<220>
<223>  Primer 223

<400>  53
ctacaggaca cacattcatc gt                                               22

<210>  54
<211>  18
<212>  DNA
<213>  Artificial

<220>
<223>  Primer 224

<400>  54
tgccagccct cctattcc                                                    18


<210>  55
<211>  447
<212>  PRT
<213>  Cupriavidus basilensis

<400>  55

Met Glu Ala Val Ala Lys Lys Arg Thr Glu Thr Ile Ser Glu Ala Leu
1               5                   10                  15

Pro Ala Ala Thr Asn Arg Gln Val Phe Gly Ala Val Thr Ala Ser Cys
            20                  25                  30

Met Gly Trp Ala Leu Asp Leu Phe Asp Leu Phe Ile Leu Leu Phe Val
        35                  40                  45

Ala Pro Val Ile Gly Arg Leu Phe Phe Pro Ser Glu His Ala Met Leu
    50                  55                  60

Ser Leu Ala Ala Val Tyr Ala Ser Phe Ala Val Thr Leu Leu Met Arg
65                  70                  75                  80

Pro Leu Gly Ser Ala Ile Phe Gly Thr Tyr Ala Asp Arg His Gly Arg
                85                  90                  95

Lys Gly Ala Met Val Val Ala Val Thr Gly Val Gly Leu Ser Thr Ala
            100                 105                 110

Ala Phe Gly Leu Leu Pro Thr Val Gly Gln Val Gly Leu Leu Ala Pro
        115                 120                 125

Ala Leu Phe Ile Leu Leu Arg Leu Val Gln Gly Ile Phe Val Gly Gly
    130                 135                 140

Val Val Ala Ser Thr His Thr Ile Gly Thr Glu Ser Val Pro Pro Ser
145                 150                 155                 160

Trp Arg Gly Ala Val Ser Gly Leu Val Gly Gly Gly Gly Ala Gly Ile
                165                 170                 175

Gly Ala Leu Leu Ala Ser Ile Thr Tyr Met Ala Met Thr Ala Leu Phe
            180                 185                 190

Pro Gly Glu Ala Phe Asp Ala Trp Gly Trp Arg Cys Met Phe Phe Ser
        195                 200                 205

Gly Ile Ile Ser Ser Val Leu Gly Leu Phe Ile Phe Asn Ser Leu Glu
    210                 215                 220

Glu Ser Pro Leu Trp Lys Gln Leu Gln Ala Ala Lys Gly His Ala Ala
225                 230                 235                 240

Pro Val Glu Asn Pro Leu Arg Val Ile Phe Ser Arg Gln Tyr Arg Gly
                245                 250                 255

Val Leu Phe Val Asn Ile Leu Leu Thr Val Gly Gly Gly Ser Ala Tyr
            260                 265                 270

Tyr Leu Thr Ser Gly Tyr Leu Pro Thr Phe Leu Lys Val Val Val Lys
        275                 280                 285

Ala Pro Ala Gly Ala Ser Ala Ala Ile Leu Met Ala Ser Ser Val Gly
    290                 295                 300

Val Ile Val Ala Ser Ile Ile Ala Gly His Leu Ser Thr Leu Ile Gly
305                 310                 315                 320

Arg Lys Arg Ala Phe Leu Leu Ile Gly Ala Leu Asn Val Val Leu Leu
                325                 330                 335

Pro Leu Ile Tyr Gln Arg Met Pro Ala Ala Pro Asp Val Thr Thr Leu
            340                 345                 350

Gly Ile Tyr Ala Val Ala Leu Ala Met Leu Gly Ser Thr Gly Phe Ala
        355                 360                 365

Pro Ile Leu Ile Phe Leu Asn Glu Arg Phe Pro Thr Ser Ile Arg Ala
    370                 375                 380

Thr Gly Thr Gly Leu Ser Trp Asn Ile Gly Phe Ala Ile Gly Gly Met
385                 390                 395                 400

Met Pro Thr Phe Ala Ser Leu Cys Ala Ser Thr Pro Ala Asp Leu Pro
                405                 410                 415

Lys Val Leu Gly Ile Phe Val Ala Val Val Thr Ala Ile Tyr Leu Ala
            420                 425                 430

Gly Ala Ala Ile Val Pro Glu Thr Ala Gly Arg Leu Gly Glu Lys
        435                 440                 445

## Description

### Field of the invention

The invention relates to the fields of molecular genetics, metabolic engineering, biotransformation and fermentation technology. In particular, the invention relates to fungi that are genetically modified to produce 2,5-furandicarboxylic acid from hydroxymethylfurfural. The invention further relates to the use of such fungi in processes for the biotransformation of hydroxymethylfurfural into 2,5-furandicarboxylic acid.

### Background of the invention

2,5-furandicarboxylic acid (FDCA) is a monomeric compound which can be applied in the production of polyesters which have a tremendous economic impact. A very important compound in the field is polyethyleneterephthalate (PET) which is produced from terephthalic acid (PTA) and ethylene glycol. FDCA may substitute for PTA in the polyester PET in which case polyethylenefurandicarboxylate (PEF) results. PEF has a good potential in replacing PET in the large polyester market. Not only because it has superior properties when compared to PET, but also because it can be derived from renewable feedstocks. FDCA can be produced from sugars either chemically (De Jong et al., 2012. In: Biobased Monomers, Polymers, and Materials; Smith, P., et al.; ACS Symposium Series; American Chemical Society: Washington, DC) or in a combined chemical-biological route (Wiercks et al., 2011. Appl Microbiol Biotechnol 92:1095-1105). In the latter case, a monomeric sugar such as glucose or fructose is chemically transformed into 5-(hydroxymethyl)-2-furaldehyde (HMF) which subsequently can be oxidized by enzymes into FDCA.

A biological route for producing FDCA from HMF has been developed based on the isolation of the HMF-degrading strain of *Cupriavidus basilensis* HMF14 (Wierckx et al., 2010. Microbial Technology 3:336-343). A cluster of genes encoding enzymes involved in the HMF degradation route in C. *basilensis* HMF14 was identified and relevant genes heterologously expressed in a *Pseudomonas putida* S12 strain (Koopman et al., 2010. PNAS 107:4919-4924) which thereby acquired the ability to metabolize HMF. The heterologous expression of only the *hmfH* gene - encoding a HMF oxidoreductase that acts as an oxidase mainly at HMF-acid (HMFCA), but it also may oxidize HMF or FFCA - enables *P. putida* S12 to produce FDCA from HMF (Koopman et al., 2010. Bioresource Technology 101:6291-6296; and WO 2011/026913). In further optimization work (Wierckx *et al.,* 2011, *supra*; and WO 2012/064195), two additional genes were expressed in P. *putida* S12 that encode for an HMFCA transporter and for an aldehyde dehydrogenase with unknown specificity, respectively.

US 7,067,303 disclose that the fungus *Coniochaeta ligniaria* (teleomorph), or its *Lecythophora* anamorph state, are capable of significantly depleting the toxic levels of furans, particularly furfural and HMF, in agricultural biomass hydrolysate. The use of C. *ligniaria* as a biological agent in detoxifying sugar-containing hydrolysates was further demonstrated in a number of subsequent papers (López et al., 2004. Appl. Microbiol Biotechnol 64:125-131; Nichols et al., 2005. Appl Biochem Biotechnol. Spring; 121-124:379-90; Nichols et al., 2008. Enzyme and Microbial Technology 42:624-630; Nichols et al., 2010. Bioresource Technol 19:7545-50; Nichols et al., 2014. Biomass and Bioenergy 67:79-88). Apart from detoxification of HMF to less toxic compounds, the organism was also able to metabolize HMF for growth.

Zhang et al. (2010, Biotechnology for Biofuels 3:26) described the isolation of two HMF-metabolizing fungi that detoxified their corn stover hydrolysate, which were identified as *Amorphotheca resinae* and *Eupenicillium baarnense*, respectively. In a subsequent paper (Ran et al., 2014, Biotechnology for Biofuels 7:51) growth of the *A. resinae* strain, designated as ZN1, was reported to be supported by many compounds including HMF. HMF was degraded and HMF alcohol and HMFCA accumulated over time but no accumulation of FDCA was reported.

Govinda Rajulu et al. (2014, Mycological Progress 13:1049-1056) similarly isolated a number of fungi with the ability to utilize furfural and/or HMF as sole carbon source but again, no accumulation of FDCA was reported.

Thus, several fungi have been described that either grow at the expense of HMF or detoxify HMF-containing feedstocks. As with yeasts, the organisms were studied from the perspective of reducing HMF into HMF-alcohol for the purpose of detoxifying feedstocks. Production of FDCA by yeast or filamentous fungi, however, has not been described. Yet, fungal production of FDCA from HMF would offer several intrinsic advantages over the bacterial processes in the art. E.g., many fungi tolerate low pH values down to pH = 3 or lower for growth, whereas most bacteria prefer neutral pH environments. In the specific situation of large-scale production of FDCA it would be of great advantage if whole-cell production methodologies at low pH-values would be available because of advantages in downstream processing (DSP) and for combating infections.

It is therefore an object of the present invention to provide for fungal cells and their use in processes for the production of FDCA from HMF.

### Summary of the invention

In a first aspect the present invention relates to a fungal cell comprising a genetic modification that reduces specific 2,5-furandicarboxylic acid (FDCA) decarboxylating monooxygenase activity in the cell, as compared to a corresponding parent cell lacking the genetic modification, wherein the genetic modification eliminates the expression of an endogenous gene encoding an FDCA decarboxylating monooxygenase by deletion of at least a part of at least one of the promoter and the coding sequence of the gene. Preferably, in the cell, the endogenous gene in the corresponding parent cell encodes a FDCA decarboxylating monooxygenase comprising an amino acid sequence with at least 45% sequence identity to at least one of SEQ ID NO : 4. It is further preferred that the genetic modification comprises or consists of the deletion of at least the complete coding sequence of an endogenous gene encoding an FDCA decarboxylating monooxygenase from the gene in the cell's genome. In cells comprising more than one copy of the endogenous gene, preferably, the expression of all copies of the endogenous gene encoding an FDCA decarboxylating monooxygenase is eliminated.

The fungal cell according to the invention preferably is a cell that has the natural ability to oxidise HMF to FDCA.

A preferred fungal cell according to the invention preferably comprises a further genetic modification that is at least one of: a) a genetic modification that confers to the cell the ability to oxidize 5-hydroxymethyl-2-furancarboxylic acid (HMFCA) to 5-formyl-2-furoic acid (FFCA) or that increases in the cell the specific activity of a enzyme that oxidizes HMFCA to FFCA as compared to a corresponding wild type cell lacking the genetic modification; and, b) a genetic modification that confers to the cell the ability to oxidize furanic aldehydes to the corresponding furanic carboxylic acids or a genetic modification that increases in the cell the specific activity of a enzyme that oxidizes furanic aldehydes to the corresponding furanic carboxylic acids, as compared to a corresponding wild type cell lacking the genetic modification. Preferably, in the cell, the genetic modification in a) is a modification that increases expression of a nucleotide sequence encoding a polypeptide with HMFCA dehydrogenase activity, which polypeptide comprises an amino acid sequence that has at least 45% sequence identity with the amino acid sequence of at least one of SEQ ID NO.'s: 1 and 2; and/or, the genetic modification in b) is a modification that increases expression of a nucleotide sequence encoding a polypeptide having furanic aldehyde dehydrogenase activity, which aldehyde dehydrogenase has at least one of the abilities of i) oxidizing HMF to HMFCA, ii) oxidizing DFF to FFCA, and, iii) oxidizing FFCA into FDCA, which polypeptide comprises an amino acid sequence that has at least 45% sequence identity with the amino acid sequence of SEQ ID NO.: 3.

A fungal cell according to the invention preferably further comprises a genetic modification selected from: a) a genetic modification that reduces or eliminates the expression of a gene encoding a short chain dehydrogenase that reduces HMF and/or FFCA to the corresponding alcohol, wherein preferably the gene is at least one of a gene encoding polypeptide comprising an amino acid sequence with at least 45% sequence identity to at least one of SEQ ID NO: 5 and 25; b) a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 6 - 10; and, c) a genetic modification that alters the expression of a gene encoding a transcriptional activator of genes involved in furan catabolism, wherein preferably the gene is a gene encoding a polypeptide comprising an amino acid sequence with at least 45% sequence identity to SEQ ID NO: 11.

In a separate aspect the invention pertains to a fungal cell that has the ability to oxidise HMF to FDCA and which cell comprises a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 9 and 10. Preferably a cell according to this aspect comprises a further genetic modification selected from: a) a genetic modification that eliminates or reduces specific FDCA decarboxylating monooxygenase activity in the cell, as compared to a corresponding parent cell lacking the genetic modification; b) a genetic modification that confers to the cell the ability to oxidize HMFCA to FFCA or that increases in the cell the specific activity of a enzyme that oxidizes HMFCA to FFCA as compared to a corresponding wild type cell lacking the genetic modification; c) a genetic modification that confers to the cell the ability to oxidize furanic aldehydes to the corresponding furanic carboxylic acids or a genetic modification that increases in the cell the specific activity of a enzyme that oxidizes furanic aldehydes to the corresponding furanic carboxylic acids, as compared to a corresponding wild type cell lacking the genetic modification; d) a genetic modification that reduces or eliminates the expression of a gene encoding a short chain dehydrogenase that reduces HMF and/or FFCA to the corresponding alcohol, wherein preferably the gene is at least one of a gene encoding polypeptide comprising an amino acid sequence with at least 45% sequence identity to at least one of SEQ ID NO: 5 and 25; e) a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 6 - 8; and, f) a genetic modification that alters the expression of a gene encoding a transcriptional activator of genes involved in furan catabolism, wherein preferably the gene is a gene encoding a polypeptide comprising an amino acid sequence with at least 45% sequence identity to SEQ ID NO: 11.

A cell according to the above aspects of the invention preferably is a filamentous fungal cell selected from a genus from the group consisting of: *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium, Trichoderma,* and *Ustilago,* more preferably the cell is a filamentous fungal cell selected from a species from the group consisting of: *Aspergillus niger, Aspergillus awamori, Aspergillus foetidus, Aspergillus sojae, Aspergillus fumigatus, Talaromyces emersonii, Aspergillus oryzae, Myceliophthora thermophila, Trichoderma reesei, Penicillium chrysogenum, Penicillium simplicissimum* and *Penicillium brasilianum*; or, the cell is a yeast cell selected from a genus from the group consisting of: *Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula,* and *Sporobolomyces,* more preferably the cell is a yeast cell selected from a species from the group consisting of *Kluyveromyces lactis, S. cerevisiae, Hansenula polymorpha, Yarrowia lipolytica, Candida tropicalis* and *Pichia pastoris.*

In a further aspect the invention relates to a process for oxidizing HMFCA to FFCA, the process comprising the step of incubating a fungal cell according to the invention, in the presence of HMFCA, under conditions conducive to the oxidation of HMFCA by the cell, wherein the cell expresses enzymes that have the ability to oxidize HMFCA to FFCA.

In another aspect, the invention relates to a process for producing FDCA, the process comprising the step of incubating a fungal cell according to the invention, in a medium comprising one or more furanic precursors of FDCA, preferably under conditions conducive to the oxidation of furanic precursors of FDCA by the cell to FDCA, and, optionally recovery of the FDCA, wherein preferably, at least one furanic precursor of FDCA is selected from the group consisting of HMF, 2,5-dihydroxymethyl furan (DHF), HMFCA, FFCA and 2,5-diformyl furan (DFF), of which HMF is most preferred, wherein the furanic precursors of FDCA are obtained from one or more hexose sugars, preferably one or more hexose sugars obtained from lignocellulosic biomass, preferably by acid-catalyzed dehydration, and, wherein preferably the FDCA is recovered from the medium by a process comprising acid precipitation followed by cooling crystallization and/or solvent extraction. Preferably, in the process, the medium has a pH in the range of 2.0 - 3.0, wherein preferably the FDCA precipitates from the acidic medium in which it is produced and is recovered from the medium by a process comprising acid precipitation followed by cooling crystallization.

In yet a further aspect the invention pertians to a process for producing a polymer from at least two FDCA monomers, the process comprising the steps of: a) preparing an FDCA monomer in a process according to the invention; and, b) producing a polymer from the FDCA monomer obtained in a).

In a final aspect, the invention relates to the use of a fungal cell according to the invention, for the biotransformation of one or more of furanic precursors to FDCA or a fungal cell expressing one or more bacterial enzymes with the ability to convert a furanic precursors of FDCA into FDCA, wherein preferably, at least one furanic precursor of FDCA is selected from the group consisting of HMF, DHF, HMFCA, FFCA and DFF, of which HMF is most preferred.

### Description of the invention

### Definitions

The terms "homology", "sequence identity" and the like are used interchangeably herein. Sequence identity is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length (full length), maximizing the number of matches and minimizing the number of gaps. A global alignment is suitably used to determine sequence identity when the two sequences have similar lengths. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blossum62 for proteins and DNAFull for DNA). When sequences have a substantially different overall lengths, local alignments, such as those using the Smith Waterman algorithm, are preferred.

Alternatively percentage similarity or identity may be determined by searching against public databases, using algorithms such as FASTA, BLAST, etc. Thus, the nucleic acid and protein sequences of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to oxidoreductase nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., BLASTx and BLASTn) can be used. See the homepage of the National Center for Biotechnology Information at http://www.ncbi.nlm.nih.gov/.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. Examples of classes of amino acid residues for conservative substitutions are given in the Tables below.

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gin (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes.

| | | | |
|---|---|---|---|
| 1 | A | S | T |
| 2 | D | E | |
| 3 | N | Q | |
| 4 | R | K | |
| 5 | I | L | M |
| 6 | F | Y | W |

Alternative Physical and Functional Classifications of Amino Acid Residues.

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

As used herein, the term "selectively hybridizing", "hybridizes selectively" and similar terms are intended to describe conditions for hybridization and washing under which nucleotide sequences at least 66%, at least 70%, at least 75%, at least 80%, more preferably at least 85%, even more preferably at least 90%, preferably at least 95%, more preferably at least 98% or more preferably at least 99% homologous to each other typically remain hybridized to each other. That is to say, such hybridizing sequences may share at least 45%, at least 50%, at least 55%, at least 60%, at least 65, at least 70%, at least 75%, at least 80%, more preferably at least 85%, even more preferably at least 90%, more preferably at least 95%, more preferably at least 98% or more preferably at least 99% sequence identity.

A preferred, non-limiting example of such hybridization conditions is hybridization in 6X sodium chloride/sodium citrate (SSC) at about 45°C, followed by one or more washes in 1 X SSC, 0.1 % SDS at about 50°C, preferably at about 55°C, preferably at about 60°C and even more preferably at about 65°C.

Highly stringent conditions include, for example, hybridization at about 68°C in 5x SSC/5x Denhardt's solution / 1.0% SDS and washing in 0.2x SSC/0.1% SDS at room temperature. Alternatively, washing may be performed at 42°C.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N.Y.; and Ausubel et al. (eds.), Sambrook and Russell (2001) "Molecular Cloning: A Laboratory Manual (3rd edition), Cold Spring Harbor Laboratory, Cold Spring Harbor Laboratory Press, New York 1995, Current Protocols in Molecular Biology, (John Wiley & Sons, N.Y.).

Of course, a polynucleotide which hybridizes only to a poly A sequence (such as the 3' terminal poly(A) tract of mRNAs), or to a complementary stretch of T (or U) resides, would not be included in a polynucleotide of the invention used to specifically hybridize to a portion of a nucleic acid of the invention, since such a polynucleotide would hybridize to any nucleic acid molecule containing a poly (A) stretch or the complement thereof (e.g., practically any doublestranded cDNA clone).

A "nucleic acid construct" or "nucleic acid vector" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules. The terms "expression vector" or "expression construct" refer to nucleotide sequences that are capable of effecting expression of a gene in host cells or host organisms compatible with such sequences. These expression vectors typically include at least suitable transcription regulatory sequences and optionally, 3' transcription termination signals. Additional factors necessary or helpful in effecting expression may also be present, such as expression enhancer elements. The expression vector will be introduced into a suitable host cell and be able to effect expression of the coding sequence in an in vitro cell culture of the host cell. The expression vector will be suitable for replication in the host cell or organism of the invention.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer.

The term "selectable marker" is a term familiar to one of ordinary skill in the art and is used herein to describe any genetic entity which, when expressed, can be used to select for a cell or cells containing the selectable marker. The term "reporter" may be used interchangeably with marker, although it is mainly used to refer to visible markers, such as green fluorescent protein (GFP). Selectable markers may be dominant or recessive or bidirectional.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

The terms "protein" or "polypeptide" are used interchangeably and refer to molecules consisting of a chain of amino acids, without reference to a specific mode of action, size, 3-dimensional structure or origin.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-nontranslated sequence (3'-end) comprising a polyadenylation site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide. The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence and, if applicable, another (heterologous) secretory signal sequence and/or terminator sequence than in its natural environment. It is understood that the regulatory sequences, signal sequences, terminator sequences, etc. may also be homologous to the host cell. In this context, the use of only "homologous" sequence elements allows the construction of "self-cloned" genetically modified organisms (GMO's) (self-cloning is defined herein as in European Directive 98/81/EC Annex II). When used to indicate the relatedness of two nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later.

The terms "heterologous" and "exogenous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous and exogenous nucleic acids or proteins are not endogenous to the cell into which it is introduced, but have been obtained from another cell or synthetically or recombinantly produced. Generally, though not necessarily, such nucleic acids encode proteins, i.e. exogenous proteins, that are not normally produced by the cell in which the DNA is transcribed or expressed. Similarly exogenous RNA encodes for proteins not normally expressed in the cell in which the exogenous RNA is present. Heterologous/exogenous nucleic acids and proteins may also be referred to as foreign nucleic acids or proteins. Any nucleic acid or protein that one of skill in the art would recognize as foreign to the cell in which it is expressed is herein encompassed by the term heterologous or exogenous nucleic acid or protein. The terms heterologous and exogenous also apply to non-natural combinations of nucleic acid or amino acid sequences, i.e. combinations where at least two of the combined sequences are foreign with respect to each other.

The "specific activity" of an enzyme is herein understood to mean the amount of activity of a particular enzyme per amount of total host cell protein, usually expressed in units of enzyme activity per mg total host cell protein. In the context of the present invention, the specific activity of a particular enzyme may be increased or decreased as compared to the specific activity of that enzyme in an (otherwise identical) wild type host cell.

"Furanic compounds" are herein understood to be 2,5-furan-dicarboxylic acid (FDCA) as well as any compound having a furan group which may be oxidized to FDCA, the latter being referred to herein as a "precursor of FDCA" or a "furanic precursor of FDCA". Precursors of FDCA at least include: 5-hydroxymethylfurfural (HMF), 2,5-dihydroxymethyl furan (DHF) or 2,5-bis(hydroxymethyl)furan (BHF) referred to as HMF-OH, 5-hydroxymethyl-2-furancarboxylic acid or 5-hydroxymethyl-2-furoic acid (HMFCA), 5-formyl-2-furoic acid (FFCA), and 2,5-diformyl furan (DFF). It is further understood that in the "furanic compounds", the furan ring or any or its substitutable sidegroup may be substituted, e.g. with OH, C1-C10 alkyl, alkyl, allyl, aryl or RO-ether moiety, including cyclic groups, in the furan ring on any available position.

"Aerobic conditions" "Oxic conditions" or an aerobic or oxic fermentation process is herein defined as conditions or a fermentation process run in the presence of oxygen and in which oxygen is consumed, preferably at a rate of at least 0.5, 1, 2, 5, 10, 20 or 50 mmol/L/h, and wherein organic molecules serve as electron donor and oxygen serves as electron acceptor.

"Anaerobic or anoxic conditions" or an "anaerobic or anoxic fermentation process" is herein defined as conditions or a fermentation process run substantially in the absence of oxygen and wherein organic molecules serve as both electron donor and electron acceptors. Under anoxic conditions substantially no oxygen is consumed, preferably less than 5, 2, 1, or 0.5 mmol/L/h, more preferably 0 mmol/L/h is consumed (i.e. oxygen consumption is not detectable), or substantially no dissolved oxygen can be detected in the fermentation medium, preferably the dissolved oxygen concentration in the medium is less than 2, 1, 0.5, 0.2, 0.1% of air saturation, i.e. below the detection limit of commercial oxygen probes.

Any reference to nucleotide or amino acid sequences accessible in public sequence databases herein refers to the version of the sequence entry as available on the filing date of this document.

### Description of the embodiments

### The parent host cell

The present invention concerns the genetic modification of a host cell so as to enable the host cell to produce 2,5-furandicarboxylic acid (FDCA) from suitable furanic precursors. To this end a number of genetic modifications can be introduced in a parent host cell in accordance with the invention. These modifications include the introduction of expression of a number of heterologous genes, as well as, the modification of the expression of a number of endogenous genes already present in the parent host cell, by reducing or eliminating of some endogenous genes and/or by increasing the expression, i.e. overexpressing, other endogenous genes. These genetic modification are further set out below herein. A parent host cell is thus understood to be a host cell prior to that any of the genetic modifications in accordance with the invention have been introduced in the host cell.

A parent host cell of the invention preferably is a host cell that naturally has the ability to metabolise furanic compounds. More preferably the parent host cell at least has the natural ability to degrade FDCA. The parent host cell of the invention can further have the natural ability to oxidise HMF to FDCA. Whether or not a given host cell strain naturally has the ability to oxidise HMF to FDCA and/or to degrade FDCA can be tested by determining the strain's ability to grow at the expense of one or more of HMF, HMF-alcohol, HMFCA and FDCA, preferably as sole carbon source, as e.g. described in the Examples herein. Preferably, the parent host cell comprises an endogenous gene encoding an enzyme that catalyses the degradation of FDCA. Preferably the endogenous gene is a functional gene expressing an enzyme that catalyses the degradation of FDCA. Preferably, the enzyme that catalyses the degradation of FDCA is an FDCA-decarboxylating monooxygenase as further defined herein.

A parent host cell of the invention can be any suitable host cell including e.g. eukaryotic cells such as a mammalian, insect, plant, fungal, or algal cell. Preferably, however, the host cell is a microbial cell. The microbial host cell can be a prokaryotic cell, preferably a bacterial cell, including e.g. both Gram-negative and Gram-positive microorganisms.

More preferably, however, a parent host cell of the invention is a eukaryotic microbial host cell, such as e.g. a fungal host cell. A most preferred parent host cell to be modified in accordance with the invention is a yeast or filamentous fungal host cell.

"Fungi" are herein defined as eukaryotic microorganisms and include all species of the subdivision Eumycotina (Alexopoulos, C. J., 1962, In: Introductory Mycology, John Wiley & Sons, Inc., New York). The terms "fungus" and "fungal" thus include or refers to both filamentous fungi and yeast.

"Filamentous fungi" are herein defined as eukaryotic microorganisms that include all filamentous forms of the subdivision Eumycotina and Oomycota (as defined in "Dictionary of The Fungi", 10th edition, 2008, CABI, UK, www.cabi.org). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium, Trichoderma,* and *Ustilago.*

Preferred filamentous fungal species as parent host cells for the invention belong to a species of an *Aspergillus, Myceliophthora, Penicillium, Talaromyces* or *Trichoderma* genus, and more preferably a species selected from *Aspergillus niger, Aspergillus awamori, Aspergillus foetidus, Aspergillus sojae, Aspergillus fumigatus, Talaromyces emersonii, Aspergillus oryzae, Myceliophthora thermophila, Trichoderma reesei, Penicillium chrysogenum, Penicillium simplicissimum* and *Penicillium brasilianum.* Suitable strains of these filamentous fungal species are available from depository institutions known per se to the skilled person.

"Yeasts" are herein defined as eukaryotic microorganisms and include all species of the subdivision Eumycotina (Yeasts: characteristics and identification, J.A. Barnett, R.W. Payne, D. Yarrow, 2000, 3rd ed., Cambridge University Press, Cambridge UK; and, The yeasts, a taxonomic study, CP. Kurtzman and J.W. Fell (eds) 1998, 4th ed., Elsevier Science Publ. B.V., Amsterdam, The Netherlands) that predominantly grow in unicellular form. Yeasts may either grow by budding of a unicellular thallus or may grow by fission of the organism. Preferred yeasts cells for use in the present invention belong to the genera *Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula,* and *Sporobolomyces.* A parental yeast host cell can be a cell that is naturally capable of anaerobic fermentation, more preferably alcoholic fermentation and most preferably anaerobic alcoholic fermentation. More preferably yeasts from species such as *Kluyveromyces lactis, S. cerevisiae, Hansenula polymorpha* (new name: *Ogataea henricii*), *Yarrowia lipolytica, Candida tropicalis* and *Pichia pastoris* (new name: *Komagataella pastoris*).

Particularly when compared to bacteria, fungi, have many attractive features for industrial fermentation processes, including e.g. their high tolerance to acids, ethanol and other harmful compounds, their high osmo-tolerance, their high fermentative capacity and for some yeasts their capability of anaerobic growth.

The host cell further preferably has a high tolerance to low pH, i.e. capable of growth at a pH equal to or lower than 5.0, 4.0, 3.5, 3.2, 3.0, 2.9, 2.8, 2.7, 2.6, 2.5, 2.4, 2.3 or 2.28 and towards organic acids like lactic acid, acetic acid or formic acid and furanic acids and a high tolerance to elevated temperatures. Any of these characteristics or activities of the host cell may be naturally present in the host cell or may be introduced or modified by genetic modification, preferably by self cloning or by the methods of the invention described below.

A suitable cell is a cultured cell, a cell that may be cultured in fermentation process e.g. in submerged or solid state fermentation.

For specific uses of a compound produced in a fungal host cell according to the invention, the selection of the host cell may be made according to such use. Where e.g. the compound produced in a host cell according to the invention is to be used in food applications, a host cell may be selected from a food-grade organism such as e.g. a *Saccharomyces* species, e.g. S. *cerevisiae,* a food-grade *Penicillium* species or *Yarrowia lipolitica.* Specific uses include, but are not limited to, food, (animal) feed, pharmaceutical, agricultural such as crop-protection, and/or personal care applications.

### A genetically modified cell

In a first aspect, the invention pertains to a cell, preferably a fungal cell comprising a genetic modification. The genetic modification of the cell preferably is at least a genetic modification that reduces or eliminates the specific activity of an enzyme that catalyses the degradation of 2,5-furandicarboxylic acid (FDCA), as compared to a corresponding wild type cell lacking the genetic modification.

A cell of the invention further preferably comprises one of: a) a genetic modification that confers to the cell the ability to oxidize 5-hydroxymethyl-2-furancarboxylic acid (HMFCA) to 5-formyl-2-furoic acid (FFCA) or that increases in the cell the specific activity of an enzyme that oxidizes HMFCA to FFCA as compared to a corresponding wild type cell lacking the genetic modification; and/or, b) a genetic modification that confers to the cell the ability to oxidize furanic aldehydes to the corresponding furanic carboxylic acids or that increases in the cell the specific activity of an enzyme that oxidizes furanic aldehydes to the corresponding furanic carboxylic acids, as compared to a corresponding wild type cell lacking the genetic modification.

Preferred cells having these genetic modifications are further specified herein below.

### Reducing or eliminating specific 2,5-furandicarboxylic acid (FDCA) decarboxylating monooxygenase activity

A cell of the invention preferably is a cell that lacks the ability to degrade FDCA. A cell of the invention will usually be a genetically modified cell of fungal species that naturally has the ability to degrade FDCA, which cell has been genetically modified to reduce or eliminate its natural ability to degrade FDCA. Whether or not a given fungal strain naturally has the ability to degrade FDCA can be tested by determining the strains ability to grow at the expense of one or more of HMF, HMF-alcohol, HMFCA and FDCA as sole carbon source, as e.g. described in the Examples herein. An example of a fungal species that naturally has the ability to degrade FDCA is *Penicillium brasilianum* as shown in the Examples herein, or *Aspergillus niger* (Rumbold et al., 2010, Bioengineered Bugs 1:5, 359-366). In contrast, yeasts such as *Saccharomyces* and *Yarrowia* species, are examples of fungal species that naturally lack the ability to degrade FDCA.

Thus, in one embodiment of the invention, the cell is genetically modified to reduce or eliminate the cell's natural ability to degrade FDCA. A gene to be modified for reducing or eliminating the cell's ability to degrade FDCA preferably is a gene encoding an FDCA decarboxylating monooxygenase.

A gene encoding an FDCA decarboxylating monooxygenase to be modified for reducing or eliminating the specific FDCA decarboxylating monooxygenase activity in the cell of the invention, preferably is a gene encoding an amino acid sequence with at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity to at least one of SEQ ID NO.: 4 (*hmfK1*). A suitable *hmfK1* orthologue be modified for reducing or eliminating the specific FDCA decarboxylating monooxygenase activity in the cell is e.g. the *Aspergillus niger hmfK1* orthologue with acc. no. XP 001397353.2 (SEQ ID NO: 26). In the cells of the invention, the specific FDCA decarboxylating monooxygenase activity is preferably reduced by at least a factor 1.05, 1.1, 1.2, 1.5, 2.0, 5.0, 10, 20, 50 or 100 as compared to cells of a strain which is genetically identical except for the genetic modification causing the reduction in activity.

Whether or not an orthologue of the *Penicillium brasilianum hmfK1* encodes a polypeptide that has FDCA decarboxylating monooxygenase activity can be assayed by expression of the polypeptide in a suitable host cell that is incapable of degrading FDCA and detecting whether or not expression of the polypeptide confers to the cell the ability to degrade FDCA. FDCA decarboxylating monooxygenase activity can e.g. be assayed using an expression construct wherein a nucleotide sequence encoding the polypeptide to be assayed for FDCA decarboxylating monooxygenase is expressed in a *P.putida* host cell and testing transformant for their ability to degrade FDCA, as is e.g. described in the Examples of PCT/EP2016/072406. Alternatively, the *hmfK1* orthologue can be assayed for its ability to complement a *Penicillium brasilianum hmfK1⁻* disruption mutant as described in the Examples herein by testing for their ability to restore the mutant ability to grow on FDCA as sole carbon source.

The nucleotide sequences of the invention, encoding an FDCA decarboxylating monooxygenase, the specific activities of which are preferably reduced or eliminated in a cell of the invention, are obtainable from and may be identified in genomic and/or cDNA of a fungus, yeast or bacterium, e.g. one that belongs to the same phylum, class or genus as the source organisms described above, using methods for isolation of nucleotide sequences that are well known in the art per se (see e.g. Sambrook and Russell (2001) "Molecular Cloning: A Laboratory Manual (3rd edition), Cold Spring Harbor Laboratory, Cold Spring Harbor Laboratory Press, New York). The nucleotide sequences of the invention are e.g. obtainable in a process wherein a) degenerate PCR primers (designed on the basis of conserved amino acid sequences) are used on genomic and/or cDNA of a suitable organism to generate a PCR fragment comprising part of the nucleotide sequences encoding enzymes the specific activities of which are preferably reduced or eliminated in a cell of the invention; b) the PCR fragment obtained in a) is used as probe to screen a cDNA and/or genomic library of the organism; and c) producing a cDNA or genomic DNA comprising the nucleotide sequence encoding the enzyme the specific activities of which is preferably reduced or eliminated in a cell of the invention. Such conserved sequences can be identified in the sequences alignments presented in Table 4, wherein invariable positions are indicated with a "*" and strongly conserved positions are indicated with a ":". Also suitable host cells of the invention can be derived from Table 4 wherein the host preferably is a non-pathogenic fungus or yeast that belongs to the same phylum, class, order, family or genus as the source organism of an orthologue identified in Table 4. Table 4 presents the amino acid sequence alignments of *Penicillium brasilianum hmfK1* with its 10 closest orthologues as available in public databases. Table 4A provides the percentages amino acid identities among the P. *brasilianum* sequence and its orthologues, as well as the accession numbers of the orthologues.

In a preferred embodiment therefore, the invention pertains to a fungal cell comprising a genetic modification that reduces specific FDCA decarboxylating monooxygenase activity in the cell, as compared to a corresponding parent cell lacking the genetic modification, wherein the genetic modification eliminates the expression of an endogenous gene encoding an FDCA decarboxylating monooxygenase by deletion of at least a part of at least one of the promoter and the coding sequence of the gene from the genome of the cell. Preferably at least a part of the coding sequence of the FDCA decarboxylating monooxygenase gene is deleted from the cell's genome, e.g. at least 10, 20, 30, 40, 50, 60, 70, 80, 90 or 100% of the coding sequence is deleted. A deletion of at least a part of the coding sequence preferably is a deletion that causes a frameshift and/or a deletion that removes the translation initiation codon. Most preferably however at least the complete coding sequence of an endogenous gene encoding an FDCA decarboxylating monooxygenase is deleted.

It is further preferred that in the genetically modified fungal cell of the invention, the expression of all copies of the endogenous gene encoding an FDCA decarboxylating monooxygenase is eliminated. Thus, if the corresponding parental cell lacking the genetic modification comprises more than one endogenous copies of the gene encoding an FDCA decarboxylating monooxygenase, e.g. as a result of di- aneu- or polyploidy and/or gene amplification, preferably the expression of each and every copy of the gene encoding an FDCA decarboxylating monooxygenase is eliminated.

Methods and means for effecting deletion of sequences from fungal genomes are described herein below.

A genetically modified fungal cell of the invention can further comprises a genetic modification that reduces or eliminates the specific activity of another hydroxylase that was found to be induced by HMF, i.e. the *P*. *brasilianum hmfK3* encoded hydroxylase or an orthologue thereof. Although under the conditions used in the Examples herein disruption of the *hmfK3-*encoded hydroxylase in *P*. *brasilianum* did not affect the ability of the fungus to degrade FDCA, this may be different in other fungi and/or under different condition. Under such circumstances it can be useful that a fungal cell of the invention comprises a genetic modification that reduces or eliminates the specific activity of and hydroxylase comprising an amino acid sequence with at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity to at least one of SEQ ID NO.: 23 *(hmfK3).* Preferably, in the cells of the invention, the specific *hmfK3*-encoded hydroxylase activity is preferably reduced by at least a factor 1.05, 1.1, 1.2, 1.5, 2.0, 5.0, 10, 20, 50 or 100 as compared to cells of a strain which is genetically identical except for the genetic modification causing the reduction in activity.

However, the Examples herein show that disruption of the *hmfK1*-encoded FDCA decarboxylating monooxygenase is sufficient for eliminating the cell's ability to degrade FDCA. Therefore, a genetically modified fungal cell comprising an intact and/or functional endogenous *hmfK3* gene or orthologue thereof is expressly included in the invention.

### Introducing or increasing HMFCA dehydrogenase activity

A cell of the invention preferably is a cell that has the ability of oxidizing 5-hydroxymethyl-2-furancarboxylic acid (HMFCA) to 5-formylfuroic acid (FFCA). The cell's ability of oxidizing HMFCA to FFCA can be an endogenous activity of the cell, such as in e.g. *Penicillium brasilianum,* or it can be an exogenous activity to be conferred to the cell, such as e.g. for *Aspergillus niger.* Preferably, the ability of oxidizing HMFCA to FFCA is conferred to the cell or increased in the cell by a genetic modification of the cell, e.g. a transformation of the cell with a nucleic acid construct comprising a nucleotide sequence encoding a dehydrogenase or an oxidase that has the ability to oxidize HMFCA to FFCA. The dehydrogenase preferably is an alcohol dehydrogenase (i.e. having EC 1.1 activity). Thus, the cell is preferably a cell comprising an expression construct for expression of a nucleotide sequence encoding a dehydrogenase or an oxidase that has the ability to oxidize HMFCA to FFCA. In a preferred cell of the invention, the expression construct is expressible in the cell and expression of the dehydrogenase or oxidase preferably confers to in the cell the ability to oxidize HMFCA to FFCA or increases in the cell the specific activity of an enzyme that oxidizes HMFCA to FFCA, as compared to a corresponding cell lacking the expression construct, e.g. a wild type cell. The specific activity of the enzyme that oxidizes HMFCA to FFCA is preferably increased in the cell by at least a factor 1.05, 1.1, 1.2, 1.5, 2.0, 5.0, 10, 20, 50 or 100 as compared to a corresponding cell lacking the expression construct.

The enzyme that has the ability to oxidize HMFCA to FFCA preferably is an alcohol dehydrogenase. A preferred enzyme that has the ability to oxidize HMFCA to FFCA is an alcohol dehydrogenase that has HMFCA dehydrogenase activity. Whether or not a polypeptide has HMFCA dehydrogenase activity can be assayed by expression of the polypeptide in a suitable host cell that is incapable of oxidizing HMFCA to FFCA and detecting whether or not expression of the polypeptide confers to the cell the ability to oxidize HMFCA to FFCA. HMFCA dehydrogenase activity can e.g. be assayed using an expression construct wherein a nucleotide sequence encoding the polypeptide to be assayed for HMFCA dehydrogenase activity replaces the *C*. *basilensis hmfH* gene in pBT'hmfH-adh (described in WO2012/064195), after which the plasmid comprising coding sequence of the polypeptide to be assayed for HMFCA dehydrogenase activity is introduced into *P*. *putida* KT2440Δgcd containing pJNNhmfT1(t) (also described in WO2012/064195). The *P*. *putida* transformants expressing the polypeptide to be assayed for HMFCA dehydrogenase activity are incubated with HMF and samples are drawn at regular intervals for analysis of FDCA. An increase of production of FDCA, as compared to corresponding *P. putida* transformants lacking the polypeptide to be assayed for HMFCA dehydrogenase activity (and the *C*. *basilensis hmfH* gene) is taken as an indication that the polypeptide has HMFCA dehydrogenase activity. Alternatively, a nucleotide sequence encoding the polypeptide to be assayed for HMFCA dehydrogenase activity can be expressed in a fungal host cell, preferably a S. *cerevisiae* host cell, as e.g. described in the Examples of PCT/EP2016/072406 and detecting whether expression of the polypeptide confers to a fungal host cell the ability to produce both FFCA and/or FDCA from HMF.

The HMFCA dehydrogenase expressed in the cell of the invention preferably is a dehydrogenase that is dependent on a cofactor selected from an adenine dinucleotide, such as NADH or NADPH, a flavin adenine dinucleotide (FAD), a flavin mononucleotide (FMN), and pyrroloquinoline quinolone (PQQ). The HMFCA dehydrogenase expressed in the cell of the invention preferably binds a divalent cation, more preferably the HMFCA dehydrogenase is Zn-binding dehydrogenase.

The HMFCA dehydrogenase expressed in the cell of the invention further preferably is an alcohol dehydrogenase that (also) has the ability of oxidizing other furanic alcohols, preferably furanic alcohols with an hydroxy group in the 2-position, to the corresponding aldehydes. Thus, HMFCA dehydrogenase preferably has the ability of oxidizing 5-hydroxymethylfurfural (HMF) to 2,5-diformyl furan (DFF).

In one embodiment the nucleotide sequence encoding the dehydrogenase with the ability to oxidize HMFCA to FFCA is selected from the group consisting of:
(a) a nucleotide sequence encoding a polypeptide with HMFCA dehydrogenase activity, which polypeptide comprises an amino acid sequence that has at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity with the amino acid sequence of at least one of SEQ ID NO.'s: 1 and 2 (*hmfL1* and *hmfL2,* respectively), more preferably SEQ ID NO.: 1;
(b)a nucleotide sequence the complementary strand of which hybridises to a nucleotide sequence of (a); and,
(c) a nucleotide sequence the sequence of which differs from the sequence of a nucleotide sequence of (b) due to the degeneracy of the genetic code.

A preferred nucleotide sequence of the invention thus encodes a HMFCA dehydrogenase with an amino acid sequence that is identical to that of a HMFCA dehydrogenase that is obtainable from (or naturally occurs in) a fungus of a genus selected from the group consisting of *Aspergillus, Byssochlamys, Coccidioides, Chaetomium, Eutypa, Endocarpon, Fusarium, Microsporum, Neosartorya, Penicillium, Sporothrix* and *Trichophyton,* more preferably, a fungus of a species selected from the group consisting of *Coccidioides immitis, Coccidioides posadasii, Endocarpon pusillum, Microsporum gypseum, Penicillium brasilianum* and *Sporothrix schenckii*, most preferably a fungus, which is the strain *P*. *brasilianum* C1 or MG11.

In one embodiment the nucleotide sequence encodes a polypeptide with HMFCA dehydrogenase activity as it occurs in nature, e.g. as it can isolated from a wild type source organism. Alternatively, the nucleotide sequence can encode engineered forms of any of the HMFCA dehydrogenase defined above and that comprise one or more amino acid substitutions, insertions and/or deletions as compared to the corresponding naturally occurring HMFCA dehydrogenase but that are within the ranges of identity or similarity as defined herein. Therefore, in one embodiment the nucleotide sequence of the invention encodes a HMFCA dehydrogenase the amino acid sequence of which at least comprises in each of the invariable positions (that are indicated in Tables 1 and 2 with a "*"), the amino acid present in a invariable position. Preferably, the amino acid sequence also comprises in the strongly conserved positions (that are indicated in Tables 1 and 2 with a ":") one of the amino acids present in a strongly conserved position. More preferably, the amino acid sequence further also comprises in the less strongly conserved positions (that are indicated in Tables 1 and 2 with a ".") one of the amino acids present in a less strongly conserved position. Amino acid substitutions outside of these invariable and conserved positions are less unlikely to affect HMFCA dehydrogenase activity. Tables 1 and 2 present the amino acid sequence alignments of each of *Penicillium brasilianum hmfL1* and *hmfL2,* respectively with their 10 closest orthologues as available in public databases. Tables 1A and 2A provide the percentages amino acid identities among the *P*. *brasilianum* sequences and their orthologues, as well as the accession numbers of the orthologues.

The nucleotide sequences of the invention, encoding polypeptides with HMFCA dehydrogenase activity, are obtainable from genomic and/or cDNA of a fungus, yeast or bacterium, e.g. one that belongs to the same phylum, class or genus as the source organisms described above, using methods for isolation of nucleotide sequences that are well known in the art per se (see e.g. Sambrook and Russell (2001) "Molecular Cloning: A Laboratory Manual (3rd edition), Cold Spring Harbor Laboratory, Cold Spring Harbor Laboratory Press, New York). The nucleotide sequences of the invention are e.g. obtainable in a process wherein a) degenerate PCR primers (designed on the basis of conserved amino acid sequences) are used on genomic and/or cDNA of a suitable organism to generate a PCR fragment comprising part of the nucleotide sequences encoding the polypeptides with HMFCA dehydrogenase activity; b) the PCR fragment obtained in a) is used as probe to screen a cDNA and/or genomic library of the organism; and c) producing a cDNA or genomic DNA comprising the nucleotide sequence encoding a polypeptide with HMFCA dehydrogenase activity.

To increase the likelihood that a HMFCA dehydrogenase of the invention is expressed at sufficient levels and in active form in the cells of the invention, the nucleotide sequence encoding these enzymes, as well as other enzymes of the invention (see below), are preferably adapted to optimise their codon usage to that of the host cell in question. The adaptiveness of a nucleotide sequence encoding a polypeptide to the codon usage of a host cell may be expressed as codon adaptation index (CAI). The codon adaptation index is herein defined as a measurement of the relative adaptiveness of the codon usage of a gene towards the codon usage of highly expressed genes in a particular host cell or organism. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Jansen et al., 2003, Nucleic Acids Res. 31(8):2242-51). An adapted nucleotide sequence preferably has a CAI of at least 0.2, 0.3, 0.4, 0.5, 0.6, 0.7, 0.8 or 0.9. Suitable codon optimised sequences are e.g. listed in SEQ ID NO's: 27 - 29, which have been codon optimised for expression in yeast cells, preferably *S*. *cerevisiae* cells.

The fungal host cell to be transformed with a nucleic acid construct for expression of the nucleotide sequence encoding a HMFCA dehydrogenase of the invention can in principle be any fungal host cell in which the HMFCA dehydrogenase invention can suitably be expressed, preferably in functional, i.e. active form. The fungal host cell of the invention, preferably is a host cell capable of active or passive transport of furanic compounds into as well as out of the cell. A preferred host cell of the invention lacks or has no detectable activities that degrade (e.g. decarboxylate) carboxylated furanic compounds, such as in particular HMFCA, FFCA and FDCA. Such a host cell preferably naturally lacks the ability to degrade carboxylated furanic compounds. Alternatively, a fungal host cell can be genetically modified to reduce or eliminate the specific activities of one or more enzymes that catalyses the degradation of carboxylated furanic compounds, as described herein below.

The expression construct for expression of a nucleotide sequence encoding a HMFCA dehydrogenase of the invention, preferably is an expression construct that is heterologous or exogenous to the host cell transformed with the construct. A construct is herein understood to be heterologous or exogenous to the host cell comprising the construct when the construct comprises at least one sequence or sequence element that does not naturally occur in the host cell and/or when construct comprises at least two sequence elements in a combination and/or order that does not naturally occur in the host cell, even if the elements themselves do naturally occur in the host cell.

Vectors and expression constructs for expression of a nucleotide sequence encoding a HMFCA dehydrogenase of the invention in appropriate host cells are described in more detail herein below.

### Introducing or increasing furanic aldehyde dehydrogenase activity

A cell expressing an HMFCA dehydrogenase of the invention, as described above, further preferably has aldehyde dehydrogenase activity (i.e. having EC 1.2 activity). Preferably, the aldehyde dehydrogenase is capable of converting furanic aldehydes. More preferably the aldehyde dehydrogenase activity is capable of oxidizing furanic aldehydes to the corresponding furanic carboxylic acids. More specifically, the aldehyde dehydrogenase activity is preferably capable of at least one of i) oxidizing HMF to HMFCA, ii) oxidizing 2,5-diformyl furan (DFF) to 5-formyl-2-furoic acid (FFCA), and iii) FFCA into FDCA. Such furanic aldehyde dehydrogenase activity can be an endogenous activity of the cell or it can be an exogenous activity conferred to the cell. Preferably, the furanic aldehyde dehydrogenase activity is conferred to or increased in the cell by transformation of the cell with an expression construct, e.g. a second expression construct if the cell already comprises a first expression construct for expression of the HMFCA dehydrogenase.

In a preferred cell of the invention, the expression construct for expression of the furanic aldehyde dehydrogenase is expressible in the cell and expression of the furanic aldehyde dehydrogenase preferably confers to the ability to oxidize at least one of i) oxidizing HMF to HMFCA, ii) oxidizing DFF to FFCA, and iii) oxidizing FFCA into FDCA, or increases in the cell the specific activity of a furanic aldehyde dehydrogenase with at least one of these abilities, as compared to a corresponding cell lacking the expression construct, e.g. a wild type cell. The specific activity of the furanic aldehyde dehydrogenase is preferably increased in the cell by at least a factor 1.05, 1.1, 1.2, 1.5, 2.0, 5.0, 10, 20, 50 or 100 as compared to a corresponding cell lacking the expression construct.

The ability of a polypeptide to oxidize at least one of i) HMF to HMFCA, ii) oxidizing DFF to FFCA, and iii) FFCA to FDCA, may be assayed by co-expression of a nucleotide sequence encoding the polypeptide in a *P*. *putida* host cell, preferably an *P*. *putida* KT2440 host cell, together with the *HmfH* and *HmfT1* genes from *C*. *basilensis* HMF 14, incubating the *P*. *putida* cells in 10mM HMF and detecting an increase in the accumulation FDCA as compared to corresponding *P*. *putida* cells that do not express the polypeptide, e.g. as described in Example IV of WO2012/064195. The ability of a polypeptide to oxidize HMF to HMFCA may also be assayed as described by Koopman et al 2010, PNAS *supra*). Strains expressing the *hmtT1* gene from *C*. *basilensis* HMF14 are herein understood to express a gene product having the amino acid sequence of SEQ ID NO: 55. Alternatively, a nucleotide sequence encoding the polypeptide to be assayed for its ability to oxidize at least one of i) HMF to HMFCA, ii) oxidizing DFF to FFCA, and iii) FFCA to FDCA can be co-expressed in a fungal host cell, preferably a *S*. *cerevisiae* host cell, with an HMFCA dehydrogenase as e.g. described in the Examples of PCT/EP2016/072406 and detecting whether expression of the polypeptide causes an increase in the accumulation FDCA as compared to corresponding fungal host cells that do not express the polypeptide.

The furanic aldehyde dehydrogenase expressed in the cell of the invention preferably is a dehydrogenase that is dependent on a cofactor selected from an adenine dinucleotide, such as NADH or NADPH, a flavin adenine dinucleotide (FAD), a flavin mononucleotide (FMN), and pyrroloquinoline quinolone (PQQ).

In one embodiment, the nucleotide sequence encoding the furanic aldehyde dehydrogenase is selected from the group consisting of:
a) a nucleotide sequence encoding a polypeptide having at least one of the abilities of i) oxidizing HMF to HMFCA, ii) oxidizing DFF to FFCA, and, iii) oxidizing FFCA into FDCA, which polypeptide comprising an amino acid sequence that has at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity with the amino acid sequence of at least one of SEQ ID NO's: 3 (the *hmfN1*-encoded aldehyde dehydrogenase);
b) a nucleotide sequence the complementary strand of which hybridises to a nucleotide sequence of (a); and,
c) a nucleotide sequence the sequence of which differs from the sequence of a nucleotide sequence of (b) due to the degeneracy of the genetic code.

A preferred nucleotide sequence of the invention thus encodes a furanic aldehyde dehydrogenase with an amino acid sequence that is identical to that of a furanic aldehyde dehydrogenase that is obtainable from (or naturally occurs in) a fungus of a genus selected from the group consisting of *Aspergillus, Eutypa, Neosartorya, Penicillium, Podospora, Scedosporium* and *Sporothrix,* more preferably, a fungus of a species selected from the group consisting of *Eutypa lata, Penicillium brasilianum, Podospora anserina, Scedosporium apiospermum* and *Sporothrix schenckii,* most preferably a fungus, which is the strain *P*. *brasilianum* C1 or MG11.

In one embodiment the nucleotide sequence encodes a polypeptide with furanic aldehyde dehydrogenase activity as it occurs in nature, e.g. as it can isolated from a wild type source organism. Alternatively, the nucleotide sequence can encode engineered forms of any of the furanic aldehyde dehydrogenase defined above and that comprise one or more amino acid substitutions, insertions and/or deletions as compared to the corresponding naturally occurring furanic aldehyde dehydrogenase but that are within the ranges of identity or similarity as defined herein. Therefore, in one embodiment the nucleotide sequence of the invention encodes a furanic aldehyde dehydrogenase, the amino acid sequence of which at least comprises in each of the invariable positions (that are indicated in Table 3 with a "*"), the amino acid present in a invariable position. Preferably, the amino acid sequence also comprises in the strongly conserved positions (that are indicated in Table 3 with a ":") one of the amino acids present in a strongly conserved position. More preferably, the amino acid sequence further also comprises in the less strongly conserved positions (that are indicated in Table 3 with a ".") one of the amino acids present in a less strongly conserved position. Amino acid substitutions outside of these invariable and conserved positions are less unlikely to affect furanic aldehyde dehydrogenase activity. Table 3 present the amino acid sequence alignments of *Penicillium brasilianum hmfN1* with its 10 closest orthologues as available in public databases. Tables 3A provide the percentages amino acid identities among the *P*. *brasilianum* sequence and its orthologues, as well as the accession numbers of the orthologues.

The nucleotide sequences of the invention, encoding polypeptides with furanic aldehyde dehydrogenase activity, are obtainable from genomic and/or cDNA of a fungus, yeast or bacterium, e.g. one that belongs to the same phylum, class or genus as the source organisms described above, using methods for isolation of nucleotide sequences that are well known in the art per se (see e.g. Sambrook and Russell (2001) "Molecular Cloning: A Laboratory Manual (3rd edition), Cold Spring Harbor Laboratory, Cold Spring Harbor Laboratory Press, New York). The nucleotide sequences of the invention are e.g. obtainable in a process wherein a) degenerate PCR primers (designed on the basis of conserved amino acid sequences) are used on genomic and/or cDNA of a suitable organism to generate a PCR fragment comprising part of the nucleotide sequences encoding the polypeptides with furanic aldehyde dehydrogenase activity; b) the PCR fragment obtained in a) is used as probe to screen a cDNA and/or genomic library of the organism; and c) producing a cDNA or genomic DNA comprising the nucleotide sequence encoding a polypeptide with furanic aldehyde dehydrogenase activity.

The fungal host cell to be transformed with a nucleic acid construct for expression of the nucleotide sequence encoding a furanic aldehyde dehydrogenase of the invention preferably is a fungal host cell as described above for transformation with a nucleic acid construct for expression of the nucleotide sequence encoding the HMFCA dehydrogenase, and wherein also the furanic aldehyde dehydrogenase can suitably be expressed, preferably in functional, i.e. active form. Preferably, the fungal host cell to be transformed with a nucleic acid construct for expression of the nucleotide sequence encoding a furanic aldehyde dehydrogenase also expresses nucleotide sequence encoding the HMFCA dehydrogenase, more preferably the cell comprises an expression construct for the HMFCA dehydrogenase that confers to or increases in the cell the ability to oxidize HMFCA to FFCA. As described above, such a fungal host cell, preferably is capable of active or passive transport of furanic compounds into as well as out of the cell and preferably lacks or has no detectable activities that degrade (e.g. decarboxylate) carboxylated furanic compounds.

The expression construct for expression of a nucleotide sequence encoding a furanic aldehyde dehydrogenase of the invention, preferably is an expression construct that is heterologous or exogenous to the host cell transformed with the construct. A construct is herein understood to be heterologous or exogenous to the host cell comprising the construct when the construct comprises at least one sequence or sequence element that does not naturally occur in the host cell and/or when construct comprises at least two sequence elements in a combination and/or order that does not naturally occur in the host cell, even if the elements themselves do naturally occur in the host cell.

Vectors and expression constructs for expression of a nucleotide sequence encoding a furanic aldehyde dehydrogenase of the invention in appropriate host cells are described in more detail herein below.

### Reduction or elimination of alternative routes for metabolism of furanic compounds

Alternative endogenous routes for metabolism of HMF and other furanic precursors of FDCA may also be present in a cell of the invention. Such alternative routes compete with the production of FDCA from HMF and other furanic precursors of FDCA. Preferably therefore the specific activity of enzymes in such alternative routes is also reduced or eliminated in a cell of the invention. One such endogenous alternative route is e.g. the reduction of HMF and/or FFCA to the corresponding alcohol by an dehydrogenase, such as e.g. a short chain dehydrogenase. A gene encoding such a short chain dehydrogenase to be modified for reducing or eliminating the specific activity of an alternative route for metabolising HMF and other furanic precursors of FDCA, preferably is a gene encoding an amino acid sequence with at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity to SEQ ID NO: 5 (*hmfM*)*.* In the cells of the invention, the specific short chain dehydrogenase activity is preferably reduced by at least a factor 1.05, 1.1, 1.2, 1.5, 2.0, 5.0, 10, 20, 50 or 100 as compared to cells of a strain which is genetically identical except for the genetic modification causing the reduction in activity.

Nucleotide sequences encoding short chain dehydrogenase the specific activities of which are preferably reduced or eliminated in a cell of the invention, are obtainable from and may be identified in genomic and/or cDNA of a fungus, yeast or bacterium, e.g. one that belongs to the same phylum, class or genus as the source organisms described above, using methods for isolation of nucleotide sequences that are well known in the art per se (see e.g. Sambrook and Russell (2001) "Molecular Cloning: A Laboratory Manual (3rd edition), Cold Spring Harbor Laboratory, Cold Spring Harbor Laboratory Press, New York). The nucleotide sequences of the invention are e.g. obtainable in a process wherein a) degenerate PCR primers (designed on the basis of conserved amino acid sequences) are used on genomic and/or cDNA of a suitable organism to generate a PCR fragment comprising part of the nucleotide sequences encoding enzymes the specific activities of which are preferably reduced or eliminated in a cell of the invention; b) the PCR fragment obtained in a) is used as probe to screen a cDNA and/or genomic library of the organism; and c) producing a cDNA or genomic DNA comprising the nucleotide sequence encoding the enzyme the specific activities of which is preferably reduced or eliminated in a cell of the invention. Such conserved sequences can be identified in the sequences alignments presented in Table 5, wherein invariable positions are indicated with a "*" and strongly conserved positions are indicated with a ":". Also suitable host cells of the invention can be derived from Table 5 wherein the host preferably is a non-pathogenic fungus or yeast that belongs to the same phylum, class, order, family or genus as the source organism of an orthologue identified in Table 5. Table 5 presents the amino acid sequence alignments of each of *Penicillium brasilianum hmfM* with its 10 closest orthologues as available in public databases. Table 5A provides the percentages amino acid identities among the *P*. *brasilianum hmfM* sequence and its orthologues, as well as the accession numbers of the orthologues.

Another endogenous dehydrogenase known to reduce HMF to HMF-alcohol is the NADPH-dependent alcohol dehydrogenase encoded by the *S*. *cerevisiae ADH6* gene as described by Petersson et al. (2006, Yeast, 23:455-464). Therefore, a gene to be modified for reducing or eliminating the specific activity of alternative route for metabolising HMF, preferably is the *S*. *cerevisiae ADH6* gene or an orthologue thereof in another fungal host species. Orthologues of the *S*. *cerevisiae ADH6* gene in filamentous fungi such as *Apergillus* and *Penicillium* with amino acid sequence identities in the range of 50-60% can be identified in public sequence databases. Preferably therefore, the gene to be modified for reducing or eliminating the specific activity of an NADPH-dependent HMF-reducing dehydrogenase is a gene encoding an amino acid sequence with at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity to SEQ ID NO: 25 (*S. cerevisiae ADH6*). In the cells of the invention, the activity specific of the NADPH-dependent HMF-reducing dehydrogenase is preferably reduced by at least a factor 1.05, 1.1, 1.2, 1.5, 2.0, 5.0, 10, 20, 50 or 100 as compared to cells of a strain which is genetically identical except for the genetic modification causing the reduction in activity.

### Cells expressing a transporter of furanic compounds

A cell of the invention, as described above, further preferably expresses one or more nucleotide sequences encoding a polypeptide having furanic compound transport capabilities. Such polypeptides having furanic compound transport capabilities can be an endogenous activity of the cell or it can be an exogenous activity conferred to the cell. Preferably, the activity of a polypeptides having furanic compound transport capabilities is conferred to or increased in the cell by transformation of the cell with an expression construct, e.g. a third expression construct if the cell already comprises a first expression construct for expression of the HMFCA dehydrogenase and a second expression construct for expression of the furanic aldehyde dehydrogenase or oxidase.

Preferably the cell is transformed with an expression construct for expression of a nucleotide sequence encoding a polypeptide having furanic compound transport capabilities. The polypeptide having furanic compound transport capabilities preferably is a polypeptide having HMFCA transport capabilities, which at least includes the capability to transport HMFCA into the cell. Preferably the cell comprises an expression construct for expression of a nucleotide sequence encoding a polypeptide having the ability to transport at least HMFCA into the cell, the polypeptide comprising an amino acid sequence with at 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity to at least one of SEQ ID NO: 6 - 10 (respectively, *hmfT3*, *hmfT4*, *hmfT5*, *hmtT6*, and *hmfT7*), wherein, the expression construct is expressible in the cell and expression of the polypeptide confers to or increases in the cell the ability to transport at least HMFCA into the cell, as compared to a corresponding wild type cell lacking the expression construct.

The ability of a polypeptide to transport furanic compounds, in particular HMFCA, into the cell may be assayed by co-expression of a nucleotide sequence encoding the transporter polypeptide in a yeast host cell, preferably a *S*. *cerevisiae* CEN.PK host cell, together with the *hmfH* gene from *C*. *basilensis* HMF 14 and a gene encoding a furanic aldehyde dehydrogenase associated with the HMF-degradation operon from *C*. *basilensis* HMF 14 (having the amino acid sequence of SEQ ID NO: 19 of WO2012/064195), incubating the transformed *S*. *cerevisiae* cells in 4 mM HMF and detecting an increase in the accumulation FDCA as compared to corresponding (i.e. otherwise identical) *S*. *cerevisiae* cells that do not express the transporter polypeptide, as e.g. described in the Examples of PCT/EP2016/072406.

A preferred nucleotide sequence of the invention thus encodes a furanic compound transporter polypeptide with an amino acid sequence that is identical to that of a furanic compound transporter polypeptide that is obtainable from (or naturally occurs in) a fungus of a genus selected from the group consisting of *Aspergillus, Fusarium, Nectria, Penicillium*, *Sporothrix* and *Togninia*, more preferably, a fungus of a species selected from the group consisting of *Aspergillus terreus, Penicillium brasilianum, Penicillium digitatum, Penicillium rubens, Sporothrix schenckii* and *Togninia minima*, most preferably a fungus, which is the strain *P. brasilianum* C1 or MG11.

In one embodiment the nucleotide sequence encodes a furanic compound transporter polypeptide as it occurs in nature, e.g. as it can isolated from a wild type source organism. Alternatively, the nucleotide sequence can encode engineered forms of any of the furanic compound transporter polypeptides defined above and that comprise one or more amino acid substitutions, insertions and/or deletions as compared to the corresponding naturally occurring furanic compound transporter polypeptide but that are within the ranges of identity or similarity as defined herein. Therefore, in one embodiment the nucleotide sequence of the invention encodes a furanic compound transporter polypeptide, the amino acid sequence of which at least comprises in each of the invariable positions (that are indicated in Tables 6 - 10 with a "*"), the amino acid present in a invariable position. Preferably, the amino acid sequence also comprises in the strongly conserved positions (that are indicated in Tables 6 - 10 with a ":") one of the amino acids present in a strongly conserved position. More preferably, the amino acid sequence further also comprises in the less strongly conserved positions (that are indicated in Tables 6 - 10 with a ".") one of the amino acids present in a less strongly conserved position. Amino acid substitutions outside of these invariable and conserved positions are less unlikely to affect furanic compound transporter polypeptide activity. Tables 6 - 10 present the amino acid sequence alignments of each of *Penicillium brasilianum hmfT3, hmfT4, hmfT5, hmfT6* and *hmfT7*, respectively, with their 10 closest orthologues as available in public databases. Tables 6A - 10A provide the percentages amino acid identities among the *P*. *brasilianum* sequences and their orthologues, as well as the accession numbers of the orthologues.

The nucleotide sequences of the invention, encoding polypeptides with furanic compound transporter activity, are obtainable from genomic and/or cDNA of a fungus, yeast or bacterium, e.g. one that belongs to the same phylum, class or genus as the source organisms described above, using methods for isolation of nucleotide sequences that are well known in the art per se (see e.g. Sambrook and Russell (2001) "Molecular Cloning: A Laboratory Manual (3rd edition), Cold Spring Harbor Laboratory, Cold Spring Harbor Laboratory Press, New York). The nucleotide sequences of the invention are e.g. obtainable in a process wherein a) degenerate PCR primers (designed on the basis of conserved amino acid sequences) are used on genomic and/or cDNA of a suitable organism to generate a PCR fragment comprising part of the nucleotide sequences encoding the polypeptides with the activity of a furanic compound transporter; b) the PCR fragment obtained in a) is used as probe to screen a cDNA and/or genomic library of the organism; and c) producing a cDNA or genomic DNA comprising the nucleotide sequence encoding a furanic compound transporter polypeptide.

The fungal host cell to be transformed with a nucleic acid construct for expression of the nucleotide sequence encoding a furanic compound transporter polypeptide preferably is a fungal host cell of the invention as described above.

The expression construct for expression of a nucleotide sequence encoding a furanic compound transporter polypeptide, preferably is an expression construct that is heterologous or exogenous to the host cell transformed with the construct. A construct is herein understood to be heterologous or exogenous to the host cell comprising the construct when the construct comprises at least one sequence or sequence element that does not naturally occur in the host cell and/or when construct comprises at least two sequence elements in a combination and/or order that does not naturally occur in the host cell, even if the elements themselves do naturally occur in the host cell.

In an separate aspect, the invention relates to a fungal cell having the ability to oxidise HMF to FDCA and comprising a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 9 and 10. Preferably, the fungal cell further comprises one or more genetic modifications selected from: a) a genetic modification that eliminates or reduces specific 2,5-furandicarboxylic acid (FDCA) decarboxylating monooxygenase activity in the cell, as compared to a corresponding parent cell lacking the genetic modification, as described herein above; b) a genetic modification that confers to the cell the ability to oxidize 5-hydroxymethyl-2-furancarboxylic acid (HMFCA) to 5-formyl-2-furoic acid (FFCA) or that increases in the cell the specific activity of a enzyme that oxidizes HMFCA to FFCA as compared to a corresponding wild type cell lacking the genetic modification, as described herein above; c) a genetic modification that confers to the cell the ability to oxidize furanic aldehydes to the corresponding furanic carboxylic acids or a genetic modification that increases in the cell the specific activity of a enzyme that oxidizes furanic aldehydes to the corresponding furanic carboxylic acids, as compared to a corresponding wild type cell lacking the genetic modification, as described herein above; d) a genetic modification that reduces or eliminates the expression of a gene encoding a short chain dehydrogenase that reduces HMF and/or FFCA to the corresponding alcohol, wherein preferably the gene is at least one of a gene encoding polypeptide comprising an amino acid sequence with at least 45% sequence identity to at least one of SEQ ID NO: 5 and 25, as described herein above; e) a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 6 - 8, as described herein above; and, f) a genetic modification that alters the expression of a gene encoding a transcriptional activator of genes involved in furan catabolism, wherein preferably the gene is a gene encoding a polypeptide comprising an amino acid sequence with at least 45% sequence identity to SEQ ID NO: 11, as described herein below.

Vectors and expression constructs for expression of a nucleotide sequence encoding a furanic compound transporter polypeptide of the invention in appropriate host cells are described in more detail herein below.

### Cell with altered regulation of expression of a transcriptional activator

In one embodiment of a cell of the invention, the regulation of expression of a transcriptional activator of genes involved in furan catabolism is altered. The expression of the transcriptional activator can be reduced or eliminated to prevent degradation of FDCA in cells containing endogenous genes for FDCA degradation, and preferably containing genes coding for enzymes for converting HMF to FDCA that expressed independent from the transcriptional activator. Alternatively, the expression of the transcriptional activator can be increased and/or be made constitutive in cells genetically modified to prevent FDCA degradation, so as to increase expression of endogenous genes for converting HMF, and/or other furanic precursors, to FDCA.

Preferably, in a cell of the invention, the transcriptional activator of which the regulation of expression is altered, is encoded by a nucleotide sequence encoding a polypeptide comprising an amino acid sequence with at least 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 95, 96, 97, 98, 99 or 100% sequence identity to SEQ ID NO: 11 (*hmfR*), wherein, the polypeptide has the ability to activate transcription of at least one gene involved in furan catabolism.

A preferred nucleotide sequence of the invention thus encodes a transcriptional activator with an amino acid sequence that is identical to that of a transcriptional activator that is obtainable from (or naturally occurs in) a fungus of a genus selected from the group consisting of *Fusarium*, *Penicillium, Scedosporium, Sporothrix* and *Stachybotrys* more preferably, a fungus of a species selected from the group consisting of *Fusarium oxysporum, Penicillium brasilianum, Scedosporium apiospermum, Sporothrix schenckii* and *Stachybotrys chlorohalonata,* most preferably a fungus, which is the strain *P*. *brasilianum* C1 or MG11.

In one embodiment the nucleotide sequence encodes a transcriptional activator as it occurs in nature, e.g. as it can isolated from a wild type source organism. Alternatively, the nucleotide sequence can encode engineered forms of any of the transcriptional activator polypeptides defined above and that comprise one or more amino acid substitutions, insertions and/or deletions as compared to the corresponding naturally occurring transcriptional activator polypeptide but that are within the ranges of identity or similarity as defined herein. Therefore, in one embodiment the nucleotide sequence of the invention encodes a transcriptional activator polypeptide, the amino acid sequence of which at least comprises in each of the invariable positions (that are indicated in Table 11 with a "*"), the amino acid present in a invariable position. Preferably, the amino acid sequence also comprises in the strongly conserved positions (that are indicated in Table 11 with a ":") one of the amino acids present in a strongly conserved position. More preferably, the amino acid sequence further also comprises in the less strongly conserved positions (that are indicated in Table 11 with a ".") one of the amino acids present in a less strongly conserved position. Amino acid substitutions outside of these invariable and conserved positions are less unlikely to affect transcriptional activator activity. Table 11 presents the amino acid sequence alignment of *Penicillium brasilianum* hmfR, with its 10 closest orthologues as available in public databases. Table 11A provides the percentages amino acid identities among the *P*. *brasilianum* sequence and its orthologues, as well as the accession numbers of the orthologues.

The nucleotide sequences of the invention, encoding polypeptides with transcriptional activator activity, are obtainable from genomic and/or cDNA of a fungus, yeast or bacterium, e.g. one that belongs to the same phylum, class or genus as the source organisms described above, using methods for isolation of nucleotide sequences that are well known in the art per se (see e.g. Sambrook and Russell (2001) "Molecular Cloning: A Laboratory Manual (3rd edition), Cold Spring Harbor Laboratory, Cold Spring Harbor Laboratory Press, New York). The nucleotide sequences of the invention are e.g. obtainable in a process wherein a) degenerate PCR primers (designed on the basis of conserved amino acid sequences) are used on genomic and/or cDNA of a suitable organism to generate a PCR fragment comprising part of the nucleotide sequences encoding the polypeptides with the activity of a transcriptional activator; b) the PCR fragment obtained in a) is used as probe to screen a cDNA and/or genomic library of the organism; and c) producing a cDNA or genomic DNA comprising the nucleotide sequence encoding a furanic transcriptional activator.

The fungal host cell to be transformed with a nucleic acid construct for expression of the nucleotide sequence encoding a furanic transcriptional activator polypeptide preferably is a fungal host cell of the invention as described above.

The expression construct for expression of a nucleotide sequence encoding a furanic transcriptional activator polypeptide, preferably is an expression construct that is heterologous or exogenous to the host cell transformed with the construct. A construct is herein understood to be heterologous or exogenous to the host cell comprising the construct when the construct comprises at least one sequence or sequence element that does not naturally occur in the host cell and/or when construct comprises at least two sequence elements in a combination and/or order that does not naturally occur in the host cell, even if the elements themselves do naturally occur in the host cell.

Vectors and expression constructs for expression of a nucleotide sequence encoding a furanic transcriptional activator polypeptide of the invention in appropriate host cells are described in more detail herein below.

### Vectors, genetic constructs and methods for genetic modifications of cells of the invention

For the genetic modification of the parent host cells of the invention, i.e. for the construction of the modified host cells of the invention, standard genetic and molecular biology techniques are used that are generally known in the art and have e.g. been described by Sambrook and Russell (2001, "Molecular cloning: a laboratory manual" (3rd edition), Cold Spring Harbor Laboratory, Cold Spring Harbor Laboratory Press) and Ausubel et al. (1987, eds., "Current protocols in molecular biology", Green Publishing and Wiley Interscience, New York).

More specifically, means and methods for genetic modification of yeasts are standard and known to those in the art, including e.g. promoters for (over-)expression of genes, episomal and/or integrating expression constructs and vectors, selectable markers, methods and genetic constructs for disrupting and/or deleting endogenous yeast genes or parts thereof and methods for transforming yeast. Such means and methods are e.g. described in: Sherman et al, Methods Yeast Genetics, Cold Spring Harbor Laboratory, NY (1978); Guthrie et al. (Eds.) Guide To Yeast Genetics and Molecular Biology Vol. 194, Academic Press, San Diego (1991); Sudbery, P. E. (2001) Genetic Engineering of Yeast, in Biotechnology Set, Second Edition (eds H.-J. Rehm and G. Reed), Wiley-VCH Verlag GmbH, Weinheim, Germany. doi: 10.1002/9783527620999.ch13a; and, Gaillardin, C. and Heslot, H. (1988), Genetic engineering in Yarrowia lipolytica. J. Basic Microbiol., 28: 161-174. doi: 10.1002/jobm.3620280303; all of which are incorporated herein by reference.

Similarly, means and methods for genetic modification of filamentous fungi are standard and known to those in the art, including e.g. promoters for (over-)expression of genes, episomal and/or integrating expression constructs and vectors, selectable markers, and methods and genetic constructs for disrupting and/or deleting endogenous fungal genes or parts thereof and methods for transforming filamentous fungi. Such means and methods are e.g. described in Moore, M. M. (2007, "Genetic engineering of fungal cells", In Biotechnology Vol. III. (Ed. H. W. Doelle and E. J. Dasilva), EOLSS, Ontario, Canada. pp. 36-63; Lubertozzi, D., & Keasling, J. D. (2009), "Developing Aspergillus as a host for heterologous expression", Biotechnology advances, 27(1), 53-75; Meyer, V. (2008) "Genetic engineering of filamentous fungi--progress, obstacles and future trends", Biotechnology Advances, (26), 177-85; Kück and Hoff (2010) "New tools for the genetic manipulation of filamentous fungi. Applied microbiology and biotechnology", 86(1), 51-62; and, WO2014/142647, all of which are incorporated herein by reference.

Thus in another aspect, the invention pertains to nucleic acid constructs, such as vectors, including cloning and expression vectors, comprising a polynucleotide of the invention, e.g. a nucleotide sequence encoding a HMFCA dehydrogenase or a furanic aldehyde dehydrogenase of the invention or a functional equivalent thereof and methods of transforming or transfecting a suitable host cell with such vectors. As used herein, the terms "vector" and "construct" are used interchangeably and refers to a constructed nucleic acid molecule comprising a polynucleotide of the invention.

A vector according to the invention may be an autonomously replicating vector, i.e. a vector which exists as an extra-chromosomal entity, the replication of which is independent of chromosomal replication, e.g. a plasmid. Alternatively, the vector may be one which, when introduced into a host cell, is integrated into the host cell genome and replicated together with the chromosome (s) into which it has been integrated. For convenience the vector can be a shuttle vector, also comprising a origin of replication and selectable marker for use in a bacterium such as *E.coli*, for ease of manipulation and production.

In one embodiment, the nucleic acid constructs is an expression construct or expression vector, comprising a nucleotide sequence encoding a polypeptide of the invention to be (over-) expressed and wherein the nucleotide sequence encoding the polypeptide is operably linked to regulatory sequences that are capable of effecting and controlling (the rate of) expression of the coding nucleotide sequence in the host cells in question. Such regulatory sequences typically at least include a promoter that functions to control the transcription of the coding sequence, which is usually located upstream of, and preferably operably linked the coding sequence. In addition to the promoter, the upstream transcription regulatory sequences may comprises further elements such as enhancers, upstream activating sequences, transcription factor binding sites, repressor and activator protein binding sites and the like. The promoter sequence will usually include the transcription initiation site(s). Suitable promoters and transcription regulatory sequences for expression of coding sequences in yeast or filamentous fungi are described in the above-cited references. Downstream of the promoter and transcription initiation site(s), the expression construct will comprise the translation initiation sequences, such as the eukaryotic Kozak consensus sequence, surrounding the translation initiation codon, i.e. the first codon of the coding sequence. The coding sequence is terminated with a translation stop codon. Downstream of the coding sequence, the expression construct may comprise a 3'-untranslated region containing one or more transcription termination sites, e.g. a terminator, which preferably also includes a polyadenylation site. The origin of the terminator is less critical. The terminator can, for example, be native to the DNA sequence encoding the polypeptide. However, preferably a yeast terminator is used in yeast host cells and a filamentous fungal terminator is used in filamentous fungal host cells. More preferably, the terminator is endogenous to the host cell (in which the nucleotide sequence encoding the polypeptide is to be expressed). A functional expression unit comprising a coding sequence operably linked to the appropriate upstream- and downstream regulatory sequences may be referred to as an expression cassette. An expression vector or expression construct of the invention may comprise more than one expression cassette, optionally for the expression of more than one different coding sequences.

In addition to at least one expression cassette, an expression vector or expression construct of the invention preferably also comprises a selectable marker for selection of host cells transformed with the vector or construct. In a preferred embodiment, the selectable marker in the expression vector or expression construct in a configuration that allows excision of the marker from the expression construct/vector, once in the host cell after initial selection of the transformants, e.g. using homologous recombination as described in EP 0 635 574, or using the *Cre-lox* system as described by Güldener et al. (1996, Nucleic Acids Res. 24:2519-2524).

The invention further relates to method for the preparation of a polypeptide of the invention, e.g. a polypeptide having HMFCA dehydrogenase activity, a polypeptide having furanic aldehyde dehydrogenase activity and including polypeptides the expression of which is to be reduced/eliminated in the cell of the invention. The method comprises cultivating a cell according to the invention under conditions conducive to expression of the polypeptide and, optionally, recovering the expressed polypeptide. The invention also relates to a polypeptide obtainable by such a method.

Thus in another aspect, the invention pertains to means and methods for modifying endogenous target genes in the cells of the invention so as to reduce or eliminate the expression and/or activity of the encoded target proteins. Modifications that may be used to reduce or eliminate expression of a target protein are disruptions that include, but are not limited to, deletion of the entire gene or a portion of the gene encoding the target protein, inserting a DNA fragment into the target gene (in either the promoter or coding region) so that the protein is not expressed or expressed at lower levels, introducing a mutation into the target coding region which adds a stop codon or frame shift such that a functional protein is not expressed, and introducing one or more mutations into a target coding region to alter amino acids so that a non-functional target protein, or a target protein with reduced enzymatic activity is expressed. In addition, expression of the target gene may be blocked by expression of an antisense RNA or an interfering RNA, and constructs may be introduced that result in co-suppression. Moreover, a target coding sequence may be synthesized whose expression will be low because rare codons are substituted for plentiful ones, when this suboptimal coding sequence is substituted for the corresponding endogenous target coding sequence. Preferably such a suboptimal coding sequence will have a codon adaptation index (see above) of less than 0.5, 0.4, 0.3 0.2, or 0.1. Such a suboptimal coding sequence will produce the same polypeptide but at a lower rate due to inefficient translation. In addition, the synthesis or stability of the transcript may be reduced by mutation. Similarly the efficiency by which a protein is translated from mRNA may be modulated by mutation, e.g. by using suboptimal translation initiation codons. All of these methods may be readily practiced by one skilled in the art making use of the sequences encoding target proteins.

In particular, genomic DNA sequences surrounding a target coding sequence are useful for modification methods using homologous recombination. For example, in this method sequences flanking the target gene are placed on either site of a selectable marker gene to mediate homologous recombination whereby the marker gene replaces the target gene. Also partial target gene sequences and target gene flanking sequences bounding a selectable marker gene may be used to mediate homologous recombination whereby the marker gene replaces a portion of the target gene. In addition, the selectable marker in the inactivation construct can be configured in such a way so as to allow excision of the marker from the inactivation construct expression construct/vector, once integrated in the host cell's genome, e.g. using homologous recombination as described in EP 0 635 574, or using the *Cre-lox* system as described by Güldener et al. (1996, Nucleic Acids Res. 24:2519-2524).

Deletions of target genes may also be effected using mitotic recombination as described in Wach et al. (1994, Yeast 10:1793-1808). This method involves preparing a DNA fragment that contains a selectable marker between genomic regions that may be as short as 20 bp, and which bound, i.e. flank the target DNA sequence. This DNA fragment can be prepared by PCR amplification of the selectable marker gene using as primers oligonucleotides that hybridize to the ends of the marker gene and that include the genomic regions that can recombine with the fungal genome. The linear DNA fragment can be efficiently transformed into yeast or filamentous fungi and recombined into the genome resulting in gene replacement including with deletion of the target DNA sequence (as described in Methods in Enzymology, 1991, v 194, pp 281-301). Moreover, promoter replacement methods may be used to exchange the endogenous transcriptional control elements allowing another means to modulate expression such as described in Mnaimneh et al. (2004, Cell 118(1):31-44) and in the Examples herein.

In addition, the activity of target proteins or genes in any cell may be disrupted using random mutagenesis, which is followed by screening to identify strains with reduced activity of the target proteins. Using this type of method, the DNA sequence coding for the target protein, or any other region of the genome affecting expression of the target protein, need not even be known. Methods for creating genetic mutations are common and well known in the art and may be applied to the exercise of creating mutants. Commonly used random genetic modification methods (reviewed in Methods in Yeast Genetics, 2005, Cold Spring Harbor Laboratory Press, Cold Spring Harbor, N.Y.) include spontaneous mutagenesis, mutagenesis caused by mutator genes, chemical mutagenesis, irradiation with UV or X-rays, or transposon mutagenesis.

Chemical mutagenesis of fungi commonly involves treatment of cells with one of the following DNA mutagens: ethyl methanesulfonate (EMS), nitrous acid, diethyl sulfate, or N-methyl-N'-nitro-N-nitroso-guanidine (MNNG). These methods of mutagenesis have been reviewed in Spencer et al (Mutagenesis in Yeast, 1996, Yeast Protocols: Methods in Cell and Molecular Biology. Humana Press, Totowa, N.J.). Chemical mutagenesis with EMS may be performed as described in Methods in Yeast Genetics, 2005, Cold Spring Harbor Laboratory Press, Cold Spring Harbor, N.Y. Irradiation with ultraviolet (UV) light or X-rays can also be used to produce random mutagenesis in yeast cells. The primary effect of mutagenesis by UV irradiation is the formation of pyrimidine dimers which disrupt the fidelity of DNA replication. Protocols for UV-mutagenesis of yeast can be found in Spencer et al (Mutagenesis in Yeast, 1996, Yeast Protocols: Methods in Cell and Molecular Biology. Humana Press, Totowa, N.J.). Introduction of a mutator phenotype can also be used to generate random chromosomal mutations in yeast. Common mutator phenotypes can be obtained through disruption of one or more of the following genes: *PMS1, MAG1, RAD18* or *RAD51* or their orthologues in fungi other than *S.cerevisae*. Restoration of the non-mutator phenotype can be easily obtained by insertion of the wild type allele. Collections of modified cells produced from any of these or other known random mutagenesis processes may be screened for reduced activity of the target protein (US20090305363).

### Processes for the oxidation of furanic compounds

In a further aspect, the invention pertains to processes for oxidizing furanic compounds. In particular the invention pertain to process wherein furanic precursors of FDCA are oxidized. A process of the invention may comprise a single oxidation reaction step resulting in a product (e.g. the oxidation of HMFCA to FFCA). Alternatively a process of the invention may comprise more than one oxidation reaction step, each step resulting in an intermediate, where the last intermediate is the final product. Examples of such a series of steps, wherein HMF is oxidized in sequential oxidation steps to FDCA include e.g.: 1) HMF is first oxidized to HMFCA, which in a second step is oxidized to FFCA, which is then finally oxidized to FDCA, or alternatively, as described by Dijkman et al. (2014, Angew. Chem. 53 (2014) 6515-8) 2) HMF is first oxidized to DFF, which in a second step is oxidized to FFCA, which is then finally oxidized to FDCA. Thus, in a preferred process of the invention one or more furanic precursors of FDCA are oxidized in a series of steps to ultimately FDCA.

In one embodiment, the invention relates to processes comprising at least the oxidation of HMFCA to FFCA. Preferably, the process is a process for oxidizing HMFCA to FFCA, wherein the process comprises the step of incubating a cell in the presence of HMFCA. The cell preferably is a cell expressing enzymes that have the ability to oxidize HMFCA to FFCA. The cell can be cell that is genetically modified to have the ability to oxidize HMFCA to FFCA. In a preferred embodiment, the cell is a genetically modified fungal cell as herein defined above. Preferably the cell is incubated in the presence of HMFCA under conditions conducive to the oxidation of HMFCA by the cell, as e.g. specified below.

In another embodiment, the invention relates to processes for producing FDCA. A process for producing FDCA preferably comprises the step of incubating a cell in a medium comprising one or more furanic precursors of FDCA. The cell preferably is a cell expressing one or more enzymes that have the ability to convert a furanic precursor of FDCA into FDCA. The enzymes with the ability to convert a furanic precursors of FDCA into FDCA can be an enzyme having alcohol and/or aldehyde dehydrogenase activities as described above, including the exemplified fungal enzymes. Thus, in a preferred embodiment, the cell is a genetically modified fungal cell, as herein defined above.

Preferably the cell is incubated in the presence of one or more furanic precursors of FDCA under conditions conducive to the oxidation furanic precursors of FDCA by the cell to FDCA, as e.g. specified below.

Preferably in the process, at least one furanic precursor of FDCA is selected from the group consisting of HMF, DHF, HMFCA, FFCA and DFF, of which HMF is most preferred. The furanic precursors of FDCA are preferably obtained from one or more hexose sugars, preferably by acid-catalysed dehydration, e.g. by heating in presence of acid, in a conventional manner. The technology to generate HMF from fructose is well established and robust (see e.g. van Putten et al., 2013, Chem. Rev. 113, 1499-1597). Also glucose-rich feedstock can be utilized, but the thermochemical formation of HMF proceeds more efficiently from fructose. Therefore, an additional enzymatic step can be included to convert glucose to fructose, using glucose isomerase. The latter process is well-established in food industry e.g. for producing high fructose corn syrup (HFCS) from hydrolysed starch. Glucose can also be chemically isomerised to fructose using combinations of catalysts and solvents as e.g. described in van Putten et al. (2013, *supra*).

The hexose sugars will usually be obtained from biomass. The term "biomass" is understood to mean the biodegradable fraction of products, waste and residues from biological origin from agriculture (including vegetal, such as crop residues, and animal substances), forestry (such as wood resources) and related industries including fisheries and aquaculture, as well as biodegradable fraction of industrial and municipal waste, such as municipal solid waste or wastepaper. In a preferred embodiment, the biomass is plant biomass, more preferably a (fermentable) hexose/glucose/sugar-rich biomass, such as e.g. sugarcane, a starch-containing biomass, for example, wheat grain, or corn straw, or even cereal grains, such as corn, wheat, barley or mixtures thereof. Preferred are agricultural crops naturally rich in fructans (e.g., topinambur or chicory roots).

The hexose sugars can be obtained by hydrolysis of such biomass Methods for hydrolysis of biomass are known in the art per se and include the use of e.g. vapour and/or carbohydrases such as glucoamylases.

Another preferred type of biomass for use in the process of the invention is a so-called "second generation" lignocellulosic feedstock, which are preferred if large volumes of FDCA are to be produced in a more sustainable way. Lignocellulosic feedstocks can be obtained from dedicated energy crops, e.g. grown on marginal lands, thus not competing directly with food crops. Or lignocellulosic feedstocks can be obtained as by-products, e.g. municipal solid wastes, wastepaper, wood residues (including sawmill and paper mill discards) and crop residues can be considered. Examples of crop residues include bagasse from sugar cane and also several corn and wheat wastes. In the case of corn by-products, three wastes are fiber, cobs and stover. Furthermore, forestry biomass may be used as feedstock. In order to convert second generation feedstocks into fermentation products of the invention, the cellulose and hemicellulose need to be released as monosaccharides. Hereto, either thermochemical approaches (usually referred to as pretreatment), enzymatic approaches or a combination of the two methodologies are applied. A pretreatment can serve to either completely liberate the sugars, or to make the polymeric compounds more accessible to subsequent enzymatic attack. Different types of pretreatment include liquid hot water, steam explosion, acid pretreatment, alkali pretreatment, and ionic liquid pretreatments. The relative amounts of the various compounds will depend both on the feedstock used and the pretreatment employed. For release of monosaccharide sugars from such lignocellulosic feedstock, appropriate carbohydrases are employed, including e.g. arabinases, xylanases, glucanases, amylases, cellulases, glucanases and the like.

The oxidation process of the invention is preferably conducted at temperature most optimal to the cell and/or the oxidoreductase enzymes contained is the cell. Thus, in case of thermophilic cells and/or thermophilic enzymes the temperature preferably is in the range between 40 and 65°C, e.g. at least 40, 42, or 45 °C and/or not higher than e.g. 65, 60, 55 or 50 °C. However, in the case of a mesophilic cell and/or enzymes from mesophiles, the oxidation reactions are preferably conducted at a relatively mild temperature, e.g. in the range of 10 - 45 °C, more preferably 20 - 40 °C, e.g. at least 10, 15, 18, 20, 22 or 25 °C and/or not higher than e.g. 45, 42, 40, 38, 35, 32 or 30 °C.

The oxidation process of the invention is preferably conducted at acidic pH. Downstream processing (DSP), i.e. recovery and purification, is of general concern in any biotechnological process but in particular in productions of monomeric compounds for polymer productions because the purity of the monomer is essential in controlled polymer formation. FDCA has a very limited solubility at pH-values below 3 (with a pKₐ of around 2.28). When the process is carried out at acidic pH, the FDCA produced will precipitate from the medium in which it is produced, preferably already during its production, thereby greatly facilitating its recovery. Preferably therefore, in the process of the invention, the cell, preferably a fungal cell is incubated in the presence of one or more furanic at a pH equal to or lower than 5.0, 4.0, 3.0, 2.9, 2.8, 2.7, 2.6, 2.5 or 2.4, and preferably at a pH that is equal to or higher than 2.0, 2.1, 2.2 or 2.25, 2.27 or 2.28. Preferably, in the process of the invention a cell, preferably a fungal host cell is selected that has a high tolerance to a pH in this range. An additional advantage of carrying out the process at acidic pH is that microbial contaminations of the process will be less of a problem since almost all bacteria are adversely affected at low pH. Yeasts and fungi are less of a problem compared to bacteria as source of infections and will be relatively easy to deal with.

The reaction time may be 6 - 150 hrs, more preferably 6 - 18 hrs. Preferably oxygen is supplied to the cells in the reaction medium from an oxygen source, such as molecular oxygen, e.g. as pure oxygen or in air, or water, or a different source of oxygen depending on the requirements of the furanic oxidizing enzyme. Air may be used conveniently as a source of molecular oxygen.

The reactor may be any suitable (aerated) bioreactor. It may be operated in batch, continuous or preferably in fed-batch.

The process of the invention further preferably comprises the step of recovery of the oxidation product(s) produced in the process, such as FDCA, or HMFCA. Preferably, the oxidation product is recovered from the medium in which the cell carrying out the oxidation steps is incubated. Oxidation products such as FDCA, HMFCA, etc. may be recovered from the reaction mixture or medium by e.g. (acid) precipitation, subsequent cooling crystallisation, and separation of the crystallized oxidation product, e.g., crystallized FDCA. However, other recovery methods are suitable, such as e.g. acid precipitation and solvent extraction, as known in the art.

The processes of the invention for oxidizing furanic compounds may advantageously be applied for the elimination of furanic compounds from feedstocks wherein furanic compounds are considered to be detrimental, such as feedstocks for fermentations for the production of biofuels and biochemicals. More preferably, the processes for oxidizing furanic compounds are applied in the bioproduction of FDCA as a monomeric precursor for the production of polyesters (plastics), wherein FDCA may substitute for PTA in the polyester PET in which case biobased polyethylenefurandicarboxylate (PEF) results. FDCA may also be used as a substrate for a large variety of valuable compounds, including e.g. as substrate for the production of succinic acid, 2,5-bis(aminomethyl)-tetrahydrofuran, 2,5-dihydroxymethyl-tetrahydrofuran, 2,5-dihydroxymethylfuran and 2,5-furandicarbaldehyde. FDCA may be used in the production of coatings, e.g. in alkyd resin and thermoplastic coatings. It may also be used as a xylene equivalent in biofuels and as solvent. FDCA may be esterified, and the esters may be used as plasticizers. FDCA may converted to its diol, that may be used in PET-like polyesters and polyurethanes. Further FDCA may be converted into its diamine, the diamine may be used as chain extender and the diamine may be converted into di-isocyanate, which can be used in the production of polyurethanes.

Thus, in a further aspect the invention relates to a process for producing a polymer from one or more, or at least two FDCA monomers, the process comprising the steps of: a) preparing an FDCA monomer in an oxidation process of the invention as described above; and, b) producing a polymer from the FDCA monomer(s) obtained in a). Preferably the polymer is polyethylenefurandicarboxylate (PEF).

In yet another aspect, the invention pertains to the use of a cell, preferably a cell of the invention, for the biotransformation of one or more of furanic precursors to FDCA to FDCA, wherein the cell is a cell expressing an HMFCA dehydrogenase as herein defined above, or a cell expressing polypeptide having furanic compound transport capabilities and further comprising a HMFCA dehydrogenase activities as herein defined above. Preferably, at least one furanic precursor of FDCA that is biotransformed to FDCA is selected from the group consisting of HMF, DHF, HMFCA, FFCA and DFF, of which HMF is most preferred.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

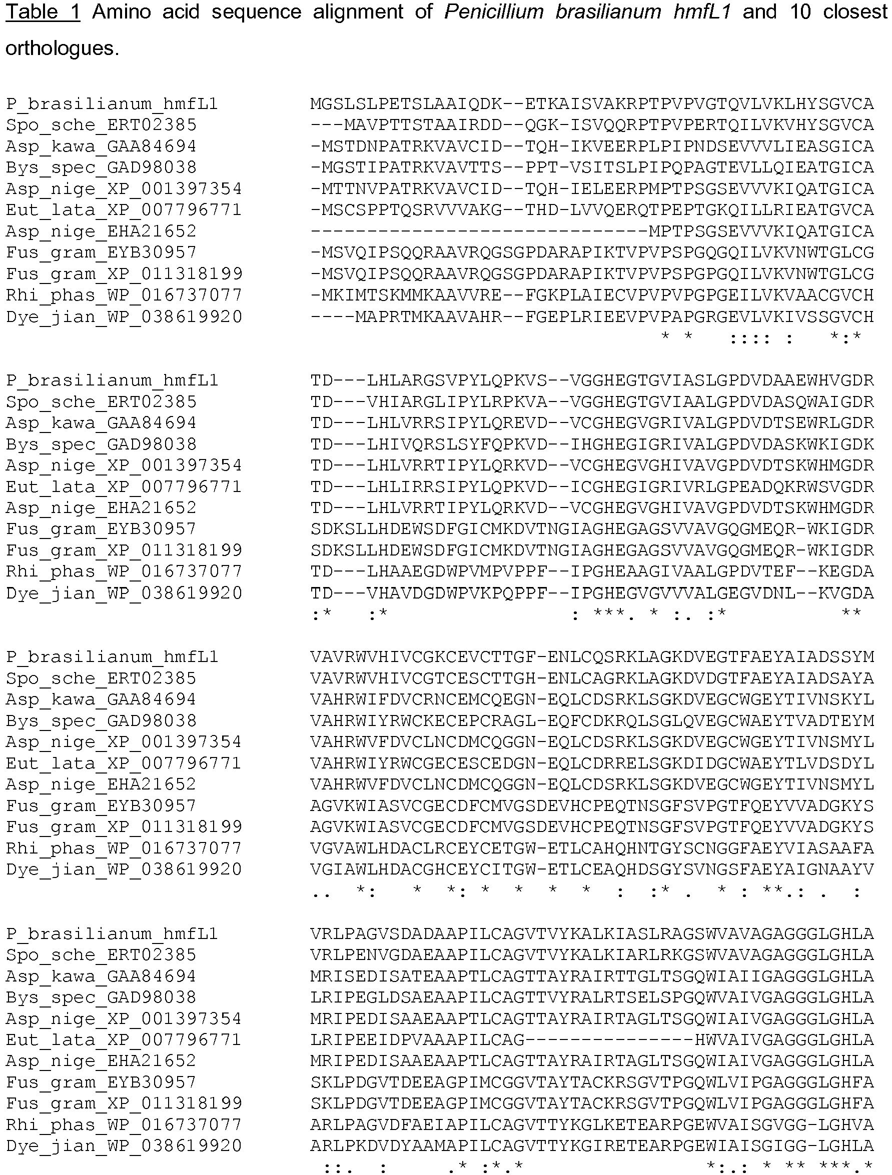

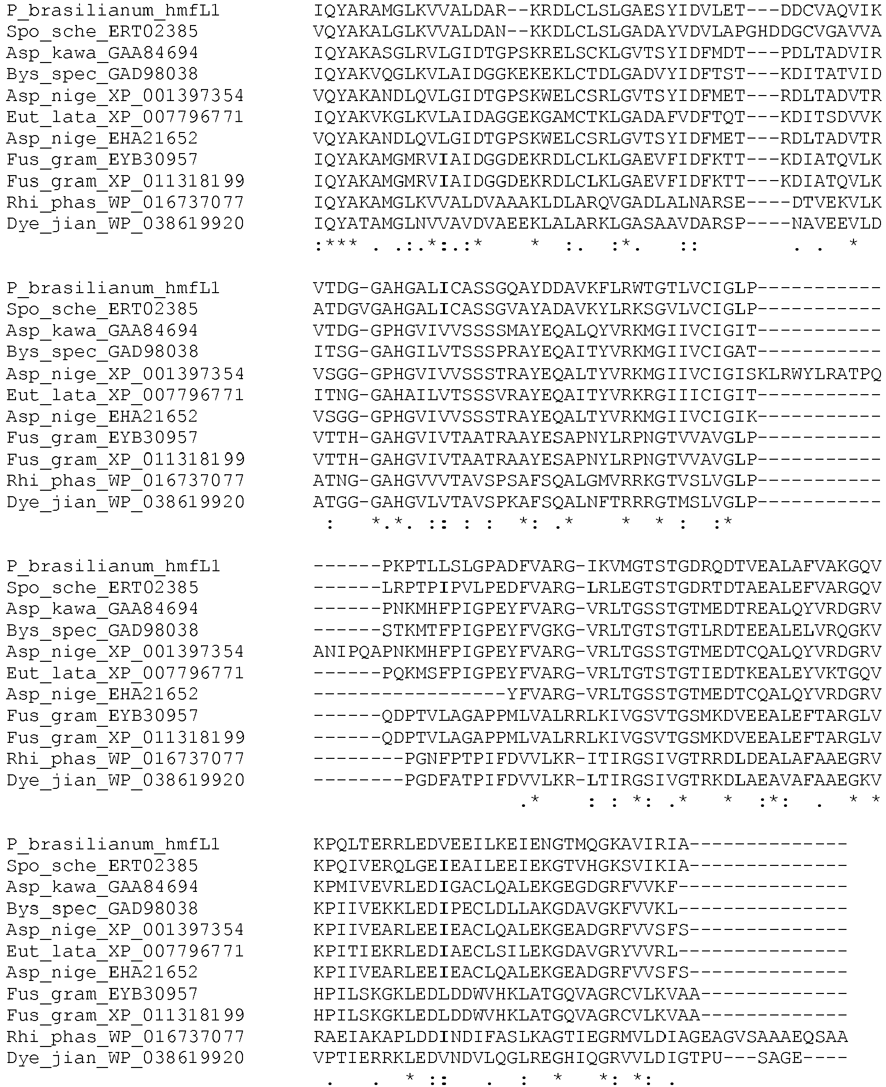

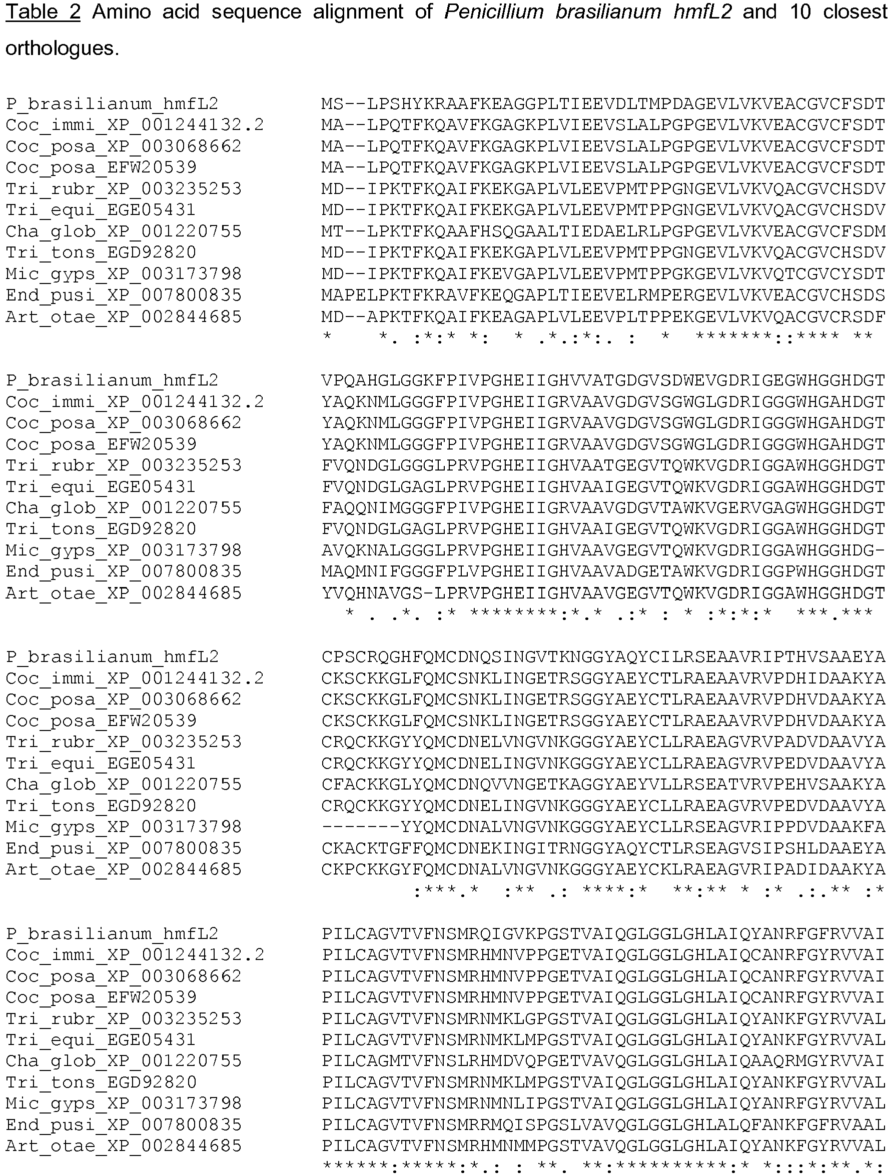

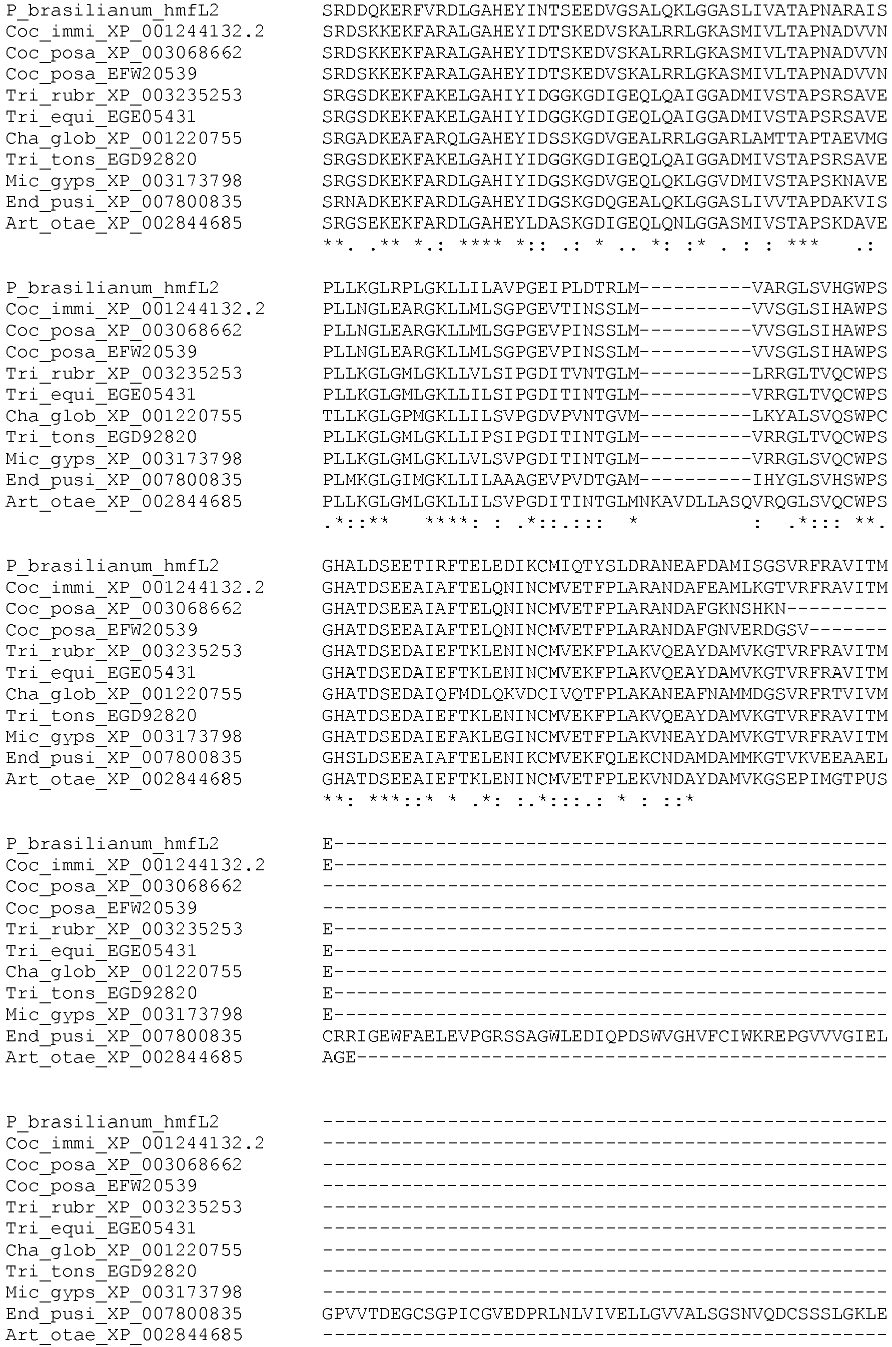

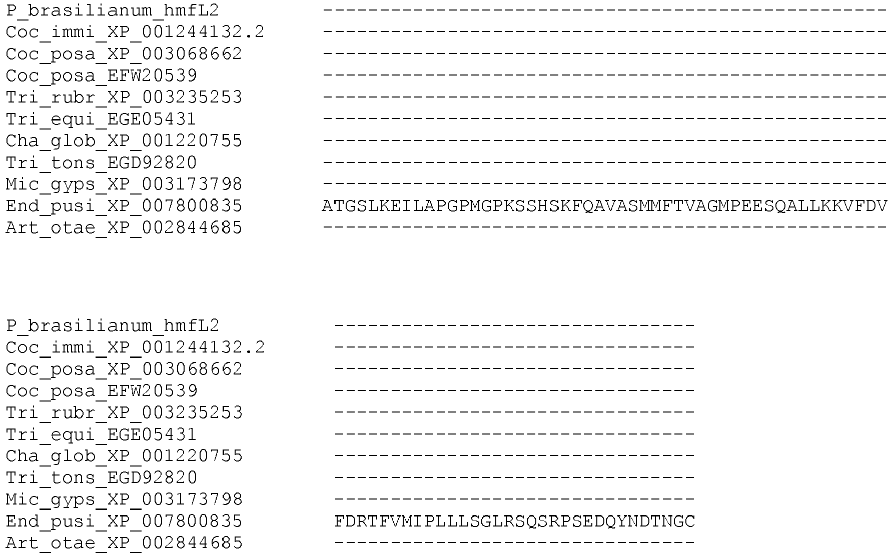

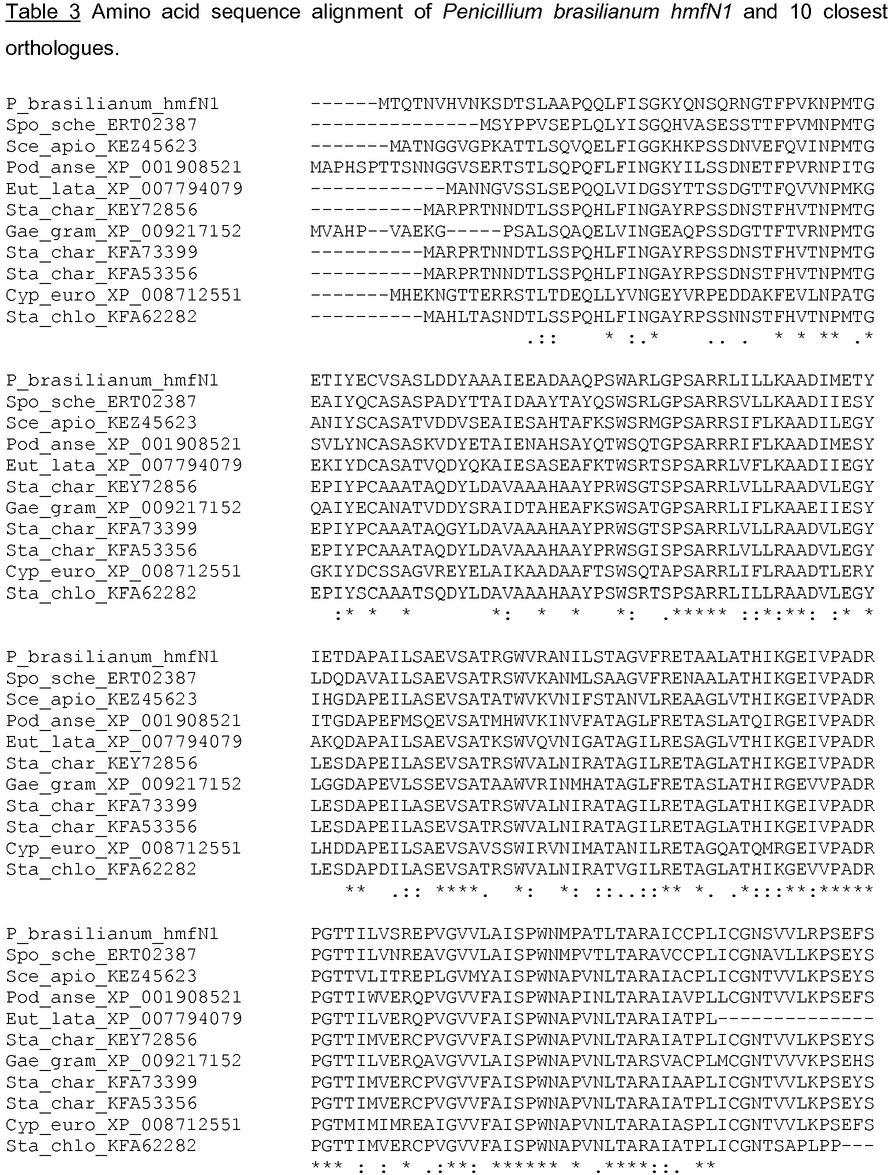

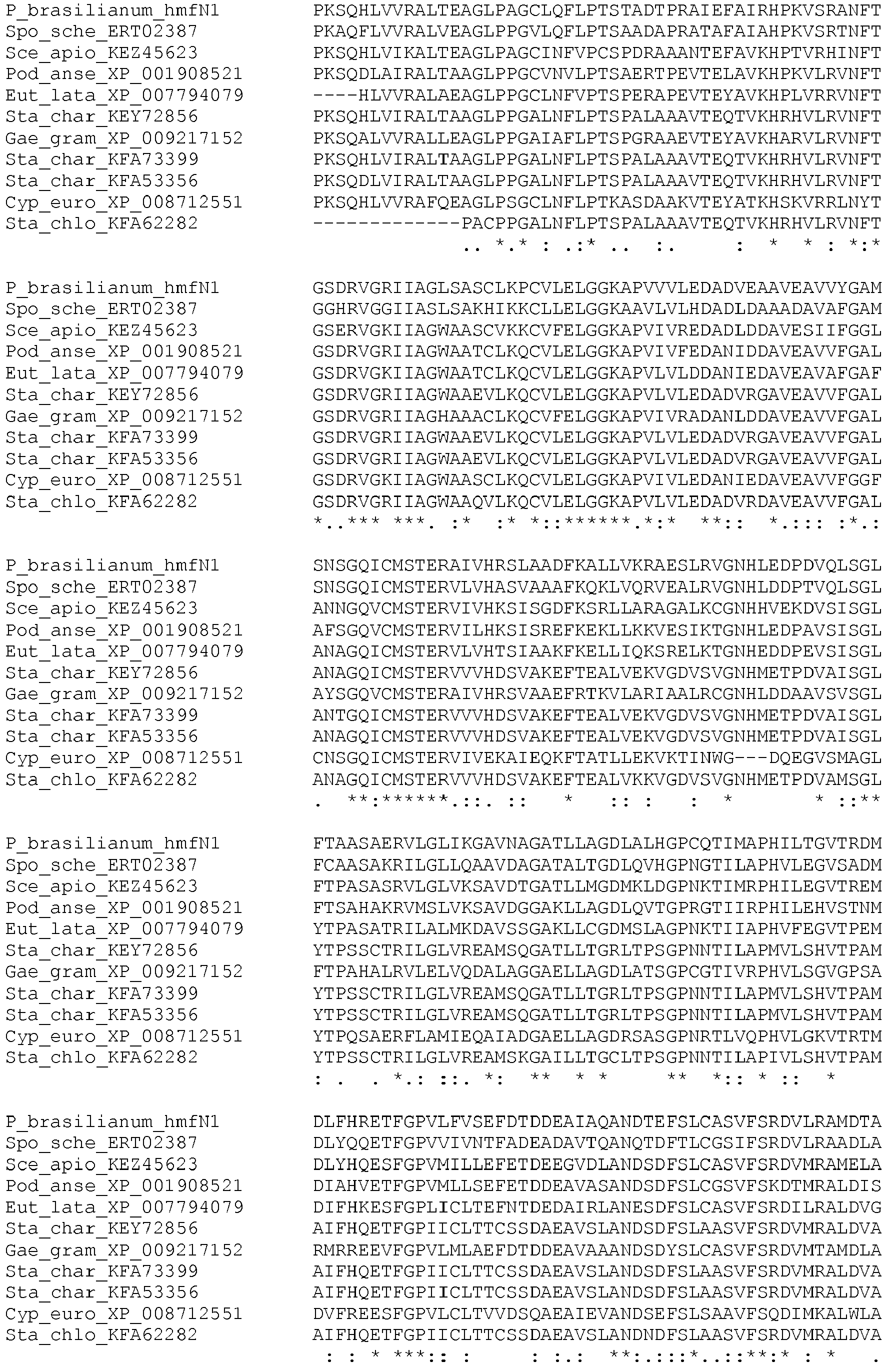

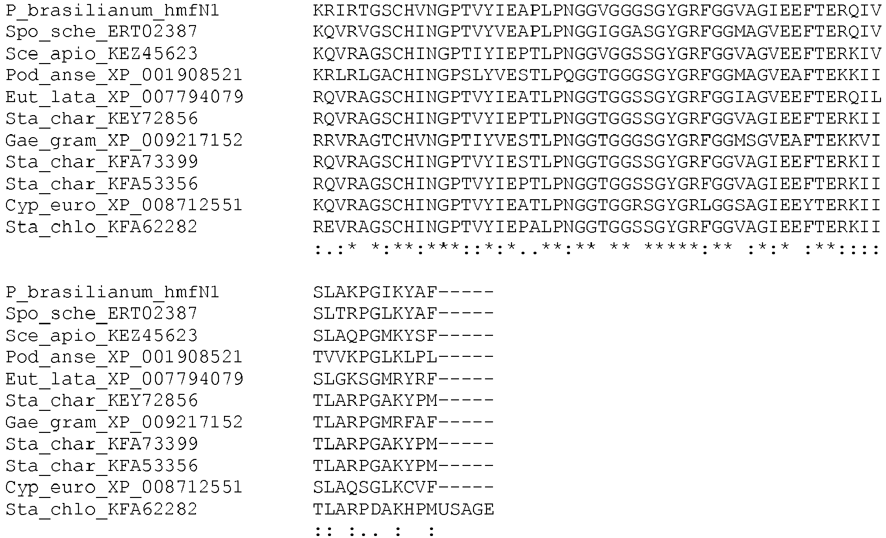

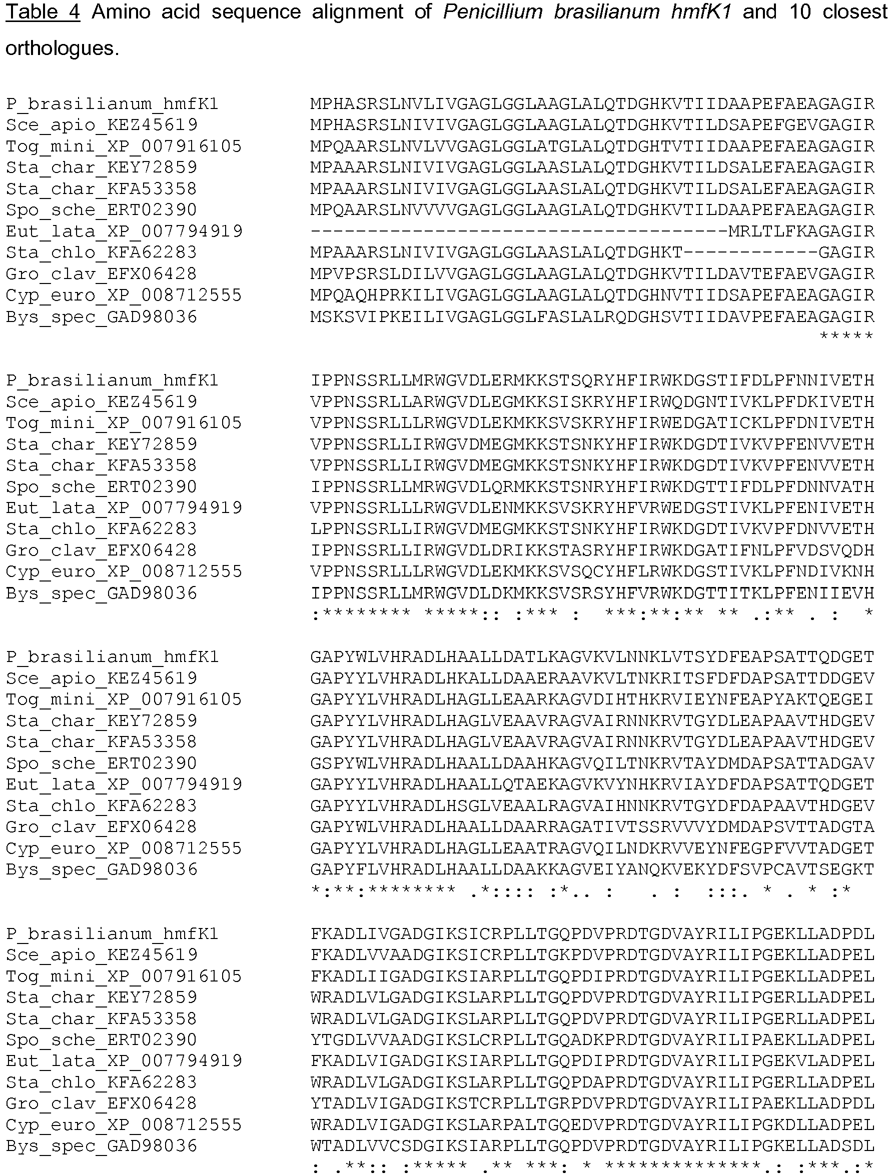

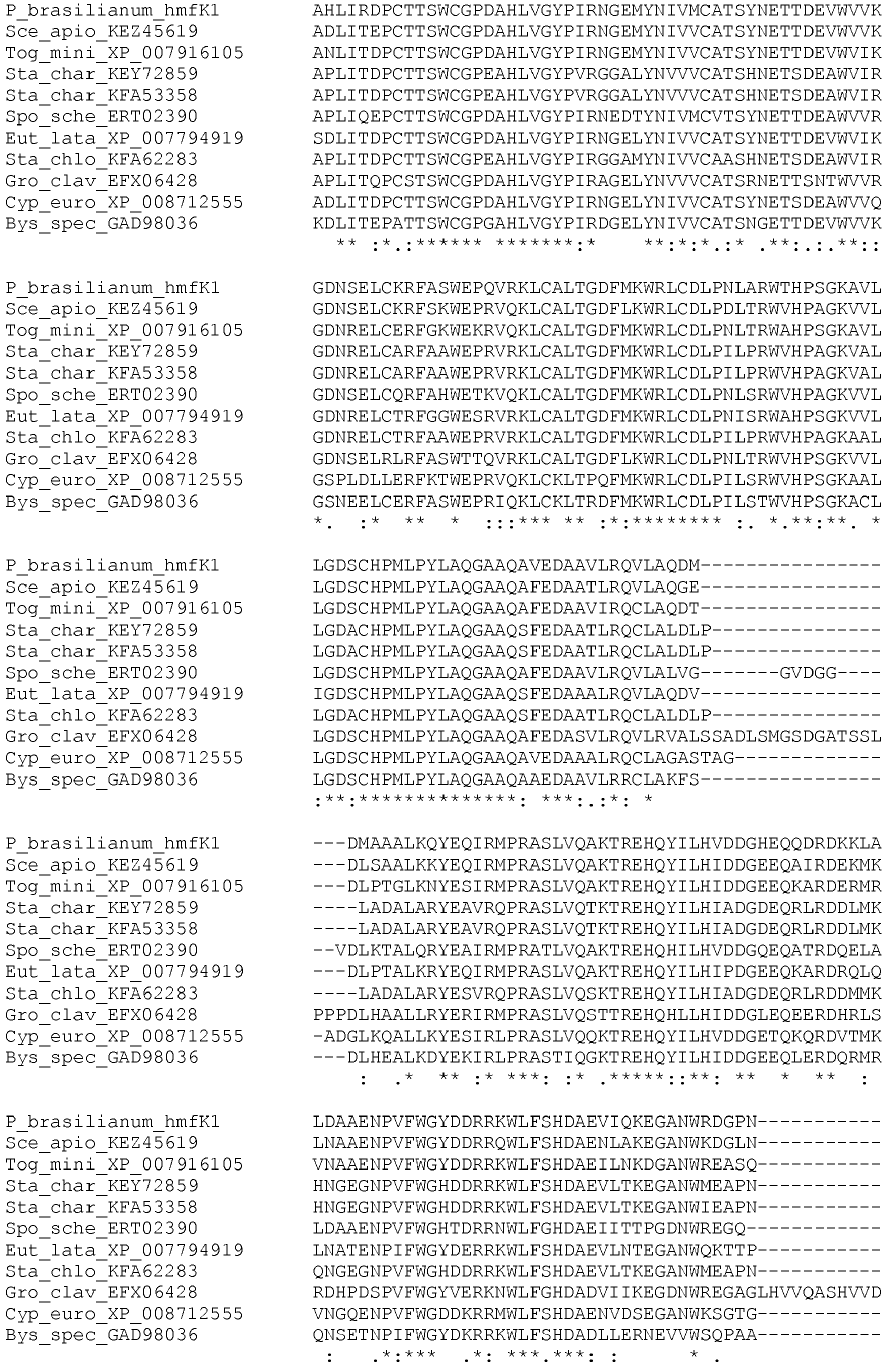

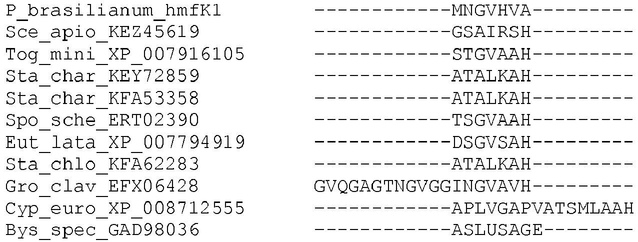

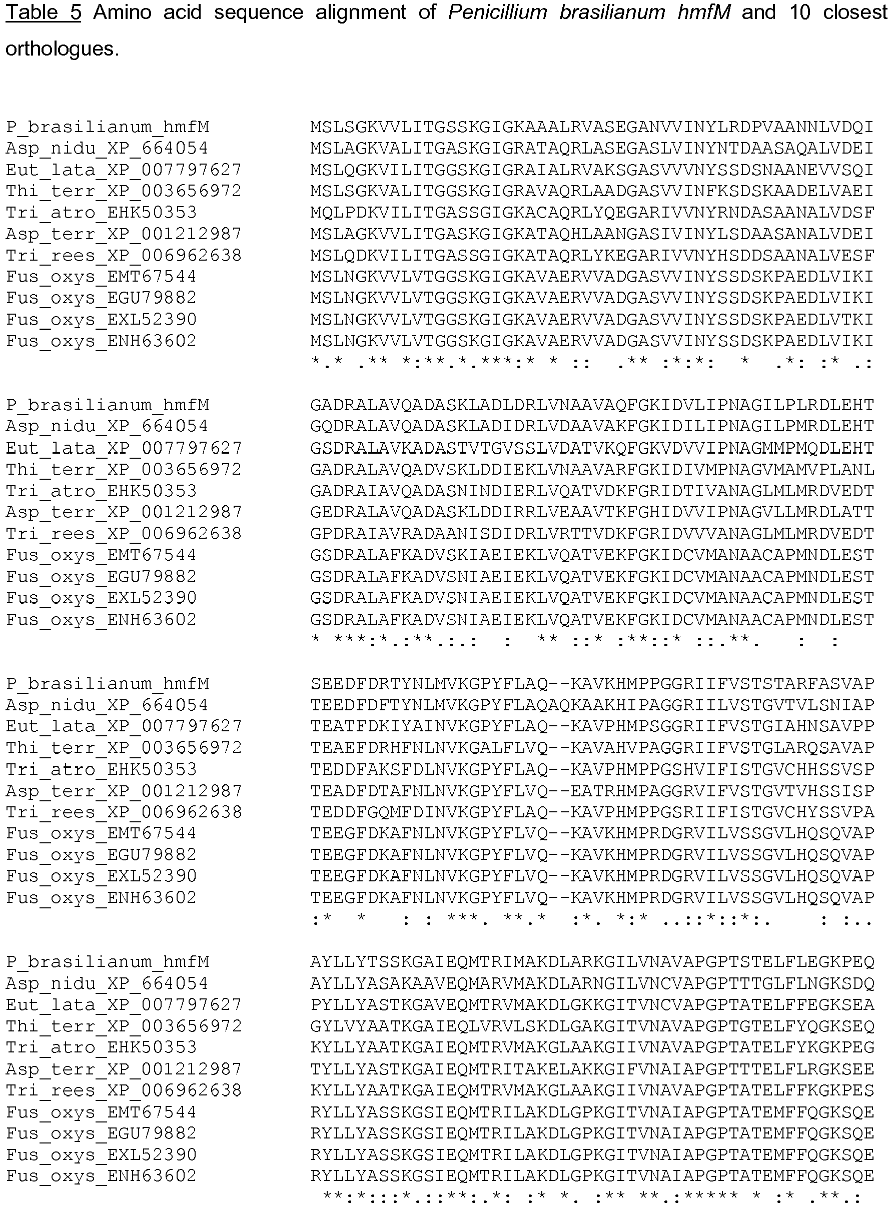

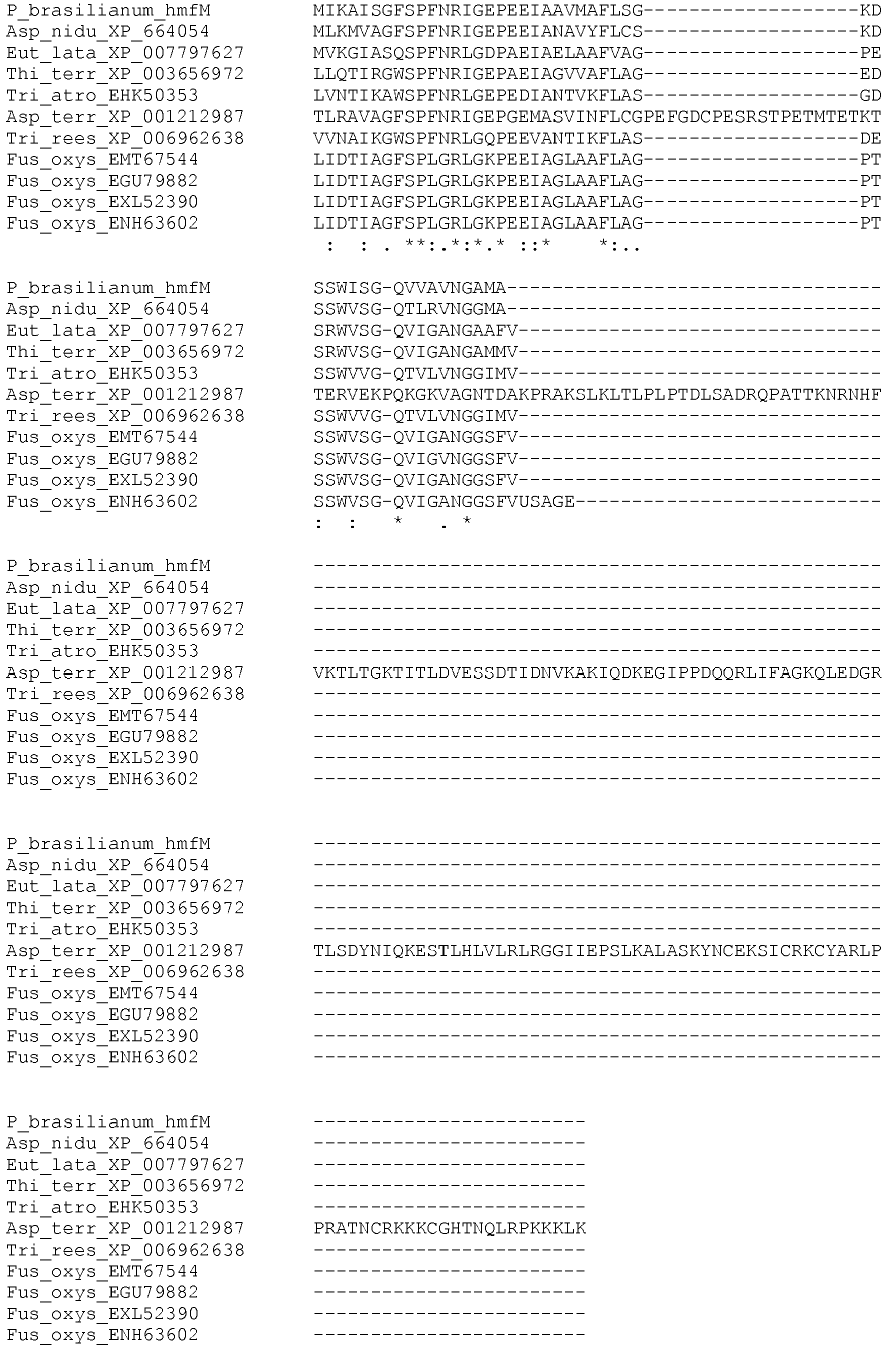

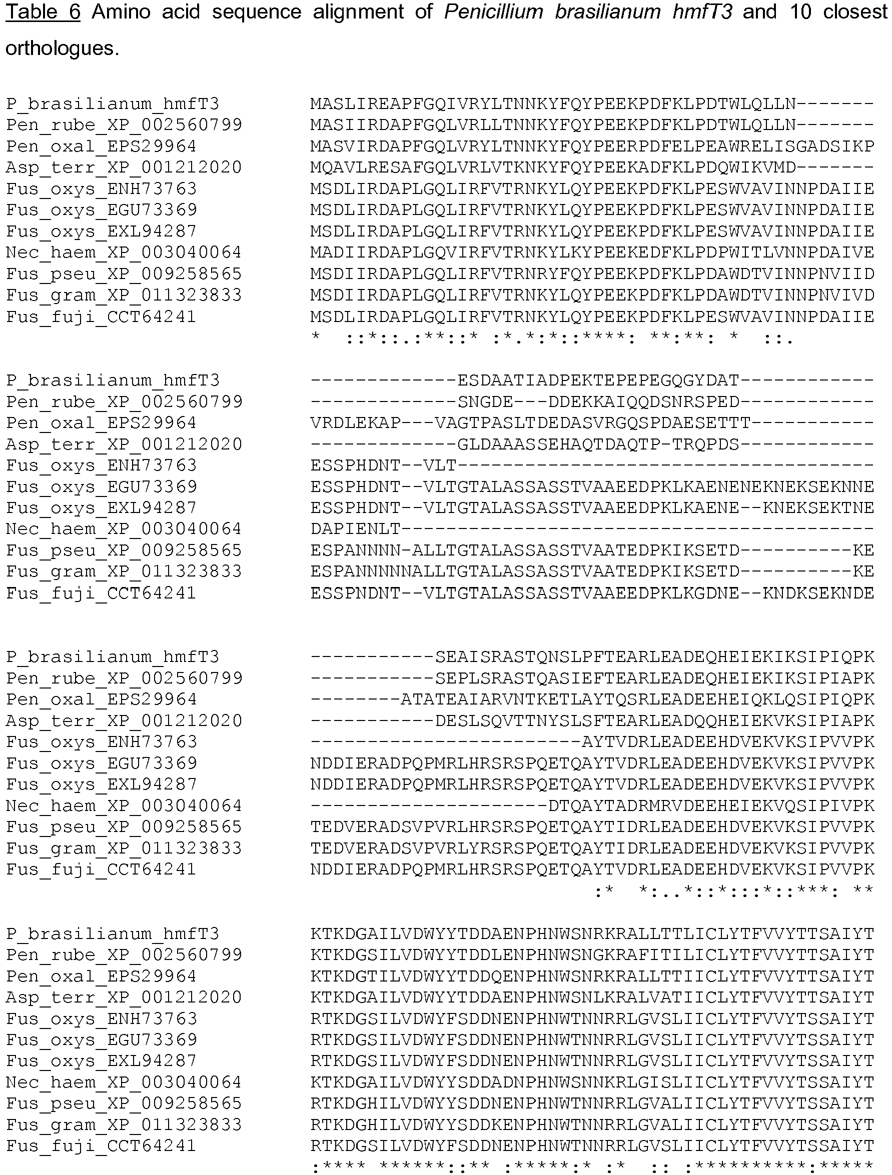

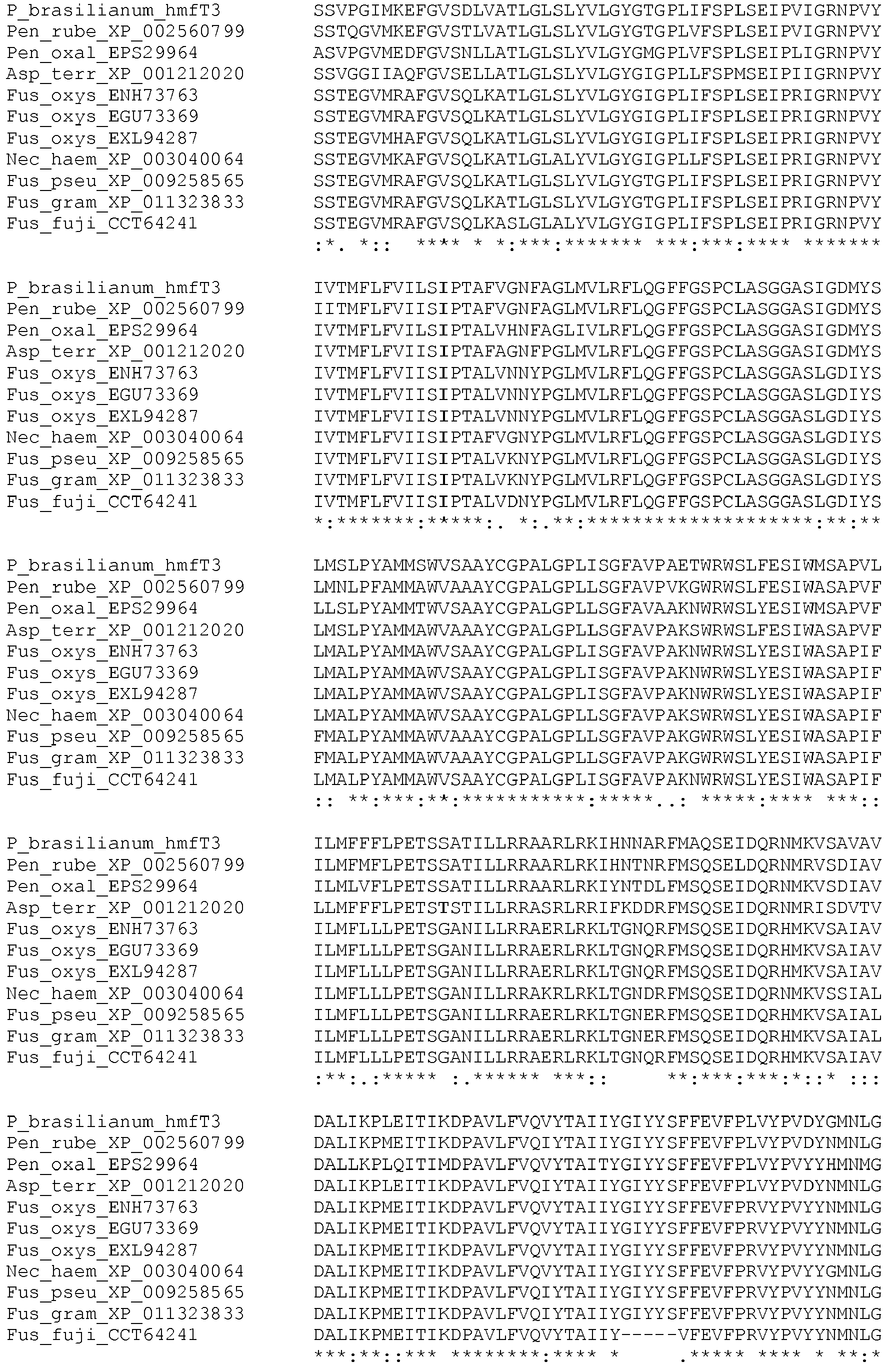

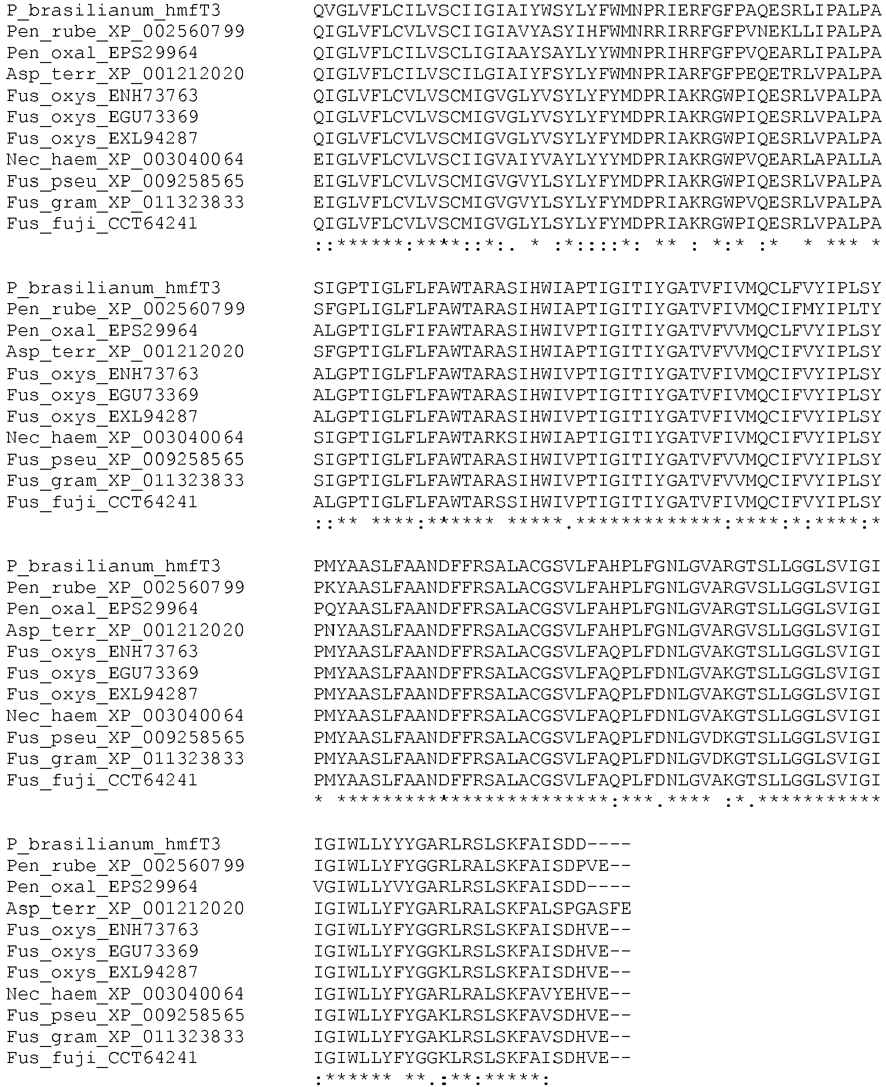

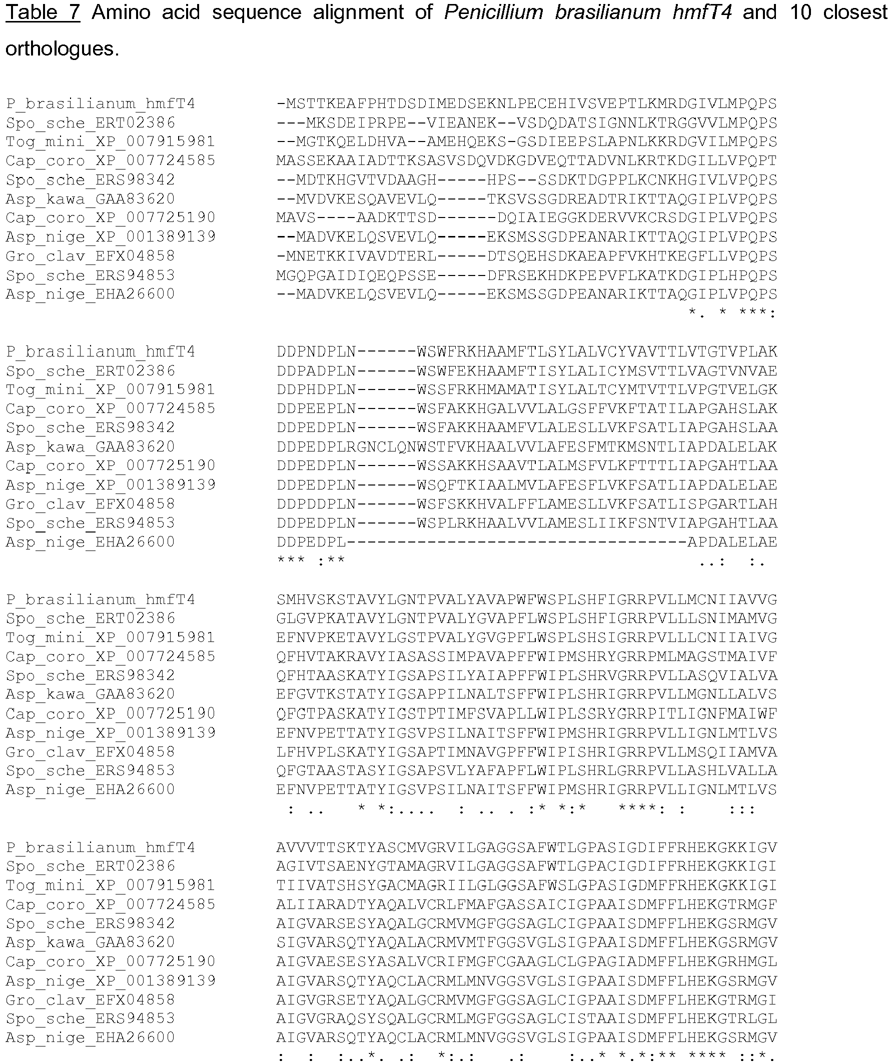

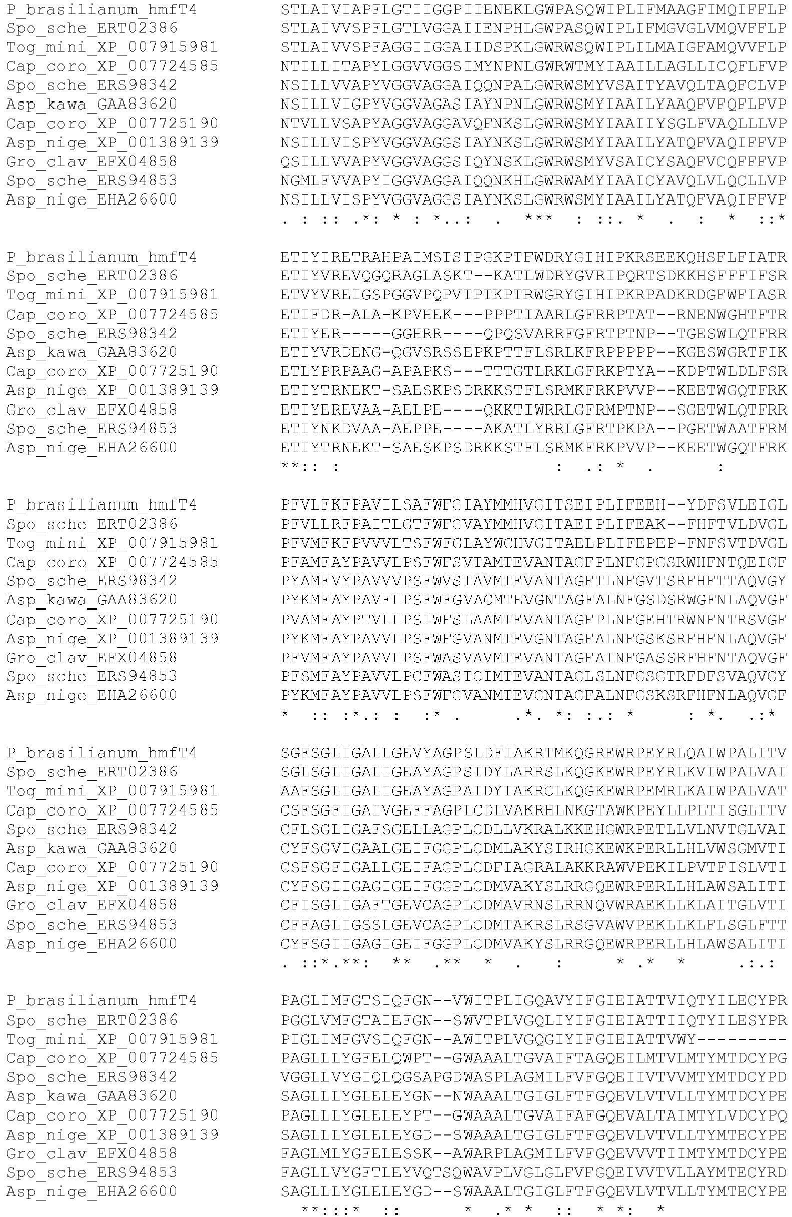

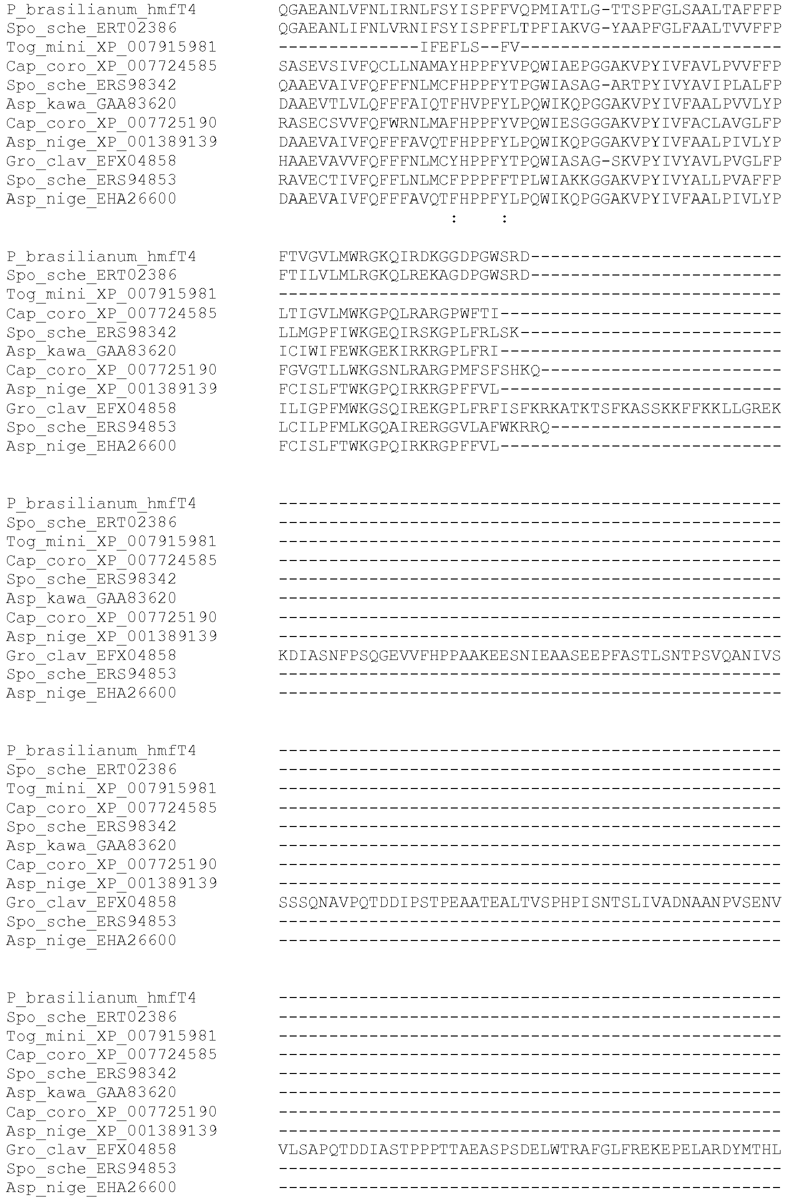

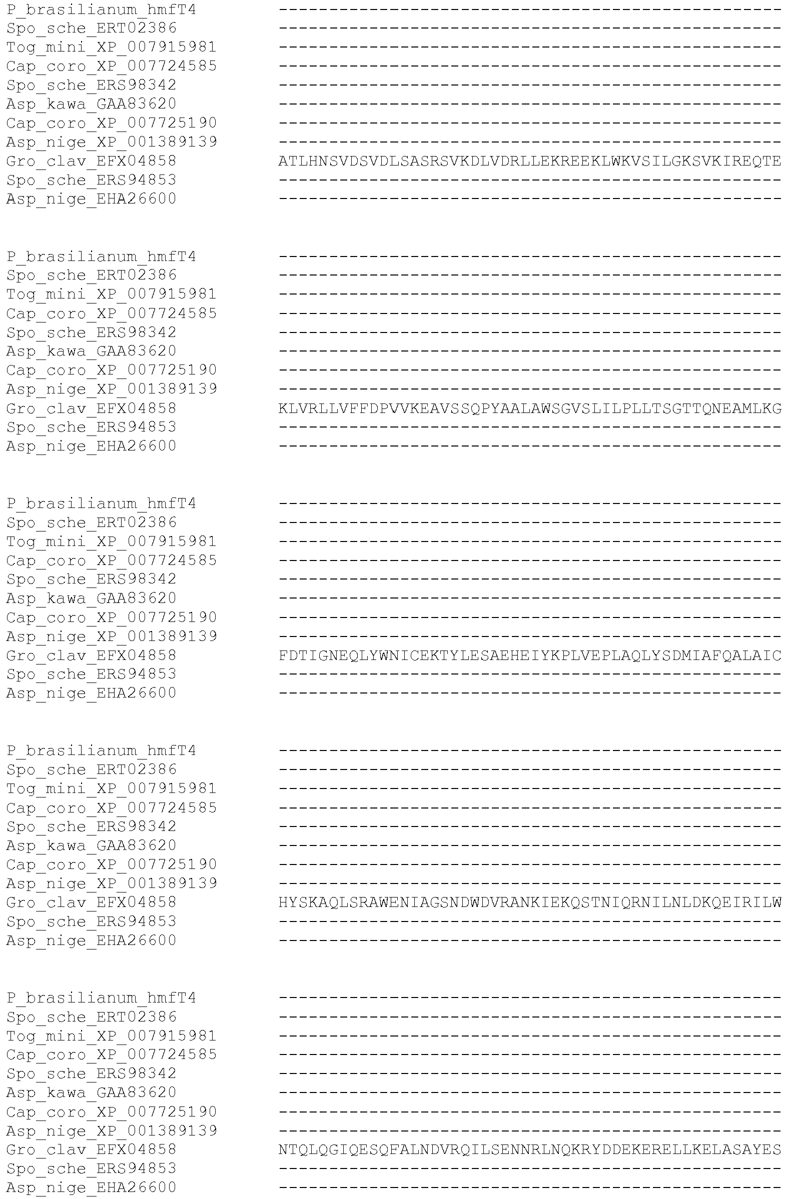

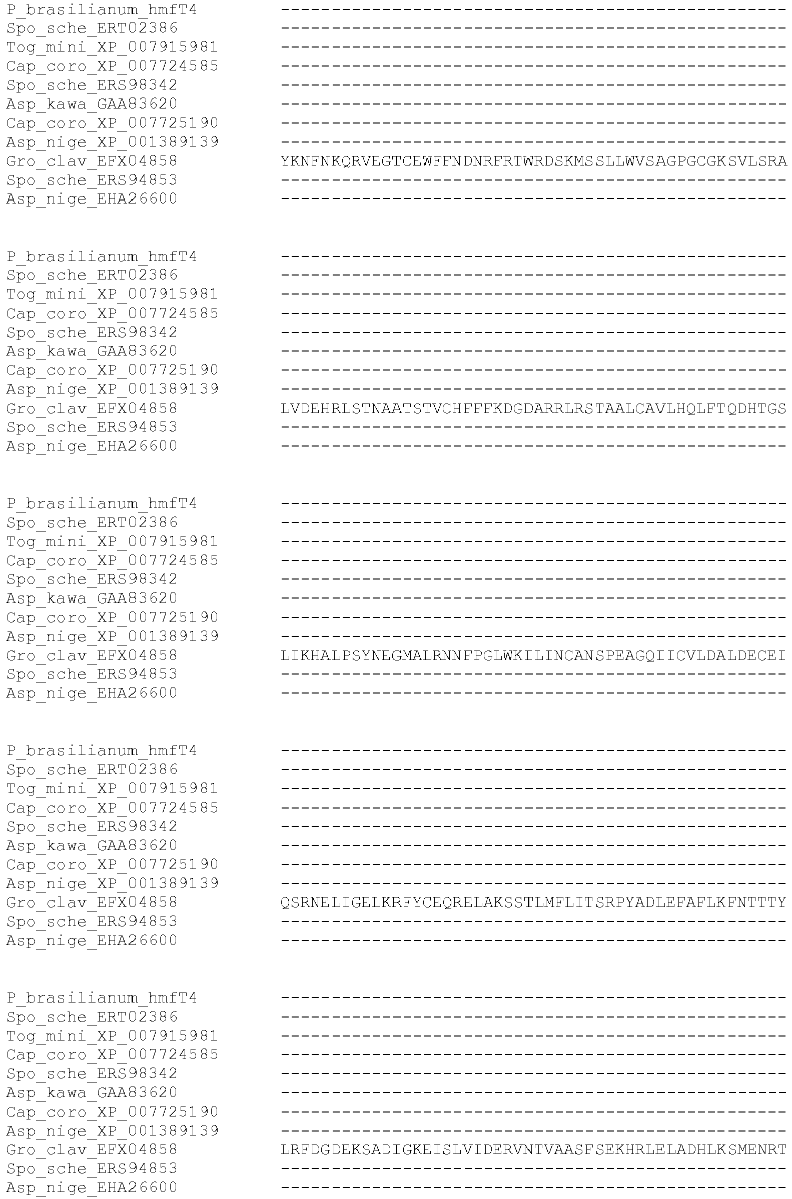

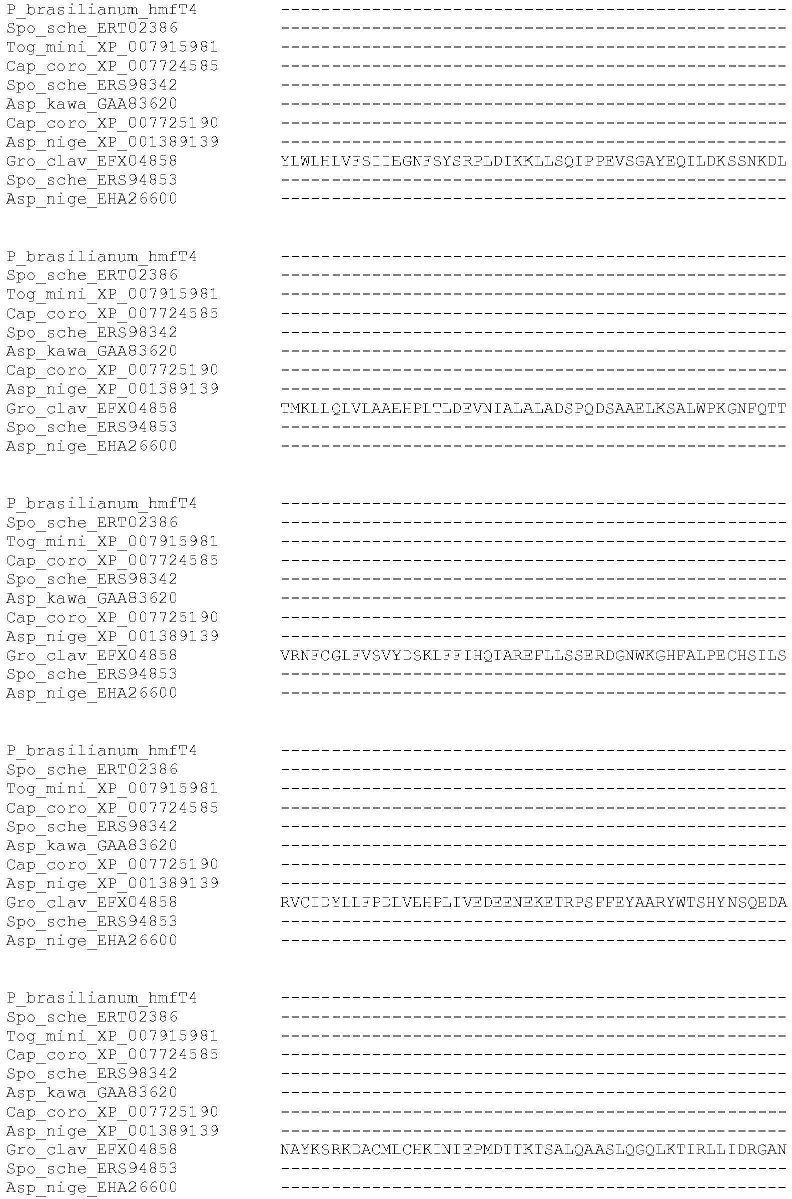

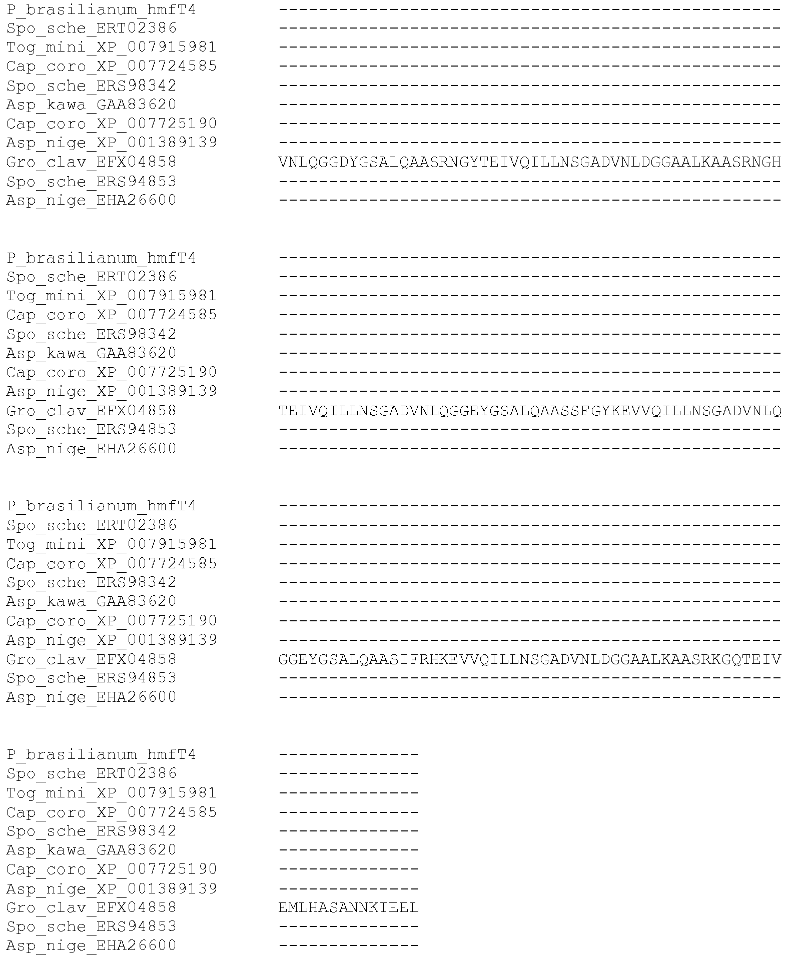

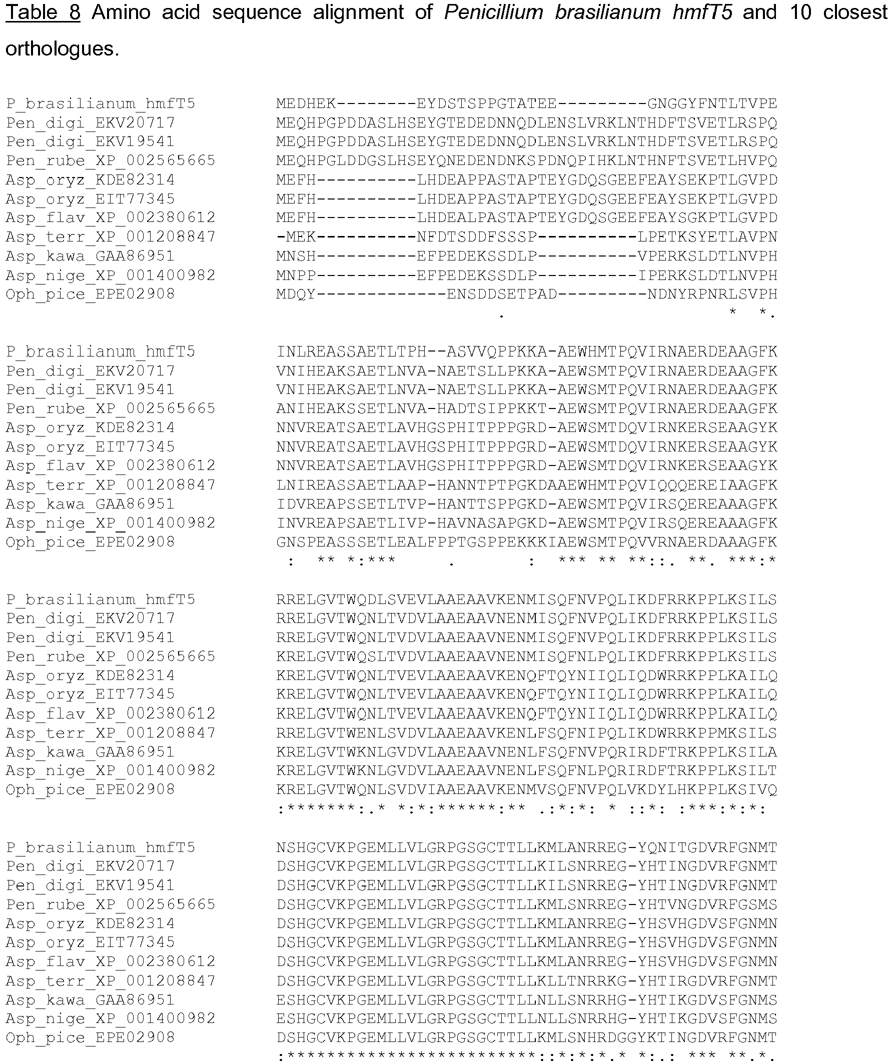

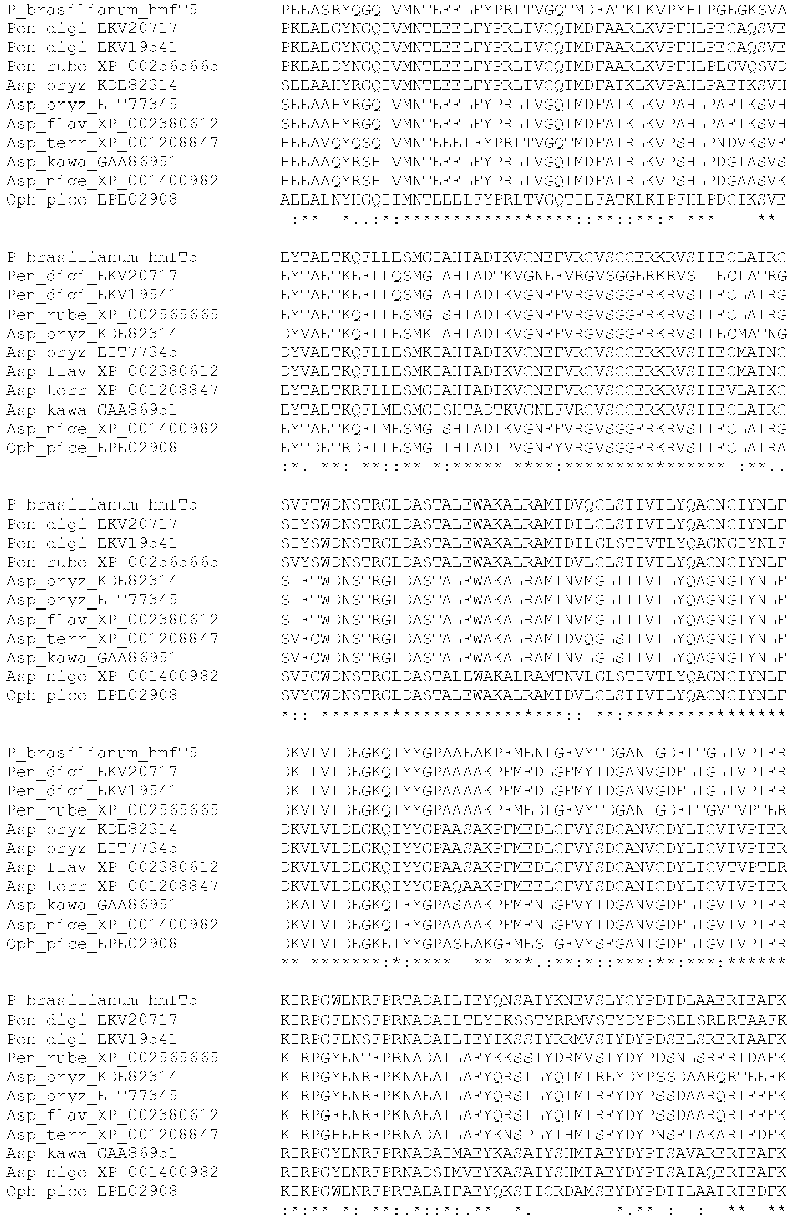

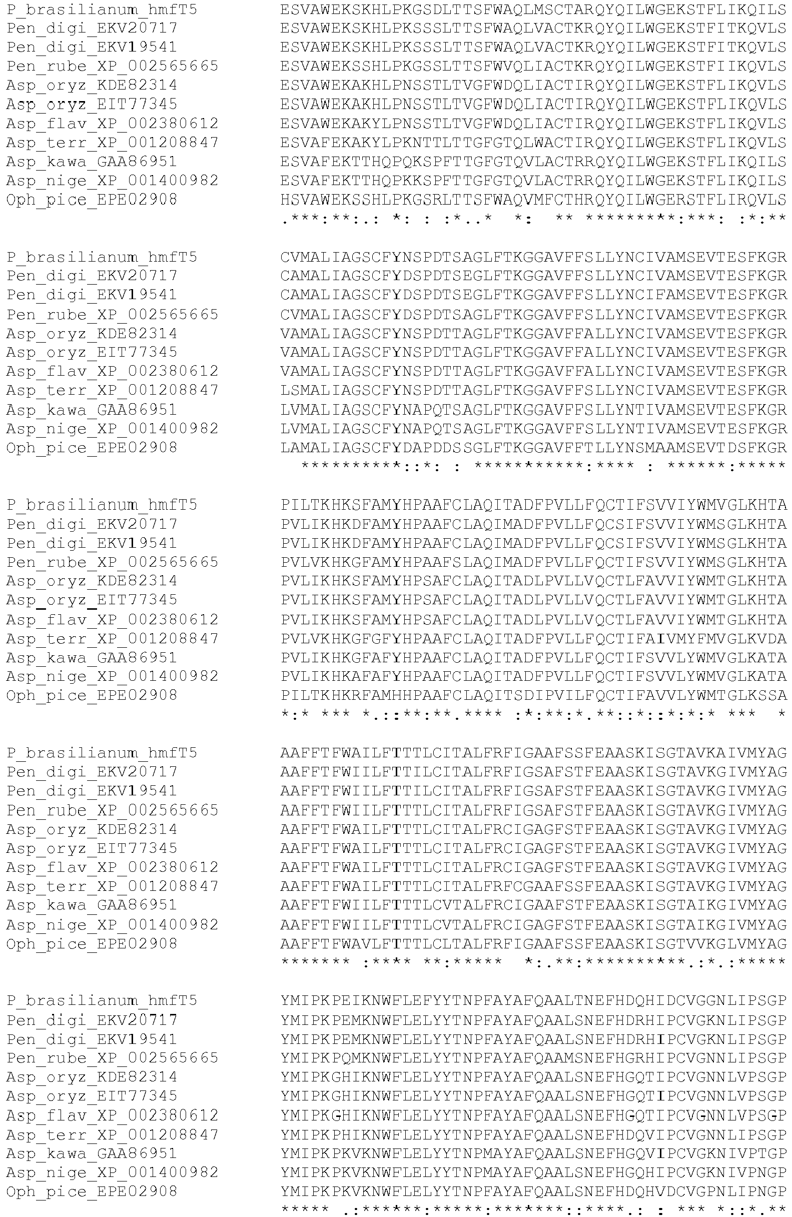

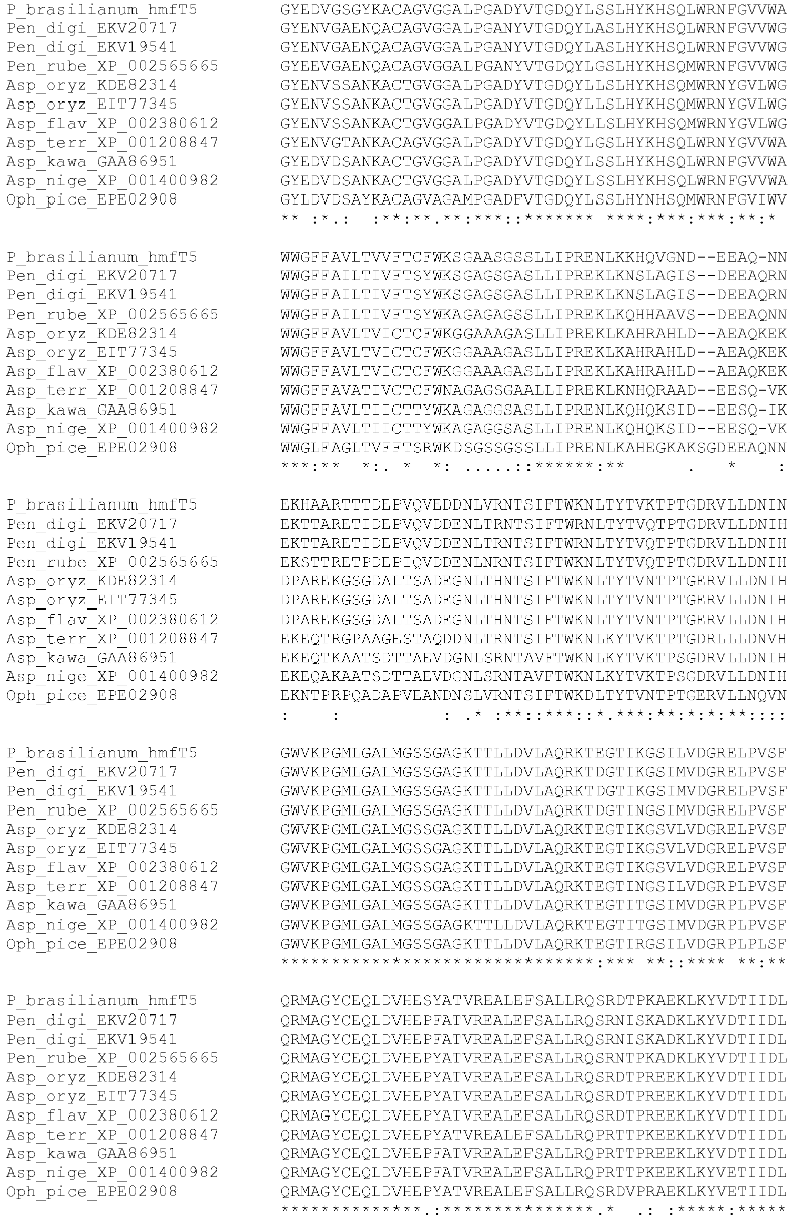

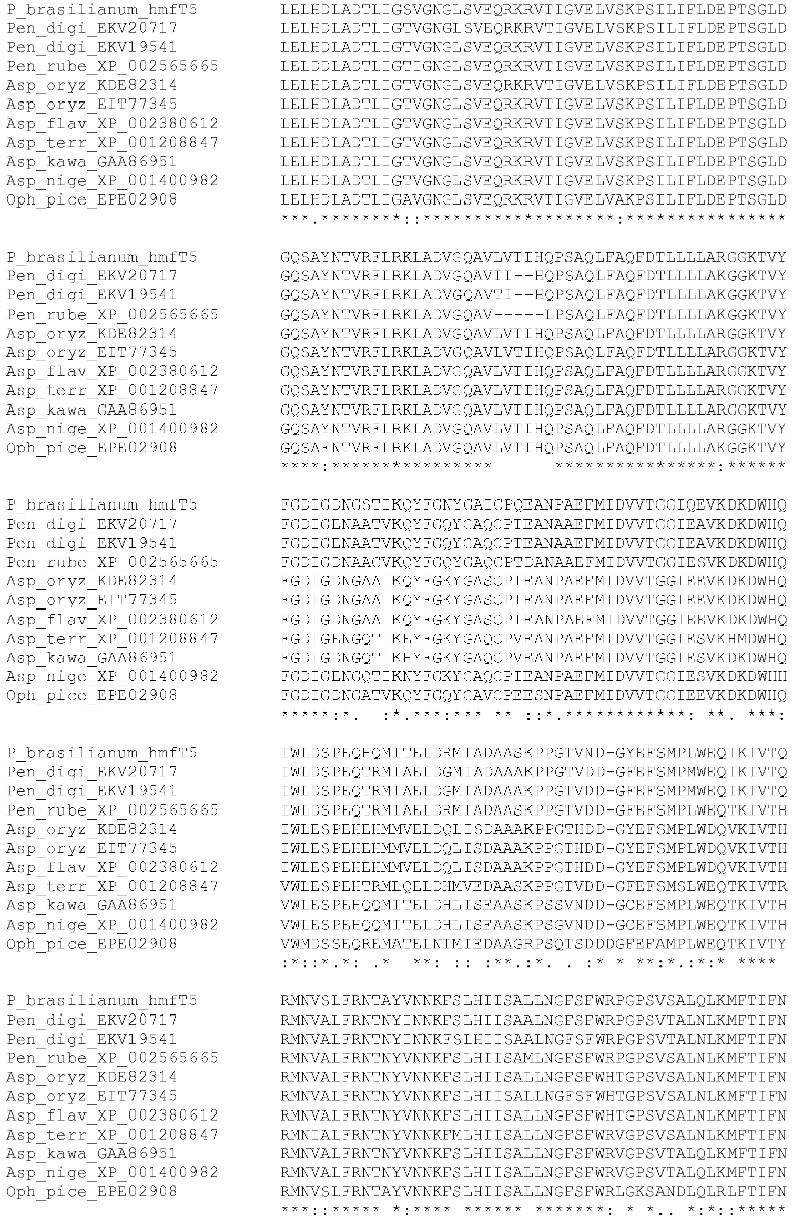

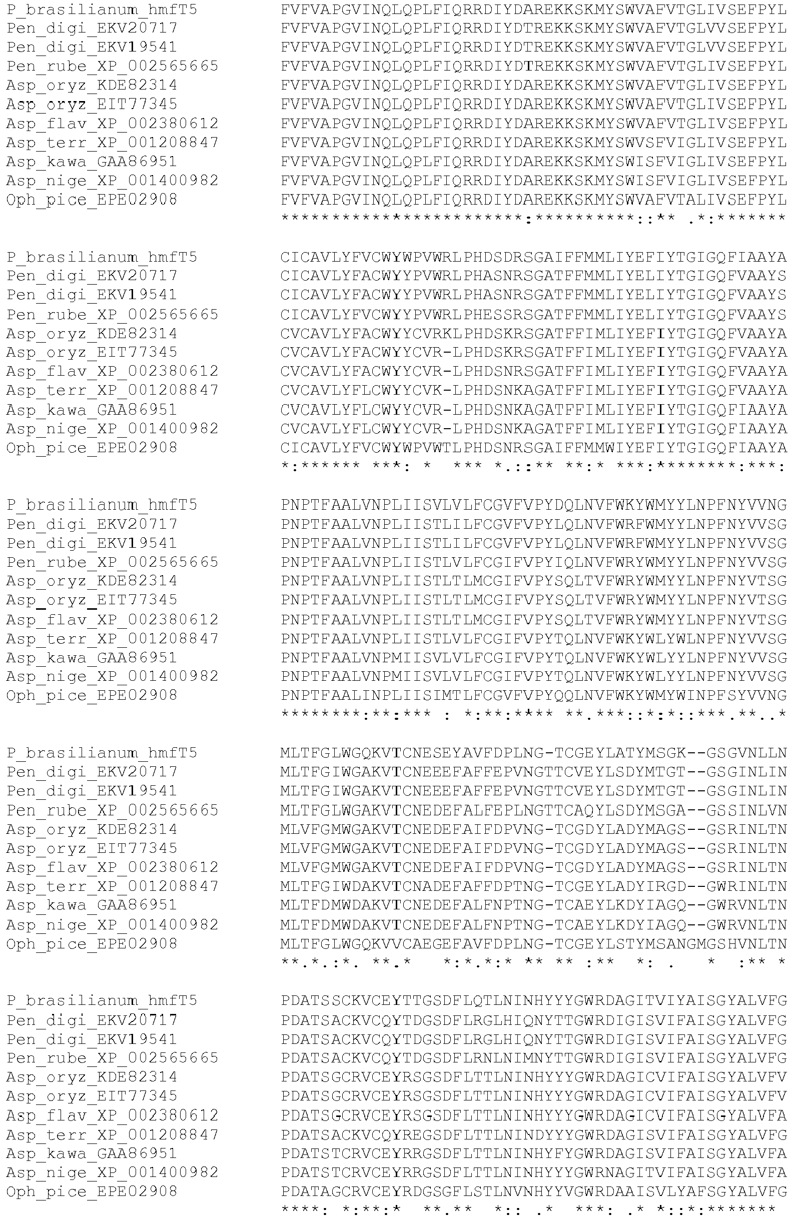

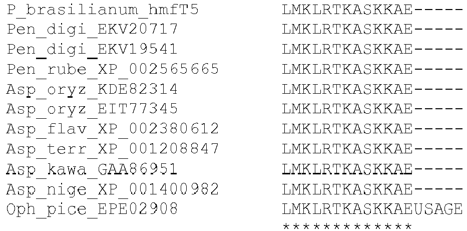

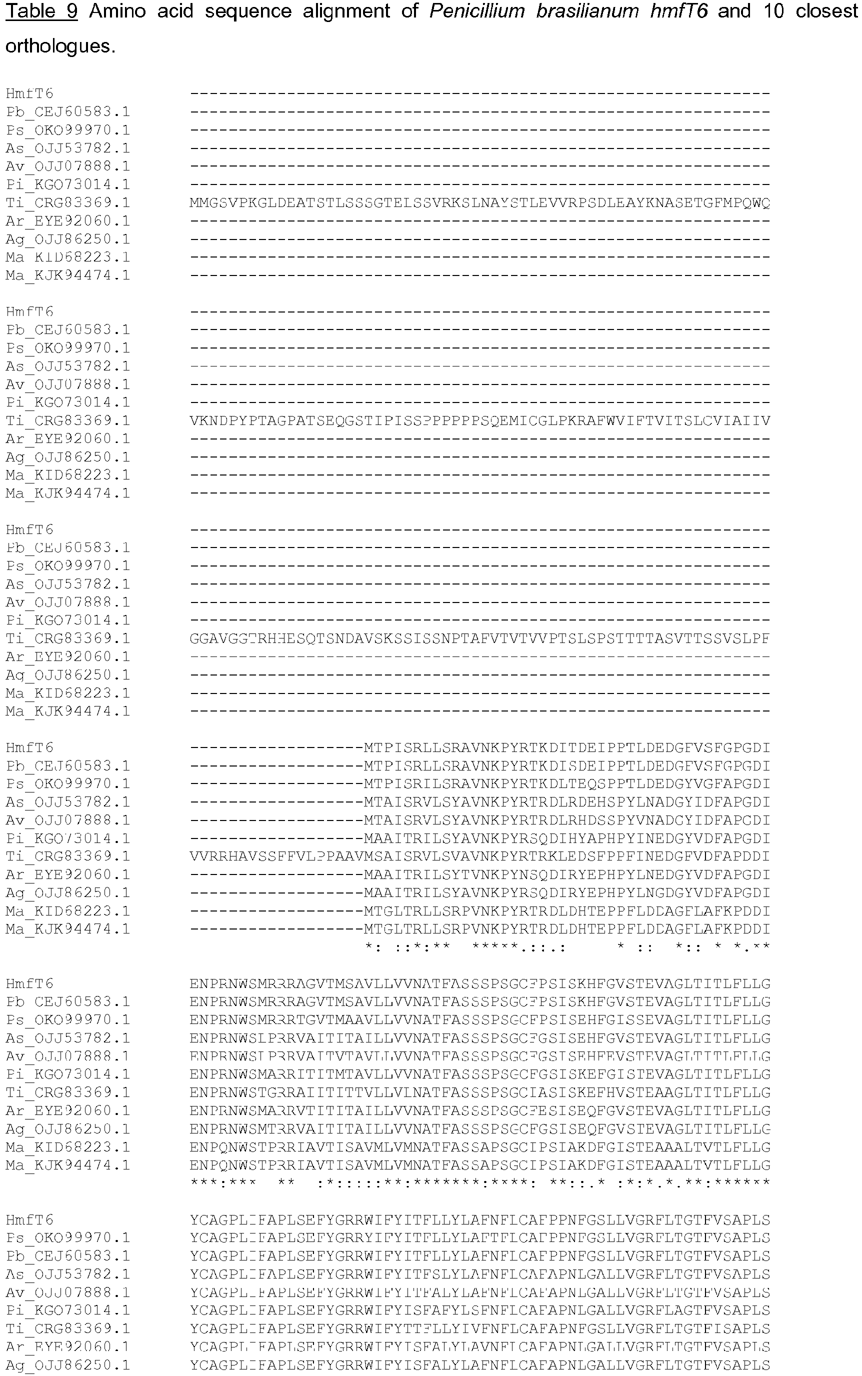

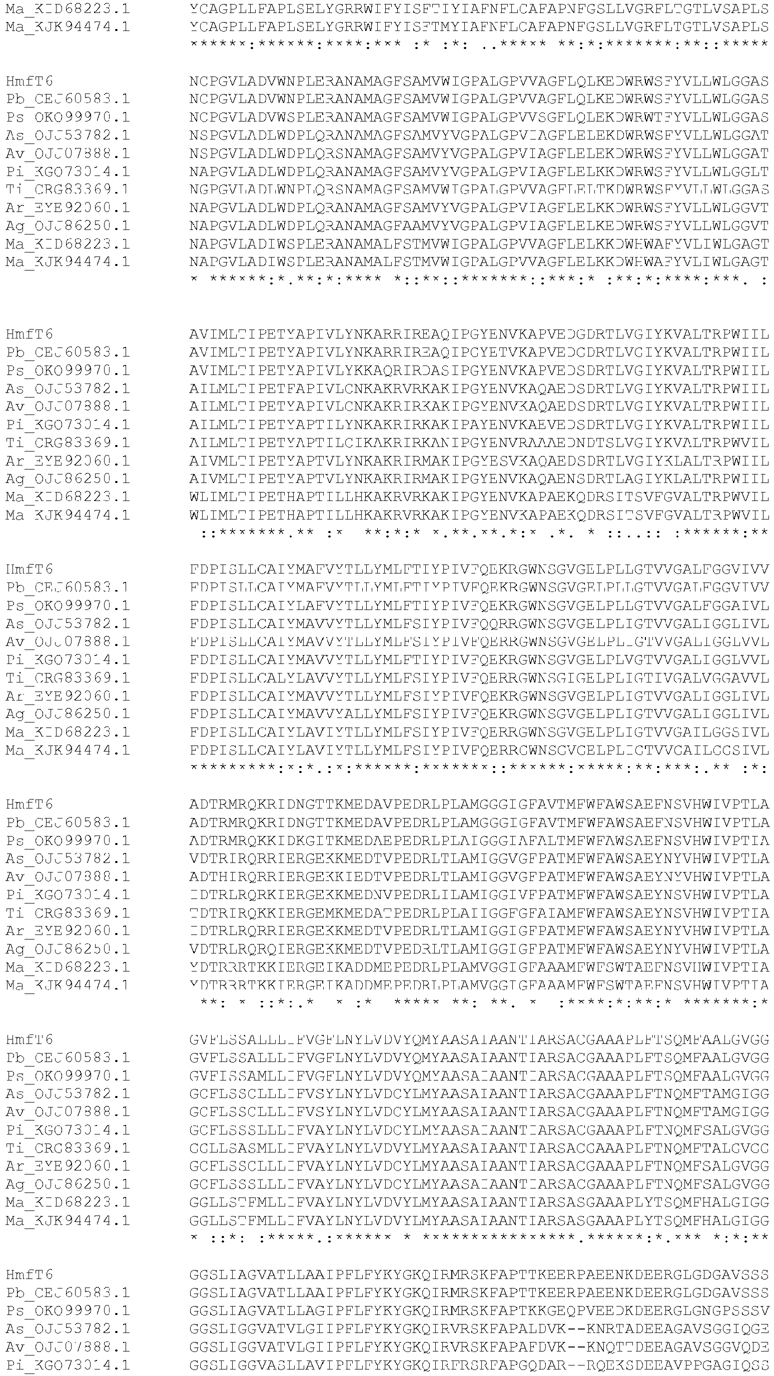

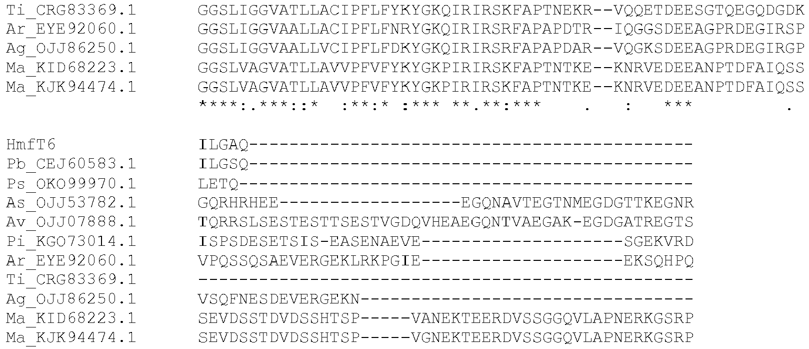

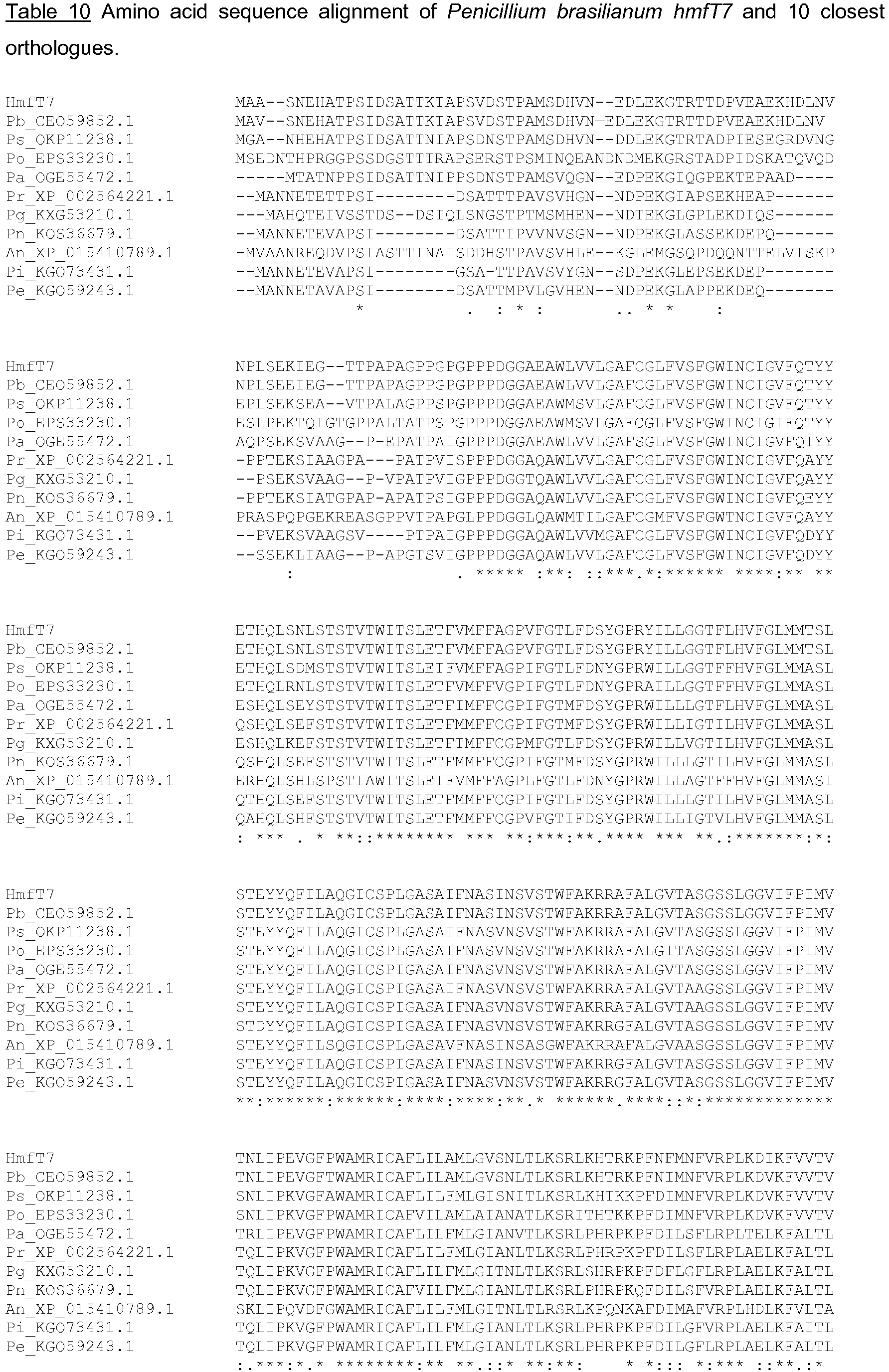

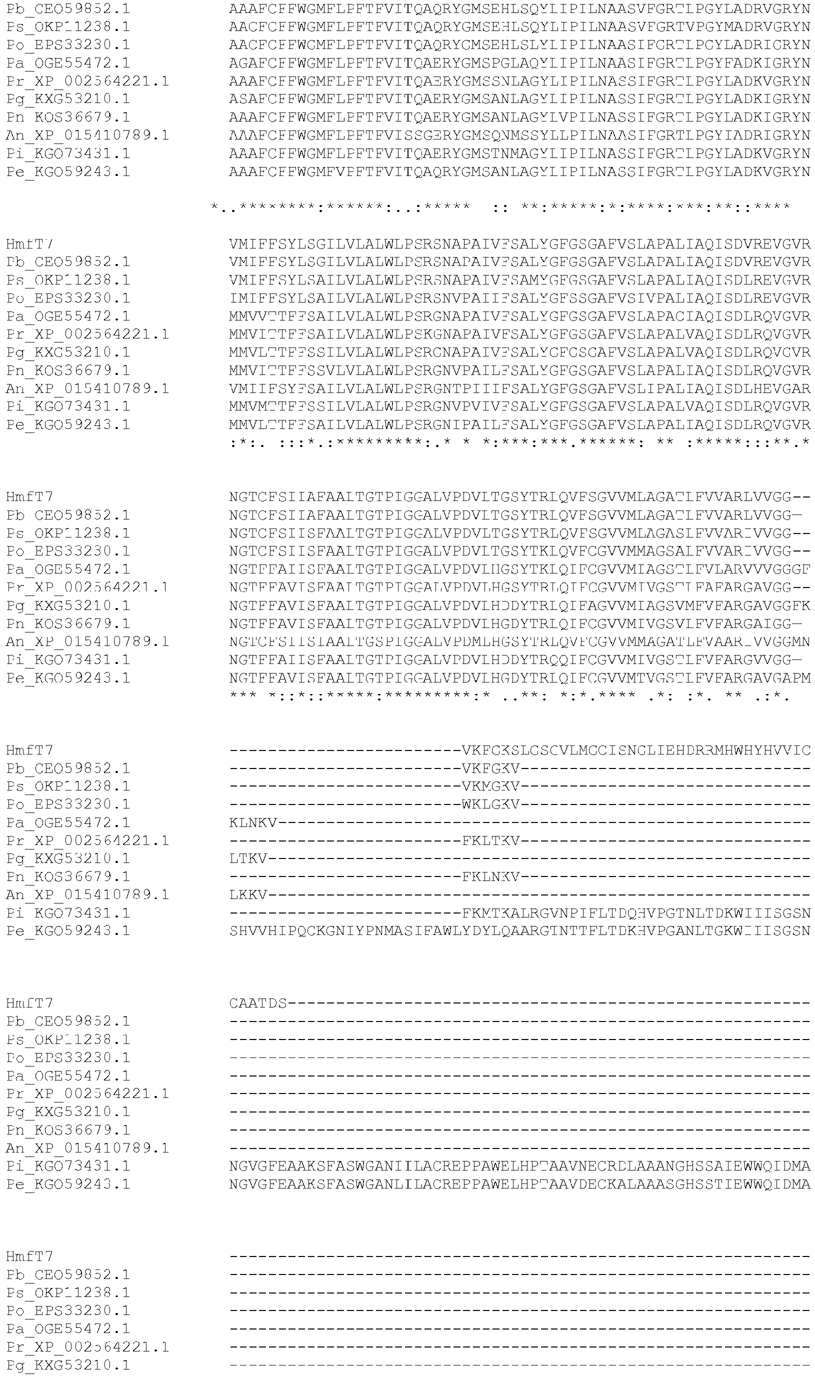

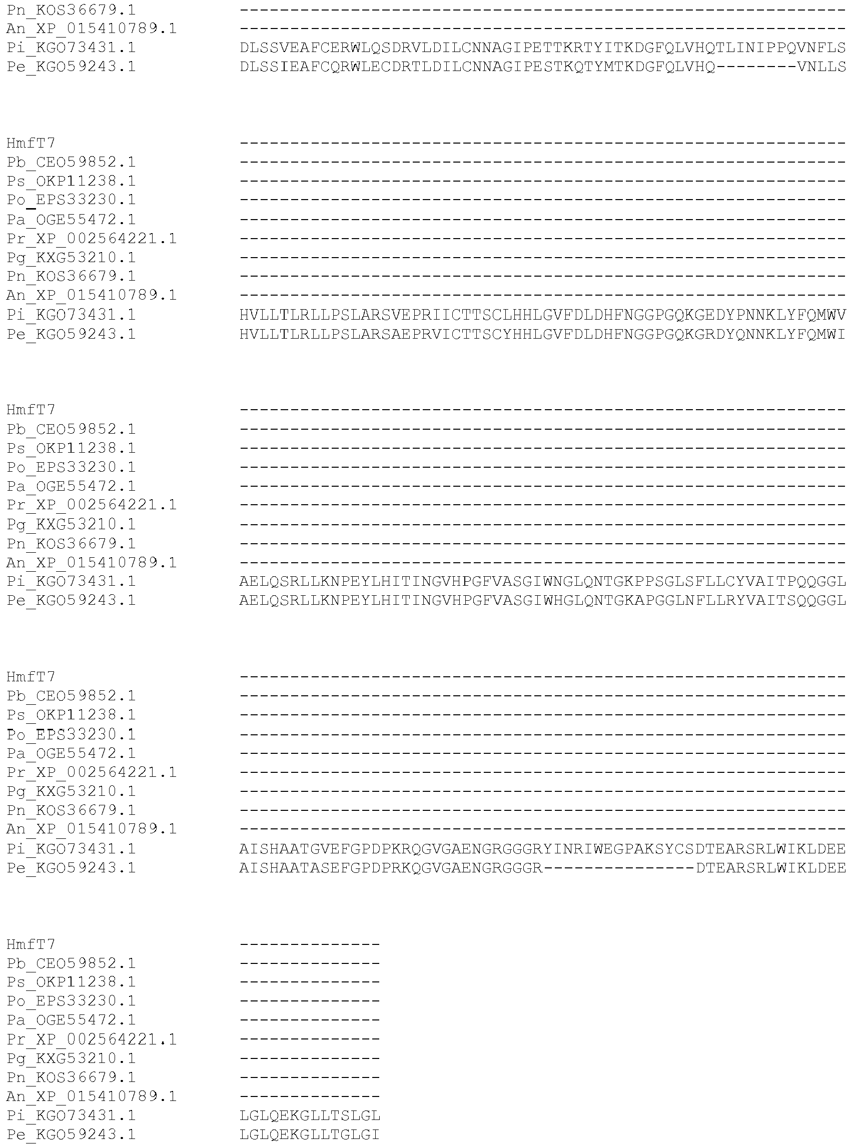

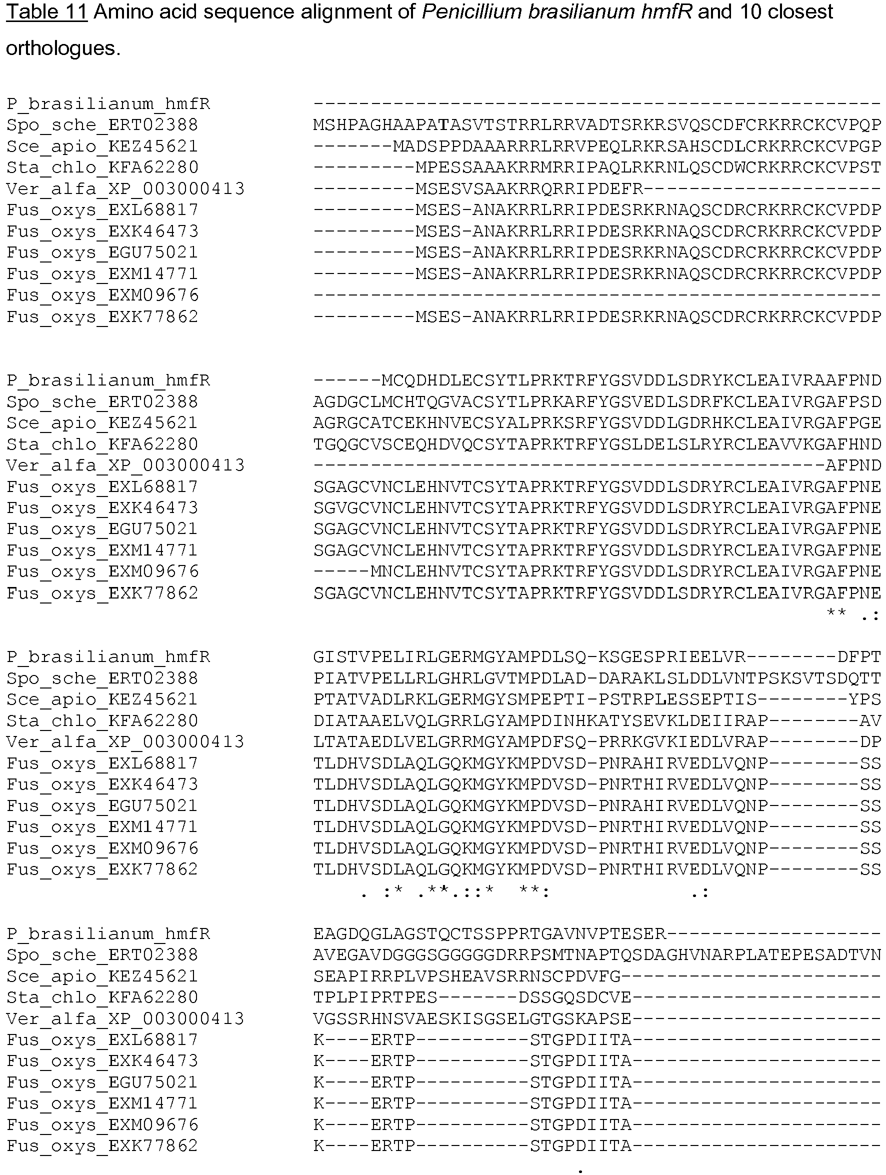

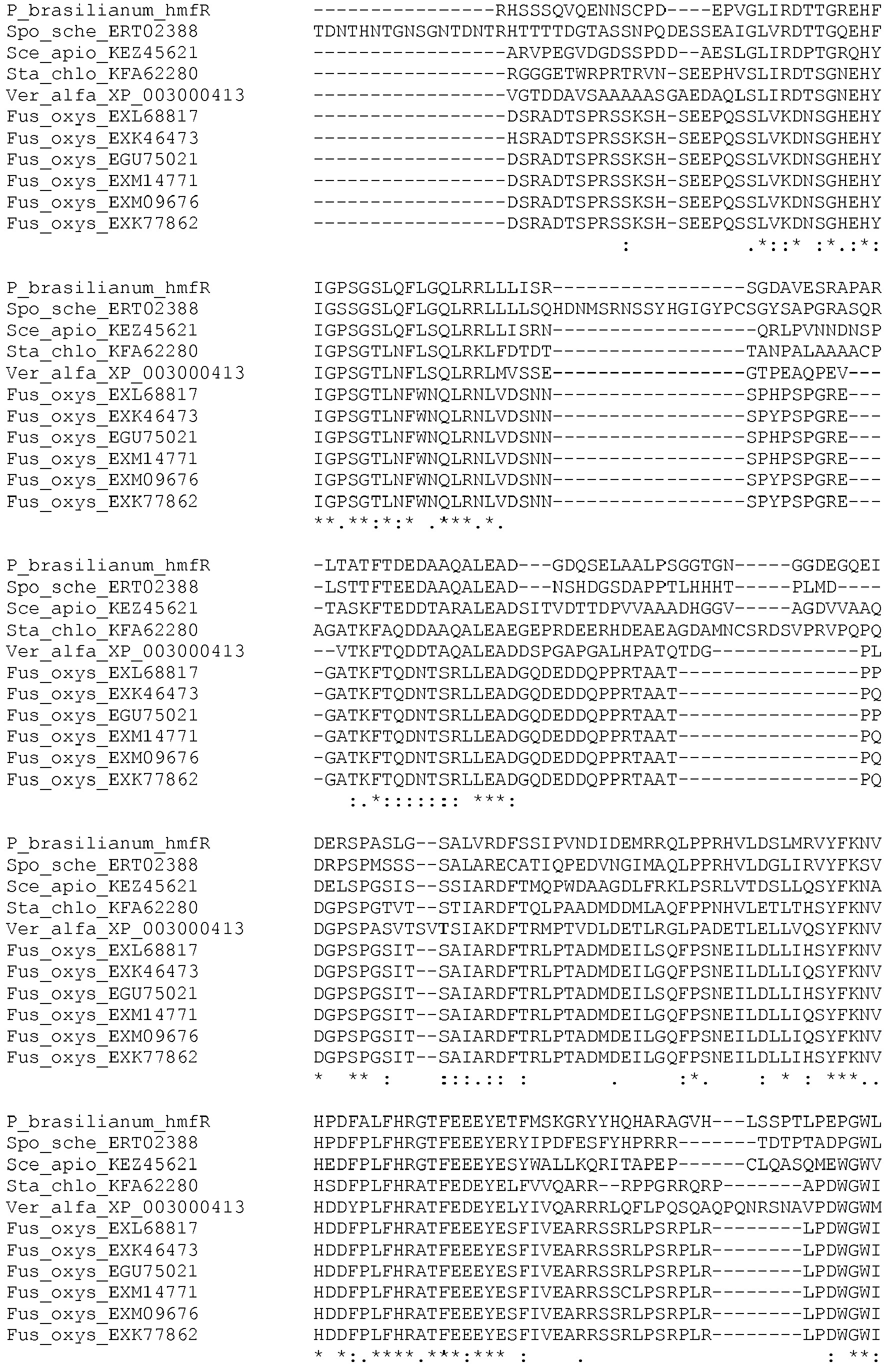

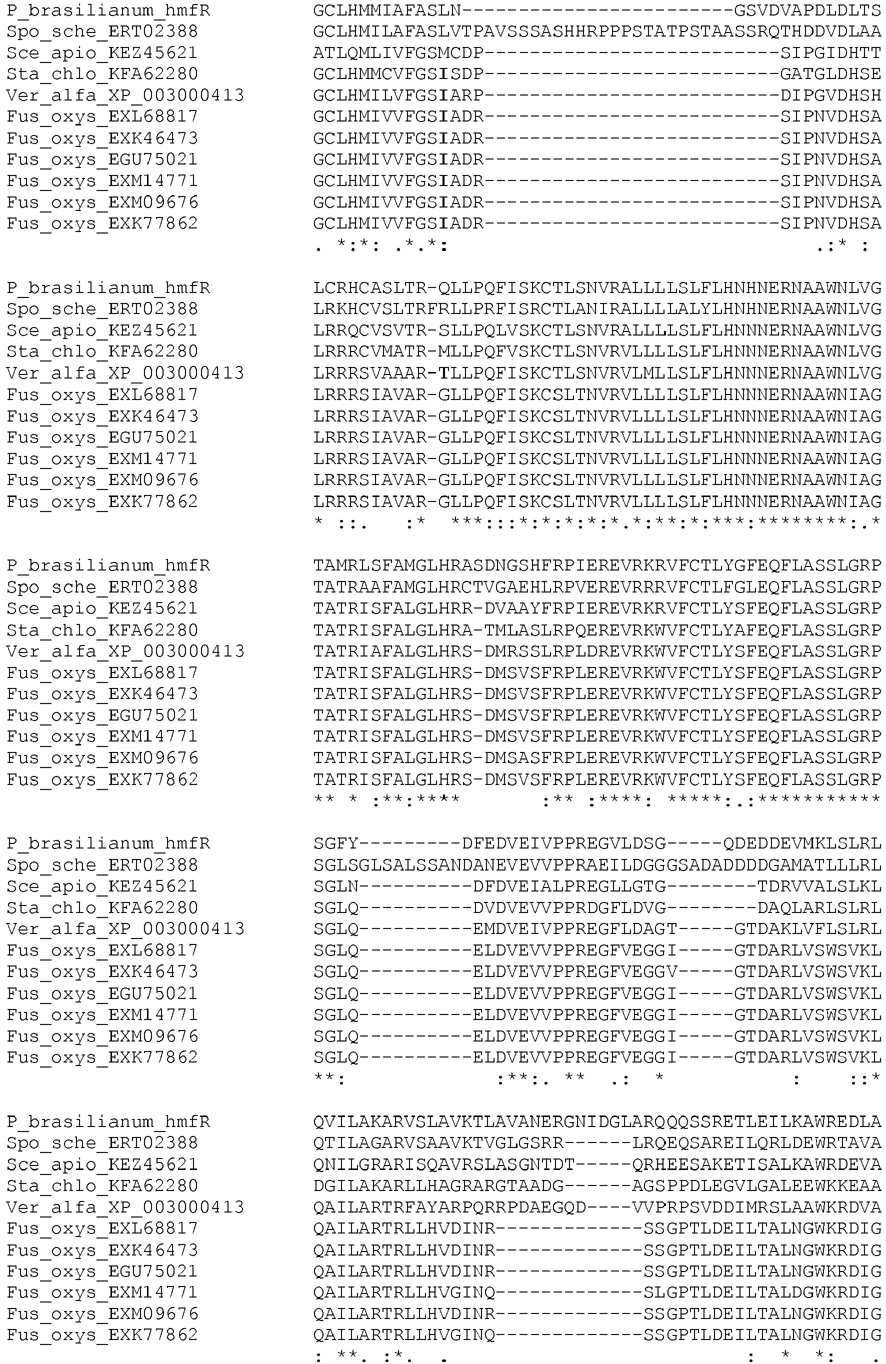

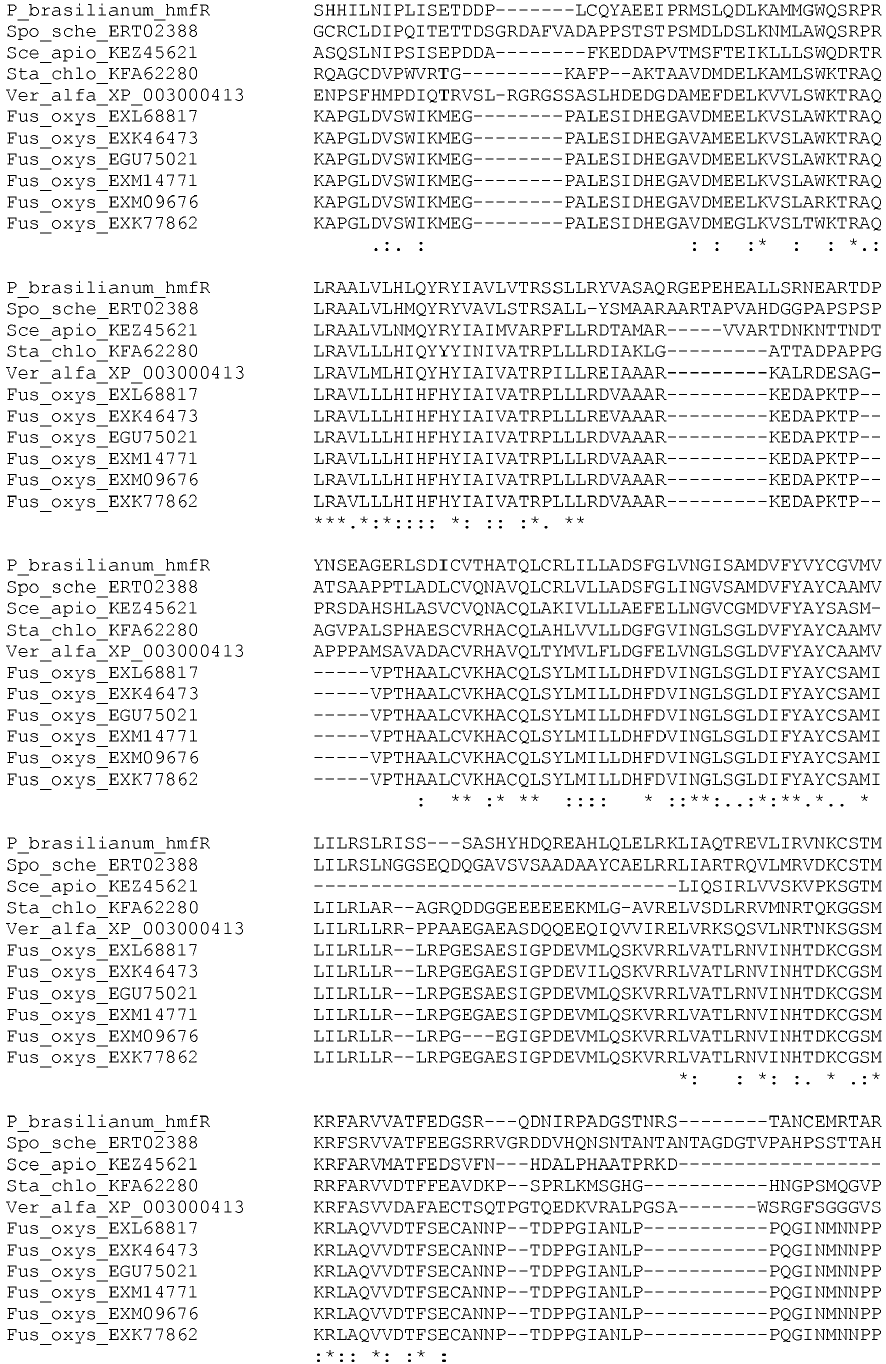

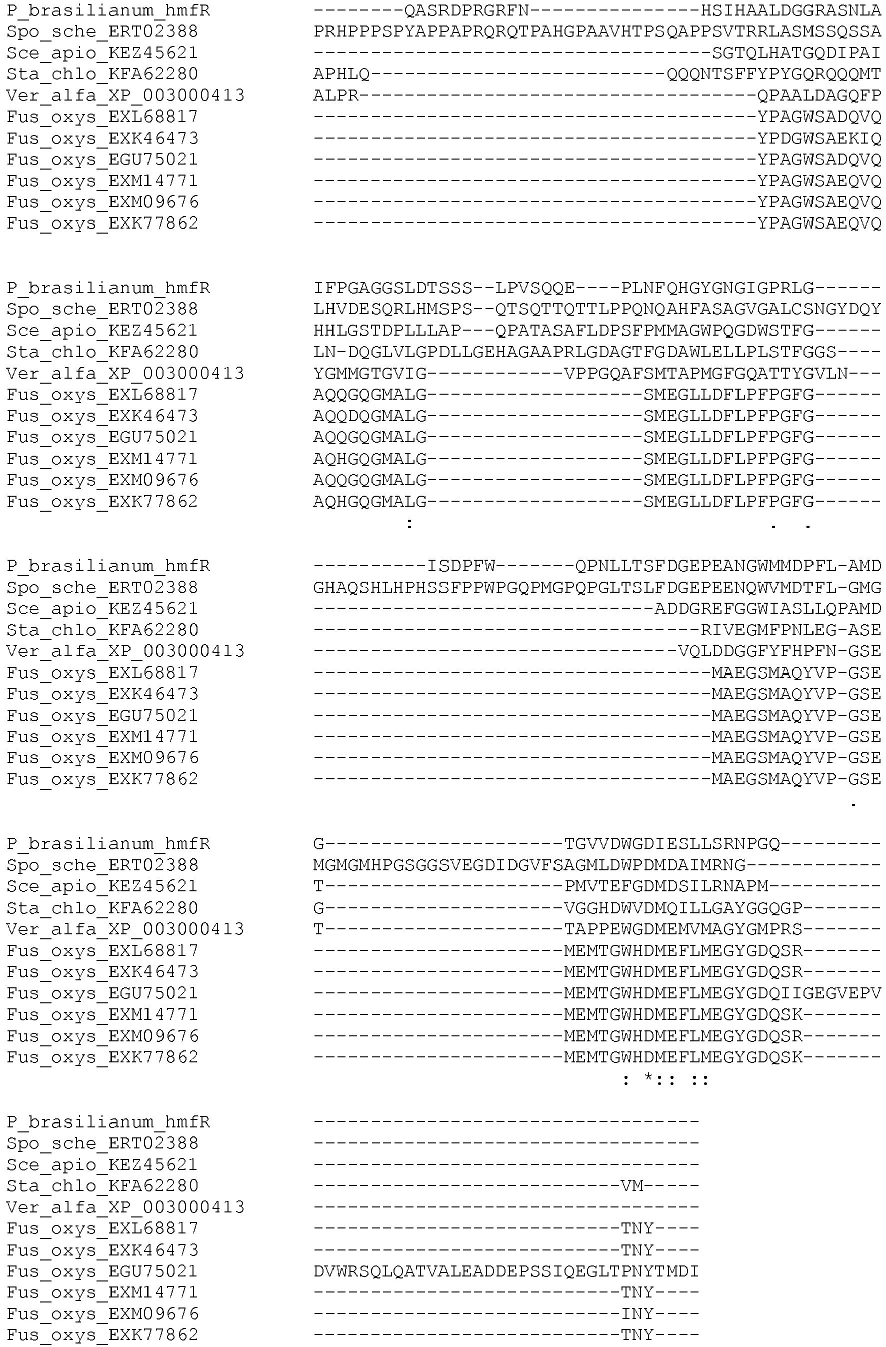

**Table 1A. Percentage amino acid sequence identity among Penicillium brasilianum hmfL1 orthologogues and accession numbers thereof.**

| Species | Accession | | | | | | | | | | | |
|---|---|---|---|---|---|---|---|---|---|---|---|---|
| Penicillium brasilianum hmfL1 | SEQ ID NO: 1 | 100,00 | 73,80 | 49,70 | 48,30 | 48,40 | 49,80 | 51,30 | 41,50 | 41,50 | 43,30 | 43,40 |
| Sporothrix schenckii ATCC 58251 | ERT02385 | 73,80 | 100,00 | 48,60 | 49,10 | 49,00 | 50,80 | 51,00 | 40,20 | 40,20 | 44,60 | 43,80 |
| Aspergillus kawachii IFO 4308 | GAA84694 | 49,70 | 48,60 | 100,00 | 61,40 | 84,90 | 61,50 | 84,70 | 38,10 | 38,10 | 35,50 | 39,90 |
| Byssochlamys spectabilis No. 5 | GAD98038 | 48,30 | 49,10 | 61,40 | 100,00 | 60,90 | 66,60 | 62,30 | 41,80 | 41,80 | 39,00 | 40,50 |
| Aspergillus niger CBS 513.88 | XP_001397354 | 48,40 | 49,00 | 84,90 | 60,90 | 100,00 | 60,00 | 99,70 | 36,60 | 36,60 | 34,50 | 38,00 |
| Eutypa lata UCREL1 | XP_007796771 | 49,80 | 50,80 | 61,50 | 66,60 | 60,00 | 100,00 | 62,10 | 38,90 | 38,90 | 35,60 | 38,30 |
| Aspergillus niger ATCC 1015 | EHA21652 | 51,30 | 51,00 | 84,70 | 62,30 | 99,70 | 62,10 | 100,00 | 39,00 | 39,00 | 35,80 | 39,00 |
| Fusarium graminearum | EYB30957 | 41,50 | 40,20 | 38,10 | 41,80 | 36,60 | 38,90 | 39,00 | 100,00 | 99,70 | 41,20 | 41,30 |
| Fusarium graminearum PH-1 | XP_011318199 | 41,50 | 40,20 | 38,10 | 41,80 | 36,60 | 38,90 | 39,00 | 99,70 | 100,00 | 41,50 | 41,30 |
| Rhizobium phaseoli | WP_016737077 | 43,30 | 44,60 | 35,50 | 39,00 | 34,50 | 35,60 | 35,80 | 41,20 | 41,50 | 100,00 | 67,70 |
| Dyella jiangningensis | WP_038619920 | 43,40 | 43,80 | 39,90 | 40,50 | 38,00 | 38,30 | 39,00 | 41,30 | 41,30 | 67,70 | 100,00 |

**Table 2A. Percentage amino acid sequence identity among Penicillium brasilianum hmfL2 orthologogues and accession numbers thereof.**

| Species | Accession | | | | | | | | | | | |
|---|---|---|---|---|---|---|---|---|---|---|---|---|
| Penicillium brasilianum hmfL2 | SEQ ID NO: 2 | 100,00 | 69,30 | 68,10 | 67,70 | 64,00 | 64,30 | 63,70 | 64,00 | 67,10 | 67,00 | 63,90 |
| Coccidioides immitis RS | XP_001244132 | 69,30 | 100,00 | 97,30 | 96,70 | 67,60 | 68,10 | 68,40 | 67,80 | 70,10 | 66,10 | 69,20 |
| Coccidioides posadasii C735 delta SOWgp | XP_003068662 | 68,10 | 97,30 | 100,00 | 98,20 | 65,00 | 65,70 | 67,80 | 65,30 | 67,60 | 66,60 | 69,50 |
| Coccidioides posadasii str. Silveira | EFW20539 | 67,70 | 96,70 | 98,20 | 100,00 | 64,70 | 65,30 | 67,40 | 65,00 | 67,20 | 66,20 | 69,10 |
| Trichophyton rubrum CBS 118892 | XP_003235253 | 64,00 | 67,60 | 65,00 | 64,70 | 100,00 | 97,60 | 65,20 | 97,30 | 89,10 | 65,20 | 82,00 |
| Trichophyton equinum CBS 127.97 | EGE05431 | 64,30 | 68,10 | 65,70 | 65,30 | 97,60 | 100,00 | 64,60 | 99,70 | 88,80 | 65,20 | 82,50 |
| Chaetomium globosum CBS 148.51 | XP_001220755 | 63,70 | 68,40 | 67,80 | 67,40 | 65,20 | 64,60 | 100,00 | 64,30 | 66,80 | 64,90 | 63,30 |
| Trichophyton tonsurans CBS 112818 | EGD92820 | 64,00 | 67,80 | 65,30 | 65,00 | 97,30 | 99,70 | 64,30 | 100,00 | 88,50 | 64,90 | 82,20 |
| Microsporum gypseum CBS 118893 | XP_003173798 | 67,10 | 70,10 | 67,60 | 67,20 | 89,10 | 88,80 | 66,80 | 88,50 | 100,00 | 65,60 | 85,20 |
| Endocarpon pusillum Z07020 | XP_007800835 | 67,00 | 66,10 | 66,60 | 66,20 | 65,20 | 65,20 | 64,90 | 64,90 | 65,60 | 100,00 | 67,60 |
| Arthroderma otae CBS 113480 | XP_002844685 | 63,90 | 69,20 | 69,50 | 69,10 | 82,00 | 82,50 | 63,30 | 82,20 | 85,20 | 67,60 | 100,00 |

**Table 3A. Percentage amino acid sequence identity among Penicillium brasilianum hmfN1 orthologogues and accession numbers thereof.**

| Species | Accession | | | | | | | | | | | |
|---|---|---|---|---|---|---|---|---|---|---|---|---|
| Penicillium brasilianum hmfN1 | SEQ ID NO: 3 | 100,00 | 70,80 | 64,90 | 62,80 | 64,20 | 62,70 | 61,90 | 62,50 | 62,50 | 57,90 | 60,80 |
| Sporothrix schenckii ATCC 58251 | ERT02387 | 70,80 | 100,00 | 59,90 | 58,10 | 62,10 | 60,10 | 59,30 | 59,90 | 60,10 | 52,90 | 57,50 |
| Scedosporium apiospermum | KEZ45623 | 64,90 | 59,90 | 100,00 | 64,30 | 67,20 | 64,70 | 65,30 | 64,30 | 64,50 | 59,90 | 61,00 |
| Podospora anserina S mat+ | XP_001908521 | 62,80 | 58,10 | 64,30 | 100,00 | 64,20 | 63,50 | 67,70 | 63,50 | 63,50 | 58,10 | 60,20 |
| Eutypa lata UCREL1 | XP_007794079 | 64,20 | 62,10 | 67,20 | 64,20 | 100,00 | 69,00 | 64,70 | 68,40 | 68,60 | 63,20 | 66,70 |
| Stachybotrys chartarum IBT 7711 | KEY72856 | 62,70 | 60,10 | 64,70 | 63,50 | 69,00 | 100,00 | 63,30 | 99,20 | 99,60 | 62,20 | 92,80 |
| Gaeumannomyces graminis var. tritici R3-111a-1 | XP_009217152 | 61,90 | 59,30 | 65,30 | 67,70 | 64,70 | 63,30 | 100,00 | 63,30 | 63,10 | 59,10 | 61,20 |
| Stachybotrys chartarum IBT 40288 | KFA73399 | 62,50 | 59,90 | 64,30 | 63,50 | 68,40 | 99,20 | 63,30 | 100,00 | 98,80 | 62,20 | 92,00 |
| Stachybotrys chartarum IBT 40293 | KFA53356 | 62,50 | 60,10 | 64,50 | 63,50 | 68,60 | 99,60 | 63,10 | 98,80 | 100,00 | 61,80 | 92,60 |
| Cyphellophora europaea CBS 101466 | XP_008712551 | 57,90 | 52,90 | 59,90 | 58,10 | 63,20 | 62,20 | 59,10 | 62,20 | 61,80 | 100,00 | 59,50 |
| Stachybotrys chlorohalonata IBT 40285 | KFA62282 | 60,80 | 57,50 | 61,00 | 60,20 | 66,70 | 92,80 | 61,20 | 92,00 | 92,60 | 59,50 | 100,00 |

**Table 4A. Percentage amino acid sequence identity among Penicillium brasilianum hmfK1 orthologogues and accession numbers thereof.**

| Species | Accession | | | | | | | | | | | |
|---|---|---|---|---|---|---|---|---|---|---|---|---|
| Penicillium brasilianum hmfK1 | SEQ ID NO: 4 | 100,00 | 82,20 | 80,60 | 73,90 | 73,90 | 80,00 | 78,50 | 74,20 | 74,50 | 72,80 | 69,80 |
| Scedosporium apiospermum | KEZ45619 | 82,20 | 100,00 | 79,60 | 76,10 | 76,10 | 77,50 | 78,50 | 76,30 | 71,70 | 73,10 | 68,40 |
| Togninia minima UCRPA7 | XP_007916105 | 80,60 | 79,60 | 100,00 | 75,60 | 75,60 | 76,10 | 82,60 | 76,30 | 70,30 | 75,60 | 70,50 |
| Stachybotrys chartarum IBT 7711 | KEY72859 | 73,90 | 76,10 | 75,60 | 100,00 | 99,80 | 73,40 | 73,50 | 95,40 | 68,40 | 73,80 | 67,40 |
| Stachybotrys chartarum IBT 40293 | KFA53358 | 73,90 | 76,10 | 75,60 | 99,80 | 100,00 | 73,40 | 73,50 | 95,20 | 68,40 | 73,80 | 67,40 |
| Sporothrix schenckii ATCC 58251 | ERT02390 | 80,00 | 77,50 | 76,10 | 73,40 | 73,40 | 100,00 | 72,50 | 72,90 | 75,50 | 69,10 | 66,00 |
| Eutypa lata UCREL1 | XP_007794919 | 78,50 | 78,50 | 82,60 | 73,50 | 73,50 | 72,50 | 100,00 | 74,30 | 67,70 | 72,10 | 69,70 |
| Stachybotrys chlorohalonata IBT 40285 | KFA62283 | 74,20 | 76,30 | 76,30 | 95,40 | 95,20 | 72,90 | 74,30 | 100,00 | 67,50 | 73,00 | 67,10 |
| Grosmannia clavigera kw1407 | EFX06428 | 74,50 | 71,70 | 70,30 | 68,40 | 68,40 | 75,50 | 67,70 | 67,50 | 100,00 | 65,30 | 64,20 |
| Cyphellophora europaea CBS 101466 | XP_008712555 | 72,80 | 73,10 | 75,60 | 73,80 | 73,80 | 69,10 | 72,10 | 73,00 | 65,30 | 100,00 | 70,60 |
| Byssochlamys spectabilis No. 5 | GAD98036 | 69,80 | 68,40 | 70,50 | 67,40 | 67,40 | 66,00 | 69,70 | 67,10 | 64,20 | 70,60 | 100,00 |

**Table 5A. Percentage amino acid sequence identity among Penicillium brasilianum hmfM orthologogues and accession numbers thereof.**

| Species | Accession | | | | | | | | | | | |
|---|---|---|---|---|---|---|---|---|---|---|---|---|
| Penicillium brasilianum hmfM | SEQ ID NO: 5 | 100,00 | 73,50 | 64,90 | 64,50 | 60,40 | 64,50 | 59,60 | 60,80 | 60,80 | 60,40 | 60,40 |
| Aspergillus nidulans FGSC A4 | XP_664054 | 73,50 | 100,00 | 60,80 | 62,90 | 60,40 | 69,00 | 58,80 | 58,40 | 58,40 | 58,00 | 58,00 |
| Eutypa lata UCREL1 | XP_007797627 | 64,90 | 60,80 | 100,00 | 65,00 | 58,50 | 56,90 | 61,80 | 65,40 | 65,00 | 65,40 | 65,40 |
| Thielavia terrestris NRRL 8126 | XP_003656972 | 64,50 | 62,90 | 65,00 | 100,00 | 58,50 | 61,80 | 55,70 | 65,40 | 64,60 | 65,00 | 65,00 |
| Trichoderma atroviride IMI 206040 | EHK50353 | 60,40 | 60,40 | 58,50 | 58,50 | 100,00 | 59,80 | 85,00 | 57,30 | 58,10 | 57,70 | 57,70 |
| Aspergillus terreus NIH2624 | XP_001212987 | 64,50 | 69,00 | 56,90 | 61,80 | 59,80 | 100,00 | 59,30 | 58,90 | 58,90 | 58,50 | 57,40 |
| Trichoderma reesei QM6a | XP_006962638 | 59,60 | 58,80 | 61,80 | 55,70 | 85,00 | 59,30 | 100,00 | 55,70 | 56,50 | 56,10 | 56,10 |
| Fusarium oxysporum f. sp. cubense race 4 | EMT67544 | 60,80 | 58,40 | 65,40 | 65,40 | 57,30 | 58,90 | 55,70 | 100,00 | 99,20 | 99,20 | 99,60 |
| Fusarium oxysporum Fo5176 | EGU79882 | 60,80 | 58,40 | 65,00 | 64,60 | 58,10 | 58,90 | 56,50 | 99,20 | 100,00 | 99,20 | 99,60 |
| Fusarium oxysporum f. sp. radicis-lycopersici 26381 | EXL52390 | 60,40 | 58,00 | 65,40 | 65,00 | 57,70 | 58,50 | 56,10 | 99,20 | 99,20 | 100,00 | 99,60 |
| Fusarium oxysporum f. sp. cubense race 1 | ENH63602 | 60,40 | 58,00 | 65,40 | 65,00 | 57,70 | 57,40 | 56,10 | 99,60 | 99,60 | 99,60 | 100,00 |

**Table 6A. Percentage amino acid sequence identity among Penicillium brasilianum hmfT3 orthologogues and accession numbers thereof.**

| | | | | | | | | | | | | |
|---|---|---|---|---|---|---|---|---|---|---|---|---|
| Species | Accession | | | | | | | | | | | |
| Penicillium brasilianum hmfT3 | SEQ ID NO: 6 | 100,00 | 85,10 | 81,90 | 81,70 | 80,50 | 75,90 | 75,90 | 80,20 | 75,20 | 75,20 | 75,20 |
| Penicillium rubens Wisconsin 54-1255 | XP_002560799 | 85,10 | 100,00 | 78,50 | 80,80 | 79,60 | 75,20 | 75,20 | 79,60 | 75,90 | 76,00 | 74,30 |
| Penicillium oxalicum 114-2 | EPS29964 | 81,90 | 78,50 | 100,00 | 77,40 | 77,10 | 72,60 | 72,70 | 76,60 | 71,90 | 72,00 | 71,60 |
| Aspergillus terreus NIH2624 | XP_001212020 | 81,70 | 80,80 | 77,40 | 100,00 | 78,30 | 73,90 | 73,90 | 79,00 | 74,60 | 74,60 | 73,00 |
| Fusarium oxysporum f. sp. cubense race 1 | ENH73763 | 80,50 | 79,60 | 77,10 | 78,30 | 100,00 | 99,80 | 99,60 | 88,00 | 93,10 | 92,90 | 98,60 |
| Fusarium oxysporum Fo5176 | EGU73369 | 75,90 | 75,20 | 72,60 | 73,90 | 99,80 | 100,00 | 99,70 | 87,70 | 91,90 | 91,50 | 98,20 |
| Fusarium oxysporum f. sp. cubense tropical race 4 54006 | EXL94287 | 75,90 | 75,20 | 72,70 | 73,90 | 99,60 | 99,70 | 100,00 | 87,70 | 91,70 | 91,40 | 97,90 |
| Nectria haematococca mpVI 77-13-4 | XP_003040064 | 80,20 | 79,60 | 76,60 | 79,00 | 88,00 | 87,70 | 87,70 | 100,00 | 87,60 | 88,30 | 87,30 |
| Fusarium pseudograminearum CS3096 | XP_009258565 | 75,20 | 75,90 | 71,90 | 74,60 | 93,10 | 91,90 | 91,70 | 87,60 | 100,00 | 99,00 | 91,30 |
| Fusarium graminearum PH-1 | XP_011323833 | 75,20 | 76,00 | 72,00 | 74,60 | 92,90 | 91,50 | 91,40 | 88,30 | 99,00 | 100,00 | 91,00 |
| Fusarium fujikuroi IMI 58289 | CCT64241 | 75,20 | 74,30 | 71,60 | 73,00 | 98,60 | 98,20 | 97,90 | 87,30 | 91,30 | 91,00 | 100,00 |

**Table 7A. Percentage amino acid sequence identity among Penicillium brasilianum hmfT4 orthologogues and accession numbers thereof.**

| Species | Accession | | | | | | | | | | | |
|---|---|---|---|---|---|---|---|---|---|---|---|---|
| Penicillium brasilianum hmfT4 | SEQ ID NO: 7 | 100,00 | 68,90 | 66,40 | 38,60 | 38,50 | 36,90 | 37,40 | 37,50 | 39,10 | 36,50 | 37,80 |
| Sporothrix schenckii ATCC 58251 | ERT02386 | 68,90 | 100,00 | 67,00 | 35,90 | 38,40 | 37,90 | 35,90 | 38,50 | 39,50 | 38,50 | 38,80 |
| Togninia minima UCRPA7 | XP_007915981 | 66,40 | 67,00 | 100,00 | 36,20 | 40,30 | 40,50 | 39,10 | 41,00 | 38,80 | 38,50 | 42,10 |
| Capronia coronata CBS 617.96 | XP_007724585 | 38,60 | 35,90 | 36,20 | 100,00 | 57,40 | 56,80 | 60,00 | 56,40 | 57,30 | 54,10 | 56,40 |
| Sporothrix schenckii ATCC 58251 | ERS98342 | 38,50 | 38,40 | 40,30 | 57,40 | 100,00 | 59,20 | 55,30 | 59,60 | 71,10 | 61,80 | 58,90 |
| Aspergillus kawachii IFO 4308 | GAA83620 | 36,90 | 37,90 | 40,50 | 56,80 | 59,20 | 100,00 | 53,40 | 80,60 | 60,30 | 56,20 | 81,30 |
| Capronia coronata CBS 617.96 | XP_007725190 | 37,40 | 35,90 | 39,10 | 60,00 | 55,30 | 53,40 | 100,00 | 53,40 | 55,70 | 52,90 | 53,60 |
| Aspergillus niger CBS 513.88 | XP_001389139 | 37,50 | 38,50 | 41,00 | 56,40 | 59,60 | 80,60 | 53,40 | 100,00 | 61,70 | 56,00 | 100,00 |
| Grosmannia clavigera kw1407 | EFX04858 | 39,10 | 39,50 | 38,80 | 57,30 | 71,10 | 60,30 | 55,70 | 61,70 | 100,00 | 61,40 | 61,40 |
| Sporothrix schenckii ATCC 58251 | ERS94853 | 36,50 | 38,50 | 38,50 | 54,10 | 61,80 | 56,20 | 52,90 | 56,00 | 61,40 | 100,00 | 55,70 |
| Aspergillus niger ATCC 1015 | EHA26600 | 37,80 | 38,80 | 42,10 | 56,40 | 58,90 | 81,30 | 53,60 | 100,00 | 61,40 | 55,70 | 100,00 |

**Table 8A. Percentage amino acid sequence identity among Penicillium brasilianum hmfT5 orthologogues and accession numbers thereof.**

| Species | Accession | | | | | | | | | | | |
|---|---|---|---|---|---|---|---|---|---|---|---|---|
| Penicillium brasilianum hmfT5 | SEQ ID NO: 8 | 100,00 | 84,00 | 83,90 | 84,30 | 82,20 | 82,30 | 82,20 | 82,40 | 81,00 | 81,10 | 81,00 |
| Penicillium digitatum Pd1 | EKV20717 | 84,00 | 100,00 | 99,90 | 91,40 | 79,10 | 79,20 | 79,30 | 80,80 | 79,10 | 79,10 | 76,80 |
| Penicillium digitatum PHI26 | EKV19541 | 83,90 | 99,90 | 100,00 | 91,30 | 79,10 | 79,10 | 79,20 | 80,70 | 79,00 | 79,00 | 76,80 |
| Penicillium rubens Wisconsin 54-1255 | XP_002565665 | 84,30 | 91,40 | 91,30 | 100,00 | 80,10 | 80,20 | 80,00 | 81,60 | 80,90 | 80,40 | 77,90 |
| Aspergillus oryzae 100-8 | KDE82314 | 82,20 | 79,10 | 79,10 | 80,10 | 100,00 | 100,00 | 99,60 | 82,40 | 81,60 | 81,30 | 75,60 |
| Aspergillus oryzae 3.042 | EIT77345 | 82,30 | 79,20 | 79,10 | 80,20 | 100,00 | 100,00 | 99,60 | 82,40 | 81,60 | 81,30 | 75,70 |
| Aspergillus flavus NRRL3357 | XP_002380612 | 82,20 | 79,30 | 79,20 | 80,00 | 99,60 | 99,60 | 100,00 | 82,50 | 81,60 | 81,30 | 75,70 |
| Aspergillus terreus NIH2624 | XP_001208847 | 82,40 | 80,80 | 80,70 | 81,60 | 82,40 | 82,40 | 82,50 | 100,00 | 84,90 | 84,80 | 76,10 |
| Aspergillus kawachii IFO 4308 | GAA86951 | 81,00 | 79,10 | 79,00 | 80,90 | 81,60 | 81,60 | 81,60 | 84,90 | 100,00 | 97,40 | 75,70 |
| Aspergillus niger CBS 513.88 | XP_001400982 | 81,10 | 79,10 | 79,00 | 80,40 | 81,30 | 81,30 | 81,30 | 84,80 | 97,40 | 100,00 | 75,40 |
| Ophiostoma piceae UAMH 11346 | EPE02908 | 81,00 | 76,80 | 76,80 | 77,90 | 75,60 | 75,70 | 75,70 | 76,10 | 75,70 | 75,40 | 100,00 |

**Table 9A. Percentage amino acid sequence identity among Penicillium brasilianum hmfT6 orthologues and accession numbers thereof.**

| Species | Accession | | | | | | | | | | | |
|---|---|---|---|---|---|---|---|---|---|---|---|---|
| Penicillium brasilianum hmfT6 | SEQ ID NO: 9 | 100 | 99,4 | 90,7 | 78,8 | 78,1 | 79,2 | 78,1 | 78,6 | 78,2 | 72,8 | 72,8 |
| Penicillium brasilianum | CEJ60583.1 | 99,4 | 100 | 90,3 | 78,6 | 77,9 | 79 | 77,9 | 78,6 | 78 | 72,6 | 72.,6 |
| Penicillium subrubescens PENSUB_8187 | OKO99970.1 | 90,7 | 90,3 | 100 | 77,2 | 76,9 | 77,8 | 77,8 | 76,6 | 76,2 | 72,1 | 72,1 |
| Aspergillus sydowii CBS 593.65 | OJJ53782.1 | 78,8 | 78,6 | 77,2 | 100 | 94 | 80,8 | 79,4 | 83,4 | 85,8 | 69,1 | 69,1 |
| Aspergillus versicolor CBS 583.65 | OJJ07888.1 | 78,1 | 77,9 | 76,9 | 94 | 100 | 81 | 80,4 | 82,7 | 84,3 | 67,1 | 67,1 |
| Penicillium italicum | KGO73014.1 | 79,2 | 79 | 77,8 | 80,8 | 81 | 100 | 78,8 | 86,5 | 88,2 | 69 | 69 |
| Talaromyces islandicus | CRG83369.1 | 78,1 | 77,9 | 77,8 | 79,4 | 80,4 | 78,8 | 100 | 77,7 | 77,7 | 74,6 | 74,6 |
| Aspergillus ruber CBS 135680 | EYE92060.1 | 78,6 | 78,6 | 76,6 | 83,4 | 82,7 | 86,5 | 77,7 | 100 | 94,6 | 67,8 | 67,8 |
| Aspergillus glaucus CBS 516.65 | OJJ86250.1 | 78,2 | 78 | 76,2 | 85,8 | 84,3 | 88,2 | 77,7 | 94,6 | 100 | 69,2 | 69,2 |
| Metarhizium anisopliae | KID68223.1 | 72,8 | 72,6 | 72,1 | 69,1 | 67,1 | 69 | 74,6 | 67,8 | 69,2 | 100 | 99,6 |
| Metarhizium anisopliae BRIP 53293 | KJK94474.1 | 72,8 | 72,6 | 72,1 | 69,1 | 67,1 | 69 | 74,6 | 67,8 | 69,2 | 99,6 | 100 |

**Table 10A. Percentage amino acid sequence identity among Penicillium brasilianum hmfT7 orthologues and accession numbers thereof.**

| Species | Accession | | | | | | | | | | | |
|---|---|---|---|---|---|---|---|---|---|---|---|---|
| Penicillium brasilianum hmfT7 | SEQ ID NO: 10 | 100 | 98,7 | 89,1 | 80,5 | 76,1 | 76,4 | 74,9 | 73,9 | 71,5 | 70,6 | 70 |
| Penicillium brasilianum PMG11_04505 | CEO59852.1 | 98,7 | 100 | 89,3 | 80,8 | 76,1 | 76,4 | 74,5 | 73,9 | 71,7 | 75,5 | 75 |
| Penicillium subrubescens | OKP11238.1 | 89,1 | 89,3 | 100 | 84,5 | 77 | 76,6 | 74,7 | 74,3 | 73,2 | 75,5 | 74,8 |
| Penicillium oxalicum 114-2 | EPS33230.1 | 80,5 | 80,8 | 84,5 | 100 | 74,2 | 73,4 | 72,5 | 73,4 | 69,6 | 72,7 | 72,8 |
| Penicillium arizonense PENARI_c004G06722 | OGE55472.1 | 76,1 | 76,1 | 77 | 74,2 | 100 | 86,6 | 84,9 | 84,6 | 69,7 | 84,9 | 82,4 |
| Penicillium rubens Wisconsin 54-1255 | XP_002564221.1 | 76,4 | 76,4 | 76,6 | 73,4 | 86,6 | 100 | 89,3 | 92,2 | 70,8 | 91 | 90,2 |
| Penicillium griseofulvum | KXG53210.1 | 74,9 | 74,5 | 74,7 | 72,5 | 84,9 | 89,3 | 100 | 87,2 | 69,1 | 87,5 | 86,5 |
| Penicillium nordicum | KOS36679.1 | 73,9 | 73,9 | 74,3 | 73,4 | 84,6 | 92,2 | 87,2 | 100 | 69,1 | 91 | 90 |
| Aspergillus nomius NRRL 13137 | XP_015410789.1 | 71,5 | 71,7 | 73,2 | 69,6 | 69,7 | 70,8 | 69,1 | 69,1 | 100 | 71,2 | 69,1 |
| Penicillium italicum | KGO73431.1 | 70,6 | 75,5 | 75,5 | 72,7 | 84,9 | 91 | 87,5 | 91 | 71,2 | 100 | 88,4 |
| Penicillium expansum | KGO59243.1 | 70 | 75 | 74,8 | 72,8 | 82,4 | 90,2 | 86,5 | 90 | 69,1 | 88,4 | 100 |

**Table 11A. Percentage amino acid sequence identity among Penicillium brasilianum hmfR orthologogues and accession numbers thereof.**

| Species | Accession | | | | | | | | | | | |
|---|---|---|---|---|---|---|---|---|---|---|---|---|
| Penicillium brasilianum hmfR | SEQ ID NO: 11 | 100,00 | 52,30 | 46,40 | 41,70 | 40,60 | 41,70 | 41,80 | 41,70 | 41,70 | 41,70 | 41,70 |
| Sporothrix schenckii ATCC 58251 | ERT02388 | 52,30 | 100,00 | 43,10 | 39,60 | 36,70 | 41,80 | 41,90 | 41,80 | 41,90 | 40,30 | 41,80 |
| Scedosporium apiospermum | KEZ45621 | 46,40 | 43,10 | 100,00 | 43,50 | 42,30 | 45,90 | 45,70 | 45,90 | 46,10 | 44,80 | 45,90 |
| Stachybotrys chlorohalonata IBT 40285 | KFA62280 | 41,70 | 39,60 | 43,50 | 100,00 | 51,50 | 56,30 | 55,90 | 56,30 | 56,00 | 55,30 | 56,30 |
| Verticillium alfalfae VaMs.102 | XP_003000413 | 40,60 | 36,70 | 42,30 | 51,50 | 100,00 | 53,90 | 54,00 | 53,90 | 54,00 | 53,10 | 53,70 |
| Fusarium oxysporum f. sp. conglutinans race 2 54008 | EXL68817 | 41,70 | 41,80 | 45,90 | 56,30 | 53,90 | 100,00 | 97,90 | 99,60 | 98,40 | 98,60 | 98,50 |
| Fusarium oxysporum f. sp. melonis 26406 | EXK46473 | 41,80 | 41,90 | 45,70 | 55,90 | 54,00 | 97,90 | 100,00 | 97,50 | 97,80 | 98,20 | 97,90 |
| Fusarium oxysporum Fo5176 | EGU75021 | 41,70 | 41,80 | 45,90 | 56,30 | 53,90 | 99,60 | 97,50 | 100,00 | 98,10 | 98,30 | 98,30 |
| Fusarium oxysporum f. sp. vasinfectum 25433 | EXM14771 | 41,70 | 41,90 | 46,10 | 56,00 | 54,00 | 98,40 | 97,80 | 98,10 | 100,00 | 98,30 | 99,10 |
| Fusarium oxysporum f. sp. cubense tropical race 4 54006 | EXM09676 | 41,70 | 40,30 | 44,80 | 55,30 | 53,10 | 98,60 | 98,20 | 98,30 | 98,30 | 100,00 | 98,40 |
| Fusarium oxysporum f. sp. raphani 54005 | EXK77862 | 41,70 | 41,80 | 45,90 | 56,30 | 53,70 | 98,50 | 97,90 | 98,30 | 99,10 | 98,40 | 100,00 |

### Description of the figures

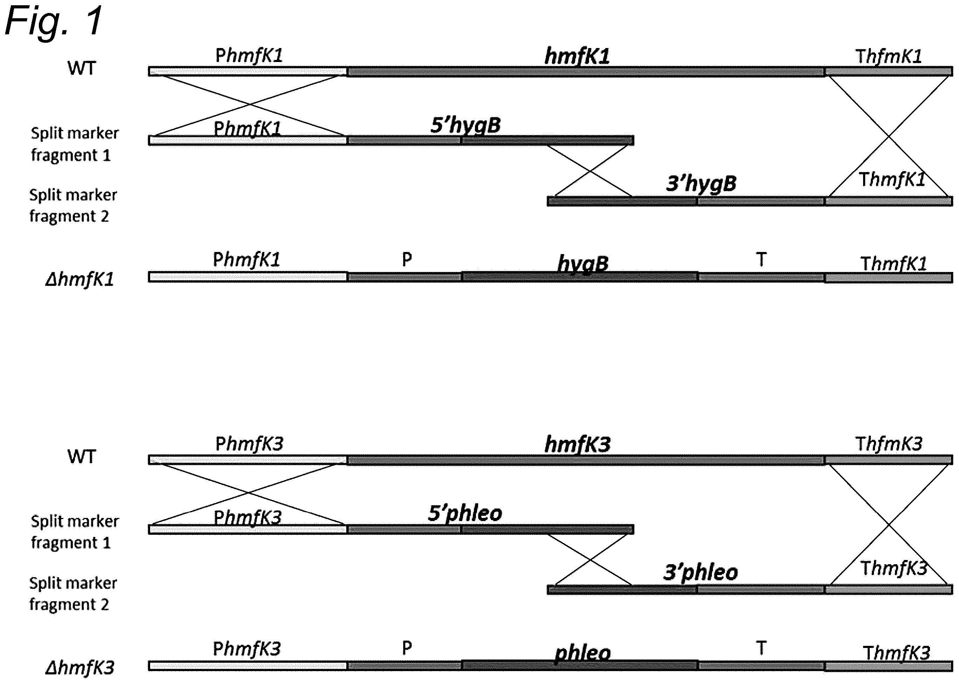

Figure 1. Split marker approach to create *hmfK1* (top) and *hmfK3* (bottom) deletions, using *hygB* and *phleo* antibiotic markers, respectively.

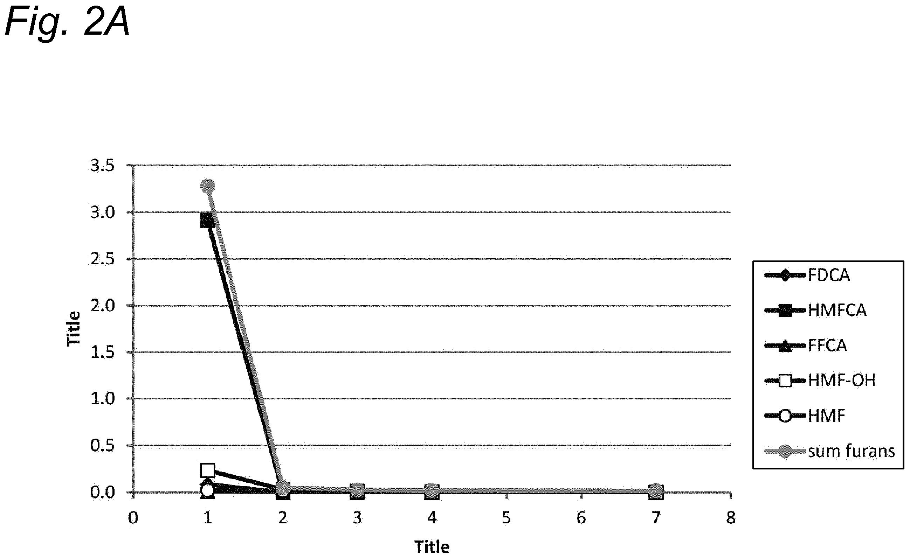

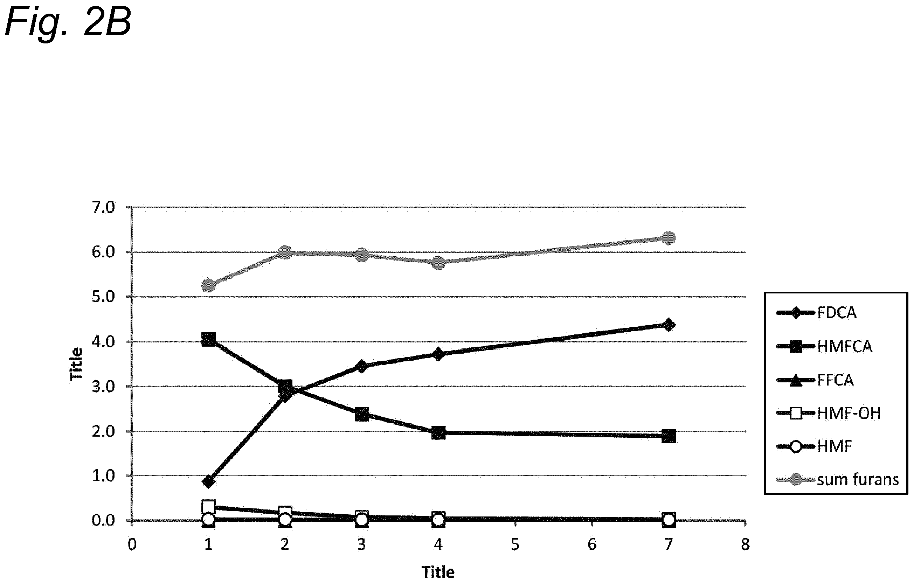

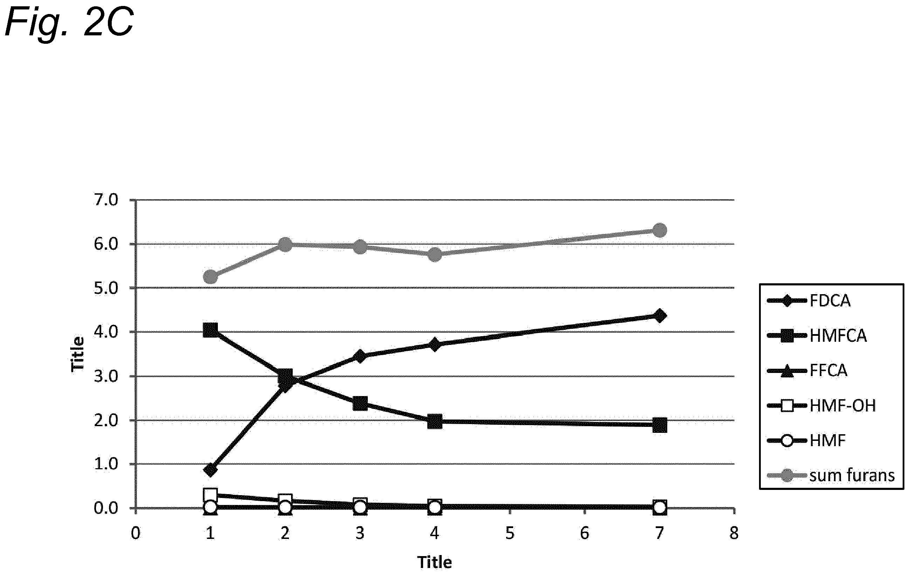

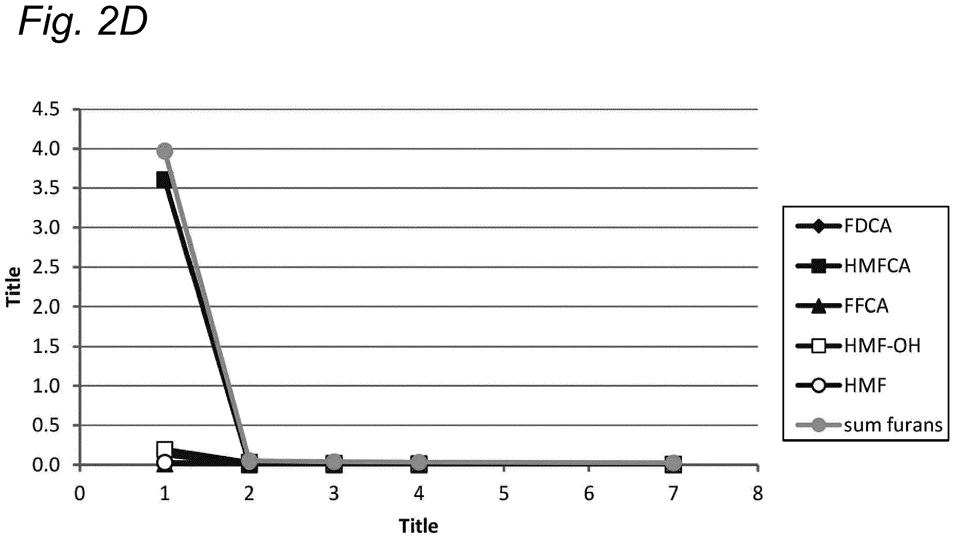

Figure 2. Concentrations of HMF, FDCA and intermediates measured in supernatants of cultures of wild type P. *brasilianum* C1 (A), and the P. *brasilianum* C1 disruption mutant strains Δ*hmfK1* #26 (B), Δ*hmfK1* #30 (C) and Δ*hmfK3* #43 (D), grown in minimal medium supplemented with 5mM glucose and 6 mM HMF at 30°C in shake flasks at 250 rpm.

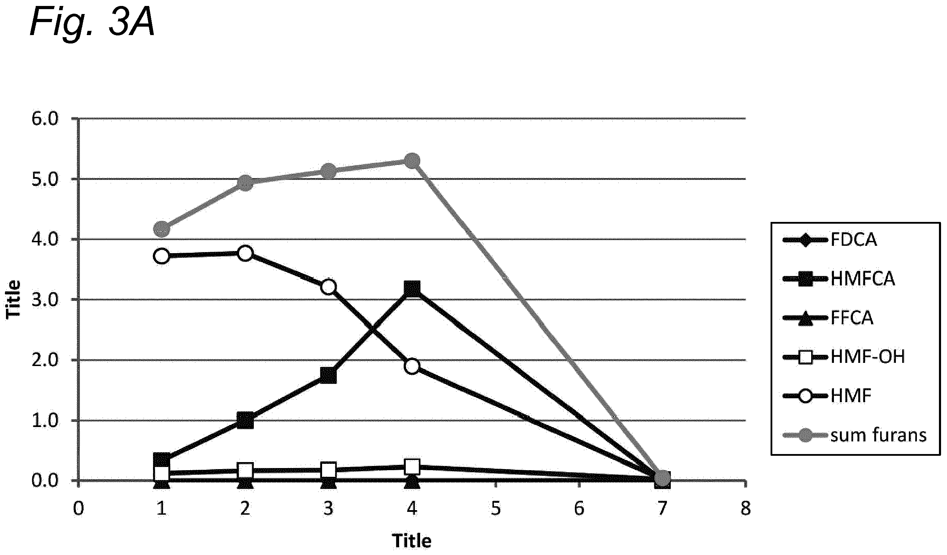

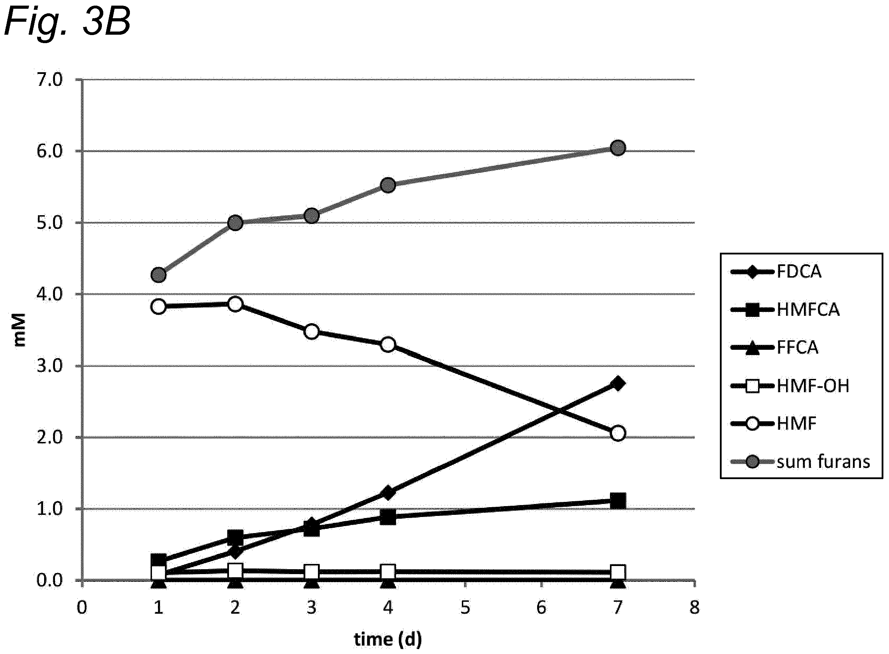

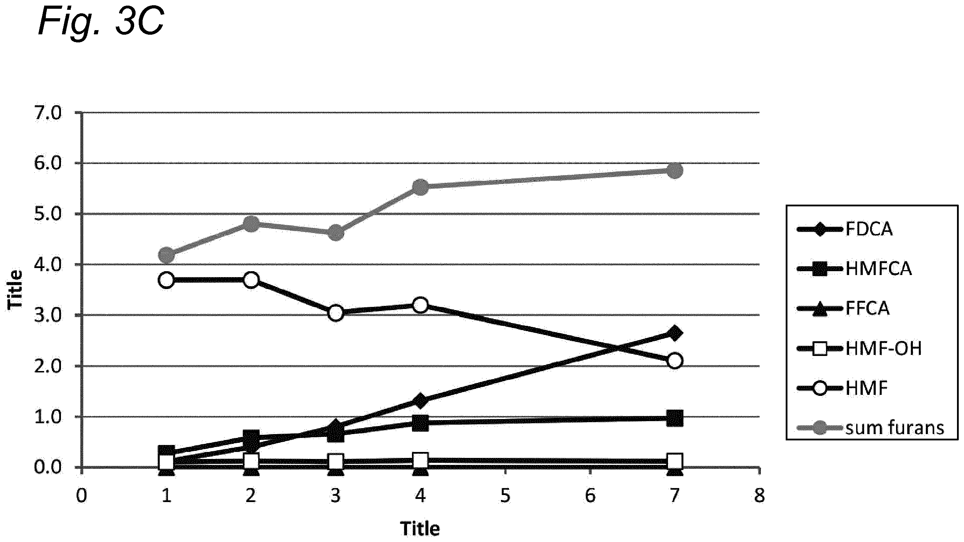

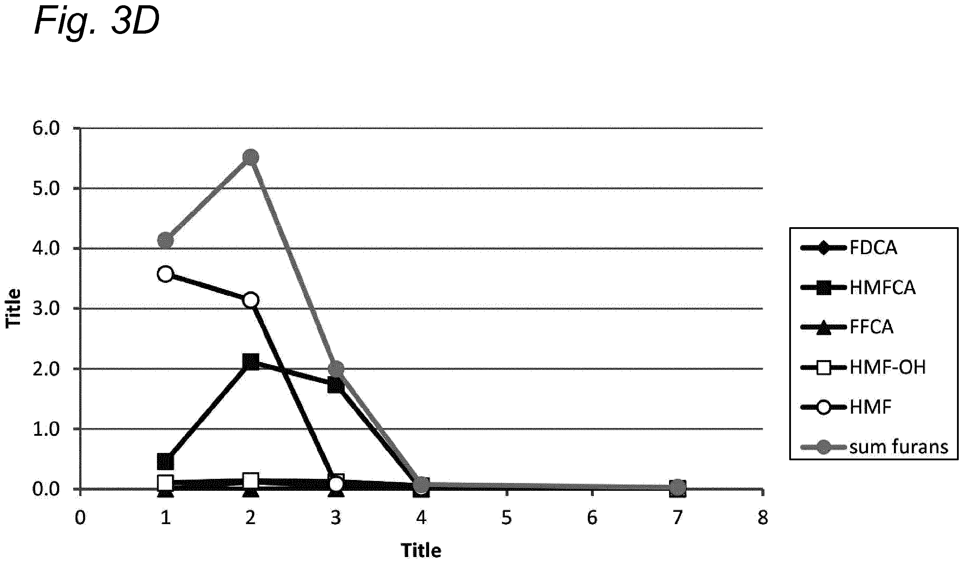

Figure 3. Concentrations of HMF, FDCA and intermediates measured in supernatants of cultures of wild type P. *brasilianum* C1 (A), and the P. *brasilianum* C1 disruption mutant strains Δ*hmfK1* #26 (B), Δ*hmfK1* #30 (C) and Δ*hmfK3* #43 (D), grown in minimal medium supplemented with 5mM citrate and 6 mM HMF at 30°C in shake flasks at 250 rpm. After 4 days ∼1% of the glucose containing culture of #26 and #30 was used to re-inoculate the citrate containing flasks.

### Examples

### Introduction

In co-pending International application PCT/EP2016/072406 the inventors have described that the isolation from Dutch soil of a *Penicillium brasilianum* strain. This *P*. *brasilianum,* referred to *P. brasilianum* C1 in application PCT/EP2016/072406, was isolated by growth selection on HMF, a precursor for FDCA. Herein, the *P*. *brasilianum* C1 strain will be referred to as the wild type (WT) WT *P*. *brasilianum* strain or the WT strain. International application PCT/EP2016/072406 describes the sequencing and annotation of the genome of *P*. *brasilianum* C1, as well as the sequencing of the transcriptome of the strain grown on HMF and on citric acid. Based on the genome annotation and blasting genes against public databases as well as on differential expression RNA-sequencing results, a list has been compiled of candidate genes that are involved in encoding enzymes that are involved in the catabolism of HMF by *P. brasilianum* C1 via a proposed HMF to furoic acid (via FDCA) pathway, including a salicylate hydroxylase *hmfK1*, two alcohol dehydrogenases *hmfL1* and *hmfL2* and a salicylaldehyde dehydrogenase *hmfN1.* By heterologous expression in a *Pseudomonas* strain, the Examples of PCT/EP2016/072406 show that the *P*. *brasilianum hmfK1* hydroxylase indeed acts as a decarboxylating monooxygenase on FDCA and thus is involved in the degradation of FDCA in *P*. *brasilianum.* Furthermore, by heterologous expression in yeast of the *P*. *brasilianum hmfL1* and *hmfL2* alcohol dehydrogenases and the *P*. *brasilianum hmfN1* salicylaldehyde dehydrogenase, the Examples of PCT/EP2016/072406 show that these enzymes indeed have ability to efficiently oxidise HMF to FDCA.

In addition the *P*. *brasilianum hmfK1*, *hmfL1, hmfL2* and *hmfN1* genes, the Examples of PCT/EP2016/072406 described a number of further genes encoding protein or enzymes involved in involved in the catabolism of HMF in *P*. *brasilianum,* which are listed in Table 12.

**Table 12 Genes in the P. brasilianum C1 genome identified as being involved in HMF catabolism**

| **Gene name** | **Contig** | **Function (annotated)** | **role in HMF catabolism** | **aa SEQ ID NO:** | **aa length** | **nt SEQ ID NO:** |
|---|---|---|---|---|---|---|
| *hmfL1* | 82 | alcohol dehydrogenase Zn-binding | HMFCA oxidation to FFCA | 1 | 351 | 12 |
| *hmfL2* | 153 | alcohol dehydrogenase Zn-binding | HMFCA oxidation to FFCA | 2 | 339 | 13 |
| *hmfN1* | 730 | salicylaldehyde dehydrogenase | HMF/FFCA oxidation to HMFCA/FDCA | 3 | 505 | 14 |
| *hmfK1* | 730 | salicylate hydroxylase FAD binding monooxygenase | FDCA decarboxylation | 4 | 427 | 15 |
| *hmfM* | 273 | short chain dehydrogenase | reduction of HMF/FFCA to the corresponding alcohol | 5 | 245 | 16 |
| *hmfT3* | 254 | major superfamily facilitator protein | furan transport | 6 | 581 | 17 |
| *hmfT4* | 730 | major superfamily facilitator protein | furan transport | 7 | 513 | 18 |
| *hmfT5* | 1 | ABC transporter | furan transport | 8 | 1435 | 19 |
| *hmfT6* | 226 | major superfamily facilitator protein | furan transport | 9 | 527 | 20 |
| *hmfT7* | 570 | major superfamily facilitator protein | furan transport | 10 | 514 | 21 |
| *hmfR* | 730 | transcriptional activator | induction furan catabolism genes | 11 | 872 | 22 |

### Example 1: attempt to delete hmfK1 and identifcation of hmfK3

Initial attempts were made to delete the first salicylate hydroxylase gene *hmfK1* using a *nia1* selection marker. Although diagnostic PCR confirmed the deletion of *hmfK1*, RNAseq analysis revealed that *hmfK1* is still expressed, indicating that the deletion was not successful. Interestingly, in the analysed presumed mutant strain yet another salicylate hydroxylase, *hmfK3* (SEQ ID NO.: 23, encoded by SEQ ID NO.: 24), was upregulated that was not identified as being upregulated in the Examples of PCT/EP2016/072406. The presumed *hmfK1* deletion transformants were next analysed in more detail. From this analysis we concluded that that both *hmfK1* deletion transformants were not correct. Most likely the deletion construct had integrated at other site(s), possibly up/downstream of *hmfK1* and/or at the *nia1* gene itself (different in both mutants).

### Example 2: Evaluation of RNAseq data

We obtained RNAseq data from the WT and the presumed *hmfK1* deletion transformant ΔSH #2E4 (which turned out not to have correct *hmfK1* deletion) from chemostat cultures grown on citrate and then replacing the citrate by HMF. A set of genes possibly involved in the degradation of HMF via FDCA was identified. The *hmfK1* gene was the gene which showed the strongest induction in the HMF-fed culture in the WT strain. A number of HMF-induced genes are located on the same contig (no. 730) as *hmfK1* is indicating the presence of a gene cluster encoding a HMF degradation pathway. As already discussed above, *hmfK1* was still strongly induced in transformant ΔSH #2E4. In addition, a second hydroxylase gene with homology to *hmfK1*, now termed *hmfK3,* was discovered to be induced in this transformant, but not in the WT strain. Since no FDCA accumulation was observed in both the WT and mutant cultures, we reanalysed the available RNAseq RPKM data for identification of potential alternative targets for improved FCDA strain design (e.g. degradation specific regulator gene).

Data re-analysis was focused on (log₂) ratios of:
1. WT_HMF vs WT_Citrate
2. M_HMF vs M_Citrate
3. M_HMF vs WT_HMF
4. M_Citr vs WT_Citr

The data was first floored (+2) to be able to look at genes with ratio "0" and "div0". Selections of genes were made by using arbitrary cut off values to get comparable amount (-100) of induced genes (see Table 2 and the Supplementary Excel File).

In the mutant (compared to the WT) culture large sets of genes were induced and repressed similarly in both HMF and citrate conditions. This suggests the induction/repression of these genes are HMF independent and mutant and/or culture specific. An exception is the induction of the salicylate hydroxylase *hmfK3.*

The previously obtained results (induction of *hmfK1*, *hmfK3,* alcohol dehydrogenases, transcriptional activator, lactonase etc.) were confirmed in the HMF-treated cultures. Salicyl related genes and decarboxylases are only induced by HMF and oxidoreductases mainly regulated by HMF. HMF also induced various transporters, like g10375 (contig 570) and g5964 (contig_226), encoding Major Facilitator Superfamily proteins, which could have a role in the transport of HMF, or its derivative into and out of the cell.

The BLAST results of the *hmf* genes previously identified in the Examples of PCT/EP2016/072406, and possibly involved in HMF degradation via FDCA were re-evaluated. Interesting to see is that the HMF-induced gene cluster around *hmfK1* (contig730) shows a clear resemblance with a fungal second metabolite cluster. The highest identity (70%) was discovered with an orthologue gene cluster in *Sporothrix schenckii* ATCC 58251. The *hmfL1* gene (encoding an alcohol dehydrogenase lies on a different contig (82) than *hmfK1* (contig 730). However, the *hmfL1* orthologue in S. *schenckii* is in the same gene cluster. In the publicly available P. *brasilianum* MG11 genome (Horn et al., 2015. Genome Announc 3(5):e00724-15. doi:10.1128/genomeA.00724-15; GenBank acc. no. CDHK01000001.1) the *hmf* gene cluster (containing *hmfK1*) also includes *hmfL1.* Therefore we conclude contigs 82 and 730 are adjacent to each other.

The co-factor dependence of dehydrogenases in the *hmf* gene cluster (contigs 730 and 82) was also investigated. For an economically viable production of FDCA in fungi NAD as the co-factor is preferred, since fungi have a higher capacity to re-generate NAD than NADP.

| Gene | NCBI | Annotation | Co-factor |
|---|---|---|---|
| *hmfL1* (contig_82) | CEJ57635 | alcohol dehydrogenase | NAD |
| *hmfN1* (contig_730) | CEJ57637 | aldehyde dehydrogenase | NAD (NADP?) |

BLAST results with *hmfL1* show a preference for NAD as the co-factor. The BLAST results with *hmfN1* does not discriminate for NAD and NADP dependence. Best match for *hmfN1* however is with a salicylaldehyde dehydrogenase (DoxF, SaliADH, EC=1.2.1.65) involved in the upper naphthalene catabolic pathway of *Pseudomonas* strain C18. For DoxF NAD seems the preferred co-factor.

Based on re-evaluation results of the RNAseq data we also conclude that salicylate hydroxylases *hmfK1* and secondly *hmfK3* are the best initial candidates to delete in order construct a host cell that oxidises HMF to FDCA but which does not degrade the FDCA produced.

### Example 3: Development of hmfK1 & hmfK3 single and double mutant strains

### Gene deletion design and synthesis

The WT P. *brasilianum* strain has been tested for phleomycin and hygromycin sensitivity to confirm the suitability of *phleo* or *hygB* antibiotic selection markers for gene disruption (see above). Gene deletion design has been performed by DDNA and is based on a split marker approach (Arentshorst et al., 2015, In: "Genetic Transformation Systems in Fungi", Volume 1. van den Berg M.A., Maruthachalam K., (eds). Switzerland: Springer International Publishing, pp. 263-272) using the *hygB* selection marker for the *hmfK1* deletion mutant and the *phleo* selection marker for the *hmfK3* deletion mutant (Figure 1). The split marker approach is applied to reduce the frequency of integration at incorrect (not *hmfK1* or *hmfK3*) sites, since only DNA fragments that have undergone homologous recombination will result in an intact antibiotic selection marker gene. An attempt to create a *hmfK1 hmfK3* double mutant was made using both selection markers in one transformation experiment. Synthesis of the gene disruption-fragments as outlined in Figure 1 was outsourced to GeneArt, ThermoFisher Scientific. Sequences of the fragments are provided in the sequence listing (SEQ ID NO.'s: 30 - 33).

### Transformation & selection

Before the transformations were performed an FDCA degradation screen was set up for the WT P. *brasilianum* strain. The WT strain is able to use FDCA as a carbon source. Creating mutants that are unable to do this will not grow on media containing FDCA as the sole C-source. This assay will facilitate screening for correct *hmfK1* (and *hmfK3*) deletion transformants (assuming deletion of these genes will impair FDCA degradation).

Corbion minimal medium was used, which contains the following per liter of demineralized water: 2.0 g of (NH₄)2SO₄, 0.1 g of MgCl₂ 6H₂0, 10 mg of EDTA, 2 mg of ZnSO₄ .7H₂0, 1 mg of CaCl₂. 2H₂0, 5 mg of FeSO₄ 7H₂0, 0.2 mg of Na₂MoO₄. 2H₂0, 0.2 mg of CuSO₄ 5H₂0, 0.4 mg of CoCl₂ 6H₂0, and 1 mg of MnCl₂ 2H₂0. 25 mM KH₂PO₄ and 25 mM NaH₂PO₄. One or more of the following C-sources were added depending on the experiment: 0.2 g/l of yeast extract, 15 mM of FDCA, 5 mM glucose, 5 mM citric acid, 6 mM HMF. pH was set at 3.0, except when FDCA was used as sole C-source, to avoid precipitation of FDCA.

WT P. *brasilianum* was able to grow on Corbion minimal medium with 15 mM FDCA as sole C-source, both in liquid medium as well as on agar plates. The negative control (minimal medium without any C-source) showed a minimal amount of background growth, which must be due to the ability of this strain to grow on agar components. This is a beneficial side-affect, because in a screen with transformants this background growth is a positive control for actual transfer of spores. We decided to initially screen the transformants (see below) on agar plates with FDCA and without YE (more clear results and faster), i.e. with 15 mM FDCA as sole C-source.

Gene fragments were transformed to the WT P. *brasilianum* strain using a standard DDNA fungal transformation protocol (Punt and van den Hondel, 1992, Methods in Enzymol. 261:447-457). Protoplasts of the WT strain were transformed with the fragments to create Δ*hmfK1* and plated on selection plates containing HygB. In addition, protoplasts of the WT strain were transformed with fragments to create both Δ*hmfK1* and Δ*hmfK3* and plated on selection plates containing HygB alone, Phleo alone, and both HygB and Phleo. In total, 48 primary transformants were generated on the various selection plates. These primary transformants were screened based on no or reduced growth on plates containing FDCA as the sole C-source. Seventeen out of the 35 (∼50%) HygB+ (Δ*hmfK1*) transformants showed a strong phenotype on plates (no growth on FDCA), suggesting FDCA degradation route involves an intact *hmfK1* gene. Potential Δ*hmfK3* mutants showed no phenotype on plates. All transformants were additionally tested for the presence of antibiotic markers.

Next, the 17 HygB+ (Δ*hmfK1*) clones that showed a phenotype on plates and all *hmfK1*+*hmfK3* transformants were purified on plates and tested in liquid medium ± 15 mM FDCA (-YE). For all Δ*hmfK1* clones, that showed a strong phenotype on agar plates, growth was completely abolished in liquid medium with FDCA as the only C-source.

Diagnostic PCR analysis was performed with the different *hmfK1* deletion transformants to discriminate between an intact and correctly deleted *hmfK1* gene. Different primer combinations were tested (see Tables 13, 14 and 15) and the results showed that all *hmfK1* clones showing a phenotype (no growth on FDCA) are correct (data not shown). Vice versa, *hmfK1*+*hmfK3* transformants #31 and #33, showing no phenotype still produced WT *hmfK1* PCR bands. Diagnostic PCR analysis was also performed on all *hmfK1*+*hmfK3* transformants to test for correct *hmfK3* deletion (see Tables 14 and 16). Only transformant #43 (Δ*hmfK1*+Δ*hmfK3*; HygB+Phleo selected) turned out to have a correct *hmfK3* deletion based on PCR (data not shown). Although this transformant is HygB resistant, it does not contain a *hmfK1* deletion (based on PCR results). All purified *hmfK1*+*hmfK3* transformants were also tested in liquid cultures similarly as the *hmfK1* transformants. Transformant #43 (which is a correct *hmfK3* deletion) did grow on FDCA.

From the results we conclude that correct *hmfK1* deletions were generated and that deletion of the *hmfK1* gene results in a 100% inability to grow on FDCA as sole C-source under the conditions tested, confirming that *hmfk1* is a key gene in FDCA degradation. Additionally, we can conclude that the *hmfK3* deletion is correct in 1 clone (#43) and shows no phenotype, suggesting *hmfk3* is not involved in FDCA degradation, at least not under the conditions tested.

**Table 13. Primers for diagnostic PCR for verification of hmfK1 deletion.**

| Primer No. | Name | Sequence | SEQ ID NO: |
|---|---|---|---|
| 180 | salHupA-430 F | GGTAGAAAAGGGGTTGCGAT | 34 |
| 181 | salHupB-1,954 F | GAAGCAATCGTCGGAAGT | 35 |
| 182 | salHdownB-3,693 R | CGCGAGGATGTATGGTATGAT | 36 |
| 183 | salHdownA-5,280 R | GAAAGTGAGATTGTGGATGGA | 37 |
| 184 | salH-53 R | TGTCGTTCATGGCTCCC | 38 |
| 185 | salH-1,583 F | AAGGTGCGAACTGGAGAG | 39 |
| 186 | niaD-2,876 F | GGAACAATCGGCAAAGAAAGTG | 40 |
| 187 | niaD-94 R | CAACTTCTTGTGGCGTTGG | 41 |

**Table 14. Primers for diagnostic PCR for verification of hmfK1::hygB and hmfK3::phleo deletion-and split marker amplification.**

| Primer No. | Name | Sequence | SEQ ID NO: |
|---|---|---|---|
| 212 | 5flank salH1-489 F | CTCGGTCGCTCTTTTGGGTA | 42 |
| 213 | 5flanksalH1-1,499 R | CAGGACATTGTTGGAGCCGA | 43 |
| 214 | 3flanksalH1-57 F | TCCGGAAGTGCTTGACATTG | 44 |
| 215 | 3flanksalH1-1,455 R | CCATCCCGAGTATTCTTTCGAG | 45 |
| 216 | 5flanksalH2-1,350 F | CCGTGCGCTATAATAACCTTCG | 46 |
| 217 | 5flanksalH2-1,156 R | GCGTCCCGGAAGTTCG | 47 |
| 218 | 3flanksalH2-15 F | GAGCGGTCGAGTTCTGGA | 48 |
| 219 | 3flanksalH2-1,508 R | GGTGAAAGAGTGATATATGAGGC | 49 |
| 220 | con.salH2 5flank-956 F | TCTCAGGACTTGCAGATGTTG | 50 |
| 221 | con.salH2 3flank-2,838 R | GCCAAGTCATCATCCTCGC | 51 |
| 222 | phleo-249 R | CTTACTGCCGGTGATTCGAT | 52 |
| 223 | phleo-712 F | CTACAGGACACACATTCATCGT | 53 |
| 224 | salH2-106 R | TGCCAGCCCTCCTATTCC | 54 |

**Table 15. Expected sizes of PCR products of the designed primer combinations for verification of the hmfK1::hygB deletion.**

| Primers | ΔhmfK3 (bp) | wild type (bp) |
|---|---|---|
| 212 + 215 | 5,504 | 4,580 |
| 180 + 213 | 3,164 | -- |
| 183 + 214 | 3.142 | -- |
| 182 + 181 | 2,641 | 1,740 |

**Table 16. Expected sizes of PCR products of the designed primer combinations for verification of the hmfK3::phleo deletion.**

| Primers | ΔhmfK3 (bp) | wild type (bp) |
|---|---|---|
| 216 + 219 | 4,900 | 4,566 |
| 216 + 224 | -- | 1,606 |
| 220 + 222 | 1,752 | -- |
| 223 + 221 | 2,126 | -- |

### Example 4: conversion of HMF and production of FDCA

We next set up experiments to monitor conversion of HMF and production of FDCA in time. From the results above the WT strain, confirmed *hmfK1* deletions #26 and #30, and confirmed *hmfK3* deletion #43 were chosen to be analysed for fermentation in shake flask cultures. Strains were grown on the Corbion minimal medium w/o yeast extract, containing 5 mM glucose or 5 mM citrate, supplemented with 6mM HMF. Cultures were grown for 7 days and samples were collected after 1, 2, 3, 4 and 7 days. Supernatant from the samples was analysed for HMF, FDCA and intermediates by HPLC as described in the Examples of PCT/EP2016/072406.

All strains grew well on glucose containing medium. The *hmfK1* deletion strains #26 and #30 did not significantly grow on citrate containing medium. Therefore, on day 4, ∼1% of the glucose containing culture (of #26 and #30) was used to re-inoculate the citrate containing flasks (=citr*). However, even than no (clear) further growth was observed in these cultures. The *hmfK3* deletion strain #43 grew on citrate containing medium comparable as the WT strain. The pH was measured after 1 week (data not shown). Interestingly, the pH of the cultures of the *hmfK1* deletion strains after 1 week incubation was lower than of the WT and *hmfK3* deletion strain cultures in both media, indicating acid (FDCA) production.

Figures 2 and 3 show the results of the HPLC analysis of the cultures grown on glucose or citrate, respectively. In the cultures containing glucose immediate HMF conversion was observed. Only low traces of HMF were measured even in the first time samples analysed. In both the WT and Δ*hmK3* cultures all furan levels dropped to very low levels in a similar fashion and as expected, no FDCA accumulation was observed (Figures 2A and 2D). On the other hand, very reproducible FDCA accumulation was measured in both *hmfK1* deletion strains (Figures 2B and 2C). HMF to FDCA conversion of up to 60-70% was reached after 7 days. The HMF-acid was a major transient intermediate compound observed in the *hmfK1* deletion cultures grown on glucose.

In the citrate containing cultures also no FDCA accumulation was measured in both the WT and Δ*hmK3* cultures (Figures 3A and 3D). All furans were degraded over time albeit at a slower rate than in the glucose containing cultures. Although no significant growth for the *hmfK1* deletions #26 and #30 in citrate containing medium was observed after 4 days, about 50% HMF → HMFCA → FDCA conversion was measured (Figures 3B and 3D). This indicates the possible bio-conversion of HMF to FDCA performed by (germinated) spores.

From these results we conclude that *hmfK1* is the key gene in FDCA degradation. Deletion of *hmfK1* leads to FDCA accumulation when HMF is provided to the cultures. (Germinated) spore-based bioconversion seems possible and suggests no growth of cells is needed. However, in cultures growing in glucose containing medium FDCA accumulation is faster. Under the conditions tested, the *hmfK3* gene is not involved in FDCA degradation.

## Claims

**1.** A fungal cell comprising a genetic modification that reduces specific 2,5-furandicarboxylic acid (FDCA) decarboxylating monooxygenase activity in the cell, as compared to a corresponding parent cell lacking the genetic modification, wherein the genetic modification eliminates the expression of an endogenous gene encoding an FDCA decarboxylating monooxygenase by deletion of at least a part of at least one of the promoter and the coding sequence of the gene.

**2.** A fungal cell according to claim 1, wherein the endogenous gene in the corresponding parent cell encodes a FDCA decarboxylating monooxygenase comprising an amino acid sequence with at least 45% sequence identity to at least one of SEQ ID NO : 4.

**3.** A fungal cell according to claim 1 or 2, wherein at least the complete coding sequence of an endogenous gene encoding an FDCA decarboxylating monooxygenase is deleted.

**4.** A fungal cell according to any one of the preceding claims, wherein the expression of all copies of the endogenous gene encoding an FDCA decarboxylating monooxygenase is eliminated.

**5.** A fungal cell according to any one of the preceding claims, wherein the cell has the natural ability to oxidise HMF to FDCA.

**6.** A fungal cell according to any one of the preceding claims, wherein the cell comprises a further genetic modification that is at least one of:
a) a genetic modification that confers to the cell the ability to oxidize 5-hydroxymethyl-2-furancarboxylic acid (HMFCA) to 5-formyl-2-furoic acid (FFCA) or that increases in the cell the specific activity of a enzyme that oxidizes HMFCA to FFCA as compared to a corresponding wild type cell lacking the genetic modification; and,
b) a genetic modification that confers to the cell the ability to oxidize furanic aldehydes to the corresponding furanic carboxylic acids or a genetic modification that increases in the cell the specific activity of a enzyme that oxidizes furanic aldehydes to the corresponding furanic carboxylic acids, as compared to a corresponding wild type cell lacking the genetic modification.

**7.** A fungal cell according to claim 6, wherein the genetic modification in a) is a modification that increases expression of a nucleotide sequence encoding a polypeptide with HMFCA dehydrogenase activity, which polypeptide comprises an amino acid sequence that has at least 45% sequence identity with the amino acid sequence of at least one of SEQ ID NO.'s: 1 and 2; and/or,
wherein the genetic modification in b) is a modification that increases expression of a nucleotide sequence encoding a polypeptide having furanic aldehyde dehydrogenase activity, which aldehyde dehydrogenase has at least one of the abilities of i) oxidizing HMF to HMFCA, ii) oxidizing DFF to FFCA, and, iii) oxidizing FFCA into FDCA, which polypeptide comprises an amino acid sequence that has at least 45% sequence identity with the amino acid sequence of SEQ ID NO.: 3.

**8.** A fungal cell according to any one of the preceding claims, wherein the cell further comprises a genetic modification selected from:
a) a genetic modification that reduces or eliminates the expression of a gene encoding a short chain dehydrogenase that reduces HMF and/or FFCA to the corresponding alcohol, wherein preferably the gene is at least one of a gene encoding polypeptide comprising an amino acid sequence with at least 45% sequence identity to at least one of SEQ ID NO: 5 and 25;
b) a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 6 - 10; and,
c) a genetic modification that alters the expression of a gene encoding a transcriptional activator of genes involved in furan catabolism, wherein preferably the gene is a gene encoding a polypeptide comprising an amino acid sequence with at least 45% sequence identity to SEQ ID NO: 11.

**9.** A fungal cell having the ability to oxidise HMF to FDCA and comprising a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 9 and 10.

**10.** A fungal cell according to claim 9, wherein the cell further comprises a genetic modification selected from:
a) a genetic modification that eliminates or reduces specific FDCA decarboxylating monooxygenase activity in the cell, as compared to a corresponding parent cell lacking the genetic modification;
b) a genetic modification that confers to the cell the ability to oxidize HMFCA to FFCA or that increases in the cell the specific activity of a enzyme that oxidizes HMFCA to FFCA as compared to a corresponding wild type cell lacking the genetic modification;
c) a genetic modification that confers to the cell the ability to oxidize furanic aldehydes to the corresponding furanic carboxylic acids or a genetic modification that increases in the cell the specific activity of a enzyme that oxidizes furanic aldehydes to the corresponding furanic carboxylic acids, as compared to a corresponding wild type cell lacking the genetic modification;
d) a genetic modification that reduces or eliminates the expression of a gene encoding a short chain dehydrogenase that reduces HMF and/or FFCA to the corresponding alcohol, wherein preferably the gene is at least one of a gene encoding polypeptide comprising an amino acid sequence with at least 45% sequence identity to at least one of SEQ ID NO: 5 and 25;
e) a genetic modification that increases expression of a nucleotide sequence encoding a polypeptide that transports at least one furanic compound, which polypeptide preferably comprises an amino acid sequence that has at least 45% sequence identity with the amino acid sequence of at least one of SEQ ID NO.'s: 6 - 8; and,
f) a genetic modification that alters the expression of a gene encoding a transcriptional activator of genes involved in furan catabolism, wherein preferably the gene is a gene encoding a polypeptide comprising an amino acid sequence with at least 45% sequence identity to SEQ ID NO: 11.

**10.** A cell according to any one of the preceding claims, wherein the cell is a filamentous fungal cell selected from a genus from the group consisting of:
*Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium, Trichoderma,* and *Ustilago,* preferably the cell is a filamentous fungal cell selected from a species from the group consisting of: *Aspergillus niger, Aspergillus awamori, Aspergillus foetidus, Aspergillus sojae, Aspergillus fumigatus, Talaromyces emersonii, Aspergillus oryzae, Myceliophthora thermophila, Trichoderma reesei, Penicillium chrysogenum, Penicillium simplicissimum* and *Penicillium brasilianum*;
or, wherein the cell is a yeast cell selected from a genus from the group consisting of:
*Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula,* and *Sporobolomyces,* preferably the cell is a yeast cell selected from a species from the group consisting of *Kluyveromyces lactis, S. cerevisiae, Hansenula polymorpha, Yarrowia lipolytica, Candida tropicalis* and *Pichia pastoris.*

**11.** A process for oxidizing HMFCA to FFCA, the process comprising the step of incubating a fungal cell according to any one of claims 5 - 10, in the presence of HMFCA, under conditions conducive to the oxidation of HMFCA by the cell, wherein the cell expresses enzymes that have the ability to oxidize HMFCA to FFCA.

**12.** A process for producing FDCA, the process comprising the step of incubating a fungal cell according to any one of claims 5 - 10, in a medium comprising one or more furanic precursors of FDCA, preferably under conditions conducive to the oxidation of furanic precursors of FDCA by the cell to FDCA, and, optionally recovery of the FDCA, wherein preferably, at least one furanic precursor of FDCA is selected from the group consisting of HMF, 2,5-dihydroxymethyl furan (DHF), HMFCA, FFCA and 2,5-diformyl furan (DFF), of which HMF is most preferred,
wherein the furanic precursors of FDCA are obtained from one or more hexose sugars, preferably one or more hexose sugars obtained from lignocellulosic biomass, preferably by acid-catalyzed dehydration, and,
wherein preferably the FDCA is recovered from the medium by a process comprising acid precipitation followed by cooling crystallization and/or solvent extraction.

**13.** A process according to claim 12, wherein the medium has a pH in the range of 2.0 - 3.0, wherein preferably the FDCA precipitates from the acidic medium in which it is produced and is recovered from the medium by a process comprising acid precipitation followed by cooling crystallization.

**14.** A process for producing a polymer from at least two FDCA monomers, the process comprising the steps of:
a) preparing an FDCA monomer in a process according to claim 12 or 13; and,
b) producing a polymer from the FDCA monomer obtained in a).

**15.** Use of a fungal cell according to any of claims 5 - 10, for the biotransformation of one or more of furanic precursors to FDCA or a fungal cell expressing one or more bacterial enzymes with the ability to convert a furanic precursors of FDCA into FDCA, wherein preferably, at least one furanic precursor of FDCA is selected from the group consisting of HMF, DHF, HMFCA, FFCA and DFF, of which HMF is most preferred.
